# EUROPEAN PATENT SPECIFICATION

(11) **EP 2 082 064 B1**
(45) Date of publication and mention of the grant of the patent: **05.10.2011**
(21) Application number: 07871494.6
(22) Date of filing: 15.11.2007
(51) Int. Cl.: C12Q 1/68

(54) **GENETIC VARIATIONS ASSOCIATED WITH TUMORS**
MIT TUMOREN ASSOZIIERTE GENETISCHE VARIATIONEN
VARIATIONS GÉNÉTIQUES ASSOCIÉES À DES TUMEURS

(30) Priority: 16.11.2006 US 866103 P; 10.07.2007 US 948818 P
(43) Date of publication of application: 29.07.2009
(73) Proprietor: Genentech, Inc., South San Francisco CA 94080-4990 (US)
(72) Inventor: SESHAGIRI, Somasekar, San Carlos, California 94070 (US); PETERS, Brock, San Francisco, California 94103 (US); KAN, Zhengyan, South San Francisco, CA 94080 (US)
(74) Representative: Walton, Seán Malcolm
(86) International application number: PCT/US2007/084888
(87) International publication number: WO 2008/061213

(56) References cited:
- WO-A-2007/109515
- US-A1- 2003 158 105
- US-A1- 2005 142 585
- US-A1- 2005 266 417
- US-A1- 2006 115 827
- DATABASE NCBI SNP [Online] NCBI; Submission ss42298260 17 July 2005 (2005-07-17), ABI: XP002498998 retrieved from WWW.NCBI.NLM.NIH.GOV/SNP Database accession no. rs13129474
- SORENSEN M ET AL: "Genetic polymorphisms in CYP1B1, GSTA1, NQO1 and NAT2 and the risk of lung cancer" CANCER LETTERS, NEW YORK, NY, US, vol. 221, no. 2, 28 April 2005 (2005-04-28), pages 185-190, XP004829537 ISSN: 0304-3835
- ROGERS ET AL: "The effect of genetic diversity on angiogenesis" EXPERIMENTAL CELL RESEARCH, ACADEMIC PRESS, US, vol. 312, no. 5, 10 March 2006 (2006-03-10), pages 561-574, XP005300756 ISSN: 0014-4827

## Description

### FIELD OF THE INVENTION

The present invention generally relates to genetic variations associated with tumors.

### BACKGROUND

Cancers may arise when cells accumulate somatic mutations that ultimately confer a growth advantage. Somatic mutations include, e.g., nucleotide base substitutions, deletions, insertions, amplifications, and rearrangements. Identification of somatic mutations that occur in cancer provides valuable information regarding the development of cancer. Such information is also useful for the identification of diagnostic markers and therapeutic targets in cancer. (See, e.g., Bamford et al. (2004) British Journal of Cancer 91:355-358.) Thus, a continuing need exists to identify somatic mutations that occur in cancer.

Germline variations, or polymorphisms, are heritable variations that are present in an organism's genome. Polymorphisms include restriction fragment length polymorphisms (RFLPs), short tandem repeats (stirs), and single nucleotide polymorphisms (SNPs). Germline variations may be associated with susceptibility to certain diseases, including cancer. (See, e.g., Vierimaa et al. (2006) Science 312:1228-1230; Landi et al. (2006) Science 313:521-522; Zhu et al. (2004) Cancer Research 64:2251-2257.) Thus, a continuing need exists to identify polymorphisms associated with cancer.

The identification of variations associated with cancer has proven valuable in clinical settings, e.g., in distinguishing patient populations that are responsive to a particular therapy. (See, e.g., Lynch et al. (2004) N. Engl..J. Med. 350:2129-2139; O'Hare (2004) Blood 104:2532-2539).

The invention described herein meets the above-described needs and provides other benefits.

### SUMMARY

The invention is defined in the claims. The methods of the invention are based, in part, on the discovery of novel variations in polynucleotides derived from tumor samples.

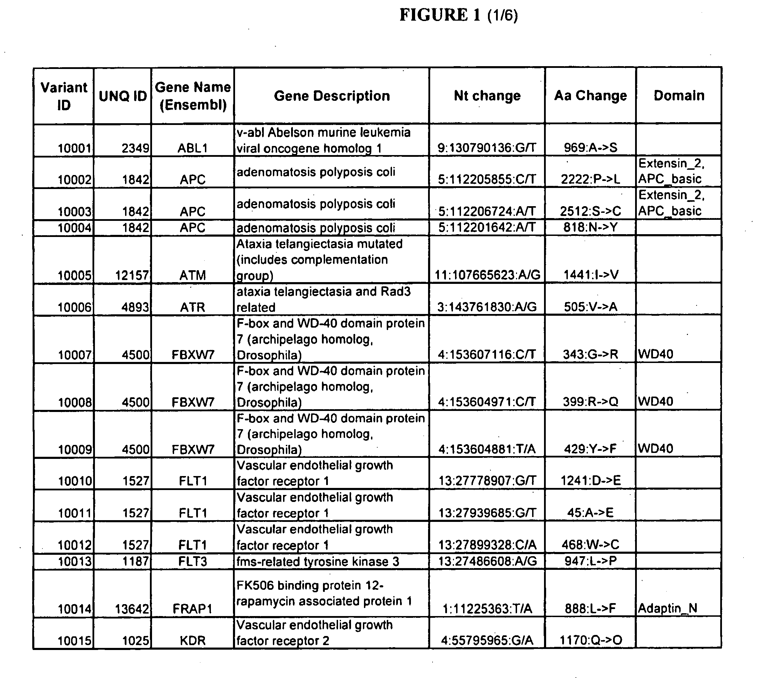

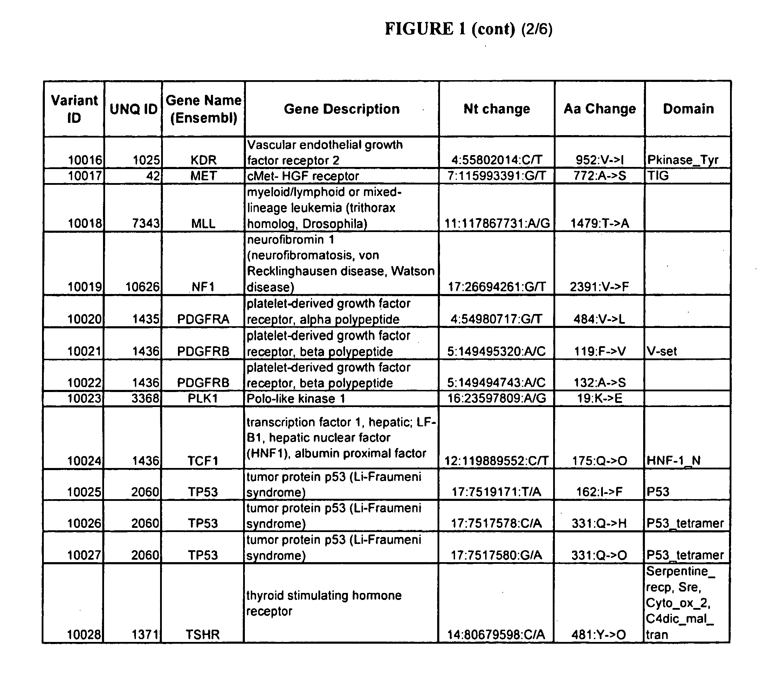

In one aspect, an isolated polynucleotide is provided, wherein the isolated polynucleotide comprises (a) a PRO polynucleotide or fragment thereof that is at least about 10 nucleotides in length, wherein the PRO polynucleotide or fragment thereof comprises a nucleotide variation at a nucleotide position selected from Figures 1-3, or (b) the complement of (a). In one embodiment, the nucleotide variation occurs in SEQ ID NOs: 1-37. In another embodiment, the nucleotide variation is a nucleotide change selected from Figures 1-3. In another embodiment, the isolated polynucleotide is a primer. In another embodiment, the isolated polynucleotide is an oligonucleotide.

In another aspect, an oligonucleotide is provided that is (a) an allele-specific oligonucleotide that hybridizes to a region of a PRO polynucleotide comprising a nucleotide variation at a nucleotide position selected from Figures 1-3, or (b) the complement of (a). In one embodiment, the nucleotide variation is a nucleotide change selected from Figures 1-3. In another embodiment, the allele-specific oligonucleotide is an allele-specific primer. In another embodiment, a kit comprising the oligonucleotide and at least one enzyme is provided. In one such embodiment, the at least one enzyme is a polymerase. In another such embodiment, the at least one enzyme is a ligase. In a further embodiment, a microarray comprising the oligonucleotide is provided.

In another aspect, a method of detecting the absence or presence of a nucleotide variation at a nucleotide position selected from Figures 1-3 is provided, the method comprising (a) contacting nucleic acid suspected of comprising the nucleotide variation with an allele-specific oligonucleotide that is specific for the nucleotide variation under conditions suitable for hybridization of the allele-specific oligonucleotide to the nucleic acid; and (b) detecting the absence or presence of allele-specific hybridization. In one embodiment, the nucleotide variation is a nucleotide change selected from Figures 1-3. In another embodiment, the nucleotide variation is a somatic mutation. In another embodiment, the nucleotide variation is a germline polymorphism.

In another aspect, a method of amplifying a nucleic acid comprising a nucleotide variation at a nucleotide position selected from Figures 1-3 is provided, the method comprising (a) contacting the nucleic acid with a primer that hybridizes to the nucleic acid at a sequence 3' of the nucleotide variation, and (b) extending the primer to generate an amplification product comprising the nucleotide variation. In one embodiment, the nucleotide variation is a nucleotide change selected from Figures 1-3. In another embodiment, the nucleotide variation is a somatic mutation. In another embodiment, the nucleotide variation is a germline polymorphism.

In another embodiment, a method of determining the genotype of a biological sample from a mammal is provided, the method comprising detecting the absence or presence of a nucleotide variation at a nucleotide position selected from Figures 1-3 in nucleic acid material derived from the biological sample. In one embodiment, the nucleotide variation is a nucleotide change selected from Figures 1-3. In another embodiment, the biological sample is suspected of comprising tumor cells. In another embodiment, the biological sample is a tumor. In one such embodiment, the tumor is a lung tumor. In one such embodiment, the lung tumor is a non-small cell lung carcinoma. In another embodiment, the detecting comprises carrying out a process selected from a primer extension assay; an allele-specific primer extension assay; an allele-specific nucleotide incorporation assay; an allele-specific oligonucleotide hybridization assay; a 5' nuclease assay; an assay employing molecular beacons; and an oligonucleotide ligation assay. In another embodiment, the nucleotide variation is a somatic mutation. In another embodiment, the nucleotide variation is a germline polymorphism.

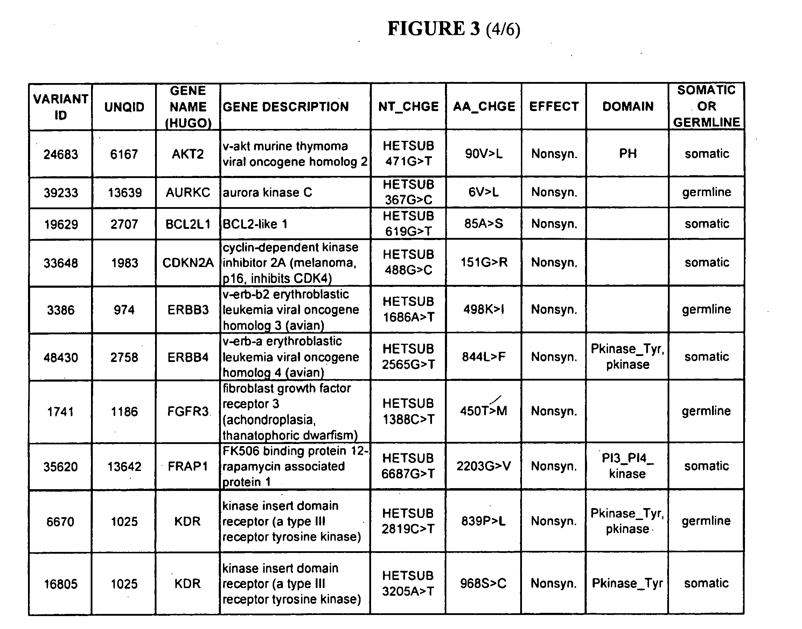

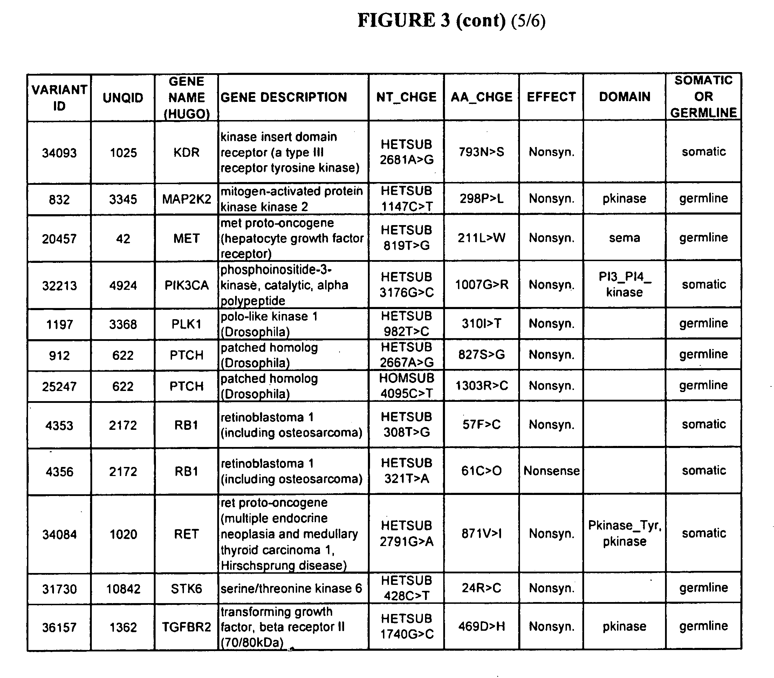

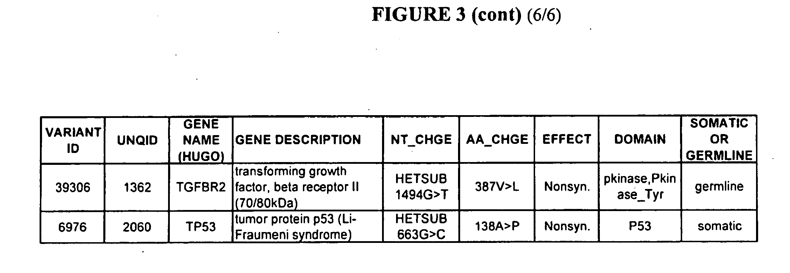

In another aspect, a method of classifying a tumor in a mammal is provided, the method comprising detecting the presence of a variation in a PRO or PRO polynucleotide in a biological sample derived from the mammal, wherein the biological sample is known to or suspected of comprising tumor cells. In one embodiment, the tumor cells are lung tumor cells. In one such embodiment, the lung tumor cells are non-small cell lung carcinoma cells. In another embodiment, the variation is a nucleotide variation. In one such embodiment, the nucleotide variation is at a nucleotide position selected from Figures 1-3. In another such embodiment, the nucleotide variation is a nucleotide change selected from Figures 1-3. In another such embodiment, the detecting comprises carrying out a process selected from a primer extension assay; an allele-specific primer extension assay; an allele-specific nucleotide incorporation assay; an allele-specific oligonucleotide hybridization assay; a 5' nuclease assay; an assay employing molecular beacons; and an oligonucleotide ligation assay. In another such embodiment, the nucleotide variation is a somatic mutation. In another such embodiment, the nucleotide variation is a germline polymorphism. In another embodiment, the variation is an amino acid variation. In one such embodiment, the amino acid variation is in a PRO functional domain selected from Figure 1 or 3. In another such embodiment, the amino acid variation is at an amino acid position selected from Figure 1 or 3. In another such embodiment, the amino acid variation is an amino acid change selected from Figure 1 or 3. In another embodiment, the variation is an amino acid variation in FBXW7. In one such embodiment, the amino acid variation in FBXW7 is in the WD40 domain. In one such embodiment, the amino acid variation is at an amino acid position selected from G343, R399, and Y429. In another embodiment, the variation is a nucleotide variation in an FBXW7 polynucleotide. In one such embodiment, the nucleotide variation is in a region of an FBXW7 polynucleotide encoding the WD40 domain. In one such embodiment, the nucleotide variation is at a nucleotide position selected from C153607116, C153604971, and T153604881 of chromosome 4. In one such embodiment, the nucleotide variation is a nucleotide change selected from C153607116T, C153604971T, and T153604R81A.

In another aspect, a method for predicting whether a tumor will respond to a therapeutic agent that targets a PRO or a PRO polynucleotide is provided, the method comprising determining whether the tumor comprises a variation in a PRO or PRO polynucleotide, wherein the presence of a variation indicates that the tumor will respond to the therapeutic agent. In one embodiment, the tumor is a lung tumor. In one such embodiment, the lung tumor is a non-small cell lung carcinoma. In another embodiment, the variation is a nucleotide variation. In one such embodiment, the nucleotide variation is at a nucleotide position selected from Figures 1-3. In another such embodiment, the nucleotide variation is a nucleotide change selected from Figures 1-3. In another such embodiment, the nucleotide variation is a somatic mutation. In another such embodiment, the nucleotide variation is a germline polymorphism. In another embodiment, the variation is an amino acid variation. In one such embodiment, the amino acid variation is in a PRO functional domain selected from Figure 1 or 3. In another such embodiment, the amino acid variation is at an amino acid position selected from Figure 1 or 3. In another such embodiment, the amino acid variation is an amino acid change selected from Figure 1 or 3.

These and further embodiments are described in the following written description.

### BRIEF DESCRIPTION OF THE FIGURES

Figure 1 is a table of nucleotide variations in the coding regions of selected genes. The resulting amino acid variations are also provided. The nucleotide variations were identified in nucleic acid derived from lung tumors by mismatch repair detection (MRD), as described in Example A.
Figure 2 is a table of nucleotide variations that affect the splicing of mRNA transcribed from selected genes. The nucleotide variations were identified in nucleic acid derived from lung tumors using mismatch repair detection (MRD), as described in Example A.
Figure 3 is a table of nucleotide variations in the coding regions of selected genes. The resulting amino acid variations are also provided. The nucleotide variations were identified in nucleic acid derived from lung tumors using Sanger sequencing, as described in Example B.

### DETAILED DESCRIPTION OF EMBODIMENTS

### I. DEFINITIONS

For purposes of interpreting this specification, the following definitions will apply and whenever appropriate, terms used in the singular will also include the plural and vice versa. In the event that any definition set forth below conflicts with any document incorporated herein by reference, the definition set forth below shall control.

The term "polynucleotide" or "nucleic acid," as used interchangeably herein, refers to polymers of nucleotides of any length, and include DNA and RNA. The nucleotides can be deoxyribonucleotides, ribonucleotides, modified nucleotides or bases, and/or their analogs, or any substrate that can be incorporated into a polymer by DNA or RNA polymerase. A polynucleotide may comprise modified nucleotides, such as methylated nucleotides and their analogs. If present, modification to the nucleotide structure may be imparted before or after assembly of the polymer. The sequence of nucleotides may be interrupted by non-nucleotide components. A polynucleotide may be further modified after polymerization, such as by conjugation with a labeling component. Other types of modifications include, for example, "caps", substitution of one or more of the naturally occurring nucleotides with an analog, internucleotide modifications such as, for example, those with uncharged linkages (e.g., methyl phosphonates, phosphotriesters, phosphoamidates, cabamates, etc.) and with charged linkages (e.g., phosphorothioates, phosphorodithioates, etc.), those containing pendant moieties, such as, for example, proteins (e.g., nucleases; toxins, antibodies, signal peptides, poly-L-lysine, etc. ), those with intercalators (e.g., acridine, psoralen, etc.), those containing chelators (e.g., metals, radioactive metals, boron, oxidative metals, etc.), those containing alkylators, those with modified linkages (e.g., alpha anomeric nucleic acids, etc.), as well as unmodified forms of the polynucleotide(s). Further, any of the hydroxyl groups ordinarily present in the sugars may be replaced, for example, by phosphonate groups, phosphate groups, protected by standard protecting groups, or activated to prepare additional linkages to additional nucleotides, or may be conjugated to solid supports. The 5' and 3' terminal OH can be phosphorylated or substituted with amines or organic capping groups moieties of from 1 to 20 carbon atoms. Other hydroxyls may also be derivatized to standard protecting groups. Polynucleotides can also contain analogous forms of ribose or deoxyribose sugars that are generally known in the art, including, for example, 2'-O-methyl-2'-O- allyl, 2'-fluoro- or 2'-azido-ribose, carbocyclic sugar analogs, α- anomeric sugars, epimeric sugars such as arabinose, xyloses or lyxoses, pyranose sugars, furanose sugars, sedoheptuloses, acyclic analogs and abasic nucleoside analogs such as methyl riboside. One or more phosphodiester linkages may be replaced by alternative linking groups. These alternative linking groups include, but are not limited to, embodiments wherein phosphate is replaced by P(O)S("thioate"), P(S)S ("dithioate"), "(O)NR 2 ("amidate"), P(O)R, P(O)OR', CO or CH 2 ("formacetal"), in which each R or R' is independently H or substituted or unsubstituted alkyl (1-20 C) optionally containing an ether (--O--) linkage, aryl, alkenyl, cycloalkyl, cycloalkenyl or araldyl. Not all linkages in a polynucleotide need be identical. The preceding description applies to all polynucleotides referred to herein, including RNA and DNA.

"Oligonucleotide," as used herein, refers to short, single stranded polynucleotides that are at least about seven nucleotides in length and less than about 250 nucleotides in length. Oligonucleotides may be synthetic. The terms "oligonucleotide" and "polynucleotide" are not mutually exclusive. The description above for polynucleotides is equally and fully applicable to oligonucleotides.

The term "primer" refers to a single stranded polynucleotide that is capable of hybridizing to a nucleic acid and allowing the polymerization of a complementary nucleic acid, generally by providing a free 3'-OH group.

The term "PRO" refers to a protein encoded by any of the genes listed in Figures 1-3, including wild-type and variant forms thereof. Such forms include "full-length," unprocessed forms of PRO; forms of PRO that result from cellular processing; naturally occurring PRO variants, e.g., PRO resulting from alternative splicing, allelic variations, or spontaneous mutation; and fragments or variants of a native PRO that maintain at least one biological activity of PRO, unless otherwise indicated.

The term "PRO polynucleotide" or "nucleic acid encoding PRO" refers to a gene or coding sequence (e.g., an mRNA or cDNA coding sequence) that encodes a PRO, unless otherwise indicated.

The term "nucleotide variation" refers to a change in a nucleotide sequence (e.g., an insertion, deletion, inversion, or substitution of one or more nucleotides, such as a single nucleotide polymorphism (SNP)) relative to a reference sequence (e.g., a wild type sequence). The term also encompasses the corresponding change in the complement of the nucleotide sequence, unless otherwise indicated. A nucleotide variation may be a somatic mutation or a germline polymorphism.

The term "amino acid variation" refers to a change in an amino acid sequence (e.g., an insertion, substitution, or deletion of one or more amino acids, such as an internal deletion or an N- or C-terminal truncation) relative to a reference sequence.

The term "variation" refers to either a nucleotide variation or an amino acid variation.

The term "a nucleotide variation at a nucleotide position selected from Figure(a) ###" and grammatical variants thereof (where "Figure(s) ###" refers to any of Figures 1-3) refer to a nucleotide variation in a PRO polynucleotide sequence at any of the nucleotide positions listed in column 5 of any of Figures 1-3, including but not limited to the specific nucleotide changes listed in column 5 of any of Figures 1-3. For example and for purposes of illustration, with reference to column 5 in the first row of Figure 1, a nucleotide variation at nucleotide position 130790136 of an ABL polynucleotide encompasses any change at that nucleotide position, including but not limited to the specific nucleotide change, i.e., the G/T substitution, indicated in column 5. The term also encompasses the corresponding change in the complement of the nucleotide sequence, unless otherwise indicated.

The term "a nucleotide change selected from Figure(s) ###" and grammatical variants thereof (where "Figure(s) ###" refers to any of Figures 1-3) refer to any of the specific nucleotide changes listed in column 5 of any of Figures 1-3. For purposes of illustration, an example of a nucleotide change selected from Figure 1 is the G/T substitution at nucleotide position 130790136 of an ABL polynucleotide, as shown in column 5 in the first row of Figure 1.

The term "an amino acid variation at an amino acid position selected from Figure(s) ###" and grammatical variants thereof (where "Figure(s) ###" refers to Figure 1 or 3) refer to an amino acid variation in a PRO amino acid sequence at any of the amino acid positions listed in column 6 of Figure 1 or 3, including but not limited to the specific amino acid changes listed in column 6 of Figure 1 or 3. For example and for purposes of illustration, with reference to column 6 in the first row of Figure 1, an amino acid variation at amino acid position 969 of ABL encompasses any change at that amino acid position, including but not limited to the specific amino acid change, i.e., the A>S substitution, indicated in column 6.

The term "an amino acid change selected from Figure(s) ###" and grammatical variants thereof (where "Figure(s) ###" refers to Figure 1 or 3) refer to any of the specific amino acid changes listed in column 6 of Figure 1 or 3. For purposes of illustration, an example of an amino acid change selected from Figure 1 is the A>S substitution at amino acid position 969 of ABL, as shown in column 6 in the first row of Figure 1.

The term "PRO functional domain" refers to any of the protein domains listed in any of Figures 1-3.

The term "activating variation" refers to a variation in a gene or coding sequence that results in a more active form of the encoded polypeptide, relative to the wild type polypeptide.

The term "array" or "microarray" refers to an ordered arrangement of hybridizable array elements, preferably polynucleotide probes (e.g., oligonucleotides), on a substrate. The substrate can be a solid substrate, such as a glass slide, or a semi-solid substrate, such as nitrocellulose membrane.

The term "amplification" refers to the process of producing one or more copies of a reference nucleic acid sequence or its complement. Amplification may be linear or exponential (e.g., PCR). A "copy" does not necessarily mean perfect sequence complementarity or identity relative to the template sequence. For example, copies can include nucleotide analogs such as deoxyinosine, intentional sequence alterations (such as sequence alterations introduced through a primer comprising a sequence that is hybridizable, but not fully complementary, to the template), and/or sequence errors that occur during amplification.

The term "allele-specific oligonucleotide" refers to an oligonucleotide that hybridizes to a region of a target nucleic acid that comprises a nucleotide variation (generally a substitution). "Allele-specific hybridization" means that, when an allele-specific oligonucleotide is hybridized to its target nucleic acid, a nucleotide in the allele-specific oligonucleotide specifically base pairs with the nucleotide variation. An allele-specific oligonucleotide capable of allele-specific hybridization with respect to a particular nucleotide variation is said to be "specific for" that variation.

The term "allele-specific primer" refers to an allele-specific oligonucleotide that is a primer.

The term "primer extension assay" refers to an assay in which nucleotides are added to a nucleic acid, resulting in a longer nucleic acid, or "extension product," that is detected directly or indirectly.

The term "allele-specific nucleotide incorporation assay" refers to a primer extension assay in which a primer is (a) hybridized to target nucleic acid at a region that is 3' of a nucleotide variation and (b) extended by a polymerase, thereby incorporating into the extension product a nucleotide that is complementary to the nucleotide variation.

The term "allele-specific primer extension assay" refers to a primer extension assay in which an allele-specific primer is hybridized to a target nucleic acid and extended.

The term "allele-specific oligonucleotide hybridization assay" refers to an assay in which (a) an allele-specific oligonucleotide is hybridized to a target nucleic acid and (b) hybridization is detected directly or indirectly.

The term "5 nuclease assay" refers to an assay in which hybridization of an allele-specific oligonucleotide to a target nucleic acid allows for nucleolytic cleavage of the hybridized probe, resulting in a detectable signal.

The term "assay employing molecular beacons" refers to an assay in which hybridization of an allele-specific oligonucleotide to a target nucleic acid results in a level of detectable signal that is higher than the level of detectable signal emitted by the free oligonucleotide.

The term "oligonucleotide ligation assay" refers to an assay in which an allele-specific oligonucleotide and a second oligonucleotide are hybridized adjacent to one another on a target nucleic acid and ligated together (either directly or indirectly through intervening nucleotides), and the ligation product is detected directly or indirectly.

The term "target sequence," "target nucleic acid," or "target nucleic acid sequence" refers generally to a polynucleotide sequence of interest in which a nucleotide variation is suspected or known to reside, including copies of such target nucleic acid generated by amplification.

The term "detection" includes any means of detecting, including direct and indirect detection.

The term "diagnosis" is used herein to refer to the identification or classification of a molecular or pathological state, disease or condition. For example, "diagnosis" may refer to identification of a particular type of cancer, e.g., a lung cancer. "Diagnosis" may also refer to the classification of a particular type of cancer, e.g., by histology (e.g., a non small cell lung carcinoma), by molecular features (e.g., a lung cancer characterized by nucleotide or amino acid variation(s) in a particular gene or protein), or both.

The term "prognosis" is used herein to refer to the prediction of the likelihood of cancer-attributable death or progression, including, for example, recurrence, metastatic spread, and drug resistance, of a neoplastic disease, such as cancer.

The term "prediction" is used herein to refer to the likelihood that a patient will respond either favorably or unfavorably to a drug or set of drugs. In one embodiment, the prediction relates to the extent of those responses. In another embodiment, the prediction relates to whether and/or the probability that a patient will survive following treatment, for example treatment with a particular therapeutic agent and/or surgical removal of the primary tumor, and/or chemotherapy for a certain period of time without cancer recurrence. The predictive methods of the invention can be used clinically to make treatment decisions by choosing the most appropriate treatment modalities for any particular patient. The predictive methods of the present invention are valuable tools in predicting if a patient is likely to respond favorably to a treatment regimen, such as a given therapeutic regimen, including for example, administration of a given therapeutic agent or combination, surgical intervention, chemotherapy, etc., or whether long-term survival of the patient, following a therapeutic regimen is likely.

The terms "cell proliferative disorder" and "proliferative disorder" refer to disorders that are associate with a measurable degree of abnormal cell proliferation. In one embodiment, the cell proliferative disorder is cancer.

"Tumor," as used herein, refers to all neoplastic cell growth and proliferation, whether malignant or benign, and all pre-cancerous and cancerous cells and tissues. The terms "cancer," "cancerous," "cell proliferative disorder," "proliferative disorder" and "tumor" are not mutually exclusive as referred to herein.

The terms "cancer" and "cancerous" refer to or describe the physiological condition in mammals that is typically characterized by unregulated cell growth and proliferation. Examples of cancer include, but are not limited to, carcinoma, lymphoma (e.g., Hodgkin's and non-Hodgkin's lymphoma), blastoma, sarcoma, and leukemia. More particular examples of cancers include squamous cell cancer, small-cell lung cancer, non-small cell lung cancer, adenocarcinoma of the lung, squamous carcinoma of the lung, cancer of the peritoneum, hepatocellular cancer, renal cell carcinoma, gastrointestinal cancer, gastric cancer, esophageal cancer, pancreatic cancer, glioma, cervical cancer, ovarian cancer, liver cancer, bladder cancer, hepatoma, breast cancer, colon cancer, rectal cancer, lung cancer, endometrial or uterine carcinoma, salivary gland carcinoma, kidney cancer, liver cancer, prostate cancer, vulval cancer, thyroid cancer, hepatic carcinoma, melanoma, leukemia and other lymphoproliferative disorders, and various types of head and neck cancer.

The term "lung tumor" refers to any tumor of the lung, including but not limited to small-cell lung carcinoma and non-small cell lung carcinoma, the latter including but not limited to adenocarcinoma, squamous carcinoma, and large cell carcinoma.

The term "neoplasm" or "neoplastic cell" refers to an abnormal tissue or cell that proliferates more rapidly than corresponding normal tissues or cells and continues to grow after removal of the stimulus that initiated the growth.

A "lung tumor cell" refers to a lung tumor cell, either in vivo or in vitro, and encompasses cell lines derived from lung tumor cells.

As used herein, "treatment" (and variations such as "treat" or "treating") refers to clinical intervention in an attempt to alter the natural course of the individual or cell being treated, and can be performed either for prophylaxis or during the course of clinical pathology. Desirable effects of treatment include preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis.

An "individual" is a vertebrate. In certain embodiments, the vertebrate is a mammal. Mammals include, but are not limited to, farm animals (such as cows), sport animals, pets (such as cats, dogs, and horses), primates (including human and non-human primates), and rodents (e.g., mice and rats). In certain embodiments, a mammal is a human.

An "effective amount" refers to an amount effective, at dosages and for periods of time necessary, to achieve the desired therapeutic or prophylactic result.

A "therapeutically effective amount" of a substance/molecule of the invention may vary according to factors such as the disease state, age, sex, and weight of the individual, and the ability of the substance/molecule, to elicit a desired response in the individual. A therapeutically effective amount encompasses an amount in which any toxic or detrimental effects of the substance/molecule are outweighed by the therapeutically beneficial effects. A "prophylactically effective amount" refers to an amount effective, at dosages and for periods of time necessary, to achieve the desired prophylactic result. Typically, but not necessarily, since a prophylactic dose is used in subjects prior to or at an earlier stage of disease, the prophylactically effective amount would be less than the therapeutically effectiye amount.

The term "long-term" survival is used herein to refer to survival for at least 1 year, 5 years, 8 years, or 10 years following therapeutic treatment.

The term "increased resistance" to a particular therapeutic agent or treatment option, when used in accordance with the invention, means decreased response to a standard dose of the drug or to a standard treatment protocol.

The term "deceased sensitivity" to a particular therapeutic agent or treatment option, when used in accordance with the invention, means decreased response to a standard dose of the agent or to a standard treatment protocol, where decreased response can be compensated for (at least partially) by increasing the dose of agent, or the intensity of treatment.

"Patient response" can be assessed using any endpoint indicating a benefit to the patient, including, without limitation, (1) inhibition, to some extent, of tumor broth, including slowing down or complete growth arrest; (2) reduction in the number of tumor cells; (3) reduction in tumor size; (4) inhibition (i.e., reduction, slowing down or complete stopping) of tumor cell infiltration into adjacent peripheral organs and/or tissues; (5) inhibition (i.e. reduction, slowing down or complete stopping) of metastasis; (6) enhancement of anti-tumor immune response, which may, but does not have to, result in the regression or rejection of the tumor; (7) relief, to some extent, of one or more symptoms associated with the tumor; (8) increase in the length of survival following treatment; and/or (9) decreased mortality at a given point of time following treatment.

The term "antagonist" is used in the broadest sense, and includes any molecule that partially or fully inhibits or neutralizes a biological activity of a polypeptide, such as PRO, or that partially or fully inhibits the transcription or translation of a nucleic acid encoding the polypeptide. Exemplary antagonist molecules include, but are not limited to, antagonists antibodies, polypeptide fragments, oligopeptides, organic molecules (including small molecules), and anti-sense nucleic acids.

The term "agonist" is used in the broadest sense, and includes any molecule that partially or fully mimics a biological activity of a polypeptide, such as PRO, or that increases the transcription or translation of a nucleic acid encoding the polypeptide. Exemplary agonist molecules include, but are not limited to, agonist antibodies, polypeptide fragments, oligopeptides, organic molecules (including small molecules), PRO polynucleotides, PRO polypeptides, and PRO-Fc fusions.

A "therapeutic agent that targets a PRO or a PRO polynucleotide" means any agent that affects the expression and/or activity of PRO or a PRO polynucleotide including, but not limited to, any of the PRO agonists or antagonists described herein, including such therapeutic agents that are already known in the art as well as those that are later developed.

The term "cytotoxic agent" as used herein refers to a substance that inhibits or prevents a cellular function and/or causes cell death or destruction. The term is intended to include radioactive isotopes (e.g., At²¹¹, I¹³¹, I¹²⁵, Y⁹⁰, Re¹⁸⁶, R¹⁸⁸, Sm¹⁵³, Bi²¹², P³², Pb²¹² and radioactive isotopes of Lu), chemotherapeutic agents (e.g., methotrexate, adriamicin, vinca alkaloids (vincristine, vinblastine, etoposide), doxorubicin, melphalan, mitomycin C, chlorambucil, daunorubicin or other intercalating agents, enzymes and fragments thereof such as nucleolytic enzymes, antibiotics, and toxins such as small molecule toxins or enzymatically active toxins of bacterial, fungal, plant or animal origin, including fragments and/or variants thereof, and the various antitumor or anticancer agents disclosed below. Other cytotoxic agents are described below. A "tumoricidal" agent causes destruction of tumor cells.

A "toxin" is any substance capable of having a detrimental effect on the growth or proliferation of a cell.

A "chemotherapeutic agent" is a chemical compound useful in the treatment of cancer. Examples of chemotherapeutic agents include alkylating agents such as thiotepa and CYTOXAN® cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethylenethiophosphoramide and trimethylolomelamine; acetogenins (especially bullatacin and bullatacinone); delta-9-tetrahydrocannabinol (dronabinol, MARINOL®; beta-lapachone; lapachol; colchicines; betulinic acid; a camptothecin (including the synthetic analogue topotecan (HYCAMTlN®), CPT-11 (irinotecan, CAMPTOSAR®), acetylcamptothecin, scopolectin, and 9-aminocamptothecin); bryostatin; callystatin; CC-1065 (including its adozelesin, carzelesin and bizelesin synthetic analogues); podophyllotoxin; podophyllinic acid; teniposide; cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazinc, cholophosphamide, estramustine, ifosfamide, mechlorethamine, mechlorethamine oxide hydrochloride, melphalan, novembichin, phenesterine, prednimustine, trofosfamide, uracil mustard; nitrosoureas such as carmustine, chlorozotocin, fotemustine, lomustine, nimustine, and ranimnustine; antibiotics such as the enediyne antibiotics (e. g., calicheamicin, especially calicheamicin gammal I and calicheamicin omegaII (see, e.g., Agnew, Chem Intl. Ed. Engl., 33: 183-186 (1994)); dynemicin, including dynemicin A; an esperamicin; as well as neocarzinostatin chromophore and related chromoprotein enediyne antiobiotic chromophores), aclacinomysins, actinomycin, authramycin, azaserine, bleomycins, cactinomycin, carabicin, carminomycin, carzinophilin, chromomycins, dactinomycin, daunorubicin, detorubicin, 6-diazo-5-oxo-L-norleucine, ADRIAMYCIN® doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, porfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; Iosoxantrone; 2-ethylhydrazide; procarbazine; PSK® polysaccharide complex (JHS Natural Products, Eugene, OR); razoxane; rhizoxin; sizofiran; spirogermanium; tenuazonic acid; triaziquone; 2,2',2"-trichlorotriethylamine; trichothecenes (especially T-2 toxin, verracurin A, rofidin A and anguidine); urethan; vindesine (ELDISINE®, FILDESIN®); dacarbazine; mannomustine; mitobronitol; mitolactol; pipobroman; gacytosine; arabinoside ("Ara-C"); thiotepa; taxoids, e.g., TAXOL® paclitaxel (Bristol-Myers Squibb Oncology, Princeton, N.J.), ABRAXANE^{™} Cremophor-free, albumin-engineered nanoparticle formulation of paclitaxel (American Pharmaceutical Partners, Schaumberg, Illinois), and TAXOTERE® docetaxel (Rhône-Poulenc Rorer, Antony, France); chloranbucil; gemcitabine (GEMZAR®); 6-thioguanine; mercaptopurine; methotrexate; platinum analogs such as cisplatin and carboplatin; vinblastine (VELBAN®); platinum; etoposide (VP-16); ifosfamide; mitoxantrone; vincristine (ONCOVINR®); oxaliplatin; leucovovin; vinorelbine (NAVELBINE®); novantrone; edatrexate; daunomycin; aminopterin; ibandronate; topoisomerase inhibitor RFS 2000; difluoromethylornithine (DMFO); retinoids such as retinoic acid; capecitabine (XELODA®); pharmaceutically acceptable salts, acids or derivatives of any of the above; as well as combinations of two or more of the above such as CHOP, an abbreviation for a combined therapy of cyclophosphamide, doxorubicin, vincristine, and prednisolone, and FOLFOX, an abbreviation for a treatment regimen with oxaliplatin (ELOXATIN^{™}) combined with 5-FU and leucovovin.

Also included in this definition are anti-hormonal agents that act to regulate, reduce, block, or inhibit the effects of hormones that can promote the growth of cancer, and are often in the form of systemic, or whole-body treatment. They may be hormones themselves. Examples include anti-estrogens and selective estrogen receptor modulators (SERMs), including, for example, tamoxifen (including NOLVADEX® tamoxifen), EVISTA® raloxifene, droloxifene, 4-hydroxytamoxifen, trioxifene, keoxifene, LY 117018, onapristone, and FARESTON® toremifene; anti-progesterones; estrogen receptor down-regulators (ERDs); agents that function to suppress or shut down the ovaries, for example, leutinizing hormone-releasing hormone (LHRH) agonists such as LUPRON® and ELIGARD® leuprolide acetate, goserelin acetate, buserelin acetate and tripterelin; other anti-androgens such as flutamide, nilutamide and bicalutamide; and aromatase inhibitors that inhibit the enzyme aromatase, which regulates estrogen production in the adrenal glands, such as, for example, 4(5)-imidazoles, aminoglutethimide, MEGASE® megestrol acetate, AROMASIN® exemestane, formestanie, fadrozole, RIVISOR® vorozole, FEMARA® letrozole, and ARIMIDEX® anastrozole. In addition, such definition of chemotherapeutic agents includes bisphosphonates such as clodronate (for example, BONEFOS® or OSTAC®, DIDROCAL® etidronate, NE-58095, ZOMETA® zoledronic acid/zoledronate, FOSAMAX® alendronate, AREDLA® pamidronate, SKELID® tiludronate, or ACTONEL® risedronate; as well as troxacitabine (a 1,3-dioxolane nucleoside cytosine analog); antisense oligonucleotides, particularly those that inhibit expression of genes in signaling pathways implicated in abherant cell proliferation, such as, for example, PKC-alpha, Raf, H-Ras, and epidermal growth factor receptor (EGF-R); vaccines such as THERATOPE® vaccine and gene therapy vaccines, for example, ALLOVECTIN® vaccine, LEUVECTIN® vaccine, and VAXID® vaccine; LURTOTECAN® topoisomerase I inhibitor; ABARELIX® rmRH; lapatinib ditosylate (an ErbB-2 and EGFR dual tyrosine kinase small-molecule inhibitor also known as GW572016); and pharmaceutically acceptable salts, acids or derivatives of any of the above.

A "growth inhibitory agent" when used herein refers to a compound or composition which inhibits growth of a cell (such as a cell expressing PRO) either in vitro or in vivo. Thus, the growth inhibitory agent may be one which significantly reduces the percentage of cells (such as a cell expressing PRO) in S phase. Examples of growth inhibitory agents include agents that block cell cycle progression (at a place other than S phase), such as agents that induce G1 arrest and M-phase arrest. Classical M-phase blockers include the vincas (vincristine and vinblastine), taxanes, and topoisomerase II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogenes, and antineoplastic drugs" by Murakami et al. (WB Saunders: Philadelphia, 1995), especially p. 13. The taxanes (paclitaxel and docetaxel) are anticancer drugs both derived from the yew tree. Docetaxel (TAXOTERE®, Rhone-Poulenc Rorer), derived from the European yew, is a semisynthetic analogue of paclitaxel (TAXOL®, Bristol-Myers Squibb). Paclitaxel and docetaxel promote the assembly of microtubules from tubulin dimers and stabilize microtubules by preventing depolymerization, which results in the inhibition of mitosis in cells.

As used herein, the term "EGFR inhibitor" refers to compounds that bind to or otherwise interact directly with EGFR and prevent or reduce its signaling activity, and is alternatively referred to as an "EGFR antagonist." Examples of such agents include antibodies and small molecules that bind to EGFR. Examples of antibodies which bind to EGFR include MAb 579 (ATCC CRL HB 8506), MAb 455 (ATCC CRL HB8507), MAb 225 (ATCC CRL 8508), MAb 528 (ATCC CRL 8509) (see, US Patent No. 4,943, 533, Mendelsohn *et cal.)* and variants thereof, such as chimerized 225 (C225 or Cetuximab; ERBUTIX^{®}) and reshaped human 225 (H225) (see, WO 9b/40210, Imclone Systems Inc.); IMC-11F8, a fully human, EGFR-targeted antibody (Imclone); antibodies that bind type II mutant EGFR (US Patent No. 5,212,290); humanized and chimeric antibodies that bind EGFR as described in US Patent No. 5,891,996; and human antibodies that bind EGFR, such as ABX-EGF or Panitumumab (see WO98/50433, Abgenix/Amgen); EMD 55900 (Stragliotto et al. Eur. J. Cancer 32A:636-640 (1996)); EMD7200 (matuzumab) a humanized EGFR antibody directed against EGFR that competes with both EGF and TGF-alpha for EGFR binding (EMD/Mcrck); human EGFR antibody, HuMax-EGFR (GenMab); fully human antibodies known as E1.1, E2.4, E2.5, E6.2, E6.4, E2.11, E6. 3 and E7.6. 3 and described in US 6,235,883; MDX-447 (Medarex Inc); and mAb 806 or humanized mAb 806 (Johns et al., J. Biol. Chem. 279(29):30375-30384 (2004)). The anti-EGFR antibody may be conjugated with a cytotoxic agent, thus generating an immunoconjugate (see, *e.g*., EP659,439A2, Merck Patent GmbH). EGFR antagonists include small molecules such as compounds described in US Patent Nos: 5,616,582, 5,457,105, 5,475,001, 5,654,307, 5,679,683, 6,084,095, 6,265,410, 6,455,534, 6,521,620, 6,596,726, 6,713,484, 5,770,599, 6,140,332, 5,866,572, 6,399,602, 6,344,459, 6,602,863, 6,391,874, 6,344,455, 5,760,041, 6,002,008, and 5,747,498, as well as the following PCT publications: WO98/14451, WO98/50038, WO99/09016, and WO99/24037. Particular small molecule EGFR antagonists include OSI-774 (CP-358774, erlotinib, TARCEVA^{®} Genentech/OSI Pharmaceuticals); PD 183805 (CI 1033, 2-propenamide, N-[4-[(3-chloro-4-fluorophenyl)amino]-7-[3-(4-morpholinyl)propoxy]-6-quinazolinyl]-, dihydrochloride, Pfizer Inc.); ZD1839, gefitinib (IRESSA™) 4-(3'-Chloro-4'-fluoroanilino)-7-methoxy-6-(3-morpholinopropoxy)quinazoline, AstraZeneca); ZM 105180 ((6-amino-4-(3-methylphenyl-amino)-quinazoline, Zeneca); BIBX-1382 (N8-(3-chloro-4-fluoro-phenyl)-N2-(1-methyl-piperidin-4-yl)-pyrimido[5,4-d]pyrimidine-2,8-diamine, Boehringer Ingelheim); PKI-166 ((R)-4-[4-[(1-phenylethyl)amino]-1H-pyrrolo[2,3-d]pyrimidin-6-yl]-phenol); (R)-6-(4-hydroxyphenyl)-4-[(1-phenylethyl)amino]-7H-pyrrolo[2,3-d]pyrimidine); CL-387785 (N-[4-[(3-bromophenyl)amino]-6-quinazolinyl]-2-butynamide); EKB-569 (N-[4-[(3-chloro-4-fluorophenyl)amino]-3-cyano-7-ethoxy-6-quinolinyl]-4-(dimethylamino)-2-butenamide) (Wyeth); AG1478 (Pfizer); AG1571 (SU 5271; Pfizer); dual EGFR/HER2 tyrosine kinase inhibitors such as lapatinib (TYKERB®, GSK572016 or N-[3-chloro-4-[(3 fluorophenyl)methoxy]phenyl]6[5[[[2methylsulfonyl)ethyl]amino]methyl]-2-furanyl]-4-quinazolinamine; Glaxo-SmithKline).

A "tyrosine kinase inhibitor" is a molecule which inhibits tyrosine kinase activity of a tyrosine kinase such as a HER receptor. Examples of such inhibitors include the EGFR-targeted drugs noted in the preceding paragraph; small molecule HER2 tyrosine kinase inhibitor such as TAK165 available from Takeda; CP-724,714, an oral selective inhibitor of the ErbB2 receptor tyrosine kinase (Pfizer and OSI); dual-HER inhibitors such as EKB-569 (available from Wyeth) which preferentially binds EGFR but inhibits both HER2 and EGFR-overexpressing cells; lapatinib (GSK572016; available from Glaxo-SmithKline), an oral HER2 and EGFR tyrosine kinase inhibitor; PKI-166 (available from Novartis); pan-HER inhibitors such as canertinib (CI-1033; Pharmacia); Raf-1 inhibitors such as antisense agent ISIS-5132 available from ISIS Pharmaceuticals which inhibit Raf-1 signaling; non-HER targeted TK inhibitors such as imatinib mesylate (GLEEVEC™, available from Glaxo SmithKline); multi-targeted tyrosine kinase inhibitors such as sunitinib (SUTENT®, available from Pfizer); VEGF receptor tyrosine kinase inhibitors such as vatalanib (PTK787/ZK222584, available from Novartis/Schering AG); MAPK extracellular regulated kinase I inhibitor CI-1040 (available from Pharmacia); quinazolines, such as PD 153035,4-(3-chloroanilino) quinazoline; pyridopyrimidines; pyrimidopyrimidines; pyrrolopyrimidines, such as CGP 59326, CGP 60261 and CGP 62706; pyrazolopyrimidines, 4-(phenylamino)-7H-pyrrolo[2,3-d] pyrimidines; curcumin (diferuloyl methane, 4,5-bis (4-fluoroanilino)phthalimide); tyrphostines containing nitrothiophene moieties; PD-0183805 (Warner-Lamber); antisense molecules (*e.g*. those that bind to HER-encoding nucleic acid); quinoxalines (US Patent No. 5,804,396); tryphostins (US Patent No. 5,804,396); ZD6474 (Astra Zeneca); PTK-787 (Novartis/Schering AG); pan-HER inhibitors such as CI-1033 (Pfizer); Affinitac (ISIS 3521; Isis/Lilly); imatinib mesylate (GLEEVEC™); PKI 166 (Novartis); GW2016 (Glaxo SmithKline); CI-I033 (Pfizer); EKB-569 (Wyeth); Semaxinib (Pfizer); ZD6474 (AstraZeneca); PTK-787 (Novartis/Schering AG); INC-1C11 (Imclone); or as described in any of the following patent publications: US Patent No. 5,804,396; WO 1999/09016 (American Cyanamid); WO 1998/43960 (American Cyanamid); WO 1997/38983 (Warner Lambert); WO 1999/06378 (Warner Lambert); WO 1999/06396 (Warner Lambert); WO 1996/30347 (Pfizer, Inc); WO 1996/33978 (Zeneca); WO 1996/3397 (Zeneca); and WO 1996/33980 (Zeneca).

"Antibodies" (Abs) and "immunoglobulins" (Igs) refer to glycoproteins having similar structural characteristics. While antibodies exhibit binding specificity to a specific antigen, immunoglobulins include both antibodies and other antibody-like molecules which generally lack antigen specificity. Polypeptides of the latter kind are, for example, produced at low levels by the lymph system and at increased levels by myelomas.

The terms "antibody" and "immunoglobulin" are used interchangeably in the broadest sense and include monoclonal antibodies (*e.g*., full length or intact monoclonal antibodies), polyclonal antibodies, monovalent antibodies, multivalent antibodies, multispecific antibodies (*e.g*., bispecific antibodies so long as they exhibit the desired biological activity) and may also include certain antibody fragments (as described in greater detail herein). An antibody can be chimeric, human, humanized and/or affinity matured.

The term "anti-PRO antibody" or "an antibody that binds to PRO" refers to an antibody that is capable of binding PRO with sufficient affinity such that the antibody is useful as a diagnostic and/or therapeutic agent in targeting PRO. Preferably, the extent of binding of an anti-PRO antibody to an unrelated, non-PRO protein is less than about 10% of the binding of the antibody to PRO as measured, e.g., by a radioimmunoassay (RIA). In certain embodiments, an antibody that binds to PRO has a dissociation constant (Kd) of ≤ 1 µM, ≤ 100 nM, ≤ 10 nM, ≤ 1 nM, or ≤ 0.1 nM. In certain embodiments, an anti-PRO antibody binds to an epitope of PRO that is conserved among PRO from different species.

The terms "full length antibody," "intact antibody" and "whole antibody" are used herein interchangeably to refer to an antibody in its substantially intact form, not antibody fragments as defined below. The terms particularly refer to an antibody with heavy chains that contain the Fc region.

"Antibody fragments" comprise a portion of an intact antibody, preferably comprising the antigen binding region thereof. Examples of antibody fragments include Fab, Fab', F(ab')₂, and Fv fragments; diabodies; linear antibodies; single-chain antibody molecules; and multispecific antibodies formed from antibody fragments.

Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab')₂ fragment that has two antigen-combining sites and is still capable of cross-linking antigen.

"Fv" is a minimum antibody fragment which contains a complete antigen-binding site. In one embodiment, a two-chain Fv species consists of a dimer of one heavy- and one light-chain variable domain in tight, non-covalent association. Collectively, the six CDRs of an Fv confer antigen-binding specificity to the antibody. However, even a single variable domain (or half of an Fv comprising only three CDRs specific for an antigen) has the ability to recognize and bind antigen, although at a lower affinity than the entire binding site.

The Fab fragment contains the heavy- and light-chain variable domains and also contains the constant domain of the light chain and the first constant domain (CH1) of the heavy chain. Fab' fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear a free thiol group. F(ab')₂ antibody fragments originally were produced as pairs of Fab' fragments which have hinge cysteines between them. Other chemical couplings of antibody fragments are also known.

"Single-chain Fv" or "scFv" antibody fragments comprise the VH and VL domains of antibody, wherein these domains are present in a single polypeptide chain. Generally, the scFv polypeptide further comprises a polypeptide linker between the VH and VL domains which enables the scFv to form the desired structure for antigen binding. For a review of scFv see Pluckthun, in The Pharmacology of Monoclonal Antibodies, vol. 113, Rosenburg and Moore eds., Springer-Verlag, New York, pp. 269-315 (1994).

The term "diabodies" refers to small antibody fragments with two antigen-binding sites, which fragments comprise a heavy-chain variable domain (VH) connected to a light-chain variable domain (VL) in the same polypeptide chain (VH-VL). By using a linker that is too short to allow pairing between the two domains on the same chain, the domains are forced to pair with the complementary domains of another chain and create two antigen-binding sites. Diabodies may be bivalent or bispecific. Diabodies are described more fully in, for example, EP 404,097; W093/1161; Hudson et al. (2003) Nat. Med. 9:129-134; and Hollinger et al., Proc. Natl. Acad. Sci. USA 90: 6444-6448 (1993). Triabodies and tetrabodies are also described in Hudson et al. (2003) Nat. Med. 9:129-134.

The term "monoclonal antibody" as used herein refers to an antibody obtained from a population of substantially homogeneous antibodies, i.e., the individual antibodies comprising the population are identical except for possible mutations, e.g., naturally occurring mutations, that may be present in minor amounts. Thus, the modifier "monoclonal" indicates the character of the antibody as not being a mixture of discrete antibodies. In certain embodiments, such a monoclonal antibody typically includes an antibody comprising a polypeptide sequence that binds a target, wherein the target-binding polypeptide sequence was obtained by a process that includes the selection of a single target binding polypeptide sequence from a plurality of polypeptide sequences. For example, the selection process can be the selection of a unique clone from a plurality of clones, such as a pool of hybridoma clones, phage clones, or recombinant DNA clones. It should be understood that a selected target binding sequence can be further altered, for example, to improve affinity for the target, to humanize the target binding sequence, to improve its production in cell culture, to reduce its immunogenicity *in vivo,* to create a multispecific antibody, etc., and that an antibody comprising the altered target binding sequence is also a monoclonal antibody of this invention. In contrast to polyclonal antibody preparations which typically include different antibodies directed against different determinants (epitopes), each monoclonal antibody of a monoclonal antibody preparation is directed against a single determinant on an antigen. In addition to their specificity, monoclonal antibody preparations are advantageous in that they are typically uncontaminated by other immunoglobulins.

The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including, for example, the hybridoma method (e.g., Kohler et al., Nature, 256: 495 (1975); Harlow et al., Antibodies: A Laboratory Manual, (Cold Spring Harbor Laboratory Press, 2nd ed. 1988); Hammerling et al., in: Monoclonal Antibodies and T-Cell Hybridomas 563-681 (Elsevier, N.Y., 1981)), recombinant DNA methods (see, e.g., U.S. Patent No. 4,816,567), phage display technologies (see, e.g., Clackson et al., Nature, 352: 624-628 (1991); Marks et al., J. Mol. Biol. 222: 581-597 (1992); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132(2004), and technologies for producing human or human-like antibodies in animals that have parts or all of the human immunoglobulin loci or genes encoding human immunoglobulin sequences (see, e.g., WO98/24893; WO96/34096; WO96/33735; WO91/10741; Jakobovits et al., Proc. Natl. Acad. Sci. USA 90: 2551 (1993); Jakobovits et al., Nature 362: 255-258 (1993); Bruggemann et al., Yecar in Immunol. 7:33 (1993); U.S. Patent Nos. 5,545,807; 5,545,806; 5,569,825;.5,625,126; 5,633,425; 5,661,016; Marks et al., Bio. Technology 10: 779-783 (1992); Lonberg et al., Nature 368: 856-859 (1994); Morrison, Nature 368: 812-813 (1994); Fishwild et al., Nature Biotechnol. 14: 845-851 (1996); Neuberger, Nature Biotechnol. 14: 826 (1996) and Lonberg and Huszar, Intern. Rev. Immunol. 13: 65-93 (1995).

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; and Morrison el al., Proc. Natl. Acad. Sci. USA 81:6851-6855 (1984)).

"Humanized" forms of non-human (*e.g*., murine) antibodies are chimeric antibodies that contain minimal sequence derived from non-human immunoglobulin. In one embodiment, a humanized antibody is a human immunoglobulin (recipient antibody) in which residues from a hypervariable region of the recipient are replaced by residues from a hypervariable region of a non-human species (donor antibody) such as mouse, rat, rabbit, or nonhuman primate having the desired specificity, affinity, and/or capacity. In some instances, framework region (FR) residues of the human immunoglobulin are replaced by corresponding non-human residues. Furthermore, humanized antibodies may comprise residues that are not found in the recipient antibody or in the donor antibody. These modifications may be made to further refine antibody performance. In general, a humanized antibody will comprise substantially all of at least one, and typically two, variable domains, in which all or substantially all of the hypervariable loops correspond to those of a non-human immunoglobulin, and all or substantially all of the FRs are those of a human immunoglobulin sequence. The humanized antibody optionally will also comprise at least a portion of an immunoglobulin constant region (Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992). See also the following review articles and references cited therein: Vaswani and Hamilton, Ann. Allergy, Asthma & Immunol. 1:105-115 (1998); Harris, Biochem. Soc. Transactions 23:1035-1038 (1995); Hurle and Gross, Curr. Op. Biotech. 5:428-433 (1994).

A "human antibody" is one which comprises an amino acid sequence corresponding to that of an antibody produced by a human and/or has been made using any of the techniques for making human antibodies as disclosed herein. Such techniques include screening human-derived combinatorial libraries, such as phage display libraries (see, e.g., Marks et al., J. Mol. Biol., 222: 581-597 (1991) and Hoogenboom et al., Nucl. Acids Res., 19: 4133-4137 (1991)); using human myeloma and mouse-human heteromyeloma cell lines for the production of human monoclonal antibodies (see, e.g., Kozbor J. Immunol., 133: 3001 (1984); Brodeur et al., Monoclonal Antibody Production Techniques and Applications, pp. 51-63 (Marcel Dekker, Inc., New York, 1987); and Boemer et al., J. Immunol., 147: 86 (1991)); and generating monoclonal antibodies in transgenic animals (e.g., mice) that are capable of producing a full repertoire of human antibodies in the absence of endogenous immunoglobulin production (see, e.g., Jakobovits et al., Proc. Natl. Acad. Sci USA, 90: 2551 (1993); Jakobovits et al., Nature. 362: 255 (1993); Bruggermann et al., Year in Immunol., 7: 33 (1993)). This definition of a human antibody specifically excludes a humanized antibody comprising antigen-binding residues from a non-human animal.

An "affinity matured" antibody is one with one or more alterations in one or more CDRs thereof which result in an improvement in the affinity of the antibody for antigen, compared to a parent antibody which does not possess those alteration(s). In one embodiment, an affinity matured antibody has nanomolar or even picomolar affinities for the target antigen. Affinity matured antibodies are produced by procedures known in the art. Marks et al. Bio/Technology 10:779-783 (1992) describes affinity maturation by VH and VL domain shuffling. Random mutagenesis of HVR and/or framework residues is described by: Barbas et al. Proc Nat. Acad. Sci. USA 91:3809-3813 (1994); Schier et al. Gene 169:147-155 (1995); Yelton et al. J. Immunol. 155:1994-2004 (1995); Jackson et al., J. Immunol. 154(7):3310-9 (1995); and Hawkins et al, J. Mol. Biol. 226:889-896 (1992).

A "blocking antibody" or an "antagonist antibody" is one which inhibits or reduces a biological activity of the antigen it binds. Certain blocking antibodies or antagonist antibodies partially or completely inhibit the biological activity of the antigen.

A "small molecule" or "small organic molecule" is defined herein as an organic molecule having a molecular weight below about 500 Daltons.

An "PRO-binding oligopeptide" or an "oligopeptide that binds PRO" is an oligopeptide that is capable of binding PRO with sufficient affinity such that the oligopeptide is useful as a diagnostic and/or therapeutic agent in targeting PRO. In certain embodiments, the extent of binding of a PRO-binding oligopeptide to an unrelated, non-PRO protein is less than about 10% of the binding of the PRO-binding oligopeptide to PRO as measured, e.g., by a surface plasmon resonance assay. In certain embodiments, a PRO-binding oligopeptide has a dissociation constant (Kd) of ≤ 1 µM, ≤ 100 nM, ≤ 10 nM, ≤ 1 nM, or ≤ 0.1 nM.

An "PRO-binding organic molecule" or "an organic molecule that binds PRO" is an organic molecule other than an oligopeptide or antibody as defined herein that is capable of binding PRO with sufficient affinity such that the organic molecule is useful as a diagnostic and/or therapeutic agent in targeting PRO. In certain embodiments, the extent of binding of a PRO-binding organic molecule to an unrelated, non-PRO protein is less than about 10% of the binding of the PRO-binding organic molecule to PRO as measured, e.g., by a surface plasmon resonance assay. In certain embodiments, a PRO-binding organic molecule has a dissociation constant (Kd) of ≤ 1 µM, ≤ 100 nM, ≤ 10 nM, ≤ 1 nM, or ≤ 0.1 nM.

The dissociation constant (Kd) of any molecule that binds a target polypeptide may conveniently be measured using a surface plasmon resonance assay. Such assays may employ a BIAcore™-2000 or a BIAcore™-3000 (BIAcore, Inc., Piscataway, NJ) at 25°C with immobilized target polypeptide CM5 chips at ~10 response units (RU). Briefly, carboxymethylated dextran biosensor chips (CM5, BIAcore Inc.) are activated with *N*-ethyl-*N*'-(3-dimethylaminopropyl)-carbodiimide hydrochloride (EDC) and *N*-hydroxysuccinimide (NHS) according to the supplier's instructions. Target polypeptide is diluted with 10 mM sodium acetate, pH 4.8, to 5 µg/ml (~0.2 µM) before injection at a flow rate of 5 µl/minute to achieve approximately 10 response units (RU) of coupled protein. Following the injection of target polypeptide, 1 M ethanolamine is injected to block unreacted groups. For kinetics measurements, two-fold serial dilutions of the binding molecule (0.78 nM to 500 nM) are injected in PBS with 0.05% Tween 20 (PBST) at 25°C at a flow rate of approximately 25 µl/min. Association rates (kₒₙ) and dissociation rates (k_{off}) are calculated using a simple one-to-one Langmuir binding model (BIAcore Evaluation Software version 3.2) by simultaneously fitting the association and dissociation sensorgrams. The equilibrium dissociation constant (Kd) is calculated as the ratio k_{off}/k_{on.} See, e.g., Chen, Y., et al., (1999) J. Mol. Biol. 293:865-881. If the on-rate of an antibody exceeds 10⁶ M⁻¹ s⁻¹ by the surface plasmon resonance assay above, then the on-rate can be determined by using a fluorescent quenching technique that measures the increase or decrease in fluorescence emission intensity (excitation = 295 nm; emission = 340 nm, 16 nm band-pass) at 25°C of a 20 nM antibody (Fab form) in PBS, pH 7.2, in the presence of increasing concentrations of antigen as measured in a spectrometer, such as a stop-flow equipped spectrophometer (Aviv Instruments) or a 8000-series SLM-Aminco spectrophotometer (Thermospectronic) with a stirred cuvette.

A "liposome" is a small vesicle composed of various types of lipids, phospholipids and/or surfactant which is useful for delivery of an agent, e.g., a drug, to a mammal. The components of the liposome are commonly arranged in a bilayer formation, similar to the lipid arrangement of biological membranes.

The word "label" when used herein refers to a detectable compound or composition. The label may be detectable by itself (*e.g*., radioisotope labels or fluorescent labels) or, in the case of an enzymatic label, may catalyze chemical alteration of a substrate compound or composition which results in a detectable product. Radionuclides that can serve as detectable labels include, for example, I-131, I-123, I-125, Y-90, Re-188, Re-186, At-211, Cu-67, Bi-212, and Pd-109.

An "isolated" biological molecule, such as a nucleic acid, polypeptide, or antibody, is one which has been identified and separated and/or recovered from at least one component of its natural environment.

### II. DESCRIPTION OF CERTAIN EMBODIMENTS

Nucleotide and amino acid variations associated with tumors are provided herein. These variations provide biomarkers for cancer and/or predispose or contribute to tumorigenesis or tumor promotion. Accordingly, the variations disclosed herein are useful in a variety of settings, e.g., in methods and compositions related to cancer diagnosis and therapy.

### A. Variations

Variations were identified in 100 genes from twenty-five different human lung tumor (non-small cell lung carcinoma) specimens. Those variations are listed in the tables shown in Figures 1, 2, and 3.

### 1. Variations Identified by MRD

Variations were identified by MRD, as further described in Example A. Those variations are listed in Figure 1. In Figure 1, the first column of the table ("VARIANT ID") lists the identification numbers assigned to each variation. The second column ("UNQID") provides an additional gene-specific identification number. The third column ("GENE NAME (Ensembl)") lists the genes in which the variations occur. The gene names are in accordance with Ensembl nomenclature. (The Ensembl project is described in Hubbard et al. (2006) Nucleic Acids Res. Database issue:D1-D8.) The fourth column ("GENE DESCRIPTION") provides a brief description from Ensembl of each gene identified in the fourth column. The fifth column ("NT CHANGE") lists the nucleotide changes by their chromosomal positions. For example, the designation "9:130790136:G/T" in column five of the first row indicates that a nucleotide change in ABL1 polynucleotide occurs at nucleotide 130790136 of chromosome 9, and the nucleotide change is a substitution of G at that position with T. In Figure 1, the nucleotide changes are all single nucleotide substitutions. All nucleotide substitutions identified in Figure 1 occurred in the coding regions of the indicated genes, and all are either nonsynonymous mutations or nonsense mutations. Additionally, all nucleotide substitutions identified in Figure 1 are somatic mutations (i.e., mutations found only in the tumor sample and not in the patient matched normal sample).

The sixth column ("AA CHANGE") of the table in Figure 1 lists the amino acid changes resulting from the nucleotide substitutions listed in the previous column. The amino acid positions designated in column 6 refer to the PRO amino acid sequences provided in Table 1 below, which are translations of the corresponding PRO cDNA sequences, also provided in Table 1. The amino acid and cDNA sequences provided in Table 1 are from the Ensembl database. The designated amino acid positions in column 6 also refer to the corresponding amino acid positions of fragments or variants of PRO, (e.g., allelic variants or splice variants), which can be routinely identified using sequence alignments or similar techniques. Where a nucleotide substitution resulted in a stop codon (i.e., a nonsense mutation), the corresponding amino acid change is indicated in column 6 by an "O" (e.g., see Variant ID 10015, indicating an amino acid change of "1170Q→O"). The seventh column ("DOMAIN") indicates, for some of the variations, the protein domains in which the amino acid changes occur. Protein domains are indicated by Pfam nomenclature. (See, e.g., Bateman et al. (2004) Nucleic Acids Res. 32(1):D138-141.)

**TABLE 1**

| GENE NAME | cDNA SEQUENC E SEQ ID NO | AMINO ACID SEQUENCE SEQ ID NO: |
|---|---|---|
| ABL1 | 1 | 38 |
| APC | 2 | 39 |
| ATM | 3 | 40 |
| ATR | 4 | 41 |
| FBXW7 | 5 | 42 |
| FLT1 | 6 | 43 |
| FLT3 | 7 | 44 |
| FRAP1 | 8 | 45 |
| KDR | 9 | 46 |
| MET | 10 | 47 |
| MLL | 11 | 48 |
| NF1 | 12 | 49 |
| PDGFRA | 13 | 50 |
| PDGFRB | 14 | 51 |
| PLK1 | 15 | 52 |
| TCF1 | 16 | 53 |
| TP53 | 17 | 54 |
| TSHR | 18 | 55 |

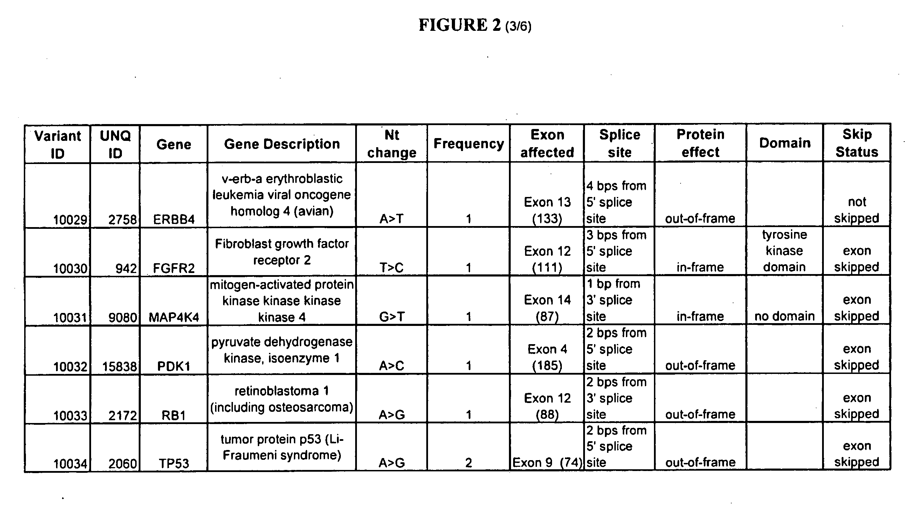

Figure 2 lists somatic nucleotide variations that fell within consensus splice site regions of six genes, In Figure 2, the first column of the table ("VARIANT ID") lists the identification numbers assigned to each variation. The second column ("UNQID") provides an additional gene-specific identification number. The third column ("GENE") lists the genes in which the variations occur. The gene names are in accordance with Ensembl nomenclature. The fourth column ("GENE DESCRIPTION") provides a brief description from Ensembl of each gene identified in the fourth column. The fifth column ("NT CHANGE") refers to the nucleotide changes, which were identified by MRD. The sixth column ("FREQUENCY") indicates the number of times that a particular variation was found. The seventh column ("EXON AFFECTED") refers to the exons associated with the consensus splice site variations. The eighth column ("SPLICE SITE") describes with particularity where in the consensus splice sites the variations occurred. The ninth column ("PROTEIN EFFECT") indicates whether the variations resulted in an in-frame or out-of-frame protein. The tenth column ("DOMAIN") indicates the protein domains affected by two of the variations. The last column ("SKIP STATUS") indicates whether the exons associated with the consensus splice site variations were skipped or not during mRNA splicing. The amino acid sequences encoded by certain genes listed in Figure 2 are provided below in Table 2:

**TABLE 2**

| GENE NAME | AMINO ACID SEQUENCE SEQ ID NO: |
|---|---|
| ERBB4 | 75 |
| FGFR2 | 76 |
| MAP4K4 | 77 |
| PDK1 | 78 |
| RB1 | 79 |

### 2. Variations Identified by Sanger Sequencing

Variations were identified by Sanger sequencing, as further described in Example B. Those variations are listed in Figure 3. The first column of the table ("VARIANT ID") lists the identification numbers assigned to each variation. The second column ("UNQID") lists additional gene-specific identification numbers. The third column ("GENE NAME (HUGO)") lists the genes in which the variations occur. The gene names are in accordance with the Human Genome Organisation (HUGO) nomenclature. *See* Wain et al. (2002) Genomics 79:464-470. The fourth column ("GENE DESCRIPTION") provides a brief description from HUGO of each gene identified in the third column. The fifth column ("NT CHGE") lists the nucleotide change found in each gene. In this study, the nucleotide changes were all single nucleotide substitutions. The term "HETSUB" in the fifth column stands for "heterozygous substitution," meaning that both the indicated variation and the wild type allele were detected in the tumor sample. The term "HOMSUB" stands for "homozygous substitution," meaning that the indicated variation was detected in the tumor sample, and the wild type allele was not detected in the tumor sample. This may occur, for example, if the tumor has undergone loss of heterozygosity (e.g., where one copy of a given gene contains a variation, and the other copy contains a deletion in the corresponding gene). Nucleotide numbers refer to nucleotide positions in the cDNA sequences corresponding to the indicated genes. For example, Variant ID 912 is a heterozygous substitution designated "2667A>G," indicating that an "A" has been substituted by a "G" in one copy of the PTCH gene, with the substitution occurring at a genomic position corresponding to nucleotide 2667 of the cDNA sequence (SEQ ID NO:32) of the PTCH gene. For each gene listed in Figure 3, the corresponding cDNA sequences and their translations are provided in the sequence listing, as indicated in Table 3 below.

**TABLE 3**

| GENE NAME | cDNA SEQUENC E SEQ ID NO | AMINO ACID SEQUENCE SEQ ID NO: |
|---|---|---|
| AKT2 | 19 | 56 |
| AURKC | 20 | 57 |
| BCL2L1 | 21 | 58 |
| CDKN2A | 22 | 59 |
| ERBB3 | 23 | 60 |
| ERBB4 | 24 | 61 |
| FGFR3 | 25 | 62 |
| FRAP1 | 26 | 63 |
| KDR | 27 | 64 |
| MAP2K2 | 28 | 65 |
| MET | 29 | 66 |
| PIK3CA | 30 | 67 |
| PLK1 | 31 | 68 |
| PTCH | 32 | 69 |
| RB1 | 33 | 70 |
| RET | 34 | 71 |
| STK6 | 35 | 72 |
| TGFBR2 | 36 | 73 |
| TP53 | 37 | 74 |

All nucleotide substitutions identified in Figure 3 occurred in the coding regions of the indicated genes. Additionally, all nucleotide substitutions identified in this study were validated (i.e., confirmed) by resequencing DNA from the tumor sample(s) in which the variation was initially discovered.

The sixth column ("AA_CHGE") of the table in Figure 3 lists the amino acid changes resulting from the nucleotide substitutions listed in the previous column. Amino acids are numbered according to their positions in the translated cDNA sequences. Where a nucleotide substitution resulted in a stop codon (i.e., a nonsense mutation), the corresponding amino acid change is indicated by an "O" (e.g., see Variant ID 4356, indicating an amino acid change of "61C>O"). The seventh column ("EFFECT") indicates whether the variation results in a nonsynonymous ("Nonsyn.") amino acid substitution or in a nonsense ("Nonsense") mutation. The eighth column ("DOMAIN") indicates, for some of the variations, the protein domains in which the amino acid changes occur. Protein domains are indicated by Pfam nomenclature. (See, e.g., Bateman et al. (2004) Nucleic Acids Res. 32(1):D138-141.)

The ninth column ("SOMATIC OR GERMLINE") indicates whether a given variation was found only in the tumor sample and not in the patient matched normal sample (i.e., a somatic mutation), or whether it was found in both the tumor sample and the patient matched normal sample (i.e., a germline polymorphism). Certain variations were found in multiple tumor samples. In such cases, a variation was classified as a somatic mutation if it was found to be absent from at least one patient matched normal sample. Likewise, a variation was classified as a germline polymorphism it was found to be present in at least one patient matched normal sample.

### 3. Tumor types and protein families

The variations listed in Figures 1, 2, and 3 were identified in non-small cell lung carcinomas. Routine screening may be used to determine whether variations occur in other types of cancers. The compositions and methods of the present invention are applicable to any cancer comprising variations in the indicated genes and/or encoded polypeptides.

Variations were found in genes encoding proteins from a variety of protein families. For example, variations were found in a number of genes encoding kinases, including preceptor tyrosine kinases (RTKs). Generally, activation of kinases is often associated with cell proliferation and tumor promotion. Thus, variations in genes encoding kinases may be "gain-of-function" mutations that increase kinase activity. Tumors in which such variations are detected may thus be responsive to kinase antagonists. A variety of kinase antagonists are currently known in the art. Such kinase antagonists include, but are not limited to antagonist antibodies and small molecule antagonists, e.g., 3-[2,4-dimethylpyrrol-5-yl)methylidene]-indolin-2-one ("SU5416"); imatinib (Gleevec®), a 2-phenylaminopyrimidine; 1-*tert*-butyl-3-[6-(3,5-dimethoxy-phenyl)-2-(4-diethylamino-butylamino)-pyrido[2,3-d]pyrimidin-7-yl]-urea ("PD173074") (see, e.g., Moffa et al. (2004) Mol. Cancer. Res. 2:643-652); and indolinones such as 3-[3-(2-carboxyethyl)-4-methylpyrrol-2-methylidenyl]-2-indolinone ("SU5402") (see, e.g., Bernard-Pierrot (2004) Oncogene 23:9201-9211). A kinase antagonist may also be an EGFR inhibitor or a tyrosine kinase inhibitor, as defined herein.

Variations were also found in tumor suppressor genes (e.g., p53 and retinoblastoma). Generally, loss of tumor suppressor function is often associated with cell proliferation and tumor promotion. Thus, variations in genes encoding tumor suppressors may be complete or partial "loss-of-function" mutations that decrease tumor suppressor function. Tumors in which such variations are detected may be responsive to tumor suppressor agonists. A tumor suppressor agonist may be, e.g., an agonist antibody, a polynucleotide encoding a tumor suppressor, the tumor suppressor itself, or a tumor suppressor-Fc fusion.

### B. Compositions

In one aspect, an isolated polynucleotide comprising at least a fragment of a PRO polynucleotide is provided, wherein the fragment comprises a nucleotide variation. In one embodiment, the nucleotide variation is at a nucleotide position selected from Figures 1-3. In one such embodiment, the nucleotide variation is a nucleotide change selected from Figures 1-3. In another embodiment, a fragment of a PRO polynucleotide is at least about 10 nucleotides in length, alternatively at least about 15 nucleotides in length, alternatively at least about 20 nucleotides in length, alternatively at least about 30 nucleotides in length, alternatively at least about 40 nucleotides in length, alternatively at least about 50 nucleotides in length, alternatively at least about 60 nucleotides in length, alternatively at least about 70 nucleotides in length, alternatively at least about 80 nucleotides in length, alternatively at least about 90 nucleotides in length, alternatively at least about 100 nucleotides in length, alternatively at least about 110 nucleotides in length, alternatively at least about 120 nucleotides in length, alternatively at least about 130 nucleotides in length, alternatively at least about 140 nucleotides in length, alternatively at least about 150 nucleotides in length, alternatively at least about 160 nucleotides in length, alternatively at least about 170 nucleotides in length, alternatively at least about 180 nucleotides in length, alternatively at least about 190 nucleotides in length, alternatively at least about 200 nucleotides in length, alternatively at least about 250 nucleotides in length, alternatively at least about 300 nucleotides in length, alternatively at least about 350 nucleotides in length, alternatively at least about 400 nucleotides in length, alternatively at least about 450 nucleotides in length, alternatively at least about 500 nucleotides in length, alternatively at least about 600 nucleotides in length, alternatively at least about 700 nucleotides in length, alternatively at least about 800 nucleotides in length, alternatively at least about 900 nucleotides in length, alternatively at least about 1000 nucleotides in length, and alternatively about the length of the full-length coding sequence. In this context the term "about" means the referenced nucleotide sequence length plus or minus 10% of that referenced length. In another embodiment, the polynucleotide comprises a nucleotide variation in a polynucleotide sequence selected from SEQ ID NO: 1-37 or a fragment thereof. In another embodiment, the complement of any of the above polynucleotides is provided. In another embodiment, a PRO encoded by the any of the above polynucleotides is provided.

In one embodiment, an isolated polynucleotide provided herein is detectably labeled, e.g., with a radioisotope, a fluorescent agent, or a chromogenic agent. In another embodiment, an isolated polynucleotide is a primer. In another embodiment, an isolated polynucleotide is an oligonucleotide, e.g., an allele-specific oligonucleotide. In another embodiment, an oligonucleotide may be, for example, from 7-60 nucleotides in length, 9-45 nucleotides in length, 15-30 nucleotides in length, or 18-25 nucleotides in length. In another embodiment, an oligonucleotide may be, e.g., PNA, morpholino-phosphoramidates, LNA, or 2'-alkoxyalkoxy. Oligonucleotides as provided herein are useful, e.g., as hybridization probes for the detection of nucleotide variations.

In another aspect, an allele-specific oligonucleotide is provided that hybridizes to a region of a PRO polynucleotide comprising a nucleotide variation (e.g., a substitution). In one embodiment, the nucleotide variation is at a nucleotide position selected from Figures 1-3. In one such embodiments, the nucleotide variation is a nucleotide change selected from Figures 1-3. The allele-specific oligonucleotide, when hybridized to the region of the PRO polynucleotide, comprises a nucleotide that base pairs with the nucleotide variation. In another embodiment, the complement of an allele-specific oligonucleotide is provided. In another embodiment, a microarray comprises an allele-specific oligonucleotide or its complement. In another embodiment, an allele-specific oligonucleotide or its complement is an allele-specific primer.

An allele-specific oligonucleotide can be used in conjunction with a control oligonucleotide that is identical to the allele-specific oligonucleotide, except that the nucleotide that specifically base pairs with the nucleotide variation is replaced with a nucleotide that specifically base pairs with the corresponding nucleotide present in the wild type PRO polynucleotide. Such oligonucleotides may be used in competitive binding assays under hybridization conditions that allow the oligonucleotides to distinguish between a PRO polynucleotide comprising a nucleotide variation and a PRO polynucleotide comprising the corresponding wild type nucleotide. Using routine methods based on, e.g., the length and base composition of the oligonucleotides, one skilled in the art can arrive at suitable hybridization conditions under which (a) an allele-specific oligonucleotide will preferentially bind to a PRO polynucleotide comprising a nucleotide variation relative to a wild type PRO polynucleotide, and (b) the control oligonucleotide will preferentially bind to a wild type PRO polynucleotide relative to a PRO polynucleotide comprising a nucleotide variation. Exemplary conditions include conditions of high stringency, e.g., hybridization conditions of 5x standard saline phosphate EDTA (SSPE) and 0.5% NaDodSO₄ (SDS) at 55°C, followed by washing with 2X SSPE and 0.1% SDS at 55°C or room temperature.

In another aspect, a binding agent is provided that preferentially binds to a PRO comprising an amino acid variation, relative to a wild-type PRO. In one embodiment, the amino acid variation is at an amino acid position selected from Figure 1 or 3. In one such embodiment, the amino acid variation is an amino acid change selected from Figure 1 or 3. In another embodiment, the binding agent is an antibody.

In another aspect, diagnostic kits are provided. In one embodiment, a kit comprises any of the foregoing polynucleotides and an enzyme. In one embodiment, the enzyme is at least one enzyme selected from a nuclease, a ligase, and a polymerase.

### C. Methods

In one aspect, a method of detecting the presence of a tumor is provided, the method comprising detecting a variation in a PRO or PRO polynucleotide derived from a biological sample. In one embodiment, the biological sample is obtained from a mammal suspected of having a tumor.

In another aspect, a method of determining the genotype of a biological sample is provided, the method comprising detecting whether a nucleotide variation is present in a PRO polynucleotide derived from the biological sample. In one embodiment, the nucleotide variation is at a nucleotide position selected from Figures 1-3. In one such embodiment, the nucleotide variation is a nucleotide change selected from Figures 1-3. In another embodiment, the biological sample is suspected of comprising tumor cells. In another embodiment, the biological sample is a cell line, e.g., an immortalized cell line. In another embodiment, the biological sample is a tumor. In one such embodiment, the genotyping of the tumor provides a basis for classifying the tumor.

In another aspect, a method of identifying tumor cells in a biological sample from a mammal is provided, the method comprising detecting a variation in a PRO or PRO polynucleotide derived from the biological sample. In one embodiment, the variation is a nucleotide variation. In one such embodiment, the nucleotide variation is at a nucleotide position selected from Figures 1-3. In one such embodiment, the nucleotide variation is a nucleotide change selected from Figures 1-3. In another embodiment, the variation is an amino acid variation. In one such embodiment, the amino acid variation is in a PRO functional domain selected from Figure 1 or 3. In another such embodiment, the amino acid variation is at an amino acid position selected from Figure 1 or 3. In another such embodiment, the amino acid variation is an amino acid change selected from Figure 1 or 3. In another embodiment, the biological sample is a biopsy (e.g., a tissue sample containing cells suspected of being cancerous).

In another aspect, a method of diagnosing a tumor in a mammal is provided, the method comprising detecting the presence of a variation in a PRO or PRO polynucleotide derived from a biological sample obtained from the mammal, wherein the biological sample is known to or suspected of comprising tumor cells. In one embodiment, the variation is a nucleotide variation. In one such embodiment, the nucleotide variation is at a nucleotide position selected from Figures 1-3. In one such embodiment, the nucleotide variation is a nucleotide change selected from Figures 1-3. In another embodiment, the variation is an amino acid variation. In one such embodiments, the amino acid variation is in a PRO functional domain selected from Figure 1 or 3. In another such embodiment, the amino acid variation is at an amino acid position selected from Figure 1 or 3. In another such embodiment, the amino acid variation is an amino acid change selected from Figure 1 or 3. In another embodiment, the method is a method of classifying a tumor, e.g., as a tumor characterized by a nucleotide or amino acid variation(s) in a particular PRO polynucleotide or PRO.

In another aspect, a method is provided for predicting whether a tumor from a mammal will respond to a therapeutic agent that targets a PRO or PRO polynucleotide, the method comprising determining whether the tumor comprises a variation in a PRO or PRO polynucleotide, wherein the presence of a variation in a PRO or PRO polynucleotide indicates that the tumor will respond to the therapeutic agent. In one embodiment, the variation is a nucleotide variation. In one such embodiment, the nucleotide variation is at a nucleotide position selected from Figures 1-3. In one such embodiment, the nucleotide variation is a nucleotide change selected from Figures 1-3. In another embodiment, the variation is an amino acid variation. In one such embodiment, the amino acid variation is in a PRO functional domain selected from Figure 1 or 3. In another such embodiment, the amino acid variation is at an amino acid position selected from Figure 1 or 3. In another such embodiment, the amino acid variation is an amino acid change selected from Figure 1 or 3. In another embodiment, the PRO is a kinase (such as a tyrosine kinase, e.g., an RTK). In another embodiment, the PRO is a tumor suppressor.

In another aspect, a method of detecting the absence or presence of a nucleotide variation at a nucleotide position in a nucleic acid encoding a PRO is provided, the method comprising (a) contacting the nucleic acid with any of the polynucleotides described above (section II.B.) under conditions suitable for formation of a hybridization complex between the nucleic acid and the polynucleotide; and (b) detecting whether the polynucleotide specifically base pairs with the nucleic acid at the nucleotide position. In one embodiment, the nucleotide variation is at a nucleotide position selected from Figures 1-3. In one such embodiment, the nucleotide variation is a nucleotide change selected from Figures 1-3.

In another aspect, a method of detecting the absence or presence of a nucleotide variation in a nucleic acid encoding a PRO is provided, the method comprising (a) contacting the nucleic acid with an allele-specific oligonucleotide that is specific for the nucleotide variation under conditions suitable for hybridization of the allele-specific oligonucleotide to the nucleic acid; and (b) detecting the absence or presence of allele-specific hybridization. In one embodiment, the nucleotide variation is at a nucleotide position selected from Figures 1-3. In one such embodiment, the nucleotide variation is a nucleotide change selected from Figures 1-3. In another embodiment, an allele-specific oligonucleotide is an allele-specific primer.

In another aspect, a method of amplifying a nucleic acid comprising a PRO polynucleotide or fragment thereof is provided, wherein the PRO polynucleotide or fragment thereof comprises a nucleotide variation, the method comprising (a) contacting the nucleic acid with a primer that hybridizes to a sequence 3' of the nucleotide variation, and (b) extending the primer to generate an amplification product comprising the nucleotide variation. In one embodiment, the method further comprises contacting the amplification product with a second primer that hybridizes to a sequence 3' of the nucleotide variation, and extending the second primer to generate a second amplification product. In one such embodiment, the method further comprises amplifying the amplification product and second amplification product, e.g., by PCR. In any of the above embodiments, the nucleotide variation is at a nucleotide position selected from Figures 1-3. In any of the above embodiments, the nucleotide variation is a nucleotide change selected from Figures 1-3.

In another aspect, a method of assessing the activity of a PRO comprising an amino acid variation is provided, the method comprising (a) determining the activity of the PRO; and (b) comparing the activity of the PRO with the activity of a wild-type PRO. In one embodiment, the activity is kinase activity. In another embodiment, the activity is tumor suppressor activity. In another embodiment, the amino acid variation is in a PRO functional domain selected from Figure 1 or 3. In another embodiment, the amino acid variation is at an amino acid position selected from Figure 1 or 3. In another embodiment, the amino acid variation is an amino acid change selected from Figure 1 or 3.

In another aspect, a method of identifying an agent for the treatment of a tumor is provided, the method comprising (a) contacting a PRO with a test agent, wherein the PRO comprises an amino acid variation, (b) assessing the activity of the PRO in the presence of the test agent with the activity of the PRO in the absence of the test agent, wherein an increase or decrease in the activity of the PRO in the presence of the test agent indicates that the test agent is an agent for the treatment of a tumor. In one embodiment, the amino acid variation is in a PRO functional domain selected from Figure 1 or 3. In another embodiment, the amino acid variation is at an amino acid position selected from Figure 1 or 3. in another embodiment, the amino acid variation is an amino acid change selected from Figure 1 or 3. In another embodiment, the PRO is a kinase (such as a tyrosine kinase, e.g., an RTK), and a decrease in the activity of the kinase in the presence of the test agent indicates that the test agent is an agent for the treatment of a tumor. In another embodiment, the PRO is a tumor suppressor, and an increase in the activity of the tumor suppressor in the presence of the test agent indicates that the test agent is an agent for the treatment of a tumor.

In another aspect, a method of inhibiting the proliferation of a tumor cell is provided, wherein the tumor cell comprises a PRO having an activating variation, the method comprising exposing the tumor cell to an antagonist of PRO. In one embodiment, the PRO is a kinase, e.g., a receptor tyrosine kinase.

In another aspect, a method of inhibiting the proliferation of a tumor cell is provided, wherein the tumor cell comprises a variation in a PRO that decreases the activity of PRO, the method comprising exposing the tumor cell to an agonist of PRO. In one embodiment, an agonist comprises a PRO polynucleotide or PRO itself. In another embodiments, the PRO is a tumor suppressor.

In another aspect, a method of treating a tumor comprising a variation in a PRO is provided, the method comprising administering to a mammal having the tumor an effective amount of a pharmaceutical formulation comprising an antagonist or agonist of PRO. In one embodiment, the variation is a nucleotide variation. In one such embodiment, the nucleotide variation is at a nucleotide position selected from Figures 1-3. In one such embodiment, the nucleotide variation is a nucleotide change selected from Figures 1-3. In another embodiment, the variation is an amino acid variation. In one such embodiment, the amino acid variation is in a PRO functional domain selected from Figure 1 or 3. In another such embodiment, the amino acid variation is at an amino acid position selected from Figure 1 or 3. In another such embodiment, the amino acid variation is an amino acid change selected from Figure 1 or 3. In another embodiment, the PRO is a kinase (such as a tyrosine kinase, e.g., an RTK). In another embodiment, the PRO is a tumor suppressor.

A tumor, according to any of the above methods, may be a tumor selected from a lung tumor (particularly non-small cell lung carcinomas), breast tumor, colon tumor, kidney tumor, liver tumor, bladder tumor, ovarian tumor, stomach tumor, skin tumor (including melanoma and non-melanoma skin cancers), and lymphoma. In one embodiment, a tumor is a lung tumor, e.g., a non-small cell lung carcinoma.

A nucleotide variation, according to any of the above methods, may be a somatic mutation or a germline polymorphism.

### D. General Techniques

Nucleic acid, according to any of the above methods, may be genomic DNA; RNA transcribed from genomic DNA; or cDNA generated from RNA. Nucleic acid may be derived from a vertebrate, e.g., a mammal. A nucleic acid is said to be "derived from" a particular source if it is obtained directly from that source or if it is a copy of a nucleic acid found in that source.

Nucleic acid includes copies of the nucleic acid, e.g., copies that result from amplification. Amplification may be desirable in certain instances, e.g., in order to obtain a desired amount of material for detecting variations. For example, a PRO polynucleotide or portion thereof may be amplified from nucleic acid material. The amplicons may then be subjected to a variation detection method, such as those described below, to determine whether a variation is present in the amplicon.

Variations may be detected by certain methods known to those skilled in the art. Such methods include, but are not limited to, DNA sequencing; primer extension assays, including allele-specific nucleotide incorporation assays and allele-specific primer extension assays (e.g., allele-specific PCR, allele-specific ligation chain reaction (LCR), and gap-LCR); allele-specific oligonucleotide hybridization assays (e.g., oligonucleotide ligation assays); cleavage protection assays in which protection from cleavage agents is used to detect mismatched bases in nucleic acid duplexes; analysis of MutS protein binding; electrophoretic analysis comparing the mobility of variant and wild type nucleic acid molecules; denaturing-gradient gel electrophoresis (DGGE, as in, e.g., Myers et al. (1985) Nature 313:495); analysis of RNase cleavage at mismatched base pairs; analysis of chemical or enzymatic cleavage of heteroduplex DNA; mass spectrometry (e.g., MALDI-TOF); genetic bit analysis (GBA); 5' nuclease assays (e.g., TaqMan^{®}); and assays employing molecular beacons. Certain of these methods are discussed in further detail below.

Detection of variations in target nucleic acids may be accomplished by molecular cloning and sequencing of the target nucleic acids using techniques well known in the art. Alternatively, amplification techniques such as the polymerase chain reaction (PCR) can be used to amplify target nucleic acid sequences directly from a genomic DNA preparation from tumor tissue. The nucleic acid sequence of the amplified sequences can then be determined and variations identified therefrom. Amplification techniques are well known in the art, e.g., polymerase chain reaction is described in Saiki et al., Science 239:487, 1988; U.S. Pat. Nos. 4,683,203 and 4,683,195.

The ligase chain reaction, which is known in the art, can also be used to amplify target nucleic acid sequences. See, e.g., Wu et al., Genomics 4:560-569 (1989). In addition, a technique known as allele-specific PCR can also be used to detect variations (e.g., substitutions). See, e.g., Ruano and Kidd (1989) Nucleic Acids Research 17:8392; McClay et al. (2002) Analytical Biochem. 301:200-206. In certain embodiments of this technique, an allele-specific primer is used wherein the 3' terminal nucleotide of the primer is complementary to (i.e., capable of specifically base-pairing with) a particular variation in the target nucleic acid. If the particular variation is not present, an amplification product is not observed. Amplification Refractory Mutation System (ARMS) can also be used to detect variations (e.g., substitutions). ARMS is described, e.g., in European Patent Application Publication No. 0332435, and in Newton et al., Nucleic Acids Research, 17:7, 1989.

Other methods useful for detecting variations (e.g., substitutions) include, but are not limited to, (1) allele-specific nucleotide incorporation assays, such as single base extension assays (see, e.g., Chen et al. (2000) Genome Res. 10:549-557; Fan et al. (2000) Genome Res. 10:853-860; Pastinen et al. (1997) Genome Res. 7:606-614; and Ye et al. (2001) Hum. Mut. 17:305-316); (2) allele-specific primer extension assays (see, e.g., Ye et al. (2001) Hum. Mut. 17:305-316; and Shen et al. Genetic Engineering News, vol. 23, Mar. 15, 2003), including allele-specific PCR; (3) 5'nuclease assays (see, e.g., De La Vega et al. (2002) BioTechniques 32:S48-S54 (describing the TaqMan® assay); Ranade et al. (2001) Genome Res. 11:1262-1268; and Shi (2001) Clin. Chem. 47:164-172); (4) assays employing molecular beacons (see, e.g., Tyagi et al. (1998) Nature Biotech. 16:49-53; and Mhlanga et al. (2001) Methods 25:463-71); and (5) oligonucleotide ligation assays (see, e.g., Grossman et al. (1994) Nuc. Acids Res. 22:4527-4534; patent application Publication No. US 2003/0119004 Al; PCT International Publication No. WO 01/92579 A2; and U.S. Pat. No. 6,027,889).

Variations may also be detected by mismatch detection methods. Mismatches are hybridized nucleic acid duplexes which are not 100% complementary. The lack of total complementarity may be due to deletions, insertions, inversions, or substitutions. One example of a mismatch detection method is the Mismatch Repair Detection (MRD) assay described, e.g., in Faham et al., Proc. Natl Acad. Sci. USA 102:14717-14722 (2005) and Faham et al., Hum. Mol. Genet. 10:1657-1664 (2001). Another example of a mismatch cleavage technique is the RNase protection method, which is described in detail in Winter et al., Proc. Natl. Acad. Sci. USA, 82:7575, 1985, and Myers et al., Science 230:1242, 1985. For example, a method of the invention may involve the use of a labeled riboprobe which is complementary to the human wild-type target nucleic acid. The riboprobe and target nucleic acid derived from the tissue sample are annealed (hybridized) together and subsequently digested with the enzyme RNase A which is able to detect some mismatches in a duplex RNA structure. If a mismatch is detected by RNase A, it cleaves at the site of the mismatch. Thus, when the annealed RNA preparation is separated on an electrophoretic gel matrix, if a mismatch has been detected and cleaved by RNase A, an RNA product will be seen which is smaller than the full-length duplex RNA for the riboprobe and the mRNA or DNA. The riboprobe need not be the full length of the target nucleic acid, but can a portion of the target nucleic acid, provided it encompasses the position suspected of having a variation.

In a similar manner, DNA probes can be used to detect mismatches, for example through enzymatic or chemical cleavage. See, e.g., Cotton et al., Proc. Natl. Acad. Sci. USA, 85:4397, 1988: and Shenk et al., Proc. Natl. Acad. Sci. USA, 72:989, 1975. Alternatively, mismatches can be detected by shifts in the electrophoretic mobility of mismatched duplexes relative to matched duplexes. See, e.g., Cariello, Human Genetics, 42:726, 1988. With either riboprobes or DNA probes, the target nucleic acid suspected of comprising a variation may be amplified before hybridization. Changes in target nucleic acid can also be detected using Southern hybridization, especially if the changes are gross rearrangements, such as deletions and insertions.

Restriction fragment length, polymorphism (RFLP) probes for the target nucleic acid or surrounding marker genes can be used to detect variations, e.g., insertions or deletions. Insertions and deletions can also be detected by cloning, sequencing and amplification of a target nucleic acid. Single stranded conformation polymorphism (SSCP) analysis can also be used to detect base change variants of an allele. See, e.g. Orita et al., Proc. Natl. Acad. Sci. USA 86:2766-2770, 1989, and Genomics, 5:874-879, 1989.

The invention also provides a variety of compositions suitable for use in performing methods of the invention. For example, the invention provides arrays that can be used in such methods. In one embodiment, an array of the invention comprises individual or collections of nucleic acid molecules useful for detecting variations of the invention. For instance, an array of the invention may comprise a series of discretely placed individual allele-specific oligonucleotides or sets of allele-specific oligonucleotides. Several techniques are well-known in the art for attaching nucleic acids to a solid substrate such as a glass slide. One method is to incorporate modified bases or analogs that contain a reactive moiety that is capable of attachment to a solid substrate, such as an amine group, a derivative of an amine group, or another group with a positive charge, into nucleic acid molecules that are synthesized. The synthesized product is then contacted with a solid substrate, such as a glass slide coated with an aldehyde or other reactive group. The aldehyde or other reactive group will form a covalent link with the reactive moiety on the amplified product, which will become covalently attached to the glass slide. Other methods, such as those using amino propryl silican surface chemistry are also known in the art.

A biological sample, according to any of the above methods, may be obtained using certain methods known to those skilled in the art. Biological samples may be obtained from vertebrate animals, and in particular, mammals. Tissue biopsy is often used to obtain a representative piece of tumor tissue. Alternatively, tumor cells can be obtained indirectly in the form of tissues or fluids that are known or thought to contain the tumor cells of interest. For instance, samples of lung cancer lesions may be obtained by resection, bronchoscopy, fine needle aspiration, bronchial brushings, or from sputum, pleural fluid or blood. Variations in target nucleic acids (or encoded polypeptides) may be detected from a tumor sample or from other body samples such as urine, sputum or serum. (Cancer cells are sloughed off from tumors and appear in such body samples.) By screening such body samples, a simple early diagnosis can be achieved for diseases such as cancer. In addition, the progress of therapy can be monitored more easily by testing such body samples for variations in target nucleic acids (or encoded polypeptides). Additionally, methods for enriching a tissue preparation for tumor cells are known in the art. For example, the tissue may be isolated from paraffin or cryostat sections. Cancer cells may also be separated from normal cells by flow cytometry or laser capture microdissection.

### 111. EXAMPLES

Twenty-five different human non-small cell lung carcinoma (NSCLC) specimens were obtained from commercial sources. The specimens were subjected to pathology review to ensure sufficient tumor content (i.e., on average >75% tumor cells). One hundred genes were analyzed for variations using Mismatch Repair Detection (MRD), and a subset of those genes was analyzed independently using Sanger sequencing, as described below. Some of the 100 selected genes were known or suspected to play a role in cancer, and some had not been previously implicated in tumorigenesis or tumor progression.

### A. Identification of Variations by MRD

Mismatch Repair Detection (MRD) was used to identify nucleotide variations in the selected genes. The details of the MRD method are described in Faham et al., Proc. Natl Acad. Sci. USA 102:14717-14722 (2005) and Faham et al., Hum. Mol. Genet. 10:1657-1664 (2001). Briefly, PCR primers were designed for each exon of interest. At the 5' end of each forward PCR primer a 25 nucleotide sequence was added. Twenty one of the nucleotides constitute a tag sequence specific to each amplicon, and the remaining four base pairs form a *Hae*III restriction enzyme digestion site, which was ultimately used to separate the tag from the genomic sequence before the array detection step.

PCR amplification was carried out with each primer pair using the homozygous reference genome (hydatiform mole) as a template. All amplicons were then pooled and cloned into a plasmid to make reference sequences. For sequences carrying a common SNP, an attempt was made to create two standards (with two distinct tag sequences) corresponding to each of the two alleles. The same PCR primers were utilized for multiplex PCR using *Pfu* polymerase (Stratagene, La Jolla, CA) from each pair of tumor and matched normal DNA. The PCR products from tumor and matched normal samples were hybridized to the set of reference sequences (as well as a control vector lacking genomic sequence), thereby resulting in formation of heteroduplex DNA molecules with one strand of the genomic region of interest from the reference genome and the other from the test sample.

The heteroduplex molecules were then transformed en masse into the Mutation Sorter *E. coli* strain. The Mutation Sorter strain is engineered such that bacteria that repair a mismatch between the reference and test DNA have a different antibiotic resistance phenotype from those that do not repair. (See Faham et al., (2001) *supra*.) After transformation, the bacterial culture was grown in two different media to select for variant and non-variant fragments. Miniprep DNA obtained from the two selected bacterial cultures was PCR amplified using primers complementary to the vector sequence. The PCR products were Hae III digested to separate the tag sequence from the rest of the genomic sequence and the tags from the two pools were mixed and hybridized onto an array in a two-color assay. Fragments that were identical to the reference sample generated signal primarily in the non-variant pool, while those that were different from the reference sample had signal primarily in the variant pool, and heterozygous fragments generated signal in both pools.

Nucleotide variations identified by MRD were determined to be somatic mutations by genotyping tumor and matched normal samples using an alternative mass spectrometry-based approach, as described in Nelson et al., Genome Res. 14:1664-1668. Nucleotide variations that were confirmed to be somatic were compared with somatic mutations in the Catalogue of Somatic Mutations in Cancer (COSMIC) database (see Bamford et al. (2004) British Journal of Cancer 91:355-358). Twelve somatic nucleotide variations were found in the COSMIC database and are not reported herein.

Novel nucleotide variations identified by MRD are shown in Figure 1. Figure 1 lists somatic nucleotide variations that are nonsynonymous or nonsense mutations. Figure 2 lists somatic nucleotide variations that fall within consensus splice site regions. Figures 1 and 2 are described in further detail in the above Detailed Description of Embodiments.

It is noted that MRD identified three somatic mutations in the WD40 domain of the FBXW7 gene (see Variation ID Nos. 10007, 10008, and 10009). FBXW7 is a known tumor suppressor shown to lead to chromosome instability in colorectal tumors when lost. *See* Rajagopalan, H. et al. (2004) Nature 428:77-81. The WD40 domain, which is important in the interaction between FBXW7 and cyclin E (*see* Orlicky, S. et al. (2003) Cell 112:243-256), has been found to be mutated in colorectal cancer. *See* Rajagopalan, *supra.* FBXW7 was sequenced in an additional 61 NSCLC tumors, but no further mutations were identified. To the inventors' knowledge, mutations in FBXW7 have not been previously reported in NSCLC.

### B. Identification of Variations by Sanger Sequencing

Sanger sequencing was also used to identify nucleotide variations in 52 of the 100 originally selected genes. Specifically, PCR was used to amplify exons in those genes from tumor genomic DNA. Amplicons were sequenced using standard Sanger sequencing, and the sequences were compared with the corresponding wild-type sequences to identify variations. The variations were compared with single nucleotide polymorphisms in the NCBI "dbSNP" database (see Sherry et al. (1999) Genome Res. 9:677-679) and with somatic mutations in the Catalogue of Somatic Mutations in Cancer (COSMIC) database (see Bamford et al. (2004) British Journal of Cancer 91:355-358) to identify the variations that were not present in those databases and were thus determined to be novel. The variations were validated (i.e., confirmed) by reamplifying and resequencing the exons from the original tumor samples. Genomic DNA of patient matched normal samples was used to determine whether the variations were somatic mutations or germline polymorphisms. The results of the foregoing analysis, including a listing of the nucleotide variations thus identified, are shown in Figure 3. Figure 3 is described in further detail in the above Detailed Description of Embodiments. Some of the variations identified in Figure 3 were also identified by MRD but were omitted from Figure 1 (listing MRD-identified mutations) to avoid redundancy.

Although the foregoing invention has been described in some detail by way of illustration and example for purposes of clarity of understanding, the descriptions and examples should not be construed as limiting the scope of the invention.

## Claims

1. A method of classifying a non-small cell lung carcinoma tumor in a mammal, the method comprising detecting the presence of an amino acid variation in the WD40 domain of FBXW7, or a nucleotide variation in a region of an FBXW7 polynucleotide encoding the WD40 domain, in a biological sample derived from the mammal, wherein the biological sample comprises non-small cell lung carcinoma cells.

2. The method of claim 1, wherein the amino acid is detected.

3. The method of claim 2, wherein the amino acid variation is at an amino acid position selected from G343, R399, and Y429.

4. The method of claim 1, wherein the nucleotide variation is detected.

5. The method of claim 4, wherein the nucleotide variation is at a nucleotide position selected from C153607116, C153604971, and T 153604881 of chromosome 4, wherein said nucleotide position corresponds to position G113, G1292 or A1382 of SEQ ID NO:5, respectively.

6. The method of claim 5, wherein the nucleotide variation is a nucleotide change selected from C153607116T, C153604971T, and T153604881A, wherein said nucleotide change corresponds to G1123A, G1292A or A1382T of SEQ ID NO:5, respectively.

7. A method for predicting whether a non-small cell lung carcinoma will respond to a therapeutic agent that targets FBXW7 or FBXW77 polynucleotide, the method comprising determining whether the non-small cell lung carcinoma comprises an amino acid variation in the WD40 domain of FBXW7, or a nucleotide variation in a region of an FBXW7 polynucleotide encoding the WD40 domain, wherein the presence of an amino acid variation or a nucleotide variation indicates that the non-small cell lung carcinoma will respond to the therapeutic agent.

8. The method of claim 7, wherein the amino acid variation in FBXW7 is detected,

9. The method of claim 8, wherein the amino acid variation is at an amino acid position selected from G343, R399, and Y429.

10. The method of claim 7, wherein the nucleotide variation is detected,

11. The method of claim 10, wherein the nucleotide variation is at a nucleotide position selected from C153607116, C153604971, and T153604881 of chromosome 4, wherein said nucleotide position corresponds to position G113, G1292 or A1382 of SEQ ID NO:5, respectively.

12. The method of claim 11, wherein the nucleotide variation is a nucleotide change selected from C153607116T, C153604971T, and T153604881A, wherein said nucleotide change corresponds to G1123A, G1292A or A1382T of SEQ ID NO:5, respectively.

## Patentansprüche

1. Verfahren zur Klassifikation eines nicht-kleinzelligen Lungenkarzinomtumors bei einem Säugetier, wobei das Verfahren das Nachweisen der Gegenwart einer Aminosäurevariation in der WD40-Domäne von FBXW7 oder einer Nucleotidvariation in einer Region eines FBXW7-Polynucleotids, das für die WD40-Domäne kodiert, in einer dem Säugetier entnommenen biologischen Probe umfasst, worin die biologische Probe Zellen des nicht-kleinzelligen Lungenkarzinoms umfasst.

2. Verfahren nach Anspruch 1, worin die Aminosäure nachgewiesen wird.

3. Verfahren nach Anspruch 2, worin die Aminosäurevariation an einer aus G343, R399 und Y429 ausgewählten Aminosäureposition liegt.

4. Verfahren nach Anspruch 1, worin die Nucleotidvariation nachgewiesen wird.

5. Verfahren nach Anspruch 4, worin die Nucleotidvariation an einer aus C153607116, C153604971 und T153604881 von Chromosom 4 ausgewählten Nucleotidposition liegt, worin die Nucleotidposition der Position G1123, G1292 bzw. A1382 aus Seq.-ID Nr. 5 entspricht.

6. Verfahren nach Anspruch 5, worin die Nucleotidvariation eine aus C153607116T, C153604971T und T153604881A ausgewählte Nucleotidänderung ist, worin die Nucleotidänderung G1123A, G1292A bzw. A1382T aus Seq.-ID Nr. 5 entspricht.

7. Verfahren zur Vorhersage, ob ein nicht-kleinzelliges Lungenkarzinom auf ein therapeutisches Mittel ansprechen wird, das auf FBXW7 oder FBXW7-Polynucleotid gerichtet ist, wobei das Verfahren das Bestimmen umfasst, ob das nicht-kleinzellige Lungenkarzinom eine Aminosäurevariation in der WD40-Domäne von FBXW7 oder eine Nucleotidvariation in einer Region eines FBXW7-Polynucleotids, das für die WD40-Domäne kodiert, umfasst, worin die Gegenwart einer Aminosäurevariation oder einer Nucleotidvariation ein Indikator dafür ist, dass das nicht-kleinzellige Lungenkarzinom auf das therapeutische Mittel ansprechen wird.

8. Verfahren nach Anspruch 7, worin die Aminosäurevariation in FBXW7 nachgewiesen wird.

9. Verfahren nach Anspruch 8, worin die Aminosäurevariation an einer aus G343, R399 und Y429 ausgewählten Aminosäureposition liegt.

10. Verfahren nach Anspruch 7, worin die Nucleotidvariation nachgewiesen wird.

11. Verfahren nach Anspruch 10, worin die Nucleotidvariation an einer aus C153607116, C153604971 und T153604881 von Chromosom 4 ausgewählten Nucleotidposition liegt, worin die Nucleotidposition der Position G1123, G1292 bzw. A1382 aus Seq.-ID Nr. 5 entspricht.

12. Verfahren nach Anspruch 11, worin die Nucleotidvariation eine aus C153607116T, C153604971T und T153604881A ausgewählte Nucleotidänderung ist, worin die Nucleotidänderung G1123A, G1292A bzw. A1382T aus Seq.-ID Nr. 5 entspricht.

## Revendications

1. Procédé de classification d'une tumeur du type carcinome pulmonaire non à petites cellules, le procédé comprenant la détection de la présence d'une variation d'aminoacide dans le domaine WD40 de FBXW7, ou d'une variation de nucléotide dans une région d'un polynucléotide FBXW7 codant pour le domaine WD40, dans un échantillon biologique provenant du mammifère, dans lequel l'échantillon biologique comprend des cellules de carcinome pulmonaire non à petites cellules.

2. Procédé suivant la revendication 1, dans lequel l'aminoacide est détecté.

3. Procédé suivant la revendication 2, dans lequel la variation d'aminoacide est à une position d'aminoacide choisie entre G343, R399 et Y429.

4. Procédé suivant la revendication 1, dans lequel la variation de nucléotide est détectée.

5. Procédé suivant la revendication 4, dans lequel la variation de nucléotide est à une position de nucléotide choisie entre C153607116, C153604971 et T153604881 du chromosome 4, ladite position de nucléotide correspondant à la position G1123, G1292, ou A1382 de la SEQ ID N° 5, respectivement.

6. Procédé suivant la revendication 5, dans lequel la variation de nucléotide est un remplacement de nucléotide choisi entre C153607116T, C153604971T et T153604881A, ledit remplacement de nucléotide correspondant à G1123A, G1292A, ou A1382T de la SEQ ID N° 5, respectivement.

7. Procédé pour prévoir si un carcinome pulmonaire non à petites cellules répondra à un agent thérapeutique qui a pour cible FBXW7 ou un polynucléotide FBXW7, le procédé comprenant l'étape consistant à déterminer si le carcinome pulmonaire non à petites cellules comprend une variation d'aminoacide dans le domaine WD40 de FBXW7, ou une variation de nucléotide dans une région d'un polynucléotide FBXW7 codant pour le domaine WD40, dans lequel la présence d'une variation d'aminoacide ou d'une variation de nucléotide indique que le carcinome pulmonaire non à petites cellules répondra à l'agent thérapeutique.

8. Procédé suivant la revendication 7, dans lequel la variation d'aminoacide dans FBXW7 est détectée.

9. Procédé suivant la revendication 8, dans lequel la variation d'aminoacide est à une position d'aminoacide choisie entre G343, R399 et Y429.

10. Procédé suivant la revendication 7, dans lequel la variation de nucléotide est détectée.

11. Procédé suivant la revendication 10, dans lequel la variation de nucléotide est à une position de nucléotide choisie entre C153607116, C153604971 et T153604881 du chromosome 4, ladite position de nucléotide correspondant à la position G1123, G1292, ou A1382 de la SEQ ID N° 5, respectivement.

12. Procédé suivant la revendication 11, dans lequel la variation de nucléotide est un remplacement de nucléotide choisi entre C153607116T, C153604971T et T153604881A, ledit remplacement de nucléotide correspondant à G1123A, G1292A, ou A1382T de la SEQ ID N° 5, respectivement.


### Sequence Listing

<110> Genent ech, Inc.
   Kan, Zhengyan
   Pet er s, Brock
   Seshagiri, Sorrasekar
<120> GENETIC VARIATIONS ASSOCIATED WITH TUMORS
<130> P4007R1
<141> 2007-11-15
<150> US 60/ 866, 103
   <151> 2006-11-16
<150> US 60/ 948, 818
   <151> 2007-07-10
<160> 79
<210> 1
   <211> 5766
   <212> DNA
   <213> Homo sapiens
<400> 1
   ggccttcccc ctgcgaggat cgccattggc ccgggttggc tttggaaagc 50
   ggcggtggct ttgggccggg ctcggcctcg ggaacgccag gggcccctgg 100
   gtgcggacgg gcgcggccag gagggggtta aggcgcaggc ggcggcgggg 150
   cgggggcggg cctggcgggc gccctctccg ggccctttgt taacaggcgc 200
   gtcccggcca ggcggagacg cggccgcggc catgggcggg cgcgggcgcg 250
   cggggcggcg gtgagggcgg ctggcggggc cgggggcgcc gggggggcgc 300
   gcgggccgag ccgggcctga gccgggcccg cggaccgagc tgggagaggg 350
   gttccggccc ccgacgtgct ggcgcgggaa aatgttggag atctgcctga 400
   agctggtggg ctgcaaatcc aagaaggggc tgtcctcgtc ctccagctgt 450
   tatctggaag aagcccttca gcggccagta gcatctgact ttgagcctca 500
   gggtctgagt gaagccgctc gttggaactc caaggaaaac cttctcgctg 550
   gacccagtga aaatgacccc aaccttttcg ttgcactgta tgattttgtg 600
   gccagtggag ataacactct aagcataact aaaggtgaaa agctccgggt 650
   cttaggctat aatcacaatg gggaatggtg tgaagcccaa accaaaaatg 700
   gccaaggctg ggtcccaagc aactacatca cgccagtcaa cagtctggag 750
   aaacactcct ggtaccatgg gcctgtgtcc cgcaatgccg ctgagtatct 800
   gctgagcagc gggatcaatg gcagctttct ggtgcgtgag agtgagagca 850
   gtcctggcca gaggtccatc tcgctgagat acgaagggag ggtgtaccat 900
   tacaggatca acactgcttc tgatggcaag ctctacgtct cctccgagag 950
   ccgcttcaac accctggccg agttggttca tcatcattca acggtggccg 1000
   acgggctcat caccacgctc cattatccag ccccaaagcg caacaagccc 1050
   actgtctatg gtgtgtcccc caactacgac aagtgggaga tggaacgcac 1100
   ggacatcacc atgaagcaca agctgggcgg gggccagtac ggggaggtgt 1150
   acgagggcgt gtggaagaaa tacagcctga cggtggccgt gaagaccttg 1200
   aaggaggaca ccatggaggt ggaagagttc ttgaaagaag ctgcagtcat 1250
   gaaagagatc aaacacccta acctggtgca gctccttggg gtctgcaccc 1300
   gggagccccc gttctatatc atcactgagt tcatgaccta cgggaacctc 1350
   ctggactacc tgagggagtg caaccggcag gaggtgaacg ccgtggtgct 1400
   gctgtacatg gccactcaga tctcgtcagc catggagtac ctggagaaga 1450
   aaaacttcat ccacagagat cttgctgccc gaaactgcct ggtaggggag 1500
   aaccacttgg tgaaggtagc tgattttggc ctgagcaggt tgatgacagg 1550
   ggacacctac acagcccatg ctggagccaa gttccccatc aaatggactg 1600
   cacccgagag cctggcctac aacaagttct ccatcaagtc cgacgtctgg 1650
   gcatttggag tattgctttg ggaaattgct acctatggca tgtcccctta 1700
   cccgggaatt gacctgtccc aggtgtatga gctgctagag aaggactacc 1750
   gcatggagcg cccagaaggc tgcccagaga aggtctatga actcatgcga 1800
   gcatgttggc agtggaatcc ctctgaccgg ccctcctttg ctgaaatcca 1850
   ccaagccttt gaaacaatgt tccaggaatc cagtatctca gacgaagtgg 1900
   aaaaggagct ggggaaacaa ggcgtccgtg gggctgtgag taccttgctg 1950
   caggccccag agctgcccac caagacgagg acctccagga gagctgcaga 2000
   gcacagagac accactgacg tgcctgagat gcctcactcc aagggccagg 2050
   gagagagcga tcctctggac catgagcctg ccgtgtctcc attgctccct 2100
   cgaaaagagc gaggtccccc ggagggcggc ctgaatgaag atgagcgcct 2150
   tctccccaaa gacaaaaaga ccaacttgtt cagcgccttg atcaagaaga 2200
   agaagaagac agccccaacc cctcccaaac gcagcagctc cttccgggag 2250
   atggacggcc agccggagcg cagaggggcc ggcgaggaag agggccgaga 2300
   catcagcaac ggggcactgg ctttcacccc cttggacaca gctgacccag 2350
   ccaagtcccc aaagcccagc aatggggctg gggtccccaa tggagccctc 2400
   cgggagtccg gggctcaggc cttccggtct ccccacctgt ggaagaagtc 2450
   cagcacgctg accagcagcc gcctagccac cggcgaggag gagggcggtg 2500
   gcagctccag caagcgcttc ctgcgctctt gctccgcctc ctgcgttccc 2550
   catggggcca aggacacgga gtggaggtca gtcacgctgc ctcgggactt 2600
   gcagtccacg ggaagacagt ttgactcgtc cacatttgga gggcacaaaa 2650
   gtgagaagcc ggctctgcct cggaagaggg caggggagaa caggtctgac 2700
   caggtgaccc gaggcacagt aacgcctccc cccaggctgg tgaaaaagaa 2750
   tgaggaagct gctgatgagg tcttcaaaga catcatggag tccagcccgg 2800
   gct ccagccc gcccaacct g act ccaaaac ccct ccggcg gcaggt cacc 2850
   gtggcccctg cctcgggcct cccccacaag gaagaagctg gaaagggcag 2900
   tgccttaggg acccctgctg cagctgagcc agtgaccccc accagcaaag 2950
   caggctcagg tgcaccaggg ggcaccagca agggccccgc cgaggagtcc 3000
   agagtgagga ggcacaagca ctcctctgag tcgccaggga gggacaaggg 3050
   gaaattgtcc aggctcaaac ctgccccgcc gcccccacca gcagcctctg 3100
   cagggaaggc tggaggaaag ccctcgcaga gcccgagcca ggaggcggcc 3150
   ggggaggcag tcctgggcgc aaagacaaaa gccacgagtc tggttgatgc 3200
   tgtgaacagt gacgctgcca agcccagcca gccgggagag ggcctcaaaa 3250
   agcccgtgct cccggccact ccaaagccac agtccgccaa gccgtcgggg 3300
   acccccatca gcccagcccc cgttccctcc acgttgccat cagcatcctc 3350
   ggccctggca ggggaccagc cgtcttccac cgccttcatc cctctcatat 3400
   caacccgagt gtctcttcgg aaaacccgcc agcctccaga gcggatcgcc 3450
   agcggcgcca tcaccaaggg cgtggtcctg gacagcaccg aggcgctgtg 3500
   cctcgccatc tctaggaact ccgagcagat ggccagccac agcgcagtgc 3550
   t ggaggccgg caaaaacct c t acacgt t ct gcgt gagct a t gt ggat t cc 3600
   atccagcaaa tgaggaacaa gtttgccttc cgagaggcca tcaacaaact 3650
   ggagaataat ctccgggagc ttcagatctg cccggcgaca gcaggcagtg 3700
   gt ccagcggc cact caggac t t cagcaagc t cct cagt t c ggt gaaggaa 3750
   at cagt gaca t agt gcagag gt agcagcag t caggggt ca ggt gt caggc 3800
   ccgt cggagc t gcct gcagc acat gcgggc t cgcccat ac ccgt gacagt 3850
   ggct gacaag ggact agt ga gt cagcacct t ggcccagga gct ct gcgcc 3900
   aggcagagct gagggccctg tggagtccag ctctactacc tacgtttgca 3950
   ccgcct gccc t cccgcacct t cct cct ccc cgct ccgt ct ct gt cct cga 4000
   at t t t at ct g t ggagt t cct gct ccgt gga ct gcagt cgg cat gccagga 4050
   cccgccagcc ccgctcccac ctagtgcccc agactgagct ctccaggcca 4100
   ggt gggaacg gct gat gt gg act gt ct t t t t cat t t t t t t ct ct ct ggag 4150
   cccct cct cc cccggct ggg cct cct t ct t ccact t ct cc aagaat ggaa 4200
   gcct gaact g aggcct t gt g t gt caggccc t ct gcct gca ct ccct ggcc 4250
   ttgcccgtcg tgtgctgaag acatgtttca agaaccgcat ttcgggaagg 4300
   gcat gcacgg gcat gcacac ggct ggt cac t ct gccct ct gct gct gccc 4350
   ggggt ggggt gcact cgcca t t t cct cacg t gcaggacag ct ct t gat t t 4400
   gggtggaaaa cagggtgcta aagccaacca gcctttgggt cctgggcagg 4450
   t gggagct ga aaaggat cga ggcat ggggc at gt cct t t c cat ct gt cca 4500
   catccccaga gcccagctct tgctctcttg tgacgtgcac tgtgaatcct 4550
   ggcaagaaag cttgagtctc aagggtggca ggtcactgtc actgccgaca 4600
   tccctccccc agcagaatgg aggcagggga caagggaggc agtggctagt 4650
   ggggtgaaca gctggtgcca aatagcccca gactgggccc aggcaggtct 4700
   gcaagggccc agagtgaacc gtcctttcac acatctgggt gccctgaaag 4750
   ggcccttccc ctcccccact cctctaagac aaagtagatt cttacaaggc 4800
   cctttccttt ggaacaagac agccttcact tttctgagtt cttgaagcat 4850
   ttcaaagccc tgcctctgtg tagccgccct gagagagaat agagctgcca 4900
   ctgggcacct gcgcacaggt gggaggaaag ggcctggcca gtcctggtcc 4950
   tggctgcact cttgaactgg gcgaatgtct tatttaatta ccgtgagtga 5000
   catagcctca tgttctgtgg gggtcatcag ggagggttag gaaaaccaca 5050
   aacggagccc ctgaaagcct cacgtatttc acagagcacg cctgccatct 5100
   tctccccgag gctgccccag gccggagccc agatacgggg gctgtgactc 5150
   tgggcaggga cccggggtct cctggacctt gacagagcag ctaactccga 5200
   gagcagtggg caggtggccg cccctgaggc ttcacgccgg gagaagccac 5250
   cttcccaccc cttcataccg cctcgtgcca gcagcctcgc acaggcccta 5300
   gctttacgct catcacctaa acttgtactt tatttttctg atagaaatgg 5350
   tttcctctgg atcgttttat gcggttctta cagcacatca cctctttgcc 5400
   cccgacggct gtgacgcagc cggagggagg cactagtcac cgacagcggc 5450
   cttgaagaca gagcaaagcg cccacccagg tcccccgact gcctgtctcc 5500
   atgaggtact ggtcccttcc ttttgttaac gtgatgtgcc actatatttt 5550
   acacgtatct cttggtatgc atcttttata gacgctcttt tctaagtggc 5600
   gtgtgcatag cgtcctgccc tgccccctcg ggggcctgtg gtggctcccc 5650
   ctctgcttct cggggtccag tgcattttgt ttctgtatat gattctctgt 5700
   ggtttttttt gaatccaaat ctgtcctctg tagtattttt taaataaatc 5750
   agtgtttaca ttagaa 5766
<210> 2
   <211> 10701
   <212> DNA
   <213> Homo sapiens
<400> 2
   tggagacaga atggaggtgc tgccggactc ggaaatgggg tccaagggta 50
   gccaaggatg gctgcagctt catatgatca gttgttaaag caagttgagg 100
   cactgaagat ggagaactca aatcttcgac aagagctaga agataattcc 150
   aatcatctta caaaactgga aactgaggca tctaatatga aggaagtact 200
   taaacaacta caaggaagta ttgaagatga agctatggct tcttctggac 250
   agattgattt attagagcgt cttaaagagc ttaacttaga tagcagtaat 300
   ttccctggag taaaactgcg gtcaaaaatg tccctccgtt cttatggaag 350
   ccgggaagga tctgtatcaa gccgttctgg agagtgcagt cctgttccta 400
   tgggttcatt tccaagaaga gggtttgtaa atggaagcag agaaagtact 450
   ggatatttag aagaacttga gaaagagagg tcattgcttc ttgctgatct 500
   tgacaaagaa gaaaaggaaa aagactggta ttacgctcaa cttcagaatc 550
   tcactaaaag aatagatagt cttcctttaa ctgaaaattt ttccttacaa 600
   acagatatga ccagaaggca attggaatat gaagcaaggc aaatcagagt 650
   tgcgatggaa gaacaactag gtacctgcca ggatatggaa aaacgagcac 700
   agcgaagaat agccagaatt cagcaaatcg aaaaggacat acttcgtata 750
   cgacagcttt tacagtccca agcaacagaa gcagagaggt catctcagaa 800
   caagcatgaa accggctcac atgatgctga gcggcagaat gaaggtcaag 850
   gagtgggaga aatcaacatg gcaacttctg gtaatggtca gggttcaact 900
   acacgaatgg accatgaaac agccagtgtt ttgagttcta gtagcacaca 950
   ctctgcacct cgaaggctga caagtcatct gggaaccaag gtggaaatgg 1000
   tgtattcatt gttgtcaatg cttggtactc atgataagga tgatatgtcg 1050
   cgaactttgc tagctatgtc tagctcccaa gacagctgta tatccatgcg 1100
   acagtctgga tgtcttcctc tcctcatcca gcttttacat ggcaatgaca 1150
   aagactctgt attgttggga aattcccggg gcagtaaaga ggctcgggcc 1200
   agggccagtg cagcactcca caacatcatt cactcacagc ctgatgacaa 1250
   gagaggcagg cgtgaaatcc gagtccttca tcttttggaa cagatacgcg 1300
   cttactgtga aacctgttgg gagtggcagg aagctcatga accaggcatg 1350
   gaccaggaca aaaatccaat gccagctcct gttgaacatc agatctgtcc 1400
   tgctgtgtgt gttctaatga aactttcatt tgatgaagag catagacatg 1450
   caatgaatga actaggggga ctacaggcca ttgcagaatt attgcaagtg 1500
   gactgtgaaa tgtatgggct tactaatgac cactacagta ttacactaag 1550
   acgatatgct ggaatggctt tgacaaactt gacttttgga gatgtagcca 1600
   acaaggctac gctatgctct atgaaaggct gcatgagagc acttgtggcc 1650
   caactaaaat ctgaaagtga agacttacag caggttattg cgagtgtttt 1700
   gaggaatttg tcttggcgag cagatgtaaa tagtaaaaag acgttgcgag 1750
   aagttggaag tgtgaaagca ttgatggaat gtgctttaga agttaaaaag 1800
   gaatcaaccc tcaaaagcgt attgagtgcc ttatggaatt tgtcagcaca 1850
   ttgcactgag aataaagctg atatatgtgc tgtagatggt gcacttgcat 1900
   ttttggttgg cactcttact taccggagcc agacaaacac tttagccatt 1950
   attgaaagtg gaggtgggat attacggaat gtgtccagct tgatagctac 2000
   aaatgaggac cacaggcaaa tcctaagaga gaacaactgt ctacaaactt 2050
   tattacaaca cttaaaatct catagtttga caatagtcag taatgcatgt 2100
   ggaactttgt ggaatctctc agcaagaaat cctaaagacc aggaagcatt 2150
   atgggacatg ggggcagtta gcatgctcaa gaacctcatt cattcaaagc 2200
   acaaaatgat tgctatggga agtgctgcag ctttaaggaa tctcatggca 2250
   aataggcctg cgaagtacaa ggatgccaat attatgtctc ctggctcaag 2300
   cttgccatct cttcatgtta ggaaacaaaa agccctagaa gcagaattag 2350
   atgctcagca cttatcagaa acttttgaca atatagacaa tttaagtccc 2400
   aaggcatctc atcgtagtaa gcagagacac aagcaaagtc tctatggtga 2450
   ttatgttttt gacaccaatc gacatgatga taataggtca gacaatttta 2500
   atactggcaa catgactgtc ctttcaccat atttgaatac tacagtgtta 2550
   cccagctcct cttcatcaag aggaagctta gatagttctc gttctgaaaa 2600
   agatagaagt ttggagagag aacgcggaat tggtctaggc aactaccatc 2650
   cagcaacaga aaatccagga acttcttcaa agcgaggttt gcagatctcc 2700
   accactgcag cccagattgc caaagtcatg gaagaagtgt cagccattca 2750
   tacctctcag gaagacagaa gttctgggtc taccactgaa ttacattgtg 2800
   tgacagatga gagaaatgca cttagaagaa gctctgctgc ccatacacat 2850
   tcaaacactt acaatttcac taagtcggaa aattcaaata ggacatgttc 2900
   tatgccttat gccaaattag aatacaagag atcttcaaat gatagtttaa 2950
   atagtgtcag tagtagtgat ggttatggta aaagaggtca aatgaaaccc 3000
   tcgattgaat cctattctga agatgatgaa agtaagtttt gcagttatgg 3050
   tcaataccca gccgacctag cccataaaat acatagtgca aatcatatgg 3100
   atgataatga tggagaacta gatacaccaa taaattatag tcttaaatat 3150
   tcagatgagc agttgaactc tggaaggcaa agtccttcac agaatgaaag 3200
   atgggcaaga cccaaacaca taatagaaga tgaaataaaa caaagtgagc 3250
   aaagacaatc aaggaatcaa agtacaactt atcctgttta tactgagagc 3300
   actgatgata aacacctcaa gttccaacca cattttggac agcaggaatg 3350
   tgtttctcca tacaggtcac ggggagccaa tggttcagaa acaaatcgag 3400
   tgggttctaa tcatggaatt aatcaaaatg taagccagtc tttgtgtcaa 3450
   gaagatgact atgaagatga taagcctacc aattatagtg aacgttactc 3500
   tgaagaagaa cagcatgaag aagaagagag accaacaaat tatagcataa 3550
   aatataatga agagaaacgt catgtggatc agcctattga ttatagttta 3600
   aaatatgcca cagatattcc ttcatcacag aaacagtcat tttcattctc 3650
   aaagagttca tctggacaaa gcagtaaaac cgaacatatg tcttcaagca 3700
   gtgagaatac gtccacacct tcatctaatg ccaagaggca gaatcagctc 3750
   catccaagtt ctgcacagag tagaagtggt cagcctcaaa aggctgccac 3800
   ttgcaaagtt tcttctatta accaagaaac aatacagact tattgtgtag 3850
   aagatactcc aatatgtttt tcaagatgta gttcattatc atctttgtca 3900
   tcagctgaag atgaaatagg atgtaatcag acgacacagg aagcagattc 3950
   tgctaatacc ctgcaaatag cagaaataaa agaaaagatt ggaactaggt 4000
   cagctgaaga tcctgtgagc gaagttccag cagtgtcaca gcaccctaga 4050
   accaaatcca gcagactgca gggttctagt ttatcttcag aatcagccag 4100
   gcacaaagct gttgaatttt cttcaggagc gaaatctccc tccaaaagtg 4150
   gtgctcagac acccaaaagt ccacctgaac actatgttca ggagacccca 4200
   ctcatgttta gcagatgtac ttctgtcagt tcacttgata gttttgagag 4250
   tcgttcgatt gccagctccg ttcagagtga accatgcagt ggaatggtaa 4300
   gtggcattat aagccccagt gatcttccag atagccctgg acaaaccatg 4350
   ccaccaagca gaagtaaaac acctccacca cctcctcaaa cagctcaaac 4400
   caagcgagaa gtacctaaaa ataaagcacc tactgctgaa aagagagaga 4450
   gtggacctaa gcaagctgca gtaaatgctg cagttcagag ggtccaggtt 4500
   cttccagatg ctgatacttt attacatttt gccacggaaa gtactccaga 4550
   tggattttct tgttcatcca gcctgagtgc tctgagcctc gatgagccat 4600
   ttatacagaa agatgtggaa ttaagaataa tgcctccagt tcaggaaaat 4650
   gacaatggga atgaaacaga atcagagcag cctaaagaat caaatgaaaa 4700
   ccaagagaaa gaggcagaaa aaactattga ttctgaaaag gacctattag 4750
   atgattcaga tgatgatgat attgaaatac tagaagaatg tattatttct 4800
   gccatgccaa caaagtcatc acgtaaagca aaaaagccag cccagactgc 4850
   ttcaaaatta cctccacctg tggcaaggaa accaagtcag ctgcctgtgt 4900
   acaaacttct accatcacaa aacaggttgc aaccccaaaa gcatgttagt 4950
   tttacaccgg gggatgatat gccacgggtg tattgtgttg aagggacacc 5000
   tataaacttt tccacagcta catctctaag tgatctaaca atcgaatccc 5050
   ctccaaatga gttagctgct ggagaaggag ttagaggagg ggcacagtca 5100
   ggtgaatttg aaaaacgaga taccattcct acagaaggca gaagtacaga 5150
   tgaggctcaa ggaggaaaaa cctcatctgt aaccatacct gaattggatg 5200
   acaataaagc agaggaaggt gatattcttg cagaatgcat taattctgct 5250
   atgcccaaag ggaaaagtca caagcctttc cgtgtgaaaa agataatgga 5300
   ccaggtccag caagcatctg cgtcttcttc tgcacccaac aaaaatcagt 5350
   tagatggtaa gaaaaagaaa ccaacttcac cagtaaaacc tataccacaa 5400
   aatactgaat ataggacacg tgtaagaaaa aatgcagact caaaaaataa 5450
   tttaaatgct gagagagttt tctcagacaa caaagattca aagaaacaga 5500
   at t t gaaaaa t aat t ccaag gt ct t caat g at aagct ccc aaat aat gaa 5550
   gat agagt ca gaggaagt t t t gct t t t gat t cacct cat c at t acacgcc 5600
   t at t gaagga act cct t act gt t t t t cacg aaat gat t ct t t gagt t ct c 5650
   tagattttga tgatgatgat gttgaccttt ccagggaaaa ggctgaatta 5700
   agaaaggcaa aagaaaataa ggaatcagag gctaaagtta ccagccacac 5750
   agaact aacc t ccaaccaac aat cagct aa t aagacacaa gct at t gcaa 5800
   agcagccaat aaatcgaggt cagcctaaac ccatacttca gaaacaatcc 5850
   acttttcccc agtcatccaa agacatacca gacagagggg cagcaactga 5900
   t gaaaagt t a cagaat t t t g ct at t gaaaa t act ccggt t t gct t t t ct c 5950
   ataattcctc tctgagttct ctcagtgaca ttgaccaaga aaacaacaat 6000
   aaagaaaatg aacctatcaa agagactgag ccccctgact cacagggaga 6050
   accaagt aaa cct caagcat caggct at gc t cct aaat ca t t t cat gt t g 6100
   aagat acccc agt t t gt t t c t caagaaaca gt t ct ct cag t t ct ct t agt 6150
   at t gact ct g aagat gacct gt t gcaggaa t gt at aagct ccgcaat gcc 6200
   aaaaaagaaa aagccttcaa gactcaaggg tgataatgaa aaacatagtc 6250
   ccagaaat at gggt ggcat a t t aggt gaag at ct gacact t gat t t gaaa 6300
   gat at acaga gaccagat t c agaacat ggt ct at cccct g at t cagaaaa 6350
   t t t t gat t gg aaagct at t c aggaaggt gc aaat t ccat a gt aagt agt t 6400
   t acat caagc t gct gct gct gcat gt t t at ct agacaagc t t cgt ct gat 6450
   t cagat t cca t cct t t ccct gaaat cagga at ct ct ct gg gat caccat t 6500
   tcatcttaca cctgatcaag aagaaaaacc ctttacaagt aataaaggcc 6550
   cacgaattct aaaaccaggg gagaaaagta cattggaaac taaaaagata 6600
   gaatctgaaa gtaaaggaat caaaggagga aaaaaagttt ataaaagttt 6650
   gat t act gga aaagt t cgat ct aat t caga aat t t caggc caaat gaaac 6700
   agcccct t ca agcaaacat g cct t caat ct ct cgaggcag gacaat gat t 6750
   cat at t ccag gagt t cgaaa t agct cct ca agt acaagt c ct gt t t ct aa 6800
   aaaaggccca ccccttaaga ctccagcctc caaaagccct agtgaaggtc 6850
   aaacagccac cacttctcct agaggagcca agccatctgt gaaatcagaa 6900
   t t aagccct g t t gccaggca gacat cccaa at aggt gggt caagt aaagc 6950
   acct t ct aga t caggat ct a gagat t cgac ccct t caaga cct gcccagc 7000
   aaccat t aag t agacct at a cagt ct cct g gccgaaact c aat t t cccct 7050
   ggt agaaat g gaat aagt cc t cct aacaaa t t at ct caac t t ccaaggac 7100
   at cat cccct agt act gct t caact aagt c ct caggt t ct ggaaaaat gt 7150
   catatacatc tccaggtaga cagatgagcc aacagaacct taccaaacaa 7200
   acaggt t t at ccaagaat gc cagt agt at t ccaagaagt g agt ct gcct c 7250
   caaaggact a aat cagat ga at aat ggt aa t ggagccaat aaaaaggt ag 7300
   aact t t ct ag aat gt ct t ca act aaat caa gt ggaagt ga at ct gat aga 7350
   t cagaaagac ct gt at t agt acgccagt ca act t t cat ca aagaagct cc 7400
   aagcccaacc ttaagaagaa aattggagga atctgcttca tttgaatctc 7450
   tttctccatc atctagacca gcttctccca ctaggtccca ggcacaaact 7500
   ccagt t t t aa gt cct t ccct t cct gat at g t ct ct at cca cacat t cgt c 7550
   t gt t caggct ggt ggat ggc gaaaact ccc acct aat ct c agt cccact a 7600
   tagagtataa tgatggaaga ccagcaaagc gccatgatat tgcacggtct 7650
   cat t ct gaaa gt cct t ct ag act t ccaat c aat aggt cag gaacct ggaa 7700
   acgt gagcac agcaaacat t cat cat ccct t cct cgagt a agcact t gga 7750
   gaagaact gg aagt t cat ct t caat t ct t t ct gct t cat c agaat ccagt 7800
   gaaaaagcaa aaagtgagga tgaaaaacat gtgaactcta tttcaggaac 7850
   caaacaaagt aaagaaaacc aagtatccgc aaaaggaaca tggagaaaaa 7900
   t aaaagaaaa t gaat t t t ct cccacaaat a gt act t ct ca gaccgt t t cc 7950
   t caggt gct a caaat ggt gc t gaat caaag act ct aat t t at caaat ggc 8000
   acct gct gt t t ct aaaacag aggat gt t t g ggt gagaat t gaggact gt c 8050
   ccat t aacaa t cct agat ct ggaagat ct c ccacaggt aa t act cccccg 8100
   gtgattgaca gtgtttcaga aaaggcaaat ccaaacatta aagattcaaa 8150
   agataatcag gcaaaacaaa atgtgggtaa tggcagtgtt cccatgcgta 8200
   ccgt gggt t t ggaaaat cgc ct gaact cct t t at t caggt ggat gcccct 8250
   gaccaaaaag gaactgagat aaaaccagga caaaataatc ctgtccctgt 8300
   at cagagact aat gaaagt t ct at agt gga acgt acccca t t cagt t ct a 8350
   gcagctcaag caaacacagt tcacctagtg ggactgttgc tgccagagtg 8400
   actcctttta attacaaccc aagccctagg aaaagcagcg cagatagcac 8450
   ttcagctcgg ccatctcaga tcccaactcc agtgaataac aacacaaaga 8500
   agcgagattc caaaactgac agcacagaat ccagtggaac ccaaagtcct 8550
   aagcgccatt ctgggtctta ccttgtgaca tctgtttaaa agagaggaag 8600
   aat gaaact a agaaaat t ct at gt t aat t a caact gct at at agacat t t 8650
   t gt t t caaat gaaact t t aa aagact gaaa aat t t t gt aa at aggt t t ga 8700
   t t ct t gt t ag agggt t t t t g t t ct ggaagc cat at t t gat agt at act t t 8750
   gtcttcactg gtcttatttt gggaggcact cttgatggtt aggaaaaaaa 8800
   t agt aaagcc aagt at gt t t gt acagt at g t t t t acat gt at t t aaagt a 8850
   gcat cccat c ccaact t cct t t aat t at t g ct t gt ct t aa aat aat gaac 8900
   act acagat a gaaaat at ga t at at t gct g t t at caat ca t t t ct agat t 8950
   at aaact gac t aaact t aca t cagggaaaa at t ggt at t t at gcaaaaaa 9000
   aaat gt t t t t gt cct t gt ga gt ccat ct aa cat cat aat t aat cat gt gg 9050
   ct gt gaaat t cacagt aat a t ggt t cccga t gaacaagt t t acccagcct 9100
   gct t t gct t t act gcat gaa t gaaact gat ggt t caat t t cagaagt aat 9150
   gat t aacagt t at gt ggt ca cat gat gt gc at agagat ag ct acagt gt a 9200
   at aat t t aca ct at t t t gt g ct ccaaacaa aacaaaaat c t gt gt aact g 9250
   t aaaacat t g aat gaaact a t t t t acct ga act agat t t t at ct gaaagt 9300
   aggt agaat t t t t gct at gc t gt aat t t gt t gt at at t ct ggt at t t gag 9350
   gt gagat ggc t gct ct t t t a t t aat gagac at gaat t gt g t ct caacaga 9400
   aact aaat ga acat t t caga at aaat t at t gct gt at gt a aact gt t act 9450
   gaaat t ggt a t t t gt t t gaa gggt ct t gt t t cacat t t gt at t aat aat t 9500
   gt t t aaaat g cct ct t t t aa aagct t at at aaat t t t t t t ct t cagct t c 9550
   t at gcat t aa gagt aaaat t cct ct t act g t aat aaaaac aat t gaagaa 9600
   gact gt t gcc act t aaccat t ccat gcgt t ggcact t at c t at t cct gaa 9650
   at t t ct t t t a t gt gat t agc t cat ct t gat t t t t aat at t t t t ccact t a 9700
   aact t t t t t t t ct t act cca ct ggagct ca gt aaaagt aa at t cat gt aa 9750
   t agcaat gca agcagcct ag cacagact aa gcat t gagca t aat aggccc 9800
   acat aat t t c ct ct t t ct t a at at t at aga at t ct gt act t gaaat t gat 9850
   t ct t agacat t gcagt ct ct t cgaggct t t acagt gt aaa ct gt ct t gcc 9900
   cct t cat ct t ct t gt t gcaa ct gggt ct ga cat gaacact t t t t at cacc 9950
   ct gt at gt t a gggcaagat c t cagcagt ga agt at aat ca gcact t t gcc 10000
   at gct cagaa aat t caaat c acat ggaact t t agaggt ag at t t aat acg 10050
   at t aagat at t cagaagt at at t t t agaat ccct gcct gt t aaggaaact 10100
   t t at t t gt gg t aggt acagt t ct ggggt ac at gt t aagt g t cccct t at a 10150
   cagt ggaggg aagt ct t cct t cct gaagga aaat aaact g acact t at t a 10200
   act aagat aa t t t act t aat at at ct t ccc t gat t t gt t t t aaaagat ca 10250
   gagggt gact gat gat acat gcat acat at t t gt t gaat a aat gaaaat t 10300
   tatttttagt gataagattc atacactctg tatttgggga gggaaaacct 10350
   t t t t aagcat ggt ggggcac t cagat agga gt gaat acac ct acct ggt g 10400
   cct t gaaaat cacat caagt agt t aat t at ct acccct t a cct gt gt t t a 10450
   t aact t ccag gt aat gagaa t gat t t t t t t t aaagct aaa at gccagt aa 10500
   at aaaagt gc t at gact t ga gct aagat at t t gact ccaa t gcct gt act 10550
   gt gt ct act g caccact t t g t aaacact t c aat t t act at ct t t gaaat g 10600
   at t gacct t t aaat t t t t gc caaat gt t at ct gaaat t gt ct at gaat ac 10650
   cat ct act t c  t gt t gt t t t c  ccaggct t cc  at aaacaat g  gagat acat g  10700
   c  10701
<210> 3
   <211> 13147
   <212> DNA
   <213> Homo sapiens
<400> 3
   ccggagcccg agccgaaggg cgagccgcaa acgctaagtc gctggccatt 50
   ggtggacatg gcgcaggcgc gtttgctccg acgggccgaa tgttttgggg 100
   cagtgttttg agcgcggaga ccgcgtgata ctggatgcgc atgggcatac 150
   cgtgctctgc ggctgcttgg cgttgcttct tcctccagaa gtgggcgctg 200
   ggcagtcacg cagggtttga accggaagcg ggagtaggta gctgcgtggc 250
   taacggagaa aagaagccgt ggccgcggga ggaggcgaga ggagtcggga 300
   tctgcgctgc agccaccgcc gcggttgata ctactttgac cttccgagtg 350
   cagtgacagt gatgtgtgtt ctgaaattgt gaaccatgag tctagtactt 400
   aatgatctgc ttatctgctg ccgtcaacta gaacatgata gagctacaga 450
   acgaaagaaa gaagttgaga aatttaagcg cctgattcga gatcctgaaa 500
   caattaaaca tctagatcgg cattcagatt ccaaacaagg aaaatatttg 550
   aattgggatg ctgtttttag atttttacag aaatatattc agaaagaaac 600
   agaatgtctg agaatagcaa aaccaaatgt atcagcctca acacaagcct 650
   ccaggcagaa aaagatgcag gaaatcagta gtttggtcaa atacttcatc 700
   aaatgtgcaa acagaagagc acctaggcta aaatgtcaag aactcttaaa 750
   ttatatcatg gatacagtga aagattcatc taatggtgct atttacggag 800
   ctgattgtag caacatacta ctcaaagaca ttctttctgt gagaaaatac 850
   tggtgtgaaa tatctcagca acagtggtta gaattgttct ctgtgtactt 900
   caggctctat ctgaaacctt cacaagatgt tcatagagtt ttagtggcta 950
   gaataattca tgctgttacc aaaggatgct gttctcagac tgacggatta 1000
   aattccaaat ttttggactt tttttccaag gctattcagt gtgcgagaca 1050
   agaaaagagc tcttcaggtc taaatcatat cttagcagct cttactatct 1100
   tcctcaagac tttggctgtc aactttcgaa ttcgagtgtg tgaattagga 1150
   gatgaaattc ttcccacttt gctttatatt tggactcaac ataggcttaa 1200
   tgattcttta aaagaagtca ttattgaatt atttcaactg caaatttata 1250
   tccatcatcc gaaaggagcc aaaacccaag aaaaaggtgc ttatgaatca 1300
   acaaaatgga gaagtatttt atacaactta tatgatctgc tagtgaatga 1350
   gataagtcat ataggaagta gaggaaagta ttcttcagga tttcgtaata 1400
   ttgccgtcaa agaaaatttg attgaattga tggcagatat ctgtcaccag 1450
   gtttttaatg aagataccag atccttggag atttctcaat cttacactac 1500
   tacacaaaga gaatctagtg attacagtgt cccttgcaaa aggaagaaaa 1550
   tagaactagg ctgggaagta ataaaagatc accttcagaa gtcacagaat 1600
   gattttgatc ttgtgccttg gctacagatt gcaacccaat taatatcaaa 1650
   gtatcctgca agtttaccta actgtgagct gtctccatta ctgatgatac 1700
   tatctcagct tctaccccaa cagcgacatg gggaacgtac accatatgtg 1750
   ttacgatgcc ttacggaagt tgcattgtgt caagacaaga ggtcaaacct 1800
   agaaagctca caaaagtcag atttattaaa actctggaat aaaatttggt 1850
   gtattacctt tcgtggtata agttctgagc aaatacaagc tgaaaacttt 1900
   ggcttacttg gagccataat tcagggtagt ttagttgagg ttgacagaga 1950
   attctggaag ttatttactg ggtcagcctg cagaccttca tgtcctgcag 2000
   tatgctgttt gactttggca ctgaccacca gtatagttcc aggaacggta 2050
   aaaatgggaa tagagcaaaa tatgtgtgaa gtaaatagaa gcttttcttt 2100
   aaaggaatca ataatgaaat ggctcttatt ctatcagtta gagggtgact 2150
   tagaaaatag cacagaagtg cctccaattc ttcacagtaa ttttcctcat 2200
   cttgtactgg agaaaattct tgtgagtctc actatgaaaa actgtaaagc 2250
   tgcaatgaat tttttccaaa gcgtgccaga atgtgaacac caccaaaaag 2300
   ataaagaaga actttcattc tcagaagtag aagaactatt tcttcagaca 2350
   acttttgaca agatggactt tttaaccatt gtgagagaat gtggtataga 2400
   aaagcaccag tccagtattg gcttctctgt ccaccagaat ctcaaggaat 2450
   cactggatcg ctgtcttctg ggattatcag aacagcttct gaataattac 2500
   tcatctgaga ttacaaattc agaaactctt gtccggtgtt cacgtctttt 2550
   ggtgggtgtc cttggctgct actgttacat gggtgtaata gctgaagagg 2600
   aagcatataa gtcagaatta ttccagaaag ccaagtctct aatgcaatgt 2650
   gcaggagaaa gtatcactct gtttaaaaat aagacaaatg aggaattcag 2700
   aattggttcc ttgagaaata tgatgcagct atgtacacgt tgcttgagca 2750
   actgtaccaa gaagagtcca aataagattg catctggctt tttcctgcga 2800
   ttgttaacat caaagctaat gaatgacatt gcagatattt gtaaaagttt 2850
   agcatccttc atcaaaaagc catttgaccg tggagaagta gaatcaatgg 2900
   aagatgatac taatggaaat ctaatggagg tggaggatca gtcatccatg 2950
   aatctattta acgattaccc tgatagtagt gttagtgatg caaacgaacc 3000
   tggagagagc caaagtacca taggtgccat taatccttta gctgaagaat 3050
   atctgtcaaa gcaagatcta cttttcttag acatgctcaa gttcttgtgt 3100
   ttgtgtgtaa ctactgctca gaccaatact gtgtccttta gggcagctga 3150
   tattcggagg aaattgttaa tgttaattga ttctagcacg ctagaaccta 3200
   ccaaatccct ccacctgcat atgtatctaa tgcttttaaa ggagcttcct 3250
   ggagaagagt accccttgcc aatggaagat gttcttgaac ttctgaaacc 3300
   actatccaat gtgtgttctt tgtatcgtcg tgaccaagat gtttgtaaaa 3350
   ctattttaaa ccatgtcctt catgtagtga aaaacctagg tcaaagcaat 3400
   atggactctg agaacacaag ggatgctcaa ggacagtttc ttacagtaat 3450
   tggagcattt tggcatctaa caaaggagag gaaatatata ttctctgtaa 3500
   gaatggccct agtaaattgc cttaaaactt tgcttgaggc tgatccttat 3550
   tcaaaatggg ccattcttaa tgtaatggga aaagactttc ctgtaaatga 3600
   agtatttaca caatttcttg ctgacaatca tcaccaagtt cgcatgttgg 3650
   ctgcagagtc aatcaataga ttgttccagg acacgaaggg agattcttcc 3700
   aggttactga aagcacttcc tttgaagctt cagcaaacag cttttgaaaa 3750
   tgcatacttg aaagctcagg aaggaatgag agaaatgtcc catagtgctg 3800
   agaaccctga aactttggat gaaatttata atagaaaatc tgttttactg 3850
   acgttgatag ctgtggtttt atcctgtagc cctatctgcg aaaaacaggc 3900
   tttgtttgcc ctgtgtaaat ctgtgaaaga gaatggatta gaacctcacc 3950
   ttgtgaaaaa ggttttagag aaagtttctg aaacttttgg atatagacgt 4000
   ttagaagact ttatggcatc tcatttagat tatctggttt tggaatggct 4050
   aaatcttcaa gatactgaat acaacttatc ttcttttcct tttattttat 4100
   taaactacac aaatattgag gatttctata gatcttgtta taaggttttg 4150
   attccacatc tggtgattag aagtcatttt gatgaggtga agtccattgc 4200
   taatcagatt caagaggact ggaaaagtct tctaacagac tgctttccaa 4250
   agattcttgt aaatattctt ccttattttg cctatgaggg taccagagac 4300
   agtgggatgg cacagcaaag agagactgct accaaggtct atgatatgct 4350
   taaaagtgaa aacttattgg gaaaacagat tgatcactta ttcattagta 4400
   atttaccaga gattgtggtg gagttattga tgacgttaca tgagccagca 4450
   aattctagtg ccagtcagag cactgacctc tgtgactttt caggggattt 4500
   ggatcctgct cctaatccac ctcattttcc atcgcatgtg attaaagcaa 4550
   catttgccta tatcagcaat tgtcataaaa ccaagttaaa aagcatttta 4600
   gaaattcttt ccaaaagccc tgattcctat cagaaaattc ttcttgccat 4650
   atgtgagcaa gcagctgaaa caaataatgt ttataagaag cacagaattc 4700
   ttaaaatata tcacctgttt gttagtttat tactgaaaga tataaaaagt 4750
   ggcttaggag gagcttgggc ctttgttctt cgagacgtta tttatacttt 4800
   gattcactat atcaaccaaa ggccttcttg tatcatggat gtgtcattac 4850
   gtagcttctc cctttgttgt gacttattaa gtcaggtttg ccagacagcc 4900
   gtgacttact gtaaggatgc tctagaaaac catcttcatg ttattgttgg 4950
   tacacttata ccccttgtgt atgagcaggt ggaggttcag aaacaggtat 5000
   tggacttgtt gaaatactta gtgatagata acaaggataa tgaaaacctc 5050
   tatatcacga ttaagctttt agatcctttt cctgaccatg ttgtttttaa 5100
   ggatttgcgt attactcagc aaaaaatcaa atacagtaga ggaccctttt 5150
   cactcttgga ggaaattaac cattttctct cagtaagtgt ttatgatgca 5200
   cttccattga caagacttga aggactaaag gatcttcgaa gacaactgga 5250
   actacataaa gatcagatgg tggacattat gagagcttct caggataatc 5300
   cgcaagatgg gattatggtg aaactagttg tcaatttgtt gcagttatcc 5350
   aagatggcaa taaaccacac tggtgaaaaa gaagttctag aggctgttgg 5400
   aagctgcttg ggagaagtgg gtcctataga tttctctacc atagctatac 5450
   aacatagtaa agatgcatct tataccaagg cccttaagtt atttgaagat 5500
   aaagaacttc agtggacctt cataatgctg acctacctga ataacacact 5550
   ggtagaagat tgtgtcaaag ttcgatcagc agctgttacc tgtttgaaaa 5600
   acattttagc cacaaagact ggacatagtt tctgggagat ttataagatg 5650
   acaacagatc caatgctggc ctatctacag ccttttagaa catcaagaaa 5700
   aaagttttta gaagtaccca gatttgacaa agaaaaccct tttgaaggcc 5750
   tggatgatat aaatctgtgg attcctctaa gtgaaaatca tgacatttgg 5800
   ataaagacac tgacttgtgc ttttttggac agtggaggca caaaatgtga 5850
   aattcttcaa ttattaaagc caatgtgtga agtgaaaact gacttttgtc 5900
   agactgtact tccatacttg attcatgata ttttactcca agatacaaat 5950
   gaatcatgga gaaatctgct ttctacacat gttcagggat ttttcaccag 6000
   ctgtcttcga cacttctcgc aaacgagccg atccacaacc cctgcaaact 6050
   tggattcaga gtcagagcac tttttccgat gctgtttgga taaaaaatca 6100
   caaagaacaa tgcttgctgt tgtggactac atgagaagac aaaagagacc 6150
   ttcttcagga acaattttta atgatgcttt ctggctggat ttaaattatc 6200
   tagaagttgc caaggtagct cagtcttgtg ctgctcactt tacagcttta 6250
   ctctatgcag aaatctatgc agataagaaa agtatggatg atcaagagaa 6300
   aagaagtctt gcatttgaag aaggaagcca gaatacaact atttctagct 6350
   tgagtgaaaa aagtaaagaa gaaactggaa taagtttaca ggatcttctc 6400
   ttagaaatct acagaagtat aggggagcca gatagtttgt atggctgtgg 6450
   tggagggaag atgttacaac ccattactag actacgaaca tatgaacacg 6500
   aagcaatgtg gggcaaagcc ctagtaacat atgacctcga aacagcaatc 6550
   ccctcatcaa cacgccaggc aggaatcatt caggccttgc agaatttggg 6600
   actctgccat attctttccg tctatttaaa aggattggat tatgaaaata 6650
   aagactggtg tcctgaacta gaagaacttc attaccaagc agcatggagg 6700
   aatatgcagt gggaccattg cacttccgtc agcaaagaag tagaaggaac 6750
   cagttaccat gaatcattgt acaatgctct acaatctcta agagacagag 6800
   aattctctac attttatgaa agtctcaaat atgccagagt aaaagaagtg 6850
   gaagagatgt gtaagcgcag ccttgagtct gtgtattcgc tctatcccac 6900
   acttagcagg ttgcaggcca ttggagagct ggaaagcatt ggggagcttt 6950
   tctcaagatc agtcacacat agacaactct ctgaagtata tattaagtgg 7000
   cagaaacact cccagcttct caaggacagt gattttagtt ttcaggagcc 7050
   tatcatggct ctacgcacag tcattttgga gatcctgatg gaaaaggaaa 7100
   tggacaactc acaaagagaa tgtattaagg acattctcac caaacacctt 7150
   gtagaactct ctatactggc cagaactttc aagaacactc agctccctga 7200
   aagggcaata tttcaaatta aacagtacaa ttcagttagc tgtggagtct 7250
   ctgagtggca gctggaagaa gcacaagtat tctgggcaaa aaaggagcag 7300
   agtcttgccc tgagtattct caagcaaatg atcaagaagt tggatgccag 7350
   ctgtgcagcg aacaatccca gcctaaaact tacatacaca gaatgtctga 7400
   gggtttgtgg caactggtta gcagaaacgt gcttagaaaa tcctgcggtc 7450
   atcatgcaga cctatctaga aaaggcagta gaagttgctg gaaattatga 7500
   tggagaaagt agtgatgagc taagaaatgg aaaaatgaag gcatttctct 7550
   cattagcccg gttttcagat actcaatacc aaagaattga aaactacatg 7600
   aaatcatcgg aatttgaaaa caagcaagct ctcctgaaaa gagccaaaga 7650
   ggaagtaggt ctccttaggg aacataaaat tcagacaaac agatacacag 7700
   taaaggttca gcgagagctg gagttggatg aattagccct gcgtgcactg 7750
   aaagaggatc gtaaacgctt cttatgtaaa gcagttgaaa attatatcaa 7800
   ctgcttatta agtggagaag aacatgatat gtgggtattc cgactttgtt 7850
   ccctctggct tgaaaattct ggagtttctg aagtcaatgg catgatgaag 7900
   gcaaacggaa tgaagattcc aacatataaa tttttgcctc ttatgtacca 7950
   attggctgct agaatgggga ccaagatgat gggaggccta ggatttcatg 8000
   aagtcctcaa taatctaatc tctagaattt caatggatca cccccatcac 8050
   actttgttta ttatactggc cttagcaaat gcaaacagag atgaatttct 8100
   gactaaacca gaggtagcca gaagaagcag aataactaaa aatgtgccta 8150
   aacaaagctc tcagcttgat gaggatcgaa cagaggctgc aaatagaata 8200
   atatgtacta tcagaagtag gagacctcag atggtcagaa gtgttgaggc 8250
   actttgtgat gcttatatta tattagcaaa cttagatgcc actcagtgga 8300
   agactcagag aaaaggcata aatattccag cagaccagcc aattactaaa 8350
   cttaagaatt tagaagatgt tgttgtccct actatggaaa ttaaggtgga 8400
   ccacacagga gaatatggaa atctggtgac tatacagtca tttaaagcag 8450
   aatttcgctt agcaggaggt gtaaatttac caaaaataat agattgtgta 8500
   ggttccgatg gcaaggagag gagacagctt gttaagggcc gtgatgacct 8550
   gagacaagat gctgtcatgc aacaggtctt ccagatgtgt aatacattac 8600
   tgcagagaaa cacggaaact aggaagagga aattaactat ctgtacttat 8650
   aaggtggttc ccctctctca gcgaagtggt gttcttgaat ggtgcacagg 8700
   aactgtcccc attggtgaat ttcttgttaa caatgaagat ggtgctcata 8750
   aaagatacag gccaaatgat ttcagtgcct ttcagtgcca aaagaaaatg 8800
   atggaggtgc aaaaaaagtc ttttgaagag aaatatgaag tcttcatgga 8850
   tgtttgccaa aattttcaac cagttttccg ttacttctgc atggaaaaat 8900
   tcttggatcc agctatttgg tttgagaagc gattggctta tacgcgcagt 8950
   gtagctactt cttctattgt tggttacata cttggacttg gtgatagaca 9000
   tgtacagaat atcttgataa atgagcagtc agcagaactt gtacatatag 9050
   atctaggtgt tgcttttgaa cagggcaaaa tccttcctac tcctgagaca 9100
   gttcctttta gactcaccag agatattgtg gatggcatgg gcattacggg 9150
   tgttgaaggt gtcttcagaa gatgctgtga gaaaaccatg gaagtgatga 9200
   gaaactctca ggaaactctg ttaaccattg tagaggtcct tctatatgat 9250
   ccactctttg actggaccat gaatcctttg aaagctttgt atttacagca 9300
   gaggccggaa gatgaaactg agcttcaccc tactctgaat gcagatgacc 9350
   aagaatgcaa acgaaatctc agtgatattg accagagttt caacaaagta 9400
   gctgaacgtg tcttaatgag actacaagag aaactgaaag gagtggaaga 9450
   aggcactgtg ctcagtgttg gtggacaagt gaatttgctc atacagcagg 9500
   ccatagaccc caaaaatctc agccgacttt tcccaggatg gaaagcttgg 9550
   gtgtgatctt cagtatatga attacccttt cattcagcct ttagaaatta 9600
   tattttagcc tttattttta acctgccaac atactttaag tagggattaa 9650
   tatttaagtg aactattgtg ggtttttttg aatgttggtt ttaatacttg 9700
   atttaatcac cactcaaaaa tgttttgatg gtcttaagga acatctctgc 9750
   tttcactctt tagaaataat ggtcattcgg gctgggcgca gcggctcacg 9800
   cctgtaatcc cagcactttg ggaggccgag gtgagcggat cacaaggtca 9850
   ggagttcgag accagcctgg ccaagagacc agcctggcca gtatggtgaa 9900
   accctgtctc tactaaaaat acaaaaatta gccgagcatg gtggcgggca 9950
   cctgtaatcc cagctactcg agaggctgag gcaggagaat ctcttgaacc 10000
   tgggaggtga aggttgctgt gggccaaaat catgccattg cactccagcc 10050
   tgggtgacaa gagcgaaact ccatctcaaa aaaaaaaaaa aaaaaacaga 10100
   aacgtatttg gatttttcct agtaagatca ctcagtgtta ctaaataatg 10150
   aagttgttat ggagaacaaa tttcaaagac acagttagtg tagttactat 10200
   ttttttaagt gtgtattaaa acttctcatt ctattctctt tatcttttaa 10250
   gcccttctgt actgtccatg tatgttatct ttctgtgata acttcataga 10300
   ttgccttcta gttcatgaat tctcttgtca gatgtatata atctctttta 10350
   ccctatccat tgggcttctt ctttcagaaa ttgtttttca tttctaatta 10400
   tgcatcattt ttcagatctc tgtttcttga tgtcattttt aatgtttttt 10450
   taatgttttt tatgtcacta attattttaa atgtctgtac ttgatagaca 10500
   ctgtaatagt tctattaaat ttagttcctg ctgtttatat ctgttgattt 10550
   ttgtatttga taggctgttc atccagtttt gtctttttga aaagtgagtt 10600
   tattttcagc aaggctttat ctatgggaat cttgagtgtc tgtttatgtc 10650
   atattcccag ggctgttgct gcacacaagc ccattcttat tttaatttct 10700
   tggctttagg gtttccatac ctgaagtgta gcataaatac tgataggaga 10750
   tttcccaggc caaggcaaac acacttcctc ctcatctcct tgtgctagtg 10800
   ggcagaatat ttgattgatg cctttttcac tgagagtata agcttccatg 10850
   tgtcccacct ttatggcagg ggtggaagga ggtacattta attcccactg 10900
   cctgcctttg gcaagccctg ggttctttgc tccccatata gatgtctaag 10950
   ctaaaagccg tgggttaatg agactggcaa attgttccag gacagctaca 11000
   gcatcagctc acatattcac ctctctggtt tttcattccc ctcatttttt 11050
   tctgagacag agtcttgctc tgtcacccag gctggagtgc agtggcatga 11100
   tctcagctca ctgaaacctc tgcctcctgg gttcaagcaa ttctcctgcc 11150
   tcagcctccc gagtagctgg gactacaggc gtgtgccaac acgcccggct 11200
   aattttttgt attttattta gagacggagt ttcaccgtgt tagccaggat 11250
   ggtctcgatc gcttgacctc gtgatccacc ctcctcggcc tcccaaagtg 11300
   ctgggattac aggtgtgagc caccgcgccc ggcctcattc ccctcatttt 11350
   tgaccgtaag gatttcccct ttcttgtaag ttctgctatg tatttaaaag 11400
   aatgttttct acattttatc cagcatttct ctgtgttctg ttggaaggga 11450
   agggcttagg tatctagttt gatacatagg tagaagtgga acatttctct 11500
   gtcccccagc tgtcatcata taagataaac atcagataaa aagccacctg 11550
   aaagtaaaac tactgactcg tgtattagtg agtataatct cttctccatc 11600
   cttaggaaaa tgttcatccc agctgcggag attaacaaat gggtgattga 11650
   gctttctcct cgtatttgga ccttgaaggt tatataaatt tttttcttat 11700
   gaagagttgg catttctttt tattgccaat ggcaggcact cattcatatt 11750
   tgatctcctc accttcccct cccctaaaac caatctccag aactttttgg 11800
   act at aaat t t ct t ggt t t g act t ct ggag aact gt t cag aat at t act t 11850
   t gcat t t caa at t acaaact t acct t ggt g t at ct t t t t c t t acaagct g 11900
   cct aaat gaa t at t t ggt at at at t ggt ag t t t t at t act at agt aaat c 11950
   aaggaaatgc agtaaactta aaatgtcttt aagaaagccc tgaaatcttc 12000
   at gggt gaaa t t agaaat t a t caact agat aat agt at ag at aaat gaat 12050
   t t gt agct aa t t ct t gct ag t t gt t gcat c cagagagct t t gaat aacat 12100
   cat t aat ct a ct ct t t agcc t t gcat ggt a t gct at gagg ct cct gt t ct 12150
   gt t caagt at t ct aat caat ggct t t gaaa agt t t at caa at t t acat ac 12200
   agat cacaag cct aggagaa at aact aat t cacagat gac agaat t aaga 12250
   ttataaaaga tttttttttt gtaattttag tagagacagg gttgccattg 12300
   tattccagcc ttggcgacag agcaagactc tgcctcaaaa aaaaaaaaaa 12350
   aaaggt t t t g gcaagct gga act ct t t ct g caaat gact a agat agaaaa 12400
   ct gccaagga caaat gagga gt agt t agat t t t gaaaat a t t aat cat ag 12450
   aat agt t gt t gt at gct aag t cact gaccc at at t at gt a cagcat t t ct 12500
   gat ct t t act t t gcaagat t agt gat act a t cccaat aca ct gct ggaga 12550
   aatcagaatt tggagaaata agttgtccaa ggcaagaaga tagtaaatta 12600
   t aagt acaag t gt aat at gg acagt at ct a act t gaaaag at t t caggcg 12650
   aaaagaat ct ggggt t t gcc agt cagt t gc t caaaaggt c aat gaaaacc 12700
   aaat agt gaa gct at cagag aagct aat aa at t at agact gct t gaacag 12750
   t t gt gt ccag at t aagggag at aat agct t t cccaccct a ct t t gt gcag 12800
   gt cat acct c cccaaagt gt t t acct aat c agt aggt t ca caaact ct t g 12850
   gt cat t at ag t at at gcct a aaat gt at gc act t aggaat gct aaaaat t 12900
   taaatatggt ctaaagcaaa taaaagcaaa gaggaaaaac tttggacagc 12950
   gt aaagact a gaat agt ct t t t aaaaagaa agccagt at a t t ggt t t gaa 13000
   at at agagat gt gt cccaat t t caagt at t t t aat t gcac ct t aat gaaa 13050
   t t at ct at t t t ct at agat t t t agt act at t gaat gt at t act t t act gt 13100
   t acct gaat t t at t at aaag t gt t t t t gaa t aaat aat t c t aaaagc 13147
<210> 4
   <211> 8228
   <212> DNA
   <213> Homo sapiens
<400> 4
   gcctccacac ggctccgtcg ggcgccgcgc tcttccggca gcggtagctt 50
   tggagacgcc gggaacccgc gttggcgtgg ttgactagtg cctcgcagcc 100
   tcagcatggg ggaacatggc ctggagctgg cttccatgat ccccgccctg 150
   cgggagctgg gcagtgccac accagaggaa tataatacag ttgtacagaa 200
   gccaagacaa attctgtgtc aattcattga ccggatactt acagatgtaa 250
   atgttgttgc tgtagaactt gtaaagaaaa ctgactctca gccaacctcc 300
   gtgatgttgc ttgatttcat ccagcatatc atgaaatcct ccccacttat 350
   gtttgtaaat gtgagtggaa gccatgaggc caaaggcagt tgtattgaat 400
   tcagtaattg gatcataacg agacttctgc ggattgcagc aactccctcc 450
   tgtcatttgt tacacaagaa aatctgtgaa gtcatctgtt cattattatt 500
   tctttttaaa agcaagagtc ctgctatttt tggggtactc acaaaagaat 550
   tattacaact ttttgaagac ttggtttacc tccatagaag aaatgtgatg 600
   ggtcatgctg tggaatggcc agtggtcatg agccgatttt taagtcaatt 650
   agatgaacac atgggatatt tacaatcagc tcctttgcag ttgatgagta 700
   tgcaaaattt agaatttatt gaagtcactt tattaatggt tcttactcgt 750
   attattgcaa ttgtgttttt tagaaggcaa gaactcttac tttggcagat 800
   aggttgtgtt ctgctagagt atggtagtcc aaaaattaaa tccctagcaa 850
   ttagcttttt aacagaactt tttcagcttg gaggactacc agcacaacca 900
   gctagcactt ttttcagctc atttttggaa ttattaaaac accttgtaga 950
   aatggatact gaccaattga aactctatga agagccatta tcaaagctga 1000
   taaagacact atttcccttt gaagcagaag cttatagaaa tattgaacct 1050
   gtctatttaa atatgctgct ggaaaaactc tgtgtcatgt ttgaagacgg 1100
   tgtgctcatg cggcttaagt ctgatttgct aaaagcagct ttgtgccatt 1150
   tactgcagta tttccttaaa tttgtgccag ctgggtatga atctgcttta 1200
   caagtcagga aggtctatgt gagaaatatt tgtaaagctc ttttggatgt 1250
   gcttggaatt gaggtagatg cagagtactt gttgggccca ctttatgcag 1300
   ctttgaaaat ggaaagtatg gaaatcattg aggagattca atgccaaact 1350
   caacaggaaa acctcagcag taatagtgat ggaatatcac ccaaaaggcg 1400
   tcgtctcagc tcgtctctaa acccttctaa aagagcacca aaacagactg 1450
   aggaaattaa acatgtggac atgaaccaaa agagcatatt atggagtgca 1500
   ctgaaacaga aagctgaatc ccttcagatt tcccttgaat acagtggcct 1550
   aaagaatcct gttattgaga tgttagaagg aattgctgtt gtcttacaac 1600
   tgactgctct gtgtactgtt cattgttctc atcaaaacat gaactgccgt 1650
   actttcaagg actgtcaaca taaatccaag aagaaacctt ctgtagtgat 1700
   aacttggatg tcattggatt tttacacaaa agtgcttaag agctgtagaa 1750
   gtttgttaga atctgttcag aaactggacc tggaggcaac cattgataag 1800
   gtggtgaaaa tttatgatgc tttgatttat atgcaagtaa acagttcatt 1850
   tgaagatcat atcctggaag atttatgtgg tatgctctca cttccatgga 1900
   tttattccca ttctgatgat ggctgtttaa agttgaccac atttgccgct 1950
   aatcttctaa cattaagctg taggatttca gatagctatt caccacaggc 2000
   acaatcacga tgtgtgtttc ttctgactct gtttccaaga agaatattcc 2050
   ttgagtggag aacagcagtt tacaactggg ccctgcagag ctcccatgaa 2100
   gtaatccggg ctagttgtgt tagtggattt tttatcttat tgcagcagca 2150
   gaattcttgt aacagagttc ccaagattct tatagataaa gtcaaagatg 2200
   attctgacat tgtcaagaaa gaatttgctt ctatacttgg tcaacttgtc 2250
   tgtactcttc acggcatgtt ttatctgaca agttctttaa cagaaccttt 2300
   ctctgaacac ggacatgtgg acctcttctg taggaacttg aaagccactt 2350
   ctcaacatga atgttcatct tctcaactaa aagcttctgt ctgcaagcca 2400
   ttccttttcc tactgaaaaa aaaaatacct agtccagtaa aacttgcttt 2450
   catagataat ctacatcatc tttgtaagca tcttgatttt agagaagatg 2500
   aaacagatgt aaaagcagtt cttggaactt tattaaattt aatggaagat 2550
   ccagacaaag atgttagagt ggcttttagt ggaaatatca agcacatatt 2600
   ggaatccttg gactctgaag atggatttat aaaggagctt tttgtcttaa 2650
   gaatgaagga agcatataca catgcccaaa tatcaagaaa taatgagctg 2700
   aaggatacct tgattcttac aacaggggat attggaaggg ccgcaaaagg 2750
   agatttggta ccatttgcac tcttacactt attgcattgt ttgttatcca 2800
   agtcagcatc tgtctctgga gcagcataca cagaaattag agctctggtt 2850
   gcagctaaaa gtgttaaact gcaaagtttt ttcagccagt ataagaaacc 2900
   catctgtcag tttttggtag aatcccttca ctctagtcag atgacagcac 2950
   ttccgaatac tccatgccag aatgctgacg tgcgaaaaca agatgtggct 3000
   caccagagag aaatggcttt aaatacgttg tctgaaattg ccaacgtttt 3050
   cgactttcct gatcttaatc gttttcttac taggacatta caagttctac 3100
   tacctgatct tgctgccaaa gcaagccctg cagcttctgc tctcattcga 3150
   actttaggaa aacaattaaa tgtcaatcgt agagagattt taataaacaa 3200
   cttcaaatat attttttctc atttggtctg ttcttgttcc aaagatgaat 3250
   tagaacgtgc ccttcattat ctgaagaatg aaacagaaat tgaactgggg 3300
   agcctgttga gacaagattt ccaaggattg cataatgaat tattgctgcg 3350
   tattggagaa cactatcaac aggtttttaa tggtttgtca atacttgcct 3400
   catttgcatc cagtgatgat ccatatcagg gcccgagaga tatcatatca 3450
   cctgaactga tggctgatta tttacaaccc aaattgttgg gcattttggc 3500
   tttttttaac atgcagttac tgagctctag tgttggcatt gaagataaga 3550
   aaatggcctt gaacagtttg atgtctttga tgaagttaat gggacccaaa 3600
   catgtcagtt ctgtgagggt gaagatgatg accacactga gaactggcct 3650
   tcgattcaag gatgattttc ctgaattgtg ttgcagagct tgggactgct 3700
   ttgttcgctg cctggatcat gcttgtctgg gctcccttct cagtcatgta 3750
   atagtagctt tgttacctct tatacacatc cagcctaaag aaactgcagc 3800
   tatcttccac tacctcataa ttgaaaacag ggatgctgtg caagattttc 3850
   ttcatgaaat atatttttta cctgatcatc cagaattaaa aaagataaaa 3900
   gccgttctcc aggaatacag aaaggagacc tctgagagca ctgatcttca 3950
   gacaactctt cagctctcta tgaaggccat tcaacatgaa aatgtcgatg 4000
   ttcgtattca tgctcttaca agcttgaagg aaaccttgta taaaaatcag 4050
   gaaaaactga taaagtatgc aacagacagt gaaacagtag aacctattat 4100
   ctcacagttg gtgacagtgc ttttgaaagg ttgccaagat gcaaactctc 4150
   aagctcggtt gctctgtggg gaatgtttag gggaattggg ggcgatagat 4200
   ccaggtcgat tagatttctc aacaactgaa actcaaggaa aagattttac 4250
   atttgtgact ggagtagaag attcaagctt tgcctatgga ttattgatgg 4300
   agctaacaag agcttacctt gcgtatgctg ataatagccg agctcaagat 4350
   tcagctgcct atgccattca ggagttgctt tctatttatg actgtagaga 4400
   gatggagacc aacggcccag gtcaccaatt gtggaggaga tttcctgagc 4450
   atgttcggga aatactagaa cctcatctaa ataccagata caagagttct 4500
   cagaagtcaa ccgattggtc tggagtaaag aagccaattt acttaagtaa 4550
   attgggtagt aactttgcag aatggtcagc atcttgggca ggttatctta 4600
   ttacaaaggt tcgacatgat cttgccagta aaattttcac ctgctgtagc 4650
   attatgatga agcatgattt caaagtgacc atctatcttc ttccacatat 4700
   tctggtgtat gtcttactgg gttgtaatca agaagatcag caggaggttt 4750
   atgcagaaat tatggcagtt ctaaagcatg acgatcagca taccataaat 4800
   acccaagaca ttgcatctga tctgtgtcaa ctcagtacac agactgtgtt 4850
   ctccatgctt gaccatctca cacagtgggc aaggcacaaa tttcaggcac 4900
   tgaaagctga gaaatgtcca cacagcaaat caaacagaaa taaggtagac 4950
   tcaatggtat ctactgtgga ttatgaagac tatcagagtg taacccgttt 5000
   tctagacctc ataccccagg atactctggc agtagcttcc tttcgctcca 5050
   aagcatacac acgagctgta atgcactttg aatcatttat tacagaaaag 5100
   aagcaaaata ttcaggaaca tcttggattt ttacagaaat tgtatgctgc 5150
   tatgcatgaa cctgatggag tggccggagt cagtgcaatt agaaaggcag 5200
   aaccatctct aaaagaacag atccttgaac atgaaagcct tggcttgctg 5250
   agggatgcca ctgcttgtta tgacagggct attcagctag aaccagacca 5300
   gatcattcat tatcatggtg tagtaaagtc catgttaggt cttggtcagc 5350
   tgtctactgt tatcactcag gtgaatggag tgcatgctaa caggtccgag 5400
   tggacagatg aattaaacac gtacagagtg gaagcagctt ggaaattgtc 5450
   acagtgggat ttggtggaaa actatttggc agcagatgga aaatctacaa 5500
   catggagtgt cagactggga cagctattat tatcagccaa aaaaagagat 5550
   atcacagctt tttatgactc actgaaacta gtgagagcag aacaaattgt 5600
   acctctttca gctgcaagct ttgaaagagg ctcctaccaa cgaggatatg 5650
   aatatattgt gagattgcac atgttatgtg agttggagca tagcatcaaa 5700
   ccacttttcc agcattctcc aggtgacagt tctcaagaag attctctaaa 5750
   ctgggtagct cgactagaaa tgacccagaa ttcctacaga gccaaggagc 5800
   ctatcctggc tctccggagg gctttactaa gcctcaacaa aagaccagat 5850
   tacaatgaaa tggttggaga atgctggctg cagagtgcca gggtagctag 5900
   aaaggctggt caccaccaga cagcctacaa tgctctcctt aatgcagggg 5950
   aatcacgact cgctgaactg tacgtggaaa gggcaaagtg gctctggtcc 6000
   aagggtgatg ttcaccaggc actaattgtt cttcaaaaag gtgttgaatt 6050
   atgttttcct gaaaatgaaa ccccacctga gggtaagaac atgttaatcc 6100
   atggtcgagc tatgctacta gtgggccgat ttatggaaga aacagctaac 6150
   tttgaaagca atgcaattat gaaaaaatat aaggatgtga ccgcgtgcct 6200
   gccagaatgg gaggatgggc atttttacct tgccaagtac tatgacaaat 6250
   tgatgcccat ggtcacagac aacaaaatgg aaaagcaagg tgatctcatc 6300
   cggtatatag ttcttcattt tggcagatct ctacaatatg gaaatcagtt 6350
   catatatcag tcaatgccac gaatgttaac tctatggctt gattatggta 6400
   caaaggcata tgaatgggaa aaagctggcc gctccgatcg tgtacaaatg 6450
   aggaatgatt tgggtaaaat aaacaaggtt atcacagagc atacaaacta 6500
   tttagctcca tatcaatttt tgactgcttt ttcacaattg atctctcgaa 6550
   tttgtcattc tcacgatgaa gtttttgttg tcttgatgga aataatagcc 6600
   aaagtatttc tagcctatcc tcaacaagca atgtggatga tgacagctgt 6650
   gtcaaagtca tcttatccca tgcgtgtgaa cagatgcaag gaaatcctca 6700
   ataaagctat tcatatgaaa aaatccttag agaagtttgt tggagatgca 6750
   actcgcctaa cagataagct tctagaattg tgcaataaac cggttgatgg 6800
   aagtagttcc acattaagca tgagcactca ttttaaaatg cttaaaaagc 6850
   tggtagaaga agcaacattt agtgaaatcc tcattcctct acaatcagtc 6900
   atgataccta cacttccatc aattctgggt acccatgcta accatgctag 6950
   ccatgaacca tttcctggac attgggccta tattgcaggg tttgatgata 7000
   tggtggaaat tcttgcttct cttcagaaac caaagaagat ttctttaaaa 7050
   ggctcagatg gaaagttcta catcatgatg tgtaagccaa aagatgacct 7100
   gagaaaggat tgtagactaa tggaattcaa ttccttgatt aataagtgct 7150
   taagaaaaga tgcagagtct cgtagaagag aacttcatat tcgaacatat 7200
   gcagttattc cactaaatga tgaatgtggg attattgaat gggtgaacaa 7250
   cactgctggt ttgagaccta ttctgaccaa actatataaa gaaaagggag 7300
   tgtatatgac aggaaaagaa cttcgccagt gtatgctacc aaagtcagca 7350
   gctttatctg aaaaactcaa agtattccga gaatttctcc tgcccaggca 7400
   tcctcctatt tttcatgagt ggtttctgag aacattccct gatcctacat 7450
   catggtacag tagtagatca gcttactgcc gttccactgc agtaatgtca 7500
   atggttggtt atattctggg gcttggagac cgtcatggtg aaaatattct 7550
   ctttgattct ttgactggtg aatgcgtaca tgtagatttc aattgtcttt 7600
   tcaataaggg agaaaccttt gaagttccag aaattgtgcc atttcgcctg 7650
   actcataata tggttaatgg aatgggtcct atgggaacag agggtctttt 7700
   tcgaagagca tgtgaagtta caatgaggct gatgcgtgat cagcgagagc 7750
   ctttaatgag tgtcttaaag acttttctac atgatcctct tgtggaatgg 7800
   agtaaaccag tgaaagggca ttccaaagcg ccactgaatg aaactggaga 7850
   agttgtcaat gaaaaggcca agacccatgt tcttgacatt gagcagcgac 7900
   tacaaggtgt aatcaagact cgaaatagag tgacaggact gccgttatct 7950
   attgaaggac atgtgcatta ccttatacag gaagctactg atgaaaactt 8000
   actatgccag atgtatcttg gttggactcc atatatgtga aatgaaatta 8050
   tgtaaaagaa tatgttaata atctaaaagt aatgcatttg gtatgaatct 8100
   gtggttgtat ctgttcaatt ctaaagtaca acataaattt acgttctcag 8150
   caactgttat ttctctctga tcattaatta tatgtaaaat aatatacatt 8200
   cagttattaa gaaataaact gctttctt 8228
<210> 5
   <211> 3603
   <212> DNA
   <213> Homo sapiens
<400> 5
   agtcaaggct ttgacagggc atagtctcct ccaataatct tctccgttct 50
   ctctcattat tccctcgagt tcttctcagt caagctgcat gtatgtatgt 100
   gtgtcccgag aagcggtttg atactgagct gcatttgcct ttactgtgga 150
   gttttgttgc cggttctgct ccctaatctt ccttttctga cgtgcctgag 200
   catgtccaca ttagaatctg tgacatacct acctgaaaaa ggtttatatt 250
   gtcagagact gccaagcagc cggacacacg ggggcacaga atcactgaag 300
   gggaaaaata cagaaaatat gggtttctac ggcacattaa aaatgatttt 350
   ttacaaaatg aaaagaaagt tggaccatgg ttctgaggtc cgctcttttt 400
   ctttgggaaa gaaaccatgc aaagtctcag aatatacaag taccactggg 450
   cttgtaccat gttcagcaac accaacaact tttggggacc tcagagcagc 500
   caatggccaa gggcaacaac gacgccgaat tacatctgtc cagccaccta 550
   caggcctcca ggaatggcta aaaatgtttc agagctggag tggaccagag 600
   aaattgcttg ctttagatga actcattgat agttgtgaac caacacaagt 650
   aaaacatatg atgcaagtga tagaacccca gtttcaacga gacttcattt 700
   cattgctccc taaagagttg gcactctatg tgctttcatt cctggaaccc 750
   aaagacctgc tacaagcagc tcagacatgt cgctactgga gaattttggc 800
   tgaagacaac cttctctgga gagagaaatg caaagaagag gggattgatg 850
   aaccattgca catcaagaga agaaaagtaa taaaaccagg tttcatacac 900
   agtccatgga aaagtgcata catcagacag cacagaattg atactaactg 950
   gaggcgagga gaactcaaat ctcctaaggt gctgaaagga catgatgatc 1000
   atgtgatcac atgcttacag ttttgtggta accgaatagt tagtggttct 1050
   gatgacaaca ctttaaaagt ttggtcagca gtcacaggca aatgtctgag 1100
   aacattagtg ggacatacag gtggagtatg gtcatcacaa atgagagaca 1150
   acatcatcat tagtggatct acagatcgga cactcaaagt gtggaatgca 1200
   gagactggag aatgtataca caccttatat gggcatactt ccactgtgcg 1250
   ttgtatgcat cttcatgaaa aaagagttgt tagcggttct cgagatgcca 1300
   ctcttagggt ttgggatatt gagacaggcc agtgtttaca tgttttgatg 1350
   ggtcatgttg cagcagtccg ctgtgttcaa tatgatggca ggagggttgt 1400
   tagtggagca tatgatttta tggtaaaggt gtgggatcca gagactgaaa 1450
   cctgtctaca cacgttgcag gggcatacta atagagtcta ttcattacag 1500
   tttgatggta tccatgtggt gagtggatct cttgatacat caatccgtgt 1550
   ttgggatgtg gagacaggga attgcattca cacgttaaca gggcaccagt 1600
   cgttaacaag tggaatggaa ctcaaagaca atattcttgt ctctgggaat 1650
   gcagattcta cagttaaaat ctgggatatc aaaacaggac agtgtttaca 1700
   aacattgcaa ggtcccaaca agcatcagag tgctgtgacc tgtttacagt 1750
   tcaacaagaa ctttgtaatt accagctcag atgatggaac tgtaaaacta 1800
   tgggacttga aaacgggtga atttattcga aacctagtca cattggagag 1850
   tggggggagt gggggagttg tgtggcggat cagagcctca aacacaaagc 1900
   tggtgtgtgc agttgggagt cggaatggga ctgaagaaac caagctgctg 1950
   gtgctggact ttgatgtgga catgaagtga agagcagaaa agatgaattt 2000
   gtccaattgt gtagacgata tactccctgc ccttccccct gcaaaaagaa 2050
   aaaaagaaaa gaaaaagaaa aaaatccctt gttctcagtg gtgcaggatg 2100
   ttggcttggg gcaacagatt gaaaagacct acagactaag aaggaaaaga 2150
   agaagagatg acaaaccata actgacaaga gaggcgtctg ctgtctcatc 2200
   acat aaaagg ct t cact t t t gact gagggc agct t t gcaa aat gagact t 2250
   t ct aaat caa accaggt gca at t at t t ct t t at t t t ct t c t ccagt ggt c 2300
   at t gggcagt gt t aat gct g aaacat cat t acagat t ct g ct agcct gt t 2350
   ct t t t accac t gacagct ag acacct agaa aggaact gca at aat at caa 2400
   aacaagt act ggt t gact t t ct aat t agag agcat ct gca acaaaaagt c 2450
   at t t t t ct gg agt ggaaaag ct t aaaaaaa t t act gt gaa t t gt t t t t gt 2500
   acagttatca tgaaaagctt tttttttttt ttttttgcca accattgcca 2550
   at gt caat ca at cacagt at t agcct ct gt t aat ct at t t act gt t gct t 2600
   ccat at acat t ct t caat gc at at gt t gct caaaggt ggc aagt t gt cct 2650
   gggt t ct gt g agt cct gaga t ggat t t aat t ct t gat gct ggt gct agaa 2700
   gt aggt ct t c aaat at ggga t t gt t gt ccc aaccct gt ac t gt act ccca 2750
   gtggccaaac ttatttatgc tgctaaatga aagaaagaaa aaagcaaatt 2800
   at t t t t t t t t at t t t t t t t c t gct gt gacg t t t t agt ccc agact gaat t 2850
   ccaaat t t gc t ct agt t t gg t t at ggaaaa aagact t t t t gccact gaaa 2900
   ct t gagccat ct gt gcct ct aagaggct ga gaat ggaaga gt t t cagat a 2950
   at aaagagt g aagt t t gcct gcaagt aaag aat t gagagt gt gt gcaaag 3000
   ct t at t t t ct t t t at ct ggg caaaaat t aa aacacat t cc t t ggaacaga 3050
   gct at t act t gcct gt t ct g t ggagaaact t t t ct t t t t g agggct gt gg 3100
   t gaat ggat g aacgt acat c gt aaaact ga caaaat at t t t aaaaat at a 3150
   t aaaacacaa aat t aaaat a aagt t gct gg t cagt ct t ag t gt t t t acag 3200
   t at t t gggaa aacaact gt t acagt t t t at t gct ct gagt aact gacaaa 3250
   gcagaaacta ttcagttttt gtagtaaagg cgtcacatgc aaacaaacaa 3300
   aatgaatgaa acagtcaaat ggtttgcctc attctccaag agccacaact 3350
   caagct gaac t gt gaaagt g gt t t aacact gt at cct agg cgat ct t t t t 3400
   t cct cct t ct gt t t at t t t t t t gt t t gt t t t at t t at agt ct gat t t aaa 3450
   acaat cagat t caagt t ggt t aat t t t agt t at gt aacaa cct gacat ga 3500
   t ggaggaaaa caacct t t aa agggat t gt g t ct at ggt t t gat t cact t a 3550
   gaaat t t t at t t t ct t at aa ct t aagt gca at aaaat gt g t t t t t t cat g 3600
   t t a 3603
<210> 6
   <211> 5783
   <212> DNA
   <213> Homo sapiens
<400> 6
   gcggacactc ctctcggctc ctccccggca gcggcggcgg ctcggagcgg 50
   gctccggggc tcgggtgcag cggccagcgg gcgcctggcg gcgaggatta 100
   cccggggaag tggttgtctc ctggctggag ccgcgagacg ggcgctcagg 150
   gcgcggggcc ggcggcggcg aacgagagga cggactctgg cggccgggtc 200
   gttggccgcg gggagcgcgg gcaccgggcg agcaggccgc gtcgcgctca 250
   ccatggtcag ctactgggac accggggtcc tgctgtgcgc gctgctcagc 300
   tgtctgcttc tcacaggatc tagttcaggt tcaaaattaa aagatcctga 350
   actgagttta aaaggcaccc agcacatcat gcaagcaggc cagacactgc 400
   atctccaatg caggggggaa gcagcccata aatggtcttt gcctgaaatg 450
   gtgagtaagg aaagcgaaag gctgagcata actaaatctg cctgtggaag 500
   aaatggcaaa caattctgca gtactttaac cttgaacaca gctcaagcaa 550
   accacactgg cttctacagc tgcaaatatc tagctgtacc tacttcaaag 600
   aagaaggaaa cagaatctgc aatctatata tttattagtg atacaggtag 650
   acctttcgta gagatgtaca gtgaaatccc cgaaattata cacatgactg 700
   aaggaaggga gctcgtcatt ccctgccggg ttacgtcacc taacatcact 750
   gttactttaa aaaagtttcc acttgacact ttgatccctg atggaaaacg 800
   cataatctgg gacagtagaa agggcttcat catatcaaat gcaacgtaca 850
   aagaaatagg gcttctgacc tgtgaagcaa cagtcaatgg gcatttgtat 900
   aagacaaact atctcacaca tcgacaaacc aatacaatca tagatgtcca 950
   aataagcaca ccacgcccag tcaaattact tagaggccat actcttgtcc 1000
   tcaattgtac tgctaccact cccttgaaca cgagagttca aatgacctgg 1050
   agttaccctg atgaaaaaaa taagagagct tccgtaaggc gacgaattga 1100
   ccaaagcaat tcccatgcca acatattcta cagtgttctt actattgaca 1150
   aaatgcagaa caaagacaaa ggactttata cttgtcgtgt aaggagtgga 1200
   ccatcattca aatctgttaa cacctcagtg catatatatg ataaagcatt 1250
   catcactgtg aaacatcgaa aacagcaggt gcttgaaacc gtagctggca 1300
   agcggtctta ccggctctct atgaaagtga aggcatttcc ctcgccggaa 1350
   gttgtatggt taaaagatgg gttacctgcg actgagaaat ctgctcgcta 1400
   tttgactcgt ggctactcgt taattatcaa ggacgtaact gaagaggatg 1450
   cagggaatta tacaatcttg ctgagcataa aacagtcaaa tgtgtttaaa 1500
   aacctcactg ccactctaat tgtcaatgtg aaaccccaga tttacgaaaa 1550
   ggccgtgtca tcgtttccag acccggctct ctacccactg ggcagcagac 1600
   aaatcctgac ttgtaccgca tatggtatcc ctcaacctac aatcaagtgg 1650
   ttctggcacc cctgtaacca taatcattcc gaagcaaggt gtgacttttg 1700
   ttccaataat gaagagtcct ttatcctgga tgctgacagc aacatgggaa 1750
   acagaattga gagcatcact cagcgcatgg caataataga aggaaagaat 1800
   aagatggcta gcaccttggt tgtggctgac tctagaattt ctggaatcta 1850
   catttgcata gcttccaata aagttgggac tgtgggaaga aacataagct 1900
   tttatatcac agatgtgcca aatgggtttc atgttaactt ggaaaaaatg 1950
   ccgacggaag gagaggacct gaaactgtct tgcacagtta acaagttctt 2000
   atacagagac gttacttgga ttttactgcg gacagttaat aacagaacaa 2050
   tgcactacag tattagcaag caaaaaatgg ccatcactaa ggagcactcc 2100
   atcactctta atcttaccat catgaatgtt tccctgcaag attcaggcac 2150
   ctatgcctgc agagccagga atgtatacac aggggaagaa atcctccaga 2200
   agaaagaaat tacaatcaga gatcaggaag caccatacct cctgcgaaac 2250
   ctcagtgatc acacagtggc catcagcagt tccaccactt tagactgtca 2300
   tgctaatggt gtccccgagc ctcagatcac ttggtttaaa aacaaccaca 2350
   aaatacaaca agagcctgga attattttag gaccaggaag cagcacgctg 2400
   tttattgaaa gagtcacaga agaggatgaa ggtgtctatc actgcaaagc 2450
   caccaaccag aagggctctg tggaaagttc agcatacctc actgttcaag 2500
   gaacctcgga caagtctaat ctggagctga tcactctaac atgcacctgt 2550
   gtggctgcga ctctcttctg gctcctatta accctcttta tccgaaaaat 2600
   gaaaaggtct tcttctgaaa taaagactga ctacctatca attataatgg 2650
   acccagatga agttcctttg gatgagcagt gtgagcggct cccttatgat 2700
   gccagcaagt gggagtttgc ccgggagaga cttaaactgg gcaaatcact 2750
   tggaagaggg gcttttggaa aagtggttca agcatcagca tttggcatta 2800
   agaaatcacc tacgtgccgg actgtggctg tgaaaatgct gaaagagggg 2850
   gccacggcca gcgagtacaa agctctgatg actgagctaa aaatcttgac 2900
   ccacattggc caccatctga acgtggttaa cctgctggga gcctgcacca 2950
   agcaaggagg gcctctgatg gtgattgttg aatactgcaa atatggaaat 3000
   ctctccaact acctcaagag caaacgtgac ttattttttc tcaacaagga 3050
   tgcagcacta cacatggagc ctaagaaaga aaaaatggag ccaggcctgg 3100
   aacaaggcaa gaaaccaaga ctagatagcg tcaccagcag cgaaagcttt 3150
   gcgagctccg gctttcagga agataaaagt ctgagtgatg ttgaggaaga 3200
   ggaggattct gacggtttct acaaggagcc catcactatg gaagatctga 3250
   tttcttacag ttttcaagtg gccagaggca tggagttcct gtcttccaga 3300
   aagtgcattc atcgggacct ggcagcgaga aacattcttt tatctgagaa 3350
   caacgtggtg aagatttgtg attttggcct tgcccgggat atttataaga 3400
   accccgatta tgtgagaaaa ggagatactc gacttcctct gaaatggatg 3450
   gctcctgaat ctatctttga caaaatctac agcaccaaga gcgacgtgtg 3500
   gtcttacgga gtattgctgt gggaaatctt ctccttaggt gggtctccat 3550
   acccaggagt acaaatggat gaggactttt gcagtcgcct gagggaaggc 3600
   atgaggatga gagctcctga gtactctact cctgaaatct atcagatcat 3650
   gctggactgc tggcacagag acccaaaaga aaggccaaga tttgcagaac 3700
   ttgtggaaaa actaggtgat ttgcttcaag caaatgtaca acaggatggt 3750
   aaagactaca tcccaatcaa tgccatactg acaggaaata gtgggtttac 3800
   atactcaact cctgccttct ctgaggactt cttcaaggaa agtatttcag 3850
   ctccgaagtt taattcagga agctctgatg atgtcagata cgtaaatgct 3900
   ttcaagttca tgagcctgga aagaatcaaa acctttgaag aacttttacc 3950
   gaatgccacc tccatgtttg atgactacca gggcgacagc agcactctgt 4000
   tggcctctcc catgctgaag cgcttcacct ggactgacag caaacccaag 4050
   gcctcgctca agattgactt gagagtaacc agtaaaagta aggagtcggg 4100
   gctgtctgat gtcagcaggc ccagtttctg ccattccagc tgtgggcacg 4150
   tcagcgaagg caagcgcagg ttcacctacg accacgctga gctggaaagg 4200
   aaaatcgcgt gctgctcccc gcccccagac tacaactcgg tggtcctgta 4250
   ctccacccca cccatctaga gtttgacacg aagccttatt tctagaagca 4300
   catgtgtatt tataccccca ggaaactagc ttttgccagt attatgcata 4350
   tataagttta cacctttatc tttccatggg agccagctgc tttttgtgat 4400
   ttttttaata gtgctttttt ttttttgact aacaagaatg taactccaga 4450
   tagagaaata gtgacaagtg aagaacacta ctgctaaatc ctcatgttac 4500
   tcagtgttag agaaatcctt cctaaaccca atgacttccc tgctccaacc 4550
   cccgccacct cagggcacgc aggaccagtt tgattgagga gctgcactga 4600
   tcacccaatg catcacgtac cccactgggc cagccctgca gcccaaaacc 4650
   cagggcaaca agcccgttag ccccagggat cactggctgg cctgagcaac 4700
   atctcgggag tcctctagca ggcctaagac atgtgaggag gaaaaggaaa 4750
   aaaagcaaaa agcaagggag aaaagagaaa ccgggagaag gcatgagaaa 4800
   gaatttgaga cgcaccatgt gggcacggag gggacggggg ctcagcaatg 4850
   ccatttcagt ggcttcccag ctctgaccct tctacatttg agggcccagc 4900
   caggagcaga tggacagcga tgaggggaca ttttctggat tctgggaggc 4950
   aagaaaagga caaatatctt ttttggaact aaagcaaatt ttagaacttt 5000
   acctatggaa gtggttctat gtccattctc attcgtggca tgttttgatt 5050
   tgtagcactg agggtggcac tcaactctga gcccatactt ttggctcctc 5100
   tagtaagatg cactgaaaac ttagccagag ttaggttgtc tccaggccat 5150
   gatggcctta cactgaaaat gtcacattct attttgggta ttaatatata 5200
   gtccagacac ttaactcaat ttcttggtat tattctgttt tgcacagtta 5250
   gttgtgaaag aaagctgaga agaatgaaaa tgcagtcctg aggagaggag 5300
   ttttctccat atcaaaacga gggctgatgg aggaaaaagg tcaataaggt 5350
   caagggaaaa ccccgtctct ataccaacca aaccaattca ccaacacagt 5400
   tgggacccaa aacacaggaa gtcagtcacg tttccttttc atttaatggg 5450
   gat t ccact a t ct cacact a at ct gaaagg at gt ggaaga gcat t agct g 5500
   gcgcat at t a agcact t t aa gct cct t gag t aaaaaggt g gt at gt aat t 5550
   t at gcaaggt at t t ct ccag t t gggact ca ggat at t agt t aat gagcca 5600
   tcactagaag aaaagcccat tttcaactgc tttgaaactt gcctggggtc 5650
   tgagcatgat gggaataggg agacagggta ggaaagggcg cctactcttc 5700
   agggt ct aaa gat caagt gg gcct t ggat c gct aagct gg ct ct gt t t ga 5750
   t gct at t t at gcaagt t agg gt ct at gt at t t a 5783
<210> 7
   <211> 3496
   <212> DNA
   <213> Homo sapiens
<400> 7
   acctgcagcg cgaggcgcgc cgctccaggc ggcatcgcag ggctgggccg 50
   gcgcggcctg gggaccccgg gctccggagg ccatgccggc gttggcgcgc 100
   gacggcggcc agctgccgct gctcgttgtt ttttctgcaa tgatatttgg 150
   gactattaca aatcaagatc tgcctgtgat caagtgtgtt ttaatcaatc 200
   ataagaacaa tgattcatca gtggggaagt catcatcata tcccatggta 250
   tcagaatccc cggaagacct cgggtgtgcg ttgagacccc agagctcagg 300
   gacagtgtac gaagctgccg ctgtggaagt ggatgtatct gcttccatca 350
   cactgcaagt gctggtcgac gccccaggga acatttcctg tctctgggtc 400
   tttaagcaca gctccctgaa ttgccagcca cattttgatt tacaaaacag 450
   aggagttgtt tccatggtca ttttgaaaat gacagaaacc caagctggag 500
   aatacctact ttttattcag agtgaagcta ccaattacac aatattgttt 550
   acagtgagta taagaaatac cctgctttac acattaagaa gaccttactt 600
   tagaaaaatg gaaaaccagg acgccctggt ctgcatatct gagagcgttc 650
   cagagccgat cgtggaatgg gtgctttgcg attcacaggg ggaaagctgt 700
   aaagaagaaa gtccagctgt tgttaaaaag gaggaaaaag tgcttcatga 750
   attatttggg acggacataa ggtgctgtgc cagaaatgaa ctgggcaggg 800
   aatgcaccag gctgttcaca atagatctaa atcaaactcc tcagaccaca 850
   ttgccacaat tatttcttaa agtaggggaa cccttatgga taaggtgcaa 900
   agctgttcat gtgaaccatg gattcgggct cacctgggaa ttagaaaaca 950
   aagcactcga ggagggcaac tactttgaga tgagtaccta ttcaacaaac 1000
   agaactatga tacggattct gtttgctttt gtatcatcag tggcaagaaa 1050
   cgacaccgga tactacactt gttcctcttc aaagcatccc agtcaatcag 1100
   ctttggttac catcgtagaa aagggattta taaatgctac caattcaagt 1150
   gaagattatg aaattgacca atatgaagag ttttgttttt ctgtcaggtt 1200
   taaagcctac ccacaaatca gatgtacgtg gaccttctct cgaaaatcat 1250
   ttccttgtga gcaaaagggt cttgataacg gatacagcat atccaagttt 1300
   tgcaatcata agcaccagcc aggagaatat atattccatg cagaaaatga 1350
   tgatgcccaa tttaccaaaa tgttcacgct gaatataaga aggaaacctc 1400
   aagtgctcgc agaagcatcg gcaagtcagg cgtcctgttt ctcggatgga 1450
   tacccattac catcttggac ctggaagaag tgttcagaca agtctcccaa 1500
   ctgcacagaa gagatcacag aaggagtctg gaatagaaag gctaacagaa 1550
   aagtgtttgg acagtgggtg tcgagcagta ctctaaacat gagtgaagcc 1600
   ataaaagggt tcctggtcaa gtgctgtgca tacaattccc ttggcacatc 1650
   ttgtgagacg atccttttaa actctccagg ccccttccct ttcatccaag 1700
   acaacatctc attctatgca acaattggtg tttgtctcct cttcattgtc 1750
   gttttaaccc tgctaatttg tcacaagtac aaaaagcaat ttaggtatga 1800
   aagccagcta cagatggtac aggtgaccgg ctcctcagat aatgagtact 1850
   tctacgttga tttcagagaa tatgaatatg atctcaaatg ggagtttcca 1900
   agagaaaatt tagagtttgg gaaggtacta ggatcaggtg cttttggaaa 1950
   agtgatgaac gcaacagctt atggaattag caaaacagga gtctcaatcc 2000
   aggttgccgt caaaatgctg aaagaaaaag cagacagctc tgaaagagag 2050
   gcactcatgt cagaactcaa gatgatgacc cagctgggaa gccacgagaa 2100
   tattgtgaac ctgctggggg cgtgcacact gtcaggacca atttacttga 2150
   tttttgaata ctgttgctat ggtgatcttc tcaactatct aagaagtaaa 2200
   agagaaaaat ttcacaggac ttggacagag attttcaagg aacacaattt 2250
   cagtttttac cccactttcc aatcacatcc aaattccagc atgcctggtt 2300
   caagagaagt tcagatacac ccggactcgg atcaaatctc agggcttcat 2350
   gggaattcat ttcactctga agatgaaatt gaatatgaaa accaaaaaag 2400
   gctggaagaa gaggaggact tgaatgtgct tacatttgaa gatcttcttt 2450
   gctttgcata tcaagttgcc aaaggaatgg aatttctgga atttaagtcg 2500
   tgtgttcaca gagacctggc cgccaggaac gtgcttgtca cccacgggaa 2550
   agtggtgaag atatgtgact ttggattggc tcgagatatc atgagtgatt 2600
   ccaactatgt tgtcaggggc aatgcccgtc tgcctgtaaa atggatggcc 2650
   cccgaaagcc tgtttgaagg catctacacc attaagagtg atgtctggtc 2700
   atatggaata ttactgtggg aaatcttctc acttggtgtg aatccttacc 2750
   ctggcattcc ggttgatgct aacttctaca aactgattca aaatggattt 2800
   aaaatggatc agccatttta tgctacagaa gaaatataca ttataatgca 2850
   atcctgctgg gcttttgact caaggaaacg gccatccttc cctaatttga 2900
   cttcgttttt aggatgtcag ctggcagatg cagaagaagc gatgtatcag 2950
   aatgtggatg gccgtgtttc ggaatgtcct cacacctacc aaaacaggcg 3000
   acctttcagc agagagatgg atttggggct actctctccg caggctcagg 3050
   tcgaagattc gtagaggaac aatttagttt taaggacttc atccctccac 3100
   ctatccctaa caggctgtag attaccaaaa caagattaat ttcatcacta 3150
   aaagaaaatc tattatcaac tgctgcttca ccagactttt ctctagaagc 3200
   tgtctgcgtt tactcttgtt ttcaaaggga cttttgtaaa atcaaatcat 3250
   cctgtcacaa ggcaggagga gctgataatg aactttattg gagcattgat 3300
   ctgcatccaa ggccttctca ggctggcttg agtgaattgt gtacctgaag 3350
   tacagtatat tcttgtaaat acataaaaca aaagcatttt gctaaggaga 3400
   agctaatatg attttttaag tctatgtttt aaaataatat gtaaattttt 3450
   cagctattta gtgatatatt ttatgggtgg gaataaaatt tctact 3496
<210> 8
   <211> 8707
   <212> DNA
   <213> Homo sapiens
<400> 8
   gcggggaagg cgggcggtgg ggcagggggc ctgaagcggc ggtaccggtg 50
   ctggcggcgg cagctgaggc cttggccgaa gccgcgcgaa cctcagggca 100
   agatgcttgg aaccggacct gccgccgcca ccaccgctgc caccacatct 150
   agcaatgtga gcgtcctgca gcagtttgcc agtggcctaa agagccggaa 200
   tgaggaaacc agggccaaag ccgccaagga gctccagcac tatgtcacca 250
   tggaactccg agagatgagt caagaggagt ctactcgctt ctatgaccaa 300
   ctgaaccatc acatttttga attggtttcc agctcagatg ccaatgagag 350
   gaaaggtggc atcttggcca tagctagcct cataggagtg gaaggtggga 400
   atgccacccg aattggcaga tttgccaact atcttcggaa cctcctcccc 450
   tccaatgacc cagttgtcat ggaaatggca tccaaggcca ttggccgtct 500
   tgccatggca ggggacactt ttaccgctga gtacgtggaa tttgaggtga 550
   agcgagccct ggaatggctg ggtgctgacc gcaatgaggg ccggagacat 600
   gcagctgtcc tggttctccg tgagctggcc atcagcgtcc ctaccttctt 650
   cttccagcaa gtgcaaccct tctttgacaa catttttgtg gccgtgtggg 700
   accccaaaca ggccatccgt gagggagctg tagccgccct tcgtgcctgt 750
   ctgattctca caacccagcg tgagccgaag gagatgcaga agcctcagtg 800
   gtacaggcac acatttgaag aagcagagaa gggatttgat gagaccttgg 850
   ccaaagagaa gggcatgaat cgggatgatc ggatccatgg agccttgttg 900
   atccttaacg agctggtccg aatcagcagc atggagggag agcgtctgag 950
   agaagaaatg gaagaaatca cacagcagca gctggtacac gacaagtact 1000
   gcaaagatct catgggcttc ggaacaaaac ctcgtcacat tacccccttc 1050
   accagtttcc aggctgtaca gccccagcag tcaaatgcct tggtggggct 1100
   gctggggtac agctctcacc aaggcctcat gggatttggg acctccccca 1150
   gtccagctaa gtccaccctg gtggagagcc ggtgttgcag agacttgatg 1200
   gaggagaaat ttgatcaggt gtgccagtgg gtgctgaaat gcaggaatag 1250
   caagaactcg ctgatccaaa tgacaatcct taatttgttg ccccgcttgg 1300
   ctgcattccg accttctgcc ttcacagata cccagtatct ccaagatacc 1350
   atgaaccatg tcctaagctg tgtcaagaag gagaaggaac gtacagcggc 1400
   cttccaagcc ctggggctac tttctgtggc tgtgaggtct gagtttaagg 1450
   tctatttgcc tcgcgtgctg gacatcatcc gagcggccct gcccccaaag 1500
   gacttcgccc ataagaggca gaaggcaatg caggtggatg ccacagtctt 1550
   cacttgcatc agcatgctgg ctcgagcaat ggggccaggc atccagcagg 1600
   atatcaagga gctgctggag cccatgctgg cagtgggact aagccctgcc 1650
   ctcactgcag tgctctacga cctgagccgt cagattccac agctaaagaa 1700
   ggacattcaa gatgggctac tgaaaatgct gtccctggtc cttatgcaca 1750
   aaccccttcg ccacccaggc atgcccaagg cctggcccat tcagctggcc 1800
   tctcctggcc tcacgaccct ccctgaggcc agcgatgtgg gcagcatcac 1850
   tcttgccctc cgaacgcttg gcagctttga atttgaaggc cactctctga 1900
   cccaatttgt tcgccactgt gcggatcatt tcctgaacag tgagcacaag 1950
   gagatccgca tggaggctgc ccgcacctgc tcccgcctgc tcacaccctc 2000
   catccacctc atcagtggcc atgctcatgt ggttagccag accgcagtgc 2050
   aagtggtggc agatgtgctt agcaaactgc tcgtagttgg gataacagat 2100
   cctgaccctg acattcgcta ctgtgtcttg gcgtccctgg acgagcgctt 2150
   tgatgcacac ctggcccagg cggagaactt gcaggccttg tttgtggctc 2200
   tgaatgacca ggtgtttgag atccgggagc tggccatctg cactgtgggc 2250
   cgactcagta gcatgaaccc tgcctttgtc atgcctttcc tgcgcaagat 2300
   gctcatccag attttgacag agttggagca cagtgggatt ggaagaatca 2350
   aagagcagag tgcccgcatg ctggggcacc tggtctccaa tgccccccga 2400
   ctcatccgcc cctacatgga gcctattctg aaggcattaa ttttgaaact 2450
   gaaagatcca gaccctgatc caaacccagg tgtgatcaat aatgtcctgg 2500
   caacaatagg agaattggca caggttagtg gcctggaaat gaggaaatgg 2550
   gttgatgaac tttttattat catcatggac atgctccagg attcctcttt 2600
   gt t ggccaaa aggcaggt gg ct ct gt ggac cct gggacag t t ggt ggcca 2650
   gcact ggct a t gt agt agag ccct acagga agt accct ac t t t gct t gag 2700
   gtgctactga attttctgaa gactgagcag aaccagggta cacgcagaga 2750
   ggccat ccgt gt gt t agggc t t t t aggggc t t t ggat cct t acaagcaca 2800
   aagt gaacat t ggcat gat a gaccagt ccc gggat gcct c t gct gt cagc 2850
   ct gt cagaat ccaagt caag t caggat t cc t ct gact at a gcact agt ga 2900
   aat gct ggt c aacat gggaa act t gcct ct ggat gagt t c t acccagct g 2950
   t gt ccat ggt ggccct gat g cggat ct t cc gagaccagt c act ct ct cat 3000
   cat cacacca t ggt t gt cca ggccat cacc t t cat ct t ca agt ccct ggg 3050
   act caaat gt gt gcagt t cc t gccccaggt cat gcccacg t t cct t aacg 3100
   t cat t cgagt ct gt gat ggg gccat ccggg aat t t t t gt t ccagcagct g 3150
   ggaatgttgg tgtcctttgt gaagagccac atcagacctt atatggatga 3200
   aat agt cacc ct cat gagag aat t ct gggt cat gaacacc t caat t caga 3250
   gcacgat cat t ct t ct cat t gagcaaat t g t ggt agct ct t gggggt gaa 3300
   t t t aagct ct acct gcccca gct gat ccca cacat gct gc gt gt ct t cat 3350
   gcatgacaac agcccaggcc gcattgtctc tatcaagtta ctggctgcaa 3400
   t ccagct gt t t ggcgccaac ct ggat gact acct gcat t t act gct gcct 3450
   cct at t gt t a agt t gt t t ga t gcccct gaa gct ccact gc cat ct cgaaa 3500
   ggcagcgct a gagact gt gg accgcct gac ggagt ccct g gat t t cact g 3550
   actatgcctc ccggatcatt caccctattg ttcgaacact ggaccagagc 3600
   ccagaact gc gct ccacagc cat ggacacg ct gt ct t cac t t gt t t t t ca 3650
   gctggggaag aagtaccaaa ttttcattcc aatggtgaat aaagttctgg 3700
   tgcgacaccg aatcaatcat cagcgctatg atgtgctcat ctgcagaatt 3750
   gt caagggat acacact t gc t gat gaagag gaggat cct t t gat t t acca 3800
   gcatcggatg cttaggagtg gccaagggga tgcattggct agtggaccag 3850
   t ggaaacagg acccat gaag aaact gcacg t cagcaccat caacct ccaa 3900
   aaggcaaccg cctggggcgc tgccaggagg gtctccaaag atgactggct 3950
   ggaat ggct g agacggct ga gcct ggagct gct gaaggac t cat cat cgc 4000
   cct ccct gcg ct cct gct gg gccct ggcac aggcct acaa cccgat ggcc 4050
   agggat ct ct t caat gct gc at t t gt gt cc t gct ggt ct g aact gaat ga 4100
   agat caacag gat gagct ca t cagaagcat cgagt t ggcc ct cacct cac 4150
   aagacat cgc t gaagt caca cagaccct ct t aaact t ggc t gaat t cat g 4200
   gaacacagtg acaagggccc cctgccactg agagatgaca atggcattgt 4250
   t ct gct gggt gagagagct g ccaagt gccg agcat at gcc aaagcact ac 4300
   actacaaaga actggagttc cagaaaggcc ccacccctgc cattctagaa 4350
   tctctcatca gcattaataa taagctacag cagccggagg cagcggccgg 4400
   agt gt t agaa t at gccat ga aacact t t gg agagct ggag at ccaggct a 4450
   cct ggt at ga gaaact gcac gagt gggagg at gccct t gt ggcct at gac 4500
   aagaaaatgg acaccaacaa ggacgaccca gagctgatgc tgggccgcat 4550
   gcgct gcct c gaggcct t gg gggaat gggg t caact ccac cagcagt gct 4600
   gtgaaaagtg gaccctggtt aatgatgaga cccaagccaa gatggcccgg 4650
   at ggct gct g cagct gcat g gggt t t aggt cagt gggaca gcat ggaaga 4700
   atacacctgt atgatccctc gggacaccca tgatggggca ttttatagag 4750
   ct gt gct ggc act gcat cag gacct ct t ct cct t ggcaca acagt gcat t 4800
   gacaaggcca gggacctgct ggatgctgaa ttaactgcga tggcaggaga 4850
   gagt t acagt cgggcat at g gggccat ggt t t ct t gccac at gct gt ccg 4900
   agct ggagga ggt t at ccag t acaaact t g t ccccgagcg acgagagat c 4950
   at ccgccaga t ct ggt ggga gagact gcag ggct gccagc gt at cgt aga 5000
   ggact ggcag aaaat cct t a t ggt gcggt c cct t gt ggt c agccct cat g 5050
   aagacat gag aacct ggct c aagt at gcaa gcct gt gcgg caagagt ggc 5100
   aggct ggct c t t gct cat aa aact t t agt g t t gct cct gg gagt t gat cc 5150
   gt ct cggcaa ct t gaccat c ct ct gccaac agt t caccct caggt gacct 5200
   atgcctacat gaaaaacatg tggaagagtg cccgcaagat cgatgccttc 5250
   cagcacatgc agcattttgt ccagaccatg cagcaacagg cccagcatgc 5300
   catcgctact gaggaccagc agcataagca ggaactgcac aagctcatgg 5350
   cccgat gct t cct gaaact t ggagagt ggc agct gaat ct acagggcat c 5400
   aatgagagca caatccccaa agtgctgcag tactacagcg ccgccacaga 5450
   gcacgaccgc agctggtaca aggcctggca tgcgtgggca gtgatgaact 5500
   tcgaagctgt gctacactac aaacatcaga accaagcccg cgatgagaag 5550
   aagaaactgc gtcatgccag cggggccaac atcaccaacg ccaccactgc 5600
   cgccaccacg gccgccactg ccaccaccac tgccagcacc gagggcagca 5650
   acagtgagag cgaggccgag agcaccgaga acagccccac cccatcgccg 5700
   ct gcagaaga aggt cact ga ggat ct gt cc aaaaccct cc t gat gt acac 5750
   ggt gcct gcc gt ccagggct t ct t ccgt t c cat ct cct t g t cacgaggca 5800
   acaacct cca ggat acact c agagt t ct ca cct t at ggt t t gat t at ggt 5850
   cact ggccag at gt caat ga ggcct t agt g gagggggt ga aagccat cca 5900
   gat t gat acc t ggct acagg t t at acct ca gct cat t gca agaat t gat a 5950
   cgcccagacc cttggtggga cgtctcattc accagcttct cacagacatt 6000
   ggtcggtacc acccccaggc cctcatctac ccactgacag tggcttctaa 6050
   gtctaccacg acagcccggc acaatgcagc caacaagatt ctgaagaaca 6100
   tgtgtgagca cagcaacacc ctggtccagc aggccatgat ggtgagcgag 6150
   gagctgatcc gagtggccat cctctggcat gagatgtggc atgaaggcct 6200
   ggaagaggca tctcgtttgt actttgggga aaggaacgtg aaaggcatgt 6250
   ttgaggtgct ggagcccttg catgctatga tggaacgggg cccccagact 6300
   ctgaaggaaa catcctttaa tcaggcctat ggtcgagatt taatggaggc 6350
   ccaagagtgg tgcaggaagt acatgaaatc agggaatgtc aaggacctca 6400
   cccaagcctg ggacctctat tatcatgtgt tccgacgaat ctcaaagcag 6450
   ctgcctcagc tcacatcctt agagctgcaa tatgtttccc caaaacttct 6500
   gatgtgccgg gaccttgaat tggctgtgcc aggaacatat gaccccaacc 6550
   agccaatcat tcgcattcag tccatagcac cgtctttgca agtcatcaca 6600
   tccaagcaga ggccccggaa attgacactt atgggcagca acggacatga 6650
   gtttgttttc cttctaaaag gccatgaaga tctgcgccag gatgagcgtg 6700
   tgatgcagct cttcggcctg gttaacaccc ttctggccaa tgacccaaca 6750
   tctcttcgga aaaacctcag catccagaga tacgctgtca tccctttatc 6800
   gaccaactcg ggcctcattg gctgggttcc ccactgtgac acactgcacg 6850
   ccctcatccg ggactacagg gagaagaaga agatccttct caacatcgag 6900
   catcgcatca tgttgcggat ggctccggac tatgaccact tgactctgat 6950
   gcagaaggtg gaggtgtttg agcatgccgt caataataca gctggggacg 7000
   acctggccaa gctgctgtgg ctgaaaagcc ccagctccga ggtgtggttt 7050
   gaccgaagaa ccaattatac ccgttcttta gcggtcatgt caatggttgg 7100
   gtatatttta ggcctgggag atagacaccc atccaacctg atgctggacc 7150
   gtctgagtgg gaagatcctg cacattgact ttggggactg ctttgaggtt 7200
   gctatgaccc gagagaagtt tccagagaag attccattta gactaacaag 7250
   aatgttgacc aatgctatgg aggttacagg cctggatggc aactacagaa 7300
   tcacatgcca cacagtgatg gaggtgctgc gagagcacaa ggacagtgtc 7350
   atggccgtgc tggaagcctt tgtctatgac cccttgctga actggaggct 7400
   gatggacaca aataccaaag gcaacaagcg atcccgaacg aggacggatt 7450
   cctactctgc tggccagtca gtcgaaattt tggacggtgt ggaacttgga 7500
   gagccagccc ataagaaaac ggggaccaca gtgccagaat ctattcattc 7550
   tttcattgga gacggtttgg tgaaaccaga ggccctaaat aagaaagcta 7600
   tccagattat taacagggtt cgagataagc tcactggtcg ggacttctct 7650
   catgatgaca ctttggatgt tccaacgcaa gttgagctgc tcatcaaaca 7700
   agcgacatcc catgaaaacc tctgccagtg ctatattggc tggtgccctt 7750
   tctggtaact ggaggcccag atgtgcccat cacgtttttt ctgaggcttt 7800
   t gt act t t ag t aaat gct t c cact aaact g aaaccat ggt gagaaagt t t 7850
   gact t t gt t a aat at t t t ga aat gt aaat g aaaagaact a ct gt at at t a 7900
   aaagt t ggt t t gaaccaact t t ct agct gc t gt t gaagaa t at at t gt ca 7950
   gaaacacaag gcttgatttg gttcccagga cagtgaaaca tagtaatacc 8000
   acgt aaat ca agccat t cat t t t ggggaac agaagat cca t aact t t aga 8050
   aat acgggt t t t gact t aac t cacaagaga act cat cat a agt act t gct 8100
   gat ggaagaa t gacct agt t gct cct ct ca acat gggt ac agcaaact ca 8150
   gcacagccaa gaagcctcag gtcgtggaga acatggatta ggatcctaga 8200
   ct gt aaagac acagaagat g ct gacct cac ccct gccacc t at cccaaga 8250
   cctcactggt ctgtggacag cagcagaaat gtttgcaaga taggccaaaa 8300
   t gagt acaaa aggt ct gt ct t ccat cagac ccagt gat gc t gcgact cac 8350
   acgct t caat t caagacct g accgct agt a gggaggt t t a t t cagat cgc 8400
   t ggcagcct c ggct gagcag at gcacagag gggat cact g t gcagt ggga 8450
   ccaccct cac t ggcct t ct g cagcagggt t ct gggat gt t t t cagt ggt c 8500
   aaaatactct gtttagagca agggctcaga aaacagaaat actgtcatgg 8550
   aggtgctgaa cacagggaag gtctggtaca tattggaaat tatgagcaga 8600
   acaaat act c aact aaat gc acaaagt at a aagt gt agcc at gt ct agac 8650
   accat gt t gt at cagaat aa t t t t t gt gcc aat aaat gac at cagaat t t 8700
   t aaacat 8707
<210> 9
   <211> 5822
   <212> DNA
   <213> Homo sapiens
<400> 9
   actgagtccc gggaccccgg gagagcggtc aatgtgtggt cgctgcgttt 50
   cctctgcctg cgccgggcat cacttgcgcg ccgcagaaag tccgtctggc 100
   agcctggata tcctctccta ccggcacccg cagacgcccc tgcagccgcg 150
   gt cggcgccc gggct ccct a gccct gt gcg ct caact gt c ct gcgct gcg 200
   gggt gccgcg agt t ccacct ccgcgcct cc t t ct ct agac aggcgct ggg 250
   agaaagaacc ggctcccgag ttctgggcat ttcgcccggc tcgaggtgca 300
   ggat gcagag caaggt gct g ct ggccgt cg ccct gt ggct ct gcgt ggag 350
   acccgggccg cctctgtggg tttgcctagt gtttctcttg atctgcccag 400
   gctcagcata caaaaagaca tacttacaat taaggctaat acaactcttc 450
   aaattacttg caggggacag agggacttgg actggctttg gcccaataat 500
   cagagt ggca gt gagcaaag ggt ggaggt g act gagt gca gcgat ggcct 550
   ct t ct gt aag acact cacaa t t ccaaaagt gat cggaaat gacact ggag 600
   cctacaagtg cttctaccgg gaaactgact tggcctcggt catttatgtc 650
   tatgttcaag attacagatc tccatttatt gcttctgtta gtgaccaaca 700
   tggagtcgtg tacattactg agaacaaaaa caaaactgtg gtgattccat 750
   gtctcgggtc catttcaaat ctcaacgtgt cactttgtgc aagataccca 800
   gaaaagagat ttgttcctga tggtaacaga atttcctggg acagcaagaa 850
   gggctttact attcccagct acatgatcag ctatgctggc atggtcttct 900
   gtgaagcaaa aattaatgat gaaagttacc agtctattat gtacatagtt 950
   gtcgttgtag ggtataggat ttatgatgtg gttctgagtc cgtctcatgg 1000
   aattgaacta tctgttggag aaaagcttgt cttaaattgt acagcaagaa 1050
   ctgaactaaa tgtggggatt gacttcaact gggaataccc ttcttcgaag 1100
   catcagcata agaaacttgt aaaccgagac ctaaaaaccc agtctgggag 1150
   tgagatgaag aaatttttga gcaccttaac tatagatggt gtaacccgga 1200
   gtgaccaagg attgtacacc tgtgcagcat ccagtgggct gatgaccaag 1250
   aagaacagca catttgtcag ggtccatgaa aaaccttttg ttgcttttgg 1300
   aagtggcatg gaatctctgg tggaagccac ggtgggggag cgtgtcagaa 1350
   tccctgcgaa gtaccttggt tacccacccc cagaaataaa atggtataaa 1400
   aatggaatac cccttgagtc caatcacaca attaaagcgg ggcatgtact 1450
   gacgattatg gaagtgagtg aaagagacac aggaaattac actgtcatcc 1500
   ttaccaatcc catttcaaag gagaagcaga gccatgtggt ctctctggtt 1550
   gtgtatgtcc caccccagat tggtgagaaa tctctaatct ctcctgtgga 1600
   ttcctaccag tacggcacca ctcaaacgct gacatgtacg gtctatgcca 1650
   ttcctccccc gcatcacatc cactggtatt ggcagttgga ggaagagtgc 1700
   gccaacgagc ccagccaagc tgtctcagtg acaaacccat acccttgtga 1750
   agaatggaga agtgtggagg acttccaggg aggaaataaa attgaagtta 1800
   ataaaaatca atttgctcta attgaaggaa aaaacaaaac tgtaagtacc 1850
   cttgttatcc aagcggcaaa tgtgtcagct ttgtacaaat gtgaagcggt 1900
   caacaaagtc gggagaggag agagggtgat ctccttccac gtgaccaggg 1950
   gtcctgaaat tactttgcaa cctgacatgc agcccactga gcaggagagc 2000
   gtgtctttgt ggtgcactgc agacagatct acgtttgaga acctcacatg 2050
   gtacaagctt ggcccacagc ctctgccaat ccatgtggga gagttgccca 2100
   cacctgtttg caagaacttg gatactcttt ggaaattgaa tgccaccatg 2150
   ttctctaata gcacaaatga cattttgatc atggagctta agaatgcatc 2200
   cttgcaggac caaggagact atgtctgcct tgctcaagac aggaagacca 2250
   agaaaagaca ttgcgtggtc aggcagctca cagtcctaga gcgtgtggca 2300
   cccacgatca caggaaacct ggagaatcag acgacaagta ttggggaaag 2350
   catcgaagtc tcatgcacgg catctgggaa tccccctcca cagatcatgt 2400
   ggtttaaaga taatgagacc cttgtagaag actcaggcat tgtattgaag 2450
   gatgggaacc ggaacctcac tatccgcaga gtgaggaagg aggacgaagg 2500
   cctctacacc tgccaggcat gcagtgttct tggctgtgca aaagtggagg 2550
   catttttcat aatagaaggt gcccaggaaa agacgaactt ggaaatcatt 2600
   attctagtag gcacggcggt gattgccatg ttcttctggc tacttcttgt 2650
   catcatccta cggaccgtta agcgggccaa tggaggggaa ctgaagacag 2700
   gctacttgtc catcgtcatg gatccagatg aactcccatt ggatgaacat 2750
   tgtgaacgac tgccttatga tgccagcaaa tgggaattcc ccagagaccg 2800
   gctgaagcta ggtaagcctc ttggccgtgg tgcctttggc caagtgattg 2850
   aagcagatgc ctttggaatt gacaagacag caacttgcag gacagtagca 2900
   gtcaaaatgt tgaaagaagg agcaacacac agtgagcatc gagctctcat 2950
   gtctgaactc aagatcctca ttcatattgg tcaccatctc aatgtggtca 3000
   accttctagg tgcctgtacc aagccaggag ggccactcat ggtgattgtg 3050
   gaattctgca aatttggaaa cctgtccact tacctgagga gcaagagaaa 3100
   tgaatttgtc ccctacaaga ccaaaggggc acgattccgt caagggaaag 3150
   actacgttgg agcaatccct gtggatctga aacggcgctt ggacagcatc 3200
   accagtagcc agagctcagc cagctctgga tttgtggagg agaagtccct 3250
   cagtgatgta gaagaagagg aagctcctga agatctgtat aaggacttcc 3300
   tgaccttgga gcatctcatc tgttacagct tccaagtggc taagggcatg 3350
   gagttcttgg catcgcgaaa gtgtatccac agggacctgg cggcacgaaa 3400
   tatcctctta tcggagaaga acgtggttaa aatctgtgac tttggcttgg 3450
   cccgggatat ttataaagat ccagattatg tcagaaaagg agatgctcgc 3500
   ctccctttga aatggatggc cccagaaaca atttttgaca gagtgtacac 3550
   aatccagagt gacgtctggt cttttggtgt tttgctgtgg gaaatatttt 3600
   ccttaggtgc ttctccatat cctggggtaa agattgatga agaattttgt 3650
   aggcgattga aagaaggaac tagaatgagg gcccctgatt atactacacc 3700
   agaaatgtac cagaccatgc tggactgctg gcacggggag cccagtcaga 3750
   gacccacgtt ttcagagttg gtggaacatt tgggaaatct cttgcaagct 3800
   aatgctcagc aggatggcaa agactacatt gttcttccga tatcagagac 3850
   tttgagcatg gaagaggatt ctggactctc tctgcctacc tcacctgttt 3900
   cctgtatgga ggaggaggaa gtatgtgacc ccaaattcca ttatgacaac 3950
   acagcaggaa tcagtcagta tctgcagaac agtaagcgaa agagccggcc 4000
   tgtgagtgta aaaacatttg aagatatccc gttagaagaa ccagaagtaa 4050
   aagtaatccc agatgacaac cagacggaca gtggtatggt tcttgcctca 4100
   gaagagctga aaactttgga agacagaacc aaattatctc catcttttgg 4150
   tggaatggtg cccagcaaaa gcagggagtc tgtggcatct gaaggctcaa 4200
   accagacaag cggctaccag tccggatatc actccgatga cacagacacc 4250
   accgtgtact ccagtgagga agcagaactt ttaaagctga tagagattgg 4300
   agtgcaaacc ggtagcacag cccagattct ccagcctgac tcggggacca 4350
   cactgagctc tcctcctgtt taaaaggaag catccacacc cccaactcct 4400
   ggacatcaca tgagaggtgc tgctcagatt ttcaagtgtt gttctttcca 4450
   ccagcaggaa gtagccgcat ttgattttca tttcgacaac agaaaaagga 4500
   cctcggactg cagggagcca gtcttctagg catatcctgg aagaggcttg 4550
   tgacccaaga atgtgtctgt gtcttctccc agtgttgacc tgatcctctt 4600
   tttcattcat ttaaaaagca tttatcatgc cccctgctgc gggtctcacc 4650
   atgggtttag aacaaagacg ttcaagaaat ggccccatcc tcaaagaagt 4700
   agcagtacct ggggagctga cacttctgta aaactagaag ataaaccagg 4750
   caatgtaagt gttcgaggtg ttgaagatgg gaaggatttg cagggctgag 4800
   tctatccaag aggctttgtt taggacgtgg gtcccaagcc aagccttaag 4850
   tgtggaattc ggattgatag aaaggaagac taacgttacc ttgctttgga 4900
   gagtactgga gcctgcaaat gcattgtgtt tgctctggtg gaggtgggca 4950
   tggggtctgt tctgaaatgt aaagggttca gacggggttt ctggttttag 5000
   aaggttgcgt gttcttcgag ttgggctaaa gtagagttcg ttgtgctgtt 5050
   tctgactcct aatgagagtt ccttccagac cgttacgtgt ctcctggcca 5100
   agccccagga aggaaatgat gcagctctgg ctccttgtct cccaggctga 5150
   tcctttattc agaataccac aaagaaagga cattcagctc aaggctccct 5200
   gccgtgttga agagttctga ctgcacaaac cagcttctgg tttcttctgg 5250
   aatgaatacc ctcatatctg tcctgatgtg atatgtctga gactgaatgc 5300
   gggaggttca atgtgaagct gtgtgtggtg tcaaagtttc aggaaggatt 5350
   ttaccctttt gttcttcccc ctgtccccaa cccactctca ccccgcaacc 5400
   catcagtatt ttagttattt ggcctctact ccagtaaacc tgattgggtt 5450
   tgttcactct ctgaatgatt attagccaga cttcaaaatt attttatagc 5500
   ccaaattata acatctattg tattatttag acttttaaca tatagagcta 5550
   tttctactga tttttgccct tgttctgtcc tttttttcaa aaaagaaaat 5600
   gtgttttttg tttggtacca tagtgtgaaa tgctgggaac aatgactata 5650
   agacatgcta tggcacatat atttatagtc tgtttatgta gaaacaaatg 5700
   taatatatta aagccttata tataatgaac tttgtactat tcacattttg 5750
   tatcagtatt atgtagcata acaaaggtca taatgctttc agcaattgat 5800
   gtcattttat taaagaacat tg 5822
<210> 10
   <211> 4632
   <212> DNA
   <213> Homo sapiens
<400> 10
   gccctcgccg cccgcggcgc cccgagcgct ttgtgagcag atgcggagcc 50
   gagtggaggg cgcgagccag atgcggggcg acagctgact tgctgagagg 100
   aggcggggag gcgcggagcg cgcgtgtggt ccttgcgccg ctgacttctc 150
   cactggttcc tgggcaccga aagataaacc tctcataatg aaggcccccg 200
   ctgtgcttgc acctggcatc ctcgtgctcc tgtttacctt ggtgcagagg 250
   agcaatgggg agtgtaaaga ggcactagca aagtccgaga tgaatgtgaa 300
   tatgaagtat cagcttccca acttcaccgc ggaaacaccc atccagaatg 350
   tcattctaca tgagcatcac attttccttg gtgccactaa ctacatttat 400
   gttttaaatg aggaagacct tcagaaggtt gctgagtaca agactgggcc 450
   tgtgctggaa cacccagatt gtttcccatg tcaggactgc agcagcaaag 500
   ccaatttatc aggaggtgtt tggaaagata acatcaacat ggctctagtt 550
   gtcgacacct actatgatga tcaactcatt agctgtggca gcgtcaacag 600
   agggacctgc cagcgacatg tctttcccca caatcatact gctgacatac 650
   agtcggaggt tcactgcata ttctccccac agatagaaga gcccagccag 700
   tgtcctgact gtgtggtgag cgccctggga gccaaagtcc tttcatctgt 750
   aaaggaccgg ttcatcaact tctttgtagg caataccata aattcttctt 800
   atttcccaga tcatccattg cattcgatat cagtgagaag gctaaaggaa 850
   acgaaagatg gttttatgtt tttgacggac cagtcctaca ttgatgtttt 900
   acctgagttc agagattctt accccattaa gtatgtccat gcctttgaaa 950
   gcaacaattt tatttacttc ttgacggtcc aaagggaaac tctagatgct 1000
   cagacttttc acacaagaat aatcaggttc tgttccataa actctggatt 1050
   gcattcctac atggaaatgc ctctggagtg tattctcaca gaaaagagaa 1100
   aaaagagatc cacaaagaag gaagtgttta atatacttca ggctgcgtat 1150
   gtcagcaagc ctggggccca gcttgctaga caaataggag ccagcctgaa 1200
   tgatgacatt cttttcgggg tgttcgcaca aagcaagcca gattctgccg 1250
   aaccaatgga tcgatctgcc atgtgtgcat tccctatcaa atatgtcaac 1300
   gacttcttca acaagatcgt caacaaaaac aatgtgagat gtctccagca 1350
   tttttacgga cccaatcatg agcactgctt taataggaca cttctgagaa 1400
   attcatcagg ctgtgaagcg cgccgtgatg aatatcgaac agagtttacc 1450
   acagctttgc agcgcgttga cttattcatg ggtcaattca gcgaagtcct 1500
   cttaacatct atatccacct tcattaaagg agacctcacc atagctaatc 1550
   ttgggacatc agagggtcgc ttcatgcagg ttgtggtttc tcgatcagga 1600
   ccatcaaccc ctcatgtgaa ttttctcctg gactcccatc cagtgtctcc 1650
   agaagtgatt gtggagcata cattaaacca aaatggctac acactggtta 1700
   tcactgggaa gaagatcacg aagatcccat tgaatggctt gggctgcaga 1750
   catttccagt cctgcagtca atgcctctct gccccaccct ttgttcagtg 1800
   tggctggtgc cacgacaaat gtgtgcgatc ggaggaatgc ctgagcggga 1850
   catggactca acagatctgt ctgcctgcaa tctacaaggt tttcccaaat 1900
   agtgcacccc ttgaaggagg gacaaggctg accatatgtg gctgggactt 1950
   tggatttcgg aggaataata aatttgattt aaagaaaact agagttctcc 2000
   ttggaaatga gagctgcacc ttgactttaa gtgagagcac gatgaataca 2050
   ttgaaatgca cagttggtcc tgccatgaat aagcatttca atatgtccat 2100
   aattatttca aatggccacg ggacaacaca atacagtaca ttctcctatg 2150
   tggatcctgt aataacaagt atttcgccga aatacggtcc tatggctggt 2200
   ggcactttac ttactttaac tggaaattac ctaaacagtg ggaattctag 2250
   acacatttca attggtggaa aaacatgtac tttaaaaagt gtgtcaaaca 2300
   gtattcttga atgttatacc ccagcccaaa ccatttcaac tgagtttgct 2350
   gttaaattga aaattgactt agccaaccga gagacaagca tcttcagtta 2400
   ccgtgaagat cccattgtct atgaaattca tccaaccaaa tcttttatta 2450
   gtacttggtg gaaagaacct ctcaacattg tcagttttct attttgcttt 2500
   gccagtggtg ggagcacaat aacaggtgtt gggaaaaacc tgaattcagt 2550
   tagtgtcccg agaatggtca taaatgtgca tgaagcagga aggaacttta 2600
   cagtggcatg tcaacatcgc tctaattcag agataatctg ttgtaccact 2650
   ccttccctgc aacagctgaa tctgcaactc cccctgaaaa ccaaagcctt 2700
   tttcatgtta gatgggatcc tttccaaata ctttgatctc atttatgtac 2750
   ataatcctgt gtttaagcct tttgaaaagc cagtgatgat ctcaatgggc 2800
   aatgaaaatg tactggaaat taagggaaat gatattgacc ctgaagcagt 2850
   taaaggtgaa gtgttaaaag ttggaaataa gagctgtgag aatatacact 2900
   tacattctga agccgtttta tgcacggtcc ccaatgacct gctgaaattg 2950
   aacagcgagc taaatataga gtggaagcaa gcaatttctt caaccgtcct 3000
   tggaaaagta atagttcaac cagatcagaa tttcacagga ttgattgctg 3050
   gtgttgtctc aatatcaaca gcactgttat tactacttgg gtttttcctg 3100
   tggctgaaaa agagaaagca aattaaagat ctgggcagtg aattagttcg 3150
   ctacgatgca agagtacaca ctcctcattt ggataggctt gtaagtgccc 3200
   gaagt gt aag cccaact aca gaaat ggt t t caaat gaat c t gt agact ac 3250
   cgagct act t t t ccagaaga t cagt t t cct aat t cat ct c agaacggt t c 3300
   at gccgacaa gt gcagt at c ct ct gacaga cat gt ccccc at cct aact a 3350
   gt ggggact c t gat at at cc agt ccat t ac t gcaaaat ac t gt ccacat t 3400
   gacct cagt g ct ct aaat cc agagct ggt c caggcagt gc agcat gt agt 3450
   gat t gggccc agt agcct ga t t gt gcat t t caat gaagt c at aggaagag 3500
   ggcat t t t gg t t gt gt at at cat gggact t t gt t ggacaa t gat ggcaag 3550
   aaaat t cact gt gct gt gaa at cct t gaac agaat cact g acat aggaga 3600
   agt t t cccaa t t t ct gaccg agggaat cat cat gaaagat t t t agt cat c 3650
   ccaat gt cct ct cgct cct g ggaat ct gcc t gcgaagt ga agggt ct ccg 3700
   ct ggt ggt cc t accat acat gaaacat gga gat ct t cgaa at t t cat t cg 3750
   aaat gagact cat aat ccaa ct gt aaaaga t ct t at t ggc t t t ggt ct t c 3800
   aagt agccaa aggcat gaaa t at ct t gcaa gcaaaaagt t t gt ccacaga 3850
   gact t ggct g caagaaact g t at gct ggat gaaaaat t ca cagt caaggt 3900
   t gct gat t t t ggt ct t gcca gagacat gt a t gat aaagaa t act at agt g 3950
   t acacaacaa aacaggt gca aagct gccag t gaagt ggat ggct t t ggaa 4000
   agt ct gcaaa ct caaaagt t t accaccaag t cagat gt gt ggt cct t t gg 4050
   cgtgctcctc tgggagctga tgacaagagg agccccacct tatcctgacg 4100
   t aaacacct t t gat at aact gt t t act t gt t gcaagggag aagact cct a 4150
   caacccgaat actgcccaga ccccttatat gaagtaatgc taaaatgctg 4200
   gcaccct aaa gccgaaat gc gcccat cct t t t ct gaact g gt gt cccgga 4250
   t at cagcgat ct t ct ct act t t cat t gggg agcact at gt ccat gt gaac 4300
   gct act t at g t gaacgt aaa at gt gt cgct ccgt at cct t ct ct gt t gt c 4350
   atcagaagat aacgctgatg atgaggtgga cacacgacca gcctccttct 4400
   gggagacatc atagtgctag tactatgtca aagcaacagt ccacactttg 4450
   t ccaat ggt t t t t t cact gc ct gacct t t a aaaggccat c gat at t ct t t 4500
   gct ct t gcca aaat t gcact at t at aggac t t gt at t gt t at t t aaat t a 4550
   ct ggat t ct a aggaat t t ct t at ct gacag agcat cagaa ccagaggct t 4600
   ggtcccacag gccacggacc aatggcctgc ag 4632
<210> 11
   <211> 15081
   <212> DNA
   <213> Homo sapiens
<400> 11
   ct gct t cact t cacggggcg aacat ggcgc acagct gt cg gt ggcgct t c 50
   cccgcccgac ccgggaccac cgggggcggc ggcggcgggg ggcgccgggg 100
   cctagggggc gccccgcggc aacgcgtccc ggccctgctg cttccccccg 150
   ggcccccggt cggcggtggc ggccccgggg cgcccccctc ccccccggct 200
   gtggcggccg cggcggcggc ggcgggaagc agcggggctg gggttccagg 250
   gggagcggcc gccgcctcag cagcctcctc gtcgtccgcc tcgtcttcgt 300
   ct t cgt cat c gt cct cagcc t ct t cagggc cggccct gct ccgggt gggc 350
   ccgggcttcg acgcggcgct gcaggtctcg gccgccatcg gcaccaacct 400
   gcgccggttc cgggccgtgt ttggggagag cggcggggga ggcggcagcg 450
   gagagctaac aacacagatc ccatgtagtt ggagaaccaa aggccacata 500
   cat gacaaaa agact gaacc gt t caggt t a ct t gcat gga gt t ggt gct t 550
   aaat gat gag caat t ct t ag gt t t t ggct c agat gaagaa gt cagagt gc 600
   gaagt cccac aaggt ct cct t cagt t aaaa ct agt cct cg aaaacct cgt 650
   gggagacct a gaagt ggct c t gaccgaaat t cagct at cc t ct cagat cc 700
   at ct gt gt t t t cccct ct aa at aaat caga gaccaaat ct ggagat aaga 750
   tcaagaagaa agattctaaa agtatagaaa agaagagagg aagacctccc 800
   acct t ccct g gagt aaaaat caaaat aaca cat ggaaagg acat t t caga 850
   gttaccaaag ggaaacaaag aagatagcct gaaaaaaatt aaaaggacac 900
   cttctgctac gtttcagcaa gccacaaaga ttaaaaaatt aagagcaggt 950
   aaactctctc ctctcaagtc taagtttaag acagggaagc ttcaaatagg 1000
   aaggaagggg gtacaaattg tacgacggag aggaaggcct ccatcaacag 1050
   aaaggat aaa gacccct t cg ggt ct cct ca t t aat t ct ga act ggaaaag 1100
   ccccagaaag tccggaaaga caaggaagga acacctccac ttacaaaaga 1150
   agat aagaca gt t gt cagac aaagccct cg aaggat t aag ccagt t agga 1200
   t t at t cct t c t t caaaaagg acagat gcaa ccat t gct aa gcaact ct t a 1250
   cagagggcaa aaaagggggc tcaaaagaaa attgaaaaag aagcagctca 1300
   gctgcaggga agaaaggtga agacacaggt caaaaatatt cgacagttca 1350
   t cat gcct gt t gt cagt gct at ct cct cgc ggat cat t aa gacccct cgg 1400
   cggt t t at ag aggat gagga t t at gaccct ccaat t aaaa t t gcccgat t 1450
   agagt ct aca ccgaat agt a gat t cagt gc cccgt cct gt ggat ct t ct g 1500
   aaaaat caag t gcagct t ct cagcact cct ct caaat gt c t t cagact cc 1550
   t ct cgat ct a gt agccccag t gt t gat acc t ccacagact ct caggct t c 1600
   t gaggagat t caggt act t c ct gaggagcg gagcgat acc cct gaagt t c 1650
   at cct ccact gcccat t t cc cagt ccccag aaaat gagag t aat gat agg 1700
   agaagcagaa ggtattcagt gtcggagaga agttttggat ctagaacgac 1750
   gaaaaaatta tcaactctac aaagtgcccc ccagcagcag acctcctcgt 1800
   ct ccacct cc acct ct gct g act ccaccgc caccact gca gccagcct cc 1850
   agtatctctg accacacacc ttggcttatg cctccaacaa tccccttagc 1900
   atcaccattt ttgcctgctt ccactgctcc tatgcaaggg aagcgaaaat 1950
   ctattttgcg agaaccgaca tttaggtgga cttctttaaa gcattctagg 2000
   tcagagccac aatacttttc ctcagcaaag tatgccaaag aaggtcttat 2050
   tcgcaaacca atatttgata atttccgacc ccctccacta actcccgagg 2100
   acgttggctt tgcatctggt ttttctgcat ctggtaccgc tgcttcagcc 2150
   cgattgtttt cgccactcca ttctggaaca aggtttgata tgcacaaaag 2200
   gagccctctt ctgagagctc caagatttac tccaagtgag gctcactcta 2250
   gaatatttga gtctgtaacc ttgcctagta atcgaacttc tgctggaaca 2300
   tcttcttcag gagtatccaa tagaaaaagg aaaagaaaag tgtttagtcc 2350
   tattcgatct gaaccaagat ctccttctca ctccatgagg acaagaagtg 2400
   gaaggcttag tagttctgag ctctcacctc tcaccccccc gtcttctgtc 2450
   tcttcctcgt taagcatttc tgttagtcct cttgccacta gtgccttaaa 2500
   cccaactttt acttttcctt ctcattccct gactcagtct ggggaatctg 2550
   cagagaaaaa tcagagacca aggaagcaga ctagtgctcc ggcagagcca 2600
   ttttcatcaa gtagtcctac tcctctcttc ccttggttta ccccaggctc 2650
   tcagactgaa agagggagaa ataaagacaa ggcccccgag gagctgtcca 2700
   aagatcgaga tgctgacaag agcgtggaga aggacaagag tagagagaga 2750
   gaccgggaga gagaaaagga gaataagcgg gagtcaagga aagagaaaag 2800
   gaaaaaggga tcagaaattc agagtagttc tgctttgtat cctgtgggta 2850
   gggtttccaa agagaaggtt gttggtgaag atgttgccac ttcatcttct 2900
   gccaaaaaag caacagggcg gaagaagtct tcatcacatg attctgggac 2950
   tgatattact tctgtgactc ttggggatac aacagctgtc aaaaccaaaa 3000
   tacttataaa gaaagggaga ggaaatctgg aaaaaaccaa cttggacctc 3050
   ggcccaactg ccccatccct ggagaaggag aaaaccctct gcctttccac 3100
   tccttcatct agcactgtta aacattccac ttcctccata ggctccatgt 3150
   tggctcaggc agacaagctt ccaatgactg acaagagggt tgccagcctc 3200
   ctaaaaaagg ccaaagctca gctctgcaag attgagaaga gtaagagtct 3250
   taaacaaacc gaccagccca aagcacaggg tcaagaaagt gactcatcag 3300
   agacctctgt gcgaggaccc cggattaaac atgtctgcag aagagcagct 3350
   gttgcccttg gccgaaaacg agctgtgttt cctgatgaca tgcccaccct 3400
   gagtgcctta ccatgggaag aacgagaaaa gattttgtct tccatgggga 3450
   atgatgacaa gtcatcaatt gctggctcag aagatgctga acctcttgct 3500
   ccacccatca aaccaattaa acctgtcact agaaacaagg caccccagga 3550
   acctccagta aagaaaggac gtcgatcgag gcggtgtggg cagtgtcccg 3600
   gct gccaggt gcct gaggac t gt ggt gt t t gt act aat t g ct t agat aag 3650
   cccaagt t t g gt ggt cgcaa t at aaagaag cagt gct gca agat gagaaa 3700
   atgtcagaat ctacaatgga tgccttccaa agcctacctg cagaagcaag 3750
   ctaaagctgt gaaaaagaaa gagaaaaagt ctaagaccag tgaaaagaaa 3800
   gacagcaaag agagcagtgt tgtgaagaac gtggtggact ctagtcagaa 3850
   acctacccca tcagcaagag aggatcctgc cccaaagaaa agcagtagtg 3900
   agcctcctcc acgaaagccc gtcgaggaaa agagtgaaga agggaatgtc 3950
   tcggcccctg ggcctgaatc caaacaggcc accactccag cttccaggaa 4000
   gtcaagcaag caggtctccc agccagcact ggtcatcccg cctcagccac 4050
   ctactacagg accgccaaga aaagaagttc ccaaaaccac tcctagtgag 4100
   cccaagaaaa agcagcctcc accaccagaa tcaggtccag agcagagcaa 4150
   acagaaaaaa gtggctcccc gcccaagtat ccctgtaaaa caaaaaccaa 4200
   aagaaaagga aaaaccacct ccggtcaata agcaggagaa tgcaggcact 4250
   ttgaacatcc tcagcactct ctccaatggc aatagttcta agcaaaaaat 4300
   t ccagcagat ggagt ccaca ggat cagagt ggact t t aag gaggat t gt g 4350
   aagcagaaaa tgtgtgggag atgggaggct taggaatctt gacttctgtt 4400
   cct at aacac ccagggt ggt t t gct t t ct c t gt gccagt a gt gggcat gt 4450
   agagt t t gt g t at t gccaag t ct gt t gt ga gccct t ccac aagt t t t gt t 4500
   tagaggagaa cgagcgccct ctggaggacc agctggaaaa ttggtgttgt 4550
   cgt cgt t gca aat t ct gt ca cgt t t gt gga aggcaacat c aggct acaaa 4600
   gcagct gct g gagt gt aat a agt gccgaaa cagct at cac cct gagt gcc 4650
   tgggaccaaa ctaccccacc aaacccacaa agaagaagaa agtctggatc 4700
   t gt accaagt gt gt t cgct g t aagagct gt ggat ccacaa ct ccaggcaa 4750
   agggt gggat gcacagt ggt ct cat gat t t ct cact gt gt cat gat t gcg 4800
   ccaagct ct t t gct aaagga aact t ct gcc ct ct ct gt ga caaat gt t at 4850
   gat gat gat g act at gagag t aagat gat g caat gt ggaa agt gt gat cg 4900
   ct gggt ccat t ccaaat gt g agaat ct t t c agat gagat g t at gagat t c 4950
   tatctaatct gccagaaagt gtggcctaca cttgtgtgaa ctgtactgag 5000
   cggcaccctg cagagtggcg actggccctt gaaaaagagc tgcagatttc 5050
   t ct gaagcaa gt t ct gacag ct t t gt t gaa t t ct cggact accagccat t 5100
   tgctacgcta ccggcaggct gccaagcctc cagacttaaa tcccgagaca 5150
   gaggagagt a t acct t cccg cagct ccccc gaaggacct g at ccaccagt 5200
   t ct t act gag gt cagcaaac aggat gat ca gcagcct t t a gat ct agaag 5250
   gagt caagag gaagat ggac caagggaat t acacat ct gt gt t ggagt t c 5300
   agtgatgata ttgtgaagat cattcaagca gccattaatt cagatggagg 5350
   acagccagaa attaaaaaag ccaacagcat ggtcaagtcc ttcttcattc 5400
   ggcaaatgga acgtgttttt ccatggttca gtgtcaaaaa gtccaggttt 5450
   tgggagccaa ataaagtatc aagcaacagt gggatgttac caaacgcagt 5500
   gcttccacct tcacttgacc ataattatgc tcagtggcag gagcgagagg 5550
   aaaacagcca cactgagcag cctcctttaa tgaagaaaat cattccagct 5600
   cccaaaccca aaggtcctgg agaaccagac tcaccaactc ctctgcatcc 5650
   tcctacacca ccaattttga gtactgatag gagtcgagaa gacagtccag 5700
   agctgaaccc acccccaggc atagaagaca atagacagtg tgcgttatgt 5750
   ttgacttatg gtgatgacag tgctaatgat gctggtcgtt tactatatat 5800
   tggccaaaat gagtggacac atgtaaattg tgctttgtgg tcagcggaag 5850
   tgtttgaaga tgatgacgga tcactaaaga atgtgcatat ggctgtgatc 5900
   aggggcaagc agctgagatg tgaattctgc caaaagccag gagccaccgt 5950
   gggttgctgt ctcacatcct gcaccagcaa ctatcacttc atgtgttccc 6000
   gagccaagaa ctgtgtcttt ctggatgata aaaaagtata ttgccaacga 6050
   catcgggatt tgatcaaagg cgaagtggtt cctgagaatg gatttgaagt 6100
   tttcagaaga gtgtttgtgg actttgaagg aatcagcttg agaaggaagt 6150
   ttctcaatgg cttggaacca gaaaatatcc acatgatgat tgggtctatg 6200
   acaatcgact gcttaggaat tctaaatgat ctctccgact gtgaagataa 6250
   gctctttcct attggatatc agtgttccag ggtatactgg agcaccacag 6300
   atgctcgcaa gcgctgtgta tatacatgca agatagtgga gtgccgtcct 6350
   ccagtcgtag agccggatat caacagcact gttgaacatg atgaaaacag 6400
   gaccattgcc catagtccaa catcttttac agaaagttca tcaaaagaga 6450
   gtcaaaacac agctgaaatt ataagtcctc catcaccaga ccgacctcct 6500
   cattcacaaa cctctggctc ctgttattat catgtcatct caaaggtccc 6550
   caggattcga acacccagtt attctccaac acagagatcc cctggctgtc 6600
   gaccgttgcc ttctgcagga agtcctaccc caaccactca tgaaatagtc 6650
   acagtaggtg atcctttact ctcctctgga cttcgaagca ttggctccag 6700
   gcgtcacagt acctcttcct tatcacccca gcggtccaaa ctccggataa 6750
   tgtctccaat gagaactggg aatacttact ctaggaataa tgtttcctca 6800
   gtctccacca ccgggaccgc tactgatctt gaatcaagtg ccaaagtagt 6850
   tgatcatgtc ttagggccac tgaattcaag tactagttta gggcaaaaca 6900
   cttccacctc ttcaaatttg caaaggacag tggttactgt aggcaataaa 6950
   aacagtcact tggatggatc ttcatcttca gaaatgaagc agtccagtgc 7000
   ttcagacttg gtgtccaaga gctcctcttt aaagggagag aagaccaaag 7050
   t gct gagt t c caagagct ca gagggat ct g cacat aat gt ggct t accct 7100
   ggaattccta aactggcccc acaggttcat aacacaacat ctagagaact 7150
   gaat gt t agt aaaat cggct cct t t gct ga accct ct t ca gt gt cgt t t t 7200
   cttctaaaga ggccctctcc ttcccacacc tccatttgag agggcaaagg 7250
   aatgatcgag accaacacac agattctacc caatcagcaa actcctctcc 7300
   agat gaagat act gaagt ca aaacct t gaa gct at ct gga at gagcaaca 7350
   gat cat ccat t at caacgaa cat at gggat ct agt t ccag agat aggaga 7400
   cagaaaggga aaaaatcctg taaagaaact ttcaaagaaa agcattccag 7450
   taaatctttt ttggaacctg gtcaggtgac aactggtgag gaaggaaact 7500
   t gaagccaga gt t t at ggat gaggt t t t ga ct cct gagt a t at gggccaa 7550
   cgaccat gt a acaat gt t t c t t ct gat aag at t ggt gat a aaggcct t t c 7600
   t at gccagga gt ccccaaag ct ccacccat gcaagt agaa ggat ct gcca 7650
   aggaattaca ggcaccacgg aaacgcacag tcaaagtgac actgacacct 7700
   ct aaaaat gg aaaat gagag t caat ccaaa aat gccct ga aagaaagt ag 7750
   t cct gct t cc cct t t gcaaa t agagt caac at ct cccaca gaaccaat t t 7800
   cagcctctga aaatccagga gatggtccag tggcccaacc aagccccaat 7850
   aat acct cat gccaggat t c t caaagt aac aact at caga at ct t ccagt 7900
   acaggacaga aacctaatgc ttccagatgg ccccaaacct caggaggatg 7950
   gct ct t t t aa aaggaggt at ccccgt cgca gt gcccgt gc acgt t ct aac 8000
   at gt t t t t t g ggct t acccc act ct at gga gt aagat cct at ggt gaaga 8050
   agacattcca ttctacagca gctcaactgg gaagaagcga ggcaagagat 8100
   cagct gaagg acaggt ggat ggggccgat g act t aagcac t t cagat gaa 8150
   gacgact t at act at t acaa ct t cact aga acagt gat t t ct t caggt gg 8200
   agaggaacga ctggcatccc ataatttatt tcgggaggag gaacagtgtg 8250
   atcttccaaa aatctcacag ttggatggtg ttgatgatgg gacagagagt 8300
   gatactagtg tcacagccac aacaaggaaa agcagccaga ttccaaaaag 8350
   aaat ggt aaa gaaaat ggaa cagagaact t aaagat t gat agacct gaag 8400
   atgctgggga gaaagaacat gtcactaaga gttctgttgg ccacaaaaat 8450
   gagccaaaga tggataactg ccattctgta agcagagtta aaacacaggg 8500
   acaagattcc ttggaagctc agctcagctc attggagtca agccgcagag 8550
   t ccacacaag t accccct cc gacaaaaat t t act ggacac ct at aat act 8600
   gagct cct ga aat cagat t c agacaat aac aacagt gat g act gt gggaa 8650
   t at cct gcct t cagacat t a t ggact t t gt act aaagaat act ccat cca 8700
   t gcaggct t t gggt gagagc ccagagt cat ct t cat caga act cct gaat 8750
   ct t ggt gaag gat t gggt ct t gacagt aat cgt gaaaaag acat gggt ct 8800
   ttttgaagta ttttctcagc agctgcctac aacagaacct gtggatagta 8850
   gtgtctcttc ctctatctca gcagaggaac agtttgagtt gcctctagag 8900
   ct accat ct g at ct gt ct gt ct t gaccacc cggagt ccca ct gt ccccag 8950
   ccagaatccc agtagactag ctgttatctc agactcaggg gagaagagag 9000
   t aaccat cac agaaaaat ct gt agcct cct ct gaaagt ga cccagcact g 9050
   ct gagcccag gagt agat cc aact cct gaa ggccacat ga ct cct gat ca 9100
   t t t t at ccaa ggacacat gg at gcagacca cat ct ct agc cct cct t gt g 9150
   gt t cagt aga gcaaggt cat ggcaacaat c aggat t t aac t aggaacagt 9200
   agcacccct g gcct t caggt acct gt t t cc ccaact gt t c ccat ccagaa 9250
   ccagaagt at gt gcccaat t ct act gat ag t cct ggcccg t ct cagat t t 9300
   ccaat gcagc t gt ccagacc act ccacccc acct gaagcc agccact gag 9350
   aaact cat ag t t gt t aacca gaacat gcag ccact t t at g t t ct ccaaac 9400
   t ct t ccaaat ggagt gaccc aaaaaat cca at t gacct ct t ct gt t agt t 9450
   ct acacccag t gt gat ggag acaaat act t cagt at t ggg acccat ggga 9500
   ggt ggt ct ca ccct t accac aggact aaat ccaagct t gc caact t ct ca 9550
   at ct t t gt t c cct t ct gct a gcaaaggat t gct acccat g t ct cat cacc 9600
   agcacttaca ttccttccct gcagctactc aaagtagttt cccaccaaac 9650
   at cagcaat c ct cct t cagg cct gct t at t ggggt t cagc ct cct ccgga 9700
   tccccaactt ttggtttcag aatccagcca gaggacagac ctcagtacca 9750
   cagtagccac tccatcctct ggactcaaga aaagacccat atctcgtcta 9800
   cagacccgaa agaataaaaa acttgctccc tctagtaccc cttcaaacat 9850
   t gcccct t ct gat gt ggt t t ct aat at gac at t gat t aac t t cacaccct 9900
   cccagct t cc t aat cat cca agt ct gt t ag at t t ggggt c act t aat act 9950
   t cat ct cacc gaact gt ccc caacat cat a aaaagat ct a aat ct agcat 10000
   catgtatttt gaaccggcac ccctgttacc acagagtgtg ggaggaactg 10050
   ctgccacagc ggcaggcaca tcaacaataa gccaggatac tagccacctc 10100
   acat cagggt ct gt gt ct gg ct t ggcat cc agt t cct ct g t ct t gaat gt 10150
   t gt at ccat g caaact acca caacccct ac aagt agt gcg t cagt t ccag 10200
   gacacgt cac ct t aaccaac ccaaggt t gc t t ggt acccc agat at t ggc 10250
   tcaataagca atcttttaat caaagctagc cagcagagcc tggggattca 10300
   ggaccagcct gtggctttac cgccaagttc aggaatgttt ccacaactgg 10350
   ggacat caca gaccccct ct act gct gcaa t aacagcggc at ct agcat c 10400
   t gt gt gct cc cct ccact ca gact acgggc at aacagccg ct t cacct t c 10450
   tggggaagca gacgaacact atcagcttca gcatgtgaac cagctccttg 10500
   ccagcaaaac tgggattcat tcttcccagc gtgatcttga ttctgcttca 10550
   gggccccagg tatccaactt tacccagacg gtagacgctc ctaatagcat 10600
   gggactggag cagaacaagg ctttatcctc agctgtgcaa gccagcccca 10650
   cctctcctgg gggttctcca tcctctccat cttctggaca gcggtcagca 10700
   agcccttcag tgccgggtcc cactaaaccc aaaccaaaaa ccaaacggtt 10750
   tcagctgcct ctagacaaag ggaatggcaa gaagcacaaa gtttcccatt 10800
   tgcggaccag ttcttctgaa gcacacattc cagaccaaga aacgacatcc 10850
   ctgacctcag gcacagggac tccaggagca gaggctgagc agcaggatac 10900
   agctagcgtg gagcagtcct cccagaagga gtgtgggcaa cctgcagggc 10950
   aagtcgctgt tcttccggaa gttcaggtga cccaaaatcc agcaaatgaa 11000
   caagaaagtg cagaacctaa aacagtggaa gaagaggaaa gtaatttcag 11050
   ctccccactg atgctttggc ttcagcaaga acaaaagcgg aaggaaagca 11100
   ttactgagaa aaaacccaag aaaggacttg tttttgaaat ttccagtgat 11150
   gatggctttc agatctgtgc agaaagtatt gaagatgcct ggaagtcatt 11200
   gacagataaa gtccaggaag ctcgatcaaa tgcccgccta aagcagctct 11250
   catttgcagg tgttaacggt ttgaggatgc tggggattct ccatgatgca 11300
   gttgtgttcc tcattgagca gctgtctggt gccaagcact gtcgaaatta 11350
   caaattccgt ttccacaagc cagaggaggc caatgaaccc cccttgaacc 11400
   ctcacggctc agccagggct gaagtccacc tcaggaagtc agcatttgac 11450
   atgtttaact tcctggcttc taaacatcgt cagcctcctg aatacaaccc 11500
   caatgatgaa gaagaggagg aggtacagct gaagtcagct cggagggcaa 11550
   ctagcatgga tctgccaatg cccatgcgct tccggcactt aaaaaagact 11600
   tctaaggagg cagttggtgt ctacaggtct cccatccatg gccggggtct 11650
   tttctgtaag agaaacattg atgcaggtga gatggtgatt gagtatgccg 11700
   gcaacgtcat ccgctccatc cagactgaca agcgggaaaa gtattacgac 11750
   agcaagggca ttggttgcta tatgttccga attgatgact cagaggtagt 11800
   ggatgccacc atgcatggaa atgctgcacg cttcatcaat cactcgtgtg 11850
   agcctaactg ctattctcgg gtcatcaata ttgatgggca gaagcacatt 11900
   gtcatctttg ccatgcgtaa gatctaccga ggagaggaac tcacttacga 11950
   ctataagttc cccattgagg atgccagcaa caagctgccc tgcaactgtg 12000
   gcgccaagaa atgccggaag ttcctaaact aaagctgctc ttctccccca 12050
   gtgttggagt gcaaggaggc ggggccatcc aaagcaacgc tgaaggcctt 12100
   ttccagcagc tgggagctcc cggattgcgt ggcacagctg aggggcctct 12150
   gtgatggctg agctctctta tgtcctatac tcacatcaga catgtgatca 12200
   t agt cccaga gacagagt t g aggt ct cgaa gaaaagat cc at gat cggct 12250
   t t ct cct ggg gcccct ccaa t t gt t t act g t t agaaagt g ggaat ggggt 12300
   ccct agcaga ct t gcct gga aggagcct at t at agagggt t ggt t at gt t 12350
   gggagat t gg gcct gaat t t ct ccacagaa at aagt t gcc at cct caggt 12400
   tggccctttc ccaagcactg taagtgagtg ggtcaggcaa agccccaaat 12450
   ggagggttgg ttagattcct gacagtttgc cagccaggcc ccacctacag 12500
   cgtctgtcga acaaacagag gtctggtggt tttccctact atcctcccac 12550
   tcgagagttc acttctggtt gggagacagg attcctagca cctccggtgt 12600
   caaaaggct g t cat ggggt t gt gccaat t a at t accaaac at t gagcct g 12650
   caggct t t ga gt gggagt gt t gcccccagg agcct t at ct cagccaat t a 12700
   cct t t ct t ga cagt aggagc ggct t ccct c t cccat t ccc t ct t cact cc 12750
   ct t t t ct t cc t t t cccct gt ct t cat gcca ct gct t t ccc at gct t ct t t 12800
   cgggt t gt ag gggagact ga ct gcct gct c aaggacact c cct gct gggc 12850
   at aggat gt g cct gcaaaaa gt t ccct gag cct gt aagca ct ccaggt gg 12900
   ggaagt ggac aggagccat t ggt cat aacc agacagaat t t ggaaacat t 12950
   t t cat aaagc t ccat ggaga gt t t t aaaga aacat at gt a gcat gat t t t 13000
   gt aggagagg aaaaagat t a t t t aaat agg at t t aaat ca t gcaacaacg 13050
   agagt at cac agccaggat g accct t gggt cccat t cct a agacat ggt t 13100
   act t t at t t t cccct t gt t a agacat agga agact t aat t t t t aaacggt 13150
   cagtgtccag ttgaaggcag aacactaatc agatttcaag gcccacaact 13200
   t ggggact ag accacct t at gt t gagggaa ct ct gccacc t gcgt gcaac 13250
   ccacagct aa agt aaat t ca at gacact ac t gccct gat t act cct t agg 13300
   atgtggtcaa aacagcatca aatgtttctt ctcttccttt ccccaagaca 13350
   gagt cct gaa cct gt t aaat t aagt cat t g gat t t t act c t gt t ct gt t t 13400
   acagt t t act at t t aaggt t t t at aaat gt aaat at at t t t gt at at t t t 13450
   tctatgagaa gcacttcata gggagaagca cttatgacaa ggctattttt 13500
   t aaaccgcgg t at t at cct a at t t aaaaga agat cggt t t t t aat aat t t 13550
   t t t at t t t ca t aggat gaag t t agagaaaa t at t cagct g t acacacaaa 13600
   gtctggtttt tcctgcccaa cttccccctg gaaggtgtac tttttgttgt 13650
   t t aat gt gt a gct t gt t t gt gccct gt t ga cat aaat gt t t cct gggt t t 13700
   gctctttgac aataaatgga gaaggaaggt cacccaactc cattgggcca 13750
   ct cccct cct t cccct at t g aagct cct ca aaaggct aca gt aat at ct t 13800
   gat acaacag at t ct ct t ct t t cccgcct c t ct cct t t cc ggcgcaact t 13850
   ccagagtggt gggagacggc aatctttaca tttccctcat ctttcttact 13900
   tcagagttag caaacaacaa gttgaatggc aacttgacat ttttgcatca 13950
   ccatctgcct cataggccac tctttccttt ccctctgccc accaagtcct 14000
   catatctgca gagaacccat tgatcacctt gtgccctctt ttggggcagc 14050
   ctgttgaaac tgaagcacag tctgaccact cacgataaag cagatttttc 14100
   tctgcctctg ccacaaggtt tcagagtagt gtagtccaag tagagggtgg 14150
   ggcacccttt tctcgccgca agaagcccat tcctatggaa gtctagcaaa 14200
   gcaatacgac tcagcccagc actctctgcc ccaggactca tggctctgct 14250
   gtgccttcca tcctgggctc ccttctctcc tgtgacctta agaactttgt 14300
   ctggtggctt tgctggaaca ttgtcactgt tttcactgtc atgcagggag 14350
   cccagcactg tggccaggat ggcagagact tccttgtcat catggagaag 14400
   tgccagcagg ggactgggaa aagcactcta cccagacctc acctcccttc 14450
   ctccttttgc ccatgaacaa gatgcagtgg ccctaggggt tccactagtg 14500
   tctgctttcc tttattattg cactgtgtga ggtttttttg taaatccttg 14550
   tattcctatt ttttttaaag aaaaaaaaaa aaccttaagc tgcatttgtt 14600
   actgaaatga ttaatgcact gatgggtcct gaattcacct tgagaaagac 14650
   ccaaaggcca gtcagggggt ggggggaact cagctaaata gacctagtta 14700
   ctgccctgct aggccatgct gtactgtgag cccctcctca ctctctacca 14750
   accctaaacc ctgaggacag gggaggaacc cacagcttcc ttctcctgcc 14800
   agctgcagat ggtttgcctt gcctttccac cccctaattg tcaaccacaa 14850
   aaatgagaaa ttcctcttct agctcagcct tgagtccatt gccaaatttt 14900
   cagcacacct gccagcaact tgggggaata agcgaaggtt tccctacaag 14950
   agggaaagaa ggcaaaaacg gcacagctat ctccaaacac atctgagttc 15000
   atttcaaaag tgaccaaggg aatctccgca caaaagtgca gattgaggaa 15050
   ttgtgatggg tcattcccaa gaatccccca a 15081
<210> 12
   <211> 9009
   <212> DNA
   <213> Homo sapiens
<400> 12
   ctccccctcc cgctcggcgc tgacccccca tccccacccc cgtgggaaca 50
   ctgggagcct gcactccaca gaccctctcc ttgcctcttc cctcacctca 100
   gcctccgctc cccgccctct tcccggccca gggcgccggc ccacccttcc 150
   ctccgccgcc ccccggccgc ggggaggaca tggccgcgca caggccggtg 200
   gaatgggtcc aggccgtggt cagccgcttc gacgagcagc ttccaataaa 250
   aacaggacag cagaacacac ataccaaagt cagtactgag cacaacaagg 300
   aatgtctaat caatatttcc aaatacaagt tttctttggt tataagcggc 350
   ctcactacta ttttaaagaa tgttaacaat atgagaatat ttggagaagc 400
   tgctgaaaaa aatttatatc tctctcagtt gattatattg gatacactgg 450
   aaaaatgtct tgctgggcaa ccaaaggaca caatgagatt agatgaaacg 500
   atgctggtca aacagttgct gccagaaatc tgccattttc ttcacacctg 550
   tcgtgaagga aaccagcatg cagctgaact tcggaattct gcctctgggg 600
   ttttattttc tctcagctgc aacaacttca atgcagtctt tagtcgcatt 650
   tctaccaggt tacaggaatt aactgtttgt tcagaagaca atgttgatgt 700
   tcatgatata gaattgttac agtatatcaa tgtggattgt gcaaaattaa 750
   aacgactcct gaaggaaaca gcatttaaat ttaaagccct aaagaaggtt 800
   gcgcagttag cagttataaa tagcctggaa aaggcatttt ggaactgggt 850
   agaaaattat ccagatgaat ttacaaaact gtaccagatc ccacagactg 900
   atatggctga atgtgcagaa aagctatttg acttggtgga tggttttgct 950
   gaaagcacca aacgtaaagc agcagtttgg ccactacaaa tcattctcct 1000
   tatcttgtgt ccagaaataa tccaggatat atccaaagac gtggttgatg 1050
   aaaacaacat gaataagaag ttatttctgg acagtctacg aaaagctctt 1100
   gctggccatg gaggaagtag gcagctgaca gaaagtgctg caattgcctg 1150
   tgtcaaactg tgtaaagcaa gtacttacat caattgggaa gataactctg 1200
   tcattttcct acttgttcag tccatggtgg ttgatcttaa gaacctgctt 1250
   tttaatccaa gtaagccatt ctcaagaggc agtcagcctg cagatgtgga 1300
   tctaatgatt gactgccttg tttcttgctt tcgtataagc cctcacaaca 1350
   accaacactt taagatctgc ctggctcaga attcaccttc tacatttcac 1400
   tatgtgctgg taaattcact ccatcgaatc atcaccaatt ccgcattgga 1450
   ttggtggcct aagattgatg ctgtgtattg tcactcggtt gaacttcgaa 1500
   atatgtttgg tgaaacactt cataaagcag tgcaaggttg tggagcacac 1550
   ccagcaatac gaatggcacc gagtcttaca tttaaagaaa aagtaacaag 1600
   ccttaaattt aaagaaaaac ctacagacct ggagacaaga agctataagt 1650
   atcttctctt gtccatggtg aaactaattc atgcagatcc aaagctcttg 1700
   ctttgtaatc caagaaaaca ggggcccgaa acccaaggca gtacagcaga 1750
   attaattaca gggctcgtcc aactggtccc tcagtcacac atgccagaga 1800
   ttgctcagga agcaatggag gctctgctgg ttcttcatca gttagatagc 1850
   attgatttgt ggaatcctga tgctcctgta gaaacatttt gggagattag 1900
   ctcacaaatg cttttttaca tctgcaagaa attaactagt catcaaatgc 1950
   ttagtagcac agaaattctc aagtggttgc gggaaatatt gatctgcagg 2000
   aataaatttc ttcttaaaaa taagcaggca gatagaagtt cctgtcactt 2050
   tctccttttt tacggggtag gatgtgatat tccttctagt ggaaatacca 2100
   gtcaaatgtc catggatcat gaagaattac tacgtactcc tggagcctct 2150
   ctccggaagg gaaaagggaa ctcctctatg gatagtgcag caggatgcag 2200
   cggaaccccc ccgatttgcc gacaagccca gaccaaacta gaagtggccc 2250
   tgtacatgtt tctgtggaac cctgacactg aagctgttct ggttgccatg 2300
   tcctgtttcc gccacctctg tgaggaagca gatatccggt gtggggtgga 2350
   tgaagtgtca gtgcataacc tcttgcccaa ctataacaca ttcatggagt 2400
   ttgcctctgt cagcaatatg atgtcaacag gaagagcagc acttcagaaa 2450
   agagtgatgg cactgctgag gcgcattgag catcccactg caggaaacac 2500
   tgaggcttgg gaagatacac atgcaaaatg ggaacaagca acaaagctaa 2550
   tccttaacta tccaaaagcc aaaatggaag atggccaggc tgctgaaagc 2600
   cttcacaaga ccattgttaa gaggcgaatg tcccatgtga gtggaggagg 2650
   atccatagat ttgtctgaca cagactccct acaggaatgg atcaacatga 2700
   ctggcttcct ttgtgccctt gggggagtgt gcctccagca gagaagcaat 2750
   tctggcctgg caacctatag cccacccatg ggtccagtca gtgaacgtaa 2800
   gggttctatg atttcagtga tgtcttcaga gggaaacgca gatacacctg 2850
   tcagcaaatt tatggatcgg ctgttgtcct taatggtgtg taaccatgag 2900
   aaagtgggac ttcaaatacg gaccaatgtt aaggatctgg tgggtctaga 2950
   attgagtcct gctctgtatc caatgctatt taacaaattg aagaatacca 3000
   tcagcaagtt ttttgactcc caaggacagg ttttattgac tgataccaat 3050
   actcaatttg tagaacaaac catagctata atgaagaact tgctagataa 3100
   tcatactgaa ggcagctctg aacatctagg gcaagctagc attgaaacaa 3150
   tgatgttaaa tctggtcagg tatgttcgtg tgcttgggaa tatggtccat 3200
   gcaattcaaa taaaaacgaa actgtgtcaa ttagttgaag taatgatggc 3250
   aaggagagat gacctctcat tttgccaaga gatgaaattt aggaataaga 3300
   tggtagaata cctgacagac tgggttatgg gaacatcaaa ccaagcagca 3350
   gatgatgatg taaaatgtct tacaagagat ttggaccagg caagcatgga 3400
   agcagtagtt tcacttctag ctggtctccc tctgcagcct gaagaaggag 3450
   atggtgtgga attgatggaa gccaaatcac agttatttct taaatacttc 3500
   acattattta tgaacctttt gaatgactgc agtgaagttg aagatgaaag 3550
   tgcgcaaaca ggtggcagga aacgtggcat gtctcggagg ctggcatcac 3600
   tgaggcactg tacggtcctt gcaatgtcaa acttactcaa tgccaacgta 3650
   gacagtggtc tcatgcactc cataggctta ggttaccaca aggatctcca 3700
   gacaagagct acatttatgg aagttctgac aaaaatcctt caacaaggca 3750
   cagaatttga cacacttgca gaaacagtat tggctgatcg gtttgagaga 3800
   ttggtggaac tggtcacaat gatgggtgat caaggagaac tccctatagc 3850
   gatggctctg gccaatgtgg ttccttgttc tcagtgggat gaactagctc 3900
   gagttctggt tactctgttt gattctcggc atttactcta ccaactgctc 3950
   tggaacatgt tttctaaaga agtagaattg gcagactcca tgcagactct 4000
   cttccgaggc aacagcttgg ccagtaaaat aatgacattc tgtttcaagg 4050
   tatatggtgc tacctatcta caaaaactcc tggatccttt attacgaatt 4100
   gtgatcacat cctctgattg gcaacatgtt agctttgaag tggatcctac 4150
   caggttagaa ccatcagaga gccttgagga aaaccagcgg aacctccttc 4200
   agatgactga aaagttcttc catgccatca tcagttcctc ctcagaattc 4250
   ccccctcaac ttcgaagtgt gtgccactgt ttataccagg caacttgcca 4300
   ctccctactg aataaagcta cagtaaaaga aaaaaaggaa aacaaaaaat 4350
   cagtggttag ccagcgtttc cctcagaaca gcatcggtgc agtaggaagt 4400
   gccatgttcc tcagatttat caatcctgcc attgtctcac cgtatgaagc 4450
   agggatttta gataaaaagc caccacctag aatcgaaagg ggcttgaagt 4500
   taatgtcaaa gatacttcag agtattgcca atcatgttct cttcacaaaa 4550
   gaagaacata tgcggccttt caatgatttt gtgaaaagca actttgatgc 4600
   agcacgcagg tttttccttg atatagcatc tgattgtcct acaagtgatg 4650
   cagtaaatca tagtctttcc ttcataagtg acggcaatgt gcttgcttta 4700
   catcgtctac tctggaacaa tcaggagaaa attgggcagt atctttccag 4750
   caacagggat cataaagctg ttggaagacg accttttgat aagatggcaa 4800
   cacttcttgc atacctgggt cctccagagc acaaacctgt ggcagataca 4850
   cactggtcca gccttaacct taccagttca aagtttgagg aatttatgac 4900
   taggcatcag gtacatgaaa aagaagaatt caaggctttg aaaacgttaa 4950
   gtattttcta ccaagctggg acttccaaag ctgggaatcc tattttttat 5000
   tatgttgcac ggaggttcaa aactggtcaa atcaatggtg atttgctgat 5050
   ataccatgtc ttactgactt taaagccata ttatgcaaag ccatatgaaa 5100
   ttgtagtgga ccttacccat accgggccta gcaatcgctt taaaacagac 5150
   tttctctcta agtggtttgt tgtttttcct ggctttgctt acgacaacgt 5200
   ctccgcagtc tatatctata actgtaactc ctgggtcagg gagtacacca 5250
   agtatcatga gcggctgctg actggcctca aaggtagcaa aaggcttgtt 5300
   ttcatagact gtcctgggaa actggctgag cacatagagc atgaacaaca 5350
   gaaactacct gctgccacct tggctttaga agaggacctg aaggtattcc 5400
   acaatgctct caagctagct cacaaagaca ccaaagtttc tattaaagtt 5450
   ggttctactg ctgtccaagt aacttcagca gagcgaacaa aagtcctagg 5500
   gcaatcagtc tttctaaatg acatttatta tgcttcggaa attgaagaaa 5550
   tctgcctagt agatgagaac cagttcacct taaccattgc aaaccagggc 5600
   acgccgctca ccttcatgca ccaggagtgt gaagccattg tccagtctat 5650
   cattcatatc cggacccgct gggaactgtc acagcccgac tctatccccc 5700
   aacacaccaa gattcggcca aaagatgtcc ctgggacact gctcaatatc 5750
   gcattactta atttaggcag ttctgacccg agtttacggt cagctgccta 5800
   taatcttctg tgtgccttaa cttgtacctt taatttaaaa atcgagggcc 5850
   agttactaga gacatcaggt ttatgtatcc ctgccaacaa caccctcttt 5900
   attgtctcta ttagtaagac actggcagcc aatgagccac acctcacgtt 5950
   agaatttttg gaagagtgta tttctggatt tagcaaatct agtattgaat 6000
   tgaaacacct ttgtttggaa tacatgactc catggctgtc aaatctagtt 6050
   cgtttttgca agcataatga tgatgccaaa cgacaaagag ttactgctat 6100
   tcttgacaag ctgataacaa tgaccatcaa tgaaaaacag atgtacccat 6150
   ctattcaagc aaaaatatgg ggaagccttg ggcagattac agatctgctt 6200
   gatgttgtac tagacagttt catcaaaacc agtgcaacag gtggcttggg 6250
   atcaataaaa gctgaggtga tggcagatac tgctgtagct ttggcttctg 6300
   gaaatgtgaa attggtttca agcaaggtta ttggaaggat gtgcaaaata 6350
   attgacaaga catgcttatc tccaactcct actttagaac aacatcttat 6400
   gtgggatgat attgctattt tagcacgcta catgctgatg ctgtccttca 6450
   acaattccct tgatgtggca gctcatcttc cctacctctt ccacgttgtt 6500
   actttcttag tagccacagg tccgctctcc cttagagctt ccacacatgg 6550
   actggtcatt aatatcattc actctctgtg tacttgttca cagcttcatt 6600
   ttagtgaaga gaccaagcaa gttttgagac tcagtctgac agagttctca 6650
   ttacccaaat tttacttgct gtttggcatt agcaaagtca agtcagctgc 6700
   tgtcattgcc ttccgttcca gttaccggga caggtcattc tctcctggct 6750
   cctatgagag agagactttt gctttgacat ccttggaaac agtcacagaa 6800
   gctttgttgg agatcatgga ggcatgcatg agagatattc caacgtgcaa 6850
   gtggctggac cagtggacag aactagctca aagatttgca ttccaatata 6900
   atccatccct gcaaccaaga gctcttgttg tctttgggtg tattagcaaa 6950
   cgagtgtctc atgggcagat aaagcagata atccgtattc ttagcaaggc 7000
   acttgagagt tgcttaaaag gacctgacac ttacaacagt caagttctga 7050
   tagaagctac agtaatagca ctaaccaaat tacagccact tcttaataag 7100
   gactcgcctc tgcacaaagc cctcttttgg gtagctgtgg ctgtgctgca 7150
   gcttgatgag gtcaacttgt attcagcagg taccgcactt cttgaacaaa 7200
   acctgcatac tttagatagt ctccgtatat tcaatgacaa gagtccagag 7250
   gaagtattta tggcaatccg gaatcctctg gagtggcact gcaagcaaat 7300
   ggatcatttt gttggactca atttcaactc taactttaac tttgcattgg 7350
   ttggacacct tttaaaaggg tacaggcatc cttcacctgc tattgttgca 7400
   agaacagtca gaattttaca tacactacta actctggtta acaaacacag 7450
   aaattgtgac aaatttgaag tgaatacaca gagcgtggcc tacttagcag 7500
   ctttacttac agtgtctgaa gaagttcgaa gtcgctgcag cctaaaacat 7550
   agaaagtcac ttcttcttac tgatatttca atggaaaatg ttcctatgga 7600
   tacatatccc attcatcatg gtgacccttc ctataggaca ctaaaggaga 7650
   ctcagccatg gtcctctccc aaaggttctg aaggatacct tgcagccacc 7700
   tatccaactg tcggccagac cagtccccga gccaggaaat ccatgagcct 7750
   ggacatgggg caaccttctc aggccaacac taagaagttg cttggaacaa 7800
   ggaaaagttt tgatcacttg atatcagaca caaaggctcc taaaaggcaa 7850
   gaaatggaat cagggatcac aacacccccc aaaatgagga gagtagcaga 7900
   aactgattat gaaatggaaa ctcagaggat ttcctcatca caacagcacc 7950
   cacatttacg taaagtttca gtgtctgaat caaatgttct cttggatgaa 8000
   gaagtactta ctgatccgaa gatccaggcg ctgcttctta ctgttctagc 8050
   tacactggta aaatatacca cagatgagtt tgatcaacga attctttatg 8100
   aatacttagc agaggccagt gttgtgtttc ccaaagtctt tcctgttgtg 8150
   cataatttgt tggactctaa gatcaacacc ctgttatcat tgtgccaaga 8200
   tccaaatttg ttaaatccaa tccatggaat tgtgcagagt gtggtgtacc 8250
   atgaagaatc cccaccacaa taccaaacat cttacctgca aagttttggt 8300
   tttaatggct tgtggcggtt tgcaggaccg ttttcaaagc aaacacaaat 8350
   tccagactat gctgagctta ttgttaagtt tcttgatgcc ttgattgaca 8400
   cgtacctgcc tggaattgat gaagaaacca gtgaagaatc cctcctgact 8450
   cccacatctc cttaccctcc tgcactgcag agccagctta gtatcactgc 8500
   caaccttaac ctttctaatt ccatgacctc acttgcaact tcccagcatt 8550
   ccccaggaat cgacaaggag aacgttgaac tctcccctac cactggccac 8600
   tgtaacagtg gacgaactcg ccacggatcc gcaagccaag tgcagaagca 8650
   aagaagcgct ggcagtttca aacgtaatag cattaagaag atcgtgtgaa 8700
   gcttgcttgc tttctttttt aaaatcaact taacatgggc tcttcactag 8750
   tgaccccttc cctgtccttg ccctttcccc ccatgttgta atgctgcact 8800
   tcctgtttta taatgaaccc atccggtttg ccatgttgcc agatgatcaa 8850
   ctcttcgaag ccttgcctaa atttaatgct gccttttctt taactttttt 8900
   tcttctactt ttggcgtgta tctggtatat gtaagtgttc agaacaactg 8950
   caaagaaagt gggaggtcag gaaactttta actgagaaat ctcaattgta 9000
   agagaggat 9009
<210> 13
   <211> 6390
   <212> DNA
   <213> Homo sapiens
<400> 13
   ggtttttgag cccattactg ttggagctac agggagagaa acagaggagg 50
   agact gcaag agat cat t gg aggccgt ggg cacgct ct t t act ccat gt g 100
   t gggacat t c at t gcggaat aacat cggag gagaagt t t c ccagagct at 150
   ggggact t cc cat ccggcgt t cct ggt ct t aggct gt ct t ct cacagggc 200
   t gagcct aat cct ct gccag ct t t cat t ac cct ct at cct t ccaaat gaa 250
   aat gaaaagg t t gt gcagct gaat t cat cc t t t t ct ct ga gat gct t t gg 300
   ggagagt gaa gt gagct ggc agt accccat gt ct gaagaa gagagct ccg 350
   atgtggaaat cagaaatgaa gaaaacaaca gcggcctttt tgtgacggtc 400
   ttggaagtga gcagtgcctc ggcggcccac acagggttgt acacttgcta 450
   t t acaaccac act cagacag aagagaat ga gct t gaaggc aggcacat t t 500
   acatctatgt gccagaccca gatgtagcct ttgtacctct aggaatgacg 550
   gat t at t t ag t cat cgt gga ggat gat gat t ct gccat t a t acct t gt cg 600
   cacaactgat cccgagactc ctgtaacctt acacaacagt gagggggtgg 650
   tacctgcctc ctacgacagc agacagggct ttaatgggac cttcactgta 700
   gggccctata tctgtgaggc caccgtcaaa ggaaagaagt tccagaccat 750
   cccat t t aat gt t t at gct t t aaaagcaac at cagagct g gat ct agaaa 800
   tggaagctct taaaaccgtg tataagtcag gggaaacgat tgtggtcacc 850
   t gt gct gt t t t t aacaat ga ggt ggt t gac ct t caat gga ct t accct gg 900
   agaagtgaaa ggcaaaggca tcacaatgct ggaagaaatc aaagtcccat 950
   ccatcaaatt ggtgtacact ttgacggtcc ccgaggccac ggtgaaagac 1000
   agt ggagat t acgaat gt gc t gcccgccag gct accaggg aggt caaaga 1050
   aat gaagaaa gt cact at t t ct gt ccat ga gaaaggt t t c at t gaaat ca 1100
   aacccacct t cagccagt t g gaagct gt ca acct gcat ga agt caaacat 1150
   t t t gt t gt ag aggt gcgggc ct acccacct cccaggat at cct ggct gaa 1200
   aaacaat ct g act ct gat t g aaaat ct cac t gagat cacc act gat gt gg 1250
   aaaagat t ca ggaaat aagg t at cgaagca aat t aaagct gat ccgt gct 1300
   aaggaagaag acagtggcca ttatactatt gtagctcaaa atgaagatgc 1350
   t gt gaagagc t at act t t t g aact gt t aac t caagt t cct t cat ccat t c 1400
   t ggact t ggt cgat gat cac cat ggct caa ct gggggaca gacggt gagg 1450
   tgcacagctg aaggcacgcc gcttcctgat attgagtgga tgatatgcaa 1500
   agat at t aag aaat gt aat a at gaaact t c ct ggact at t t t ggccaaca 1550
   atgtctcaaa catcatcacg gagatccact cccgagacag gagtaccgtg 1600
   gagggccgtg tgactttcgc caaagtggag gagaccatcg ccgtgcgatg 1650
   cctggctaag aatctccttg gagctgagaa ccgagagctg aagctggtgg 1700
   ctcccaccct gcgttctgaa ctcacggtgg ctgctgcagt cctggtgctg 1750
   ttggtgattg tgatcatctc acttattgtc ctggttgtca tttggaaaca 1800
   gaaaccgagg tatgaaattc gctggagggt cattgaatca atcagcccag 1850
   atggacatga atatatttat gtggacccga tgcagctgcc ttatgactca 1900
   agatgggagt ttccaagaga tggactagtg cttggtcggg tcttggggtc 1950
   tggagcgttt gggaaggtgg ttgaaggaac agcctatgga ttaagccggt 2000
   cccaacctgt catgaaagtt gcagtgaaga tgctaaaacc cacggccaga 2050
   tccagtgaaa aacaagctct catgtctgaa ctgaagataa tgactcacct 2100
   ggggccacat ttgaacattg taaacttgct gggagcctgc accaagtcag 2150
   gccccattta catcatcaca gagtattgct tctatggaga tttggtcaac 2200
   tatttgcata agaataggga tagcttcctg agccaccacc cagagaagcc 2250
   aaagaaagag ctggatatct ttggattgaa ccctgctgat gaaagcacac 2300
   ggagctatgt tattttatct tttgaaaaca atggtgacta catggacatg 2350
   aagcaggctg atactacaca gtatgtcccc atgctagaaa ggaaagaggt 2400
   ttctaaatat tccgacatcc agagatcact ctatgatcgt ccagcctcat 2450
   ataagaagaa atctatgtta gactcagaag tcaaaaacct cctttcagat 2500
   gataactcag aaggccttac tttattggat ttgttgagct tcacctatca 2550
   agttgcccga ggaatggagt ttttggcttc aaaaaattgt gtccaccgtg 2600
   atctggctgc tcgcaacgtc ctcctggcac aaggaaaaat tgtgaagatc 2650
   tgtgactttg gcctggccag agacatcatg catgattcga actatgtgtc 2700
   gaaaggcagt acctttctgc ccgtgaagtg gatggctcct gagagcatct 2750
   ttgacaacct ctacaccaca ctgagtgatg tctggtctta tggcattctg 2800
   ctctgggaga tcttttccct tggtggcacc ccttaccccg gcatgatggt 2850
   ggattctact ttctacaata agatcaagag tgggtaccgg atggccaagc 2900
   ctgaccacgc taccagtgaa gtctacgaga tcatggtgaa atgctggaac 2950
   agtgagccgg agaagagacc ctcctttcac cacctgagtg agattgtgga 3000
   gaatctgctg cctggacaat ataaaaagag ttatgaaaaa attcacctgg 3050
   acttcctgaa gagtgaccat cctgctgtgg cacgcatgcg tgtggactca 3100
   gacaatgcat acattggtgt cacctacaaa aacgaggaag acaagctgaa 3150
   ggactgggag ggtggtctgg atgagcagag actgagcgct gacagtggct 3200
   acatcattcc tctgcctgac attgaccctg tccctgagga ggaggacctg 3250
   ggcaagagga acagacacag ctcgcagacc tctgaagaga gtgccattga 3300
   gacgggttcc agcagttcca ccttcatcaa gagagaggac gagaccattg 3350
   aagacat cga cat gat ggat gacat cggca t agact ct t c agacct ggt g 3400
   gaagacagct tcctgtaact ggcggattcg aggggttcct tccacttctg 3450
   gggccacct c t ggat cccgt t cagaaaacc act t t at t gc aat gcagagg 3500
   ttgagaggag gacttggttg atgtttaaag agaagttccc agccaagggc 3550
   ct cggggagc gt t ct aaat a t gaat gaat g ggat at t t t g aaat gaact t 3600
   t gt cagt gt t gcct ct t gca at gcct cagt agcat ct cag t ggt gt gt ga 3650
   agtttggaga tagatggata agggaataat aggccacaga aggtgaactt 3700
   tgtgcttcaa ggacattggt gagagtccaa cagacacaat ttatactgcg 3750
   acagaact t c agcat t gt aa t t at gt aaat aact ct aacc aaggct gt gt 3800
   t t agat t gt a t t aact at ct t ct t t ggact t ct gaagaga ccact caat c 3850
   cat ccat gt a ct t ccct ct t gaaacct gat gt cagct gct gt t gaact t t 3900
   t t aaagaagt gcat gaaaaa ccat t t t t ga acct t aaaag gt act ggt ac 3950
   t at agcat t t t gct at ct t t t t t agt gt t a aagagat aaa gaat aat aat 4000
   t aaccaacct t gt t t aat ag at t t gggt ca t t t agaagcc t gacaact ca 4050
   t t t t cat at t gt aat ct at g t t t at aat ac t act act gt t at cagt aat g 4100
   ct aaat gt gt aat aat gt aa cat gat t t cc ct ccagagaa agcacaat t t 4150
   aaaacaat cc t t act aagt a ggt gat gagt t t gacagt t t t t gacat t t a 4200
   t at t aaat aa cat gt t t ct c t at aaagt at ggt aat agct t t agt gaat t 4250
   aaat t t agt t gagcat agag aacaaagt aa aagt agt gt t gt ccaggaag 4300
   t cagaat t t t t aact gt act gaat aggt t c cccaat ccat cgt at t aaaa 4350
   aacaat t aac t gccct ct ga aat aat ggga t t agaaacaa acaaaact ct 4400
   t aagt cct aa aagt t ct caa t gt agaggca t aaacct gt g ct gaacat aa 4450
   cttctcatgt atattaccca atggaaaata taatgatcag caaaaagact 4500
   ggatttgcag aagttttttt tttttttttc ttcatgcctg atgaaagctt 4550
   t ggcgacccc aat at at gt a t t t t t t gaat ct at gaacct gaaaagggt c 4600
   agaaggatgc ccagacatca gcctccttct ttcacccctt accccaaaga 4650
   gaaagagt t t gaaact cgag accat aaaga t at t ct t t ag t ggaggct gg 4700
   atgtgcatta gcctggatcc tcagttctca aatgtgtgtg gcagccagga 4750
   t gact agat c ct gggt t t cc at cct t gaga t t ct gaagt a t gaagt ct ga 4800
   gggaaaccag agtctgtatt tttctaaact ccctggctgt tctgatcggc 4850
   cagt t t t cgg aaacact gac t t aggt t t ca ggaagt t gcc at gggaaaca 4900
   aataatttga actttggaac agggttggca ttcaaccacg caggaagcct 4950
   act at t t aaa t cct t ggct t caggt t agt g acat t t aat g ccat ct agct 5000
   agcaattgcg accttaattt aactttccag tcttagctga ggctgagaaa 5050
   gctaaagttt ggttttgaca ggttttccaa aagtaaagat gctacttccc 5100
   actgtatggg ggagattgaa ctttccccgt ctcccgtctt ctgcctccca 5150
   ctccataccc cgccaaggaa aggcatgtac aaaaattatg caattcagtg 5200
   ttccaagtct ctgtgtaacc agctcagtgt tttggtggaa aaaacatttt 5250
   aagttttact gataatttga ggttagatgg gaggatgaat tgtcacatct 5300
   atccacactg tcaaacaggt tggtgtgggt tcattggcat tctttgcaat 5350
   actgcttaat tgctgatacc atatgaatga aacatgggct gtgattactg 5400
   caatcactgt gctatcggca gatgatgctt tggaagatgc agaagcaata 5450
   ataaagtact tgactaccta ctggtgtaat ctcaatgcaa gccccaactt 5500
   tcttatccaa ctttttcata gtaagtgcga agactgagcc agattggcca 5550
   attaaaaacg aaaacctgac taggttctgt agagccaatt agacttgaaa 5600
   tacgtttgtg tttctagaat cacagctcaa gcattctgtt tatcgctcac 5650
   tctcccttgt acagccttat tttgttggtg ctttgcattt tgatattgct 5700
   gtgagccttg catgacatca tgaggccgga tgaaacttct cagtccagca 5750
   gtttccagtc ctaacaaatg ctcccacctg aatttgtata tgactgcatt 5800
   tgtgtgtgtg tgtgtgtttt cagcaaattc cagatttgtt tccttttggc 5850
   ctcctgcaaa gtctccagaa gaaaatttgc caatctttcc tactttctat 5900
   ttttatgatg acaatcaaag ccggcctgag aaacactatt tgtgactttt 5950
   taaacgatta gtgatgtcct taaaatgtgg tctgccaatc tgtacaaaat 6000
   ggtcctattt ttgtgaagag ggacataaga taaaatgatg ttatacatca 6050
   atatgtatat atgtatttct atatagactt ggagaatact gccaaaacat 6100
   ttatgacaag ctgtatcact gccttcgttt atattttttt aactgtgata 6150
   atccccacag gcacattaac tgttgcactt ttgaatgtcc aaaatttata 6200
   ttttagaaat aataaaaaga aagatactta catgttccca aaacaatggt 6250
   gtggtgaatg tgtgagaaaa actaacttga tagggtctac caatacaaaa 6300
   tgtattacga atgcccctgt tcatgttttt gttttaaaac gtgtaaatga 6350
   agatctttat atttcaataa atgatatata atttaaagtt 6390
<210> 14
   <211> 5711
   <212> DNA
   <213> Homo sapiens
<400> 14
   ct cct gaggc t gccagcagc cagcagt gac t gcccgccct at ct gggacc 50
   caggatcgct ctgtgagcaa cttggagcca gagaggagat caacaaggag 100
   gaggagagag ccggcccctc agccctgctg cccagcagca gcctgtgctc 150
   gccctgccca acgcagacag ccagacccag ggcggcccct ctggcggctc 200
   t gct cct ccc gaaggat gct t ggggagt ga ggcgaagct g ggccgct cct 250
   ctcccctaca gcagccccct tcctccatcc ctctgttctc ctgagccttc 300
   aggagcct gc accagt cct g cct gt cct t c t act cagct g t t acccact c 350
   t gggaccagc agt ct t t ct g at aact ggga gagggcagt a aggaggact t 400
   cctggagggg gtgactgtcc agagcctgga actgtgccca caccagaagc 450
   catcagcagc aaggacacca tgcggcttcc gggtgcgatg ccagctctgg 500
   ccct caaagg cgagct gct g t t gct gt ct c t cct gt t act t ct ggaacca 550
   cagatctctc agggcctggt cgtcacaccc ccggggccag agcttgtcct 600
   caat gt ct cc agcacct t cg t t ct gacct g ct cgggt t ca gct ccggt gg 650
   tgtgggaacg gatgtcccag gagcccccac aggaaatggc caaggcccag 700
   gat ggcacct t ct ccagcgt gct cacact g accaacct ca ct gggct aga 750
   cacgggagaa tacttttgca cccacaatga ctcccgtgga ctggagaccg 800
   at gagcggaa acggct ct ac at ct t t gt gc cagat cccac cgt gggct t c 850
   ct ccct aat g at gccgagga act at t cat c t t t ct cacgg aaat aact ga 900
   gat caccat t ccat gccgag t aacagaccc acagct ggt g gt gacact gc 950
   acgagaagaa aggggacgtt gcactgcctg tcccctatga tcaccaacgt 1000
   ggcttttctg gtatctttga ggacagaagc tacatctgca aaaccaccat 1050
   t ggggacagg gaggt ggat t ct gat gcct a ct at gt ct ac agact ccagg 1100
   t gt cat ccat caacgt ct ct gt gaacgcag t gcagact gt ggt ccgccag 1150
   ggt gagaaca t caccct cat gt gcat t gt g at cgggaat g aggt ggt caa 1200
   cttcgagtgg acataccccc gcaaagaaag tgggcggctg gtggagccgg 1250
   t gact gact t cct ct t ggat at gcct t acc acat ccgct c cat cct gcac 1300
   at ccccagt g ccgagt t aga agact cgggg acct acacct gcaat gt gac 1350
   ggagagt gt g aat gaccat c aggat gaaaa ggccat caac at caccgt gg 1400
   t t gagagcgg ct acgt gcgg ct cct gggag aggt gggcac act acaat t t 1450
   gct gagct gc at cggagccg gacact gcag gt agt gt t cg aggcct accc 1500
   accgcccact gtcctgtggt tcaaagacaa ccgcaccctg ggcgactcca 1550
   gcgct ggcga aat cgccct g t ccacgcgca acgt gt cgga gacccggt at 1600
   gt gt cagagc t gacact ggt t cgcgt gaag gt ggcagagg ct ggccact a 1650
   caccat gcgg gcct t ccat g aggat gct ga ggt ccagct c t cct t ccagc 1700
   tacagatcaa tgtccctgtc cgagtgctgg agctaagtga gagccaccct 1750
   gacagtgggg aacagacagt ccgctgtcgt ggccggggca tgccccagcc 1800
   gaacatcatc tggtctgcct gcagagacct caaaaggtgt ccacgtgagc 1850
   tgccgcccac gctgctgggg aacagttccg aagaggagag ccagctggag 1900
   actaacgtga cgtactggga ggaggagcag gagtttgagg tggtgagcac 1950
   act gcgt ct g cagcacgt gg at cggccact gt cggt gcgc t gcacgct gc 2000
   gcaacgctgt gggccaggac acgcaggagg tcatcgtggt gccacactcc 2050
   t t gccct t t a aggt ggt ggt gat ct cagcc at cct ggccc t ggt ggt gct 2100
   caccatcatc tcccttatca tcctcatcat gctttggcag aagaagccac 2150
   gt t acgagat ccgat ggaag gt gat t gagt ct gt gagct c t gacggccat 2200
   gagt acat ct acgt ggaccc cat gcagct g ccct at gact ccacgt ggga 2250
   gct gccgcgg gaccagct t g t gct gggacg caccct cggc t ct ggggcct 2300
   t t gggcaggt ggt ggaggcc acggct cat g gcct gagcca t t ct caggcc 2350
   acgat gaaag t ggccgt caa gat gct t aaa t ccacagccc gcagcagt ga 2400
   gaagcaagcc cttatgtcgg agctgaagat catgagtcac cttgggcccc 2450
   acctgaacgt ggtcaacctg ttgggggcct gcaccaaagg aggacccatc 2500
   t at at cat ca ct gagt act g ccgct acgga gacct ggt gg act acct gca 2550
   ccgcaacaaa cacaccttcc tgcagcacca ctccgacaag cgccgcccgc 2600
   ccagcgcgga gctctacagc aatgctctgc ccgttgggct ccccctgccc 2650
   agccatgtgt ccttgaccgg ggagagcgac ggtggctaca tggacatgag 2700
   caaggacgag tcggtggact atgtgcccat gctggacatg aaaggagacg 2750
   t caaat at gc agacat cgag t cct ccaact acat ggcccc t t acgat aac 2800
   t acgt t ccct ct gcccct ga gaggacct gc cgagcaact t t gat caacga 2850
   gt ct ccagt g ct aagct aca t ggacct cgt gggct t cagc t accaggt gg 2900
   ccaat ggcat ggagt t t ct g gcct ccaaga act gcgt cca cagagacct g 2950
   gcggct agga acgt gct cat ct gt gaaggc aagct ggt ca agat ct gt ga 3000
   ct t t ggcct g gct cgagaca t cat gcggga ct cgaat t ac at ct ccaaag 3050
   gcagcacct t t t t gcct t t a aagt ggat gg ct ccggagag cat ct t caac 3100
   agcct ct aca ccaccct gag cgacgt gt gg t cct t cggga t cct gct ct g 3150
   ggagat ct t c acct t gggt g gcacccct t a cccagagct g cccat gaacg 3200
   agcagt t ct a caat gccat c aaacggggt t accgcat ggc ccagcct gcc 3250
   catgcctccg acgagatcta tgagatcatg cagaagtgct gggaagagaa 3300
   gtttgagatt cggcccccct tctcccagct ggtgctgctt ctcgagagac 3350
   t gt t gggcga aggt t acaaa aagaagt acc agcaggt gga t gaggagt t t 3400
   ctgaggagtg accacccagc catccttcgg tcccaggccc gcttgcctgg 3450
   gt t ccat ggc ct ccgat ct c ccct ggacac cagct ccgt c ct ct at act g 3500
   ccgtgcagcc caatgagggt gacaacgact atatcatccc cctgcctgac 3550
   cccaaacccg aggttgctga cgagggccca ctggagggtt cccccagcct 3600
   agccagct cc accct gaat g aagt caacac ct cct caacc at ct cct gt g 3650
   acagccccct ggagccccag gacgaaccag agccagagcc ccagcttgag 3700
   ctccaggtgg agccggagcc agagctggaa cagttgccgg attcggggtg 3750
   ccctgcgcct cgggcggaag cagaggatag cttcctgtag ggggctggcc 3800
   cctaccctgc cctgcctgaa gctccccccc tgccagcacc cagcatctcc 3850
   tggcctggcc tgaccgggct tcctgtcagc caggctgccc ttatcagctg 3900
   tccccttctg gaagctttct gctcctgacg tgttgtgccc caaaccctgg 3950
   ggctggctta ggaggcaaga aaactgcagg ggccgtgacc agccctctgc 4000
   ctccagggag gccaactgac tctgagccag ggttccccca gggaactcag 4050
   ttttcccata tgtaaaatgg gaaagttagg cttgatgacc cagaatctag 4100
   gattctctcc ctggctgaca ggtggggaga ccgaatccct ccctgggaag 4150
   attcttggag ttactgaggt ggtaaattaa cttttttctg ttcagccagc 4200
   tacccctcaa ggaatcatag ctctctcctc gcacttttat ccacccagga 4250
   gctagggaag agaccctagc ctccctggct gctggctgag ctagggccta 4300
   gccttgagca gtgttgcctc atccagaaga aagccagtct cctccctatg 4350
   atgccagtcc ctgcgttccc tggcccgagc tggtctgggg ccattaggca 4400
   gcctaattaa tgctggaggc tgagccaagt acaggacacc cccagcctgc 4450
   agcccttgcc cagggcactt ggagcacacg cagccatagc aagtgcctgt 4500
   gtccctgtcc ttcaggccca tcagtcctgg ggctttttct ttatcaccct 4550
   cagtcttaat ccatccacca gagtctagaa ggccagacgg gccccgcatc 4600
   tgtgatgaga atgtaaatgt gccagtgtgg agtggccacg tgtgtgtgcc 4650
   agtatatggc cctggctctg cattggacct gctatgaggc tttggaggaa 4700
   tccctcaccc tctctgggcc tcagtttccc cttcaaaaaa tgaataagtc 4750
   ggacttatta actctgagtg ccttgccagc actaacattc tagagtattc 4800
   caggtggttg cacatttgtc cagatgaagc aaggccatat accctaaact 4850
   tccatcctgg gggtcagctg ggctcctggg agattccaga tcacacatca 4900
   cactctgggg actcaggaac catgcccctt ccccaggccc ccagcaagtc 4950
   tcaagaacac agctgcacag gccttgactt agagtgacag ccggtgtcct 5000
   ggaaagcccc cagcagctgc cccagggaca tgggaagacc acgggacctc 5050
   tttcactacc cacgatgacc tccgggggta tcctgggcaa aagggacaaa 5100
   gagggcaaat gagatcacct cctgcagccc accactccag cacctgtgcc 5150
   gaggtctgcg tcgaagacag aatggacagt gaggacagtt atgtcttgta 5200
   aaagacaaga agcttcagat gggtacccca agaaggatgt gagaggtggg 5250
   cgctttggag gtttgcccct cacccaccag ctgccccatc cctgaggcag 5300
   cgctccatgg gggtatggtt ttgtcactgc ccagacctag cagtgacatc 5350
   t cat t gt ccc cagcccagt g ggcat t ggag gt gccagggg agt cagggt t 5400
   gtagccaaga cgcccccgca cggggagggt tgggaagggg gtgcaggaag 5450
   ct caacccct ct gggcacca accct gcat t gcaggt t ggc acct t act t c 5500
   cct gggat cc ccagagt t gg t ccaaggagg gagagt gggt t ct caat acg 5550
   gt accaaaga t at aat cacc t aggt t t aca aat at t t t t a ggact cacgt 5600
   t aact cacat t t at acagca gaaat gct at t t t gt at gct gt t gagt t t t 5650
   t ct at ct gt g t act t t t t t t t aagggaaag at t t t aat at t aaacct ggt 5700
   gct t ct cact c 5711
<210> 15
   <211> 2115
   <212> DNA
   <213> Homo sapiens
<400> 15
   atgagtgctg cagtgactgc agggaagctg gcacgggcac cggccgaccc 50
   tgggaaagcc ggggtccccg gagttgcagc tcccggagct ccggcggcgg 100
   ctccaccggc gaaagagatc ccggaggtcc tagtggaccc acgcagccgg 150
   cggcgctatg tgcggggccg ctttttgggc aagggcggct ttgccaagtg 200
   cttcgagatc tcggacgcgg acaccaagga ggtgttcgcg ggcaagattg 250
   tgcctaagtc tctgctgctc aagccgcacc agagggagaa gatgtccatg 300
   gaaatatcca ttcaccgcag cctcgcccac cagcacgtcg taggattcca 350
   cggctttttc gaggacaacg acttcgtgtt cgtggtgttg gagctctgcc 400
   gccggaggtc tctcctggag ctgcacaaga ggaggaaagc cctgactgag 450
   cctgaggccc gatactacct acggcaaatt gtgcttggct gccagtacct 500
   gcaccgaaac cgagttattc atcgagacct caagctgggc aaccttttcc 550
   tgaatgaaga tctggaggtg aaaatagggg attttggact ggcaaccaaa 600
   gtcgaatatg acggggagag gaagaagacc ctgtgtggga ctcctaatta 650
   catagctccc gaggtgctga gcaagaaagg gcacagtttc gaggtggatg 700
   tgtggtccat tgggtgtatc atgtatacct tgttagtggg caaaccacct 750
   tttgagactt cttgcctaaa agagacctac ctccggatca agaagaatga 800
   atacagtatt cccaagcaca tcaaccccgt ggccgcctcc ctcatccaga 850
   agatgcttca gacagatccc actgcccgcc caaccattaa cgagctgctt 900
   aatgacgagt tctttacttc tggctatatc cctgcccgtc tccccatcac 950
   ctgcctgacc attccaccaa ggttttcgat tgctcccagc agcctggacc 1000
   ccagcaaccg gaagcccctc acagtcctca ataaaggctt ggagaacccc 1050
   ctgcctgagc gtccccggga aaaagaagaa ccagtggttc gagagacagg 1100
   tgaggtggtc gactgccacc tcagtgacat gctgcagcag ctgcacagtg 1150
   tcaatgcctc caagccctcg gagcgtgggc tggtcaggca agaggaggct 1200
   gaggat cct g cct gcat ccc cat ct t ct gg gt cagcaagt gggt ggact a 1250
   t t cggacaag t acggcct t g ggt at cagct ct gt gat aac agcgt ggggg 1300
   t gct ct t caa t gact caaca cgcct cat cc t ct acaat ga t ggt gacagc 1350
   ct gcagt aca t agagcgt ga cggcact gag t cct acct ca ccgt gagt t c 1400
   ccat cccaac t cct t gat ga agaagat cac cct cct t aaa t at t t ccgca 1450
   attacatgag cgagcacttg ctgaaggcag gtgccaacat cacgccgcgc 1500
   gaaggt gat g agct cgcccg gct gccct ac ct acggacct ggt t ccgcac 1550
   ccgcagcgcc atcatcctgc acctcagcaa cggcagcgtg cagatcaact 1600
   t ct t ccagga t cacaccaag ct cat ct t gt gcccact gat ggcagccgt g 1650
   acctacatcg acgagaagcg ggacttccgc acataccgcc tgagtctcct 1700
   ggaggagt ac ggct gct gca aggagct ggc cagccggct c cgct acgccc 1750
   gcact at ggt ggacaagct g ct gagct cac gct cggccag caaccgt ct c 1800
   aaggcct cct aat agct gcc ct cccct ccg gact ggt gcc ct cct cact c 1850
   ccacct gcat ct ggggccca t act ggt t gg ct cccgcggt gccat gt ct g 1900
   cagtgtgccc cccagccccg gtggctgggc agagctgcat catccttgca 1950
   ggt gggggt t gct gt at aag t t at t t t t gt acat gt t cgg gt gt gggt t c 2000
   tacagccttg tccccctccc cctcaacccc accatatgaa ttgtacagaa 2050
   t at t t ct at t gaat t cggaa ct gt cct t t c ct t ggct t t a t gcacat t aa 2100
   acagat gt ga at at t 2115
<210> 16
   <211> 3236
   <212> DNA
   <213> Homo sapiens
<400> 16
   cgtggccctg tggcagccga gccatggttt ctaaactgag ccagctgcag 50
   acggagctcc tggcggccct gctcgagtca gggctgagca aagaggcact 100
   gatccaggca ctgggtgagc cggggcccta cctcctggct ggagaaggcc 150
   ccctggacaa gggggagtcc tgcggcggcg gtcgagggga gctggctgag 200
   ctgcccaatg ggctggggga gactcggggc tccgaggacg agacggacga 250
   cgatggggaa gacttcacgc cacccatcct caaagagctg gagaacctca 300
   gccctgagga ggcggcccac cagaaagccg tggtggagac ccttctgcag 350
   gaggacccgt ggcgtgtggc gaagatggtc aagtcctacc tgcagcagca 400
   caacatccca cagcgggagg tggtcgatac cactggcctc aaccagtccc 450
   acctgtccca acacctcaac aagggcactc ccatgaagac gcagaagcgg 500
   gccgccctgt acacctggta cgtccgcaag cagcgagagg tggcgcagca 550
   gttcacccat gcagggcagg gagggctgat tgaagagccc acaggtgatg 600
   agctaccaac caagaagggg cggaggaacc gtttcaagtg gggcccagca 650
   tcccagcaga tcctgttcca ggcctatgag aggcagaaga accctagcaa 700
   ggaggagcga gagacgctag tggaggagtg caatagggcg gaatgcatcc 750
   agagaggggt gtccccatca caggcacagg ggctgggctc caacctcgtc 800
   acggaggtgc gtgtctacaa ctggtttgcc aaccggcgca aagaagaagc 850
   cttccggcac aagctggcca tggacacgta cagcgggccc cccccagggc 900
   caggcccggg acctgcgctg cccgctcaca gctcccctgg cctgcctcca 950
   cctgccctct cccccagtaa ggtccacggt gtgcgctatg gacagcctgc 1000
   gaccagtgag actgcagaag taccctcaag cagcggcggt cccttagtga 1050
   cagtgtctac acccctccac caagtgtccc ccacgggcct ggagcccagc 1100
   cacagcctgc tgagtacaga agccaagctg gtctcagcag ctgggggccc 1150
   cctcccccct gtcagcaccc tgacagcact gcacagcttg gagcagacat 1200
   ccccaggcct caaccagcag ccccagaacc tcatcatggc ctcacttcct 1250
   ggggtcatga ccatcgggcc tggtgagcct gcctccctgg gtcctacgtt 1300
   caccaacaca ggtgcctcca ccctggtcat cggcctggcc tccacgcagg 1350
   cacagagtgt gccggtcatc aacagcatgg gcagcagcct gaccaccctg 1400
   cagcccgtcc agttctccca gccgctgcac ccctcctacc agcagccgct 1450
   catgccacct gtgcagagcc atgtgaccca gagccccttc atggccacca 1500
   tggctcagct gcagagcccc cacgccctct acagccacaa gcccgaggtg 1550
   gcccagtaca cccacacggg cctgctcccg cagactatgc tcatcaccga 1600
   caccaccaac ctgagcgccc tggccagcct cacgcccacc aagcaggtct 1650
   tcacctcaga cactgaggcc tccagtgagt ccgggcttca cacgccggca 1700
   tctcaggcca ccaccctcca cgtccccagc caggaccctg ccagcatcca 1750
   gcacctgcag ccggcccacc ggctcagcgc cagccccaca gtgtcctcca 1800
   gcagcctggt gctgtaccag agctcagact ccagcaatgg ccagagccac 1850
   ctgctgccat ccaaccacag cgtcatcgag accttcatct ccacccagat 1900
   ggcctcttcc tcccagtaac cacggcacct gggccctggg gcctgtactg 1950
   cctgcttggg gggtgatgag ggcagcagcc agccctgcct ggaggacctg 2000
   agcctgccga gcaaccgtgg cccttcctgg acagctgtgc ctcgctcccc 2050
   actctgctct gatgcatcag aaagggaggg ctctgaggcg ccccaacccg 2100
   tggaggctgc tcggggtgca caggaggggg tcgtggagag ctaggagcaa 2150
   agcctgttca tggcagatgt aggagggact gtcgctgctt cgtgggatac 2200
   agtcttctta cttggaactg aagggggcgg cctatgactt gggcaccccc 2250
   agcctgggcc tatggagagc cctgggaccg ctacaccact ctggcagcca 2300
   cacttctcag gacacaggcc tgtgtagctg tgacctgctg agctctgaga 2350
   ggccctggat cagcgtggcc ttgttctgtc accaatgtac ccaccgggcc 2400
   actccttcct gccccaactc cttccagcta gtgacccaca tgccatttgt 2450
   actgacccca tcacctactc acacaggcat ttcctgggtg gctactctgt 2500
   gccagagcct ggggctctaa cgcctgagcc cagggaggcc gaagctaaca 2550
   gggaaggcag gcagggctct cctggcttcc catccccagc gattccctct 2600
   cccaggcccc atgacctcca gctttcctgt atttgttccc aagagcatca 2650
   tgcctctgag gccagcctgg cctcctgcct ctactgggaa ggctacttcg 2700
   gggctgggaa gtcgtcctta ctcctgtggg agcctcgcaa cccgtgccaa 2750
   gtccaggtcc tggtggggca gctcctctgt ctcgagcgcc ctgcagaccc 2800
   tgcccttgtt tggggcagga gtagctgagc tcacaaggca gcaaggcccg 2850
   agcagctgag cagggccggg gaactggcca agctgaggtg cccaggagaa 2900
   gaaagaggtg accccagggc acaggagcta cctgtgtgga caggactaac 2950
   actcagaagc ctgggggcct ggctggctga gggcagttcg cagccaccct 3000
   gaggagtctg aggtcctgag cactgccagg agggacaaag gagcctgtga 3050
   acccaggaca agcatggtcc cacatccctg ggcctgctgc tgagaacctg 3100
   gccttcagtg taccgcgtct accctgggat tcaggaaaag gcctggggtg 3150
   acccggcacc ccctgcagct tgtagccagc cggggcgagt ggcacgttta 3200
   tttaactttt agtaaagtca aggagaaatg cggtgg 3236
<210> 17
   <211> 2621
   <212> DNA
   <213> Homo sapiens
<400> 17
<210> 18
   <211> 4400
   <212> DNA
   <213> Homo sapiens
<400> 18
   cctcctccac agtggtgagg tcacagcccc ttggagccct ccctcttccc 50
   acccctcccg ctcccgggtc tcctttggcc tggggtaacc cgaggtgcag 100
   agctgagaat gaggcgattt cggaggatgg agaaatagcc ccgagtcccg 150
   tggaaaatga ggccggcgga cttgctgcag ctggtgctgc tgctcgacct 200
   gcccagggac ctgggcggaa tggggtgttc gtctccaccc tgcgagtgcc 250
   atcaggagga ggacttcaga gtcacctgca aggatattca acgcatcccc 300
   agcttaccgc ccagtacgca gactctgaag cttattgaga ctcacctgag 350
   aactattcca agtcatgcat tttctaatct gcccaatatt tccagaatct 400
   acgtatctat agatgtgact ctgcagcagc tggaatcaca ctccttctac 450
   aatttgagta aagtgactca catagaaatt cggaatacca ggaacttaac 500
   ttacatagac cctgatgccc tcaaagagct ccccctccta aagttccttg 550
   gcattttcaa cactggactt aaaatgttcc ctgacctgac caaagtttat 600
   tccactgata tattctttat acttgaaatt acagacaacc cttacatgac 650
   gtcaatccct gtgaatgctt ttcagggact atgcaatgaa accttgacac 700
   tgaagctgta caacaatggc tttacttcag tccaaggata tgctttcaat 750
   gggacaaagc tggatgctgt ttacctaaac aagaataaat acctgacagt 800
   tattgacaaa gatgcatttg gaggagtata cagtggacca agcttgctgg 850
   acgtgtctca aaccagtgtc actgcccttc catccaaagg cctggagcac 900
   ctgaaggaac tgatagcaag aaacacctgg actcttaaga aacttccact 950
   ttccttgagt ttccttcacc tcacacgggc tgacctttct tacccaagcc 1000
   actgctgtgc ttttaagaat cagaagaaaa tcagaggaat ccttgagtcc 1050
   ttgatgtgta atgagagcag tatgcagagc ttgcgccaga gaaaatctgt 1100
   gaatgccttg aatagccccc tccaccagga atatgaagag aatctgggtg 1150
   acagcattgt tgggtacaag gaaaagtcca agttccagga tactcataac 1200
   aacgctcatt attacgtctt ctttgaagaa caagaggatg agatcattgg 1250
   ttttggccag gagctcaaaa acccccagga agagactcta caagcttttg 1300
   acagccatta tgactacacc atatgtgggg acagtgaaga catggtgtgt 1350
   acccccaagt ccgatgagtt caacccgtgt gaagacataa tgggctacaa 1400
   gttcctgaga attgtggtgt ggttcgttag tctgctggct ctcctgggca 1450
   atgtctttgt cctgcttatt ctcctcacca gccactacaa actgaacgtc 1500
   ccccgctttc tcatgtgcaa cctggccttt gcggatttct gcatggggat 1550
   gtacctgctc ctcatcgcct ctgtagacct ctactcacac tctgagtact 1600
   acaaccatgc catcgactgg cagacaggcc ctgggtgcaa cacggctggt 1650
   ttcttcactg tctttgcaag cgagttatcg gtgtatacgc tgacggtcat 1700
   caccctggag cgctggtatg ccatcacctt cgccatgcgc ctggaccgga 1750
   agatccgcct caggcacgca tgtgccatca tggttggggg ctgggtttgc 1800
   tgcttccttc tcgccctgct tcctttggtg ggaataagta gctatgccaa 1850
   agtcagtatc tgcctgccca tggacaccga gacccctctt gctctggcat 1900
   atattgtttt tgttctgacg ctcaacatag ttgccttcgt catcgtctgc 1950
   tgctgttatg tgaagatcta catcacagtc cgaaatccgc agtacaaccc 2000
   aggggacaaa gataccaaaa ttgccaagag gatggctgtg ttgatcttca 2050
   ccgacttcat atgcatggcc ccaatctcat tctatgctct gtcagcaatt 2100
   ctgaacaagc ctctcatcac tgttagcaac tccaaaatct tgctggtact 2150
   cttctatcca cttaactcct gtgccaatcc attcctctat gctattttca 2200
   ccaaggcctt ccagagggat gtgttcatcc tactcagcaa gtttggcatc 2250
   tgtaaacgcc aggctcaggc ataccggggg cagagggttc ctccaaagaa 2300
   cagcactgat attcaggttc aaaaggttac ccacgagatg aggcagggtc 2350
   tccacaacat ggaagatgtc tatgaactga ttgaaaactc ccatctaacc 2400
   ccaaagaagc aaggccaaat ctcagaagag tatatgcaaa cggttttgta 2450
   agttaacact acactactca caatggtagg ggaacttaca aaataatagt 2500
   ttcttgaata tgcattccaa tcccatgaca cccccaacac atagctgccc 2550
   tcactcttgt gcaggcgatg tttcaatgtt tcatggggca agagtttatc 2600
   tctggagagt gattagtatt aacctaatca ttgcccccaa gaaggaagtt 2650
   aggctaccag catatttgaa tgccaggtga aatcaaaata atctacacta 2700
   tctagaagac tttcttgatg ccaagtccag agatgtcatt gtgtaggatg 2750
   ttcagtaaat attaactgag ctatgtcaat atagagcttc tcagttttgt 2800
   ataacatttc atactaaaga ttcagcaaat ggaaaatgct attaatttgg 2850
   ttggtgacca caagataaaa tcagtcccac gttggctcag ttcaactaga 2900
   tgttccctga tacaaagaga acttgatttc cttaaaactg aaaagccaaa 2950
   cacagctagc tgtcatacaa gaaacagcta ttatgagaca tgaaggaggg 3000
   taagaattag ctttaagttt tgttttgctt tgttttgttt tttaactcaa 3050
   cct at t aat c at ct ct t cac aagaat ccac ct gat gt gac caagct at t a 3100
   t gt gt t gcct ggaaaaact g gcaagat t t c agct t at gt g gcct agcaaa 3150
   ct aagaat t g ct ct t ct t gg ccagcct cat agcat aaaag at gt gaact c 3200
   taggaagtct ttctgagtag caataagtgg gaattatggg cagagcacac 3250
   tcaatcccct gttgattaat aaaacaggct ggacactaat taactatggg 3300
   act t aaat ct gt agaaat ga aggagt ccaa t agct t ct t c caat t t t aaa 3350
   act ct agt ac at ccct t t cc ct caaat at a t at t t ct aag at aaagagaa 3400
   agaagagcac taagtaagta gaatctgttt ttcctatttt gtagggctgc 3450
   tgactcctag tccttgaagc ctagacacat gacccaggaa atttttcctt 3500
   t gt t t cact t t t gat t at ga t gt ct gagcc aaaaat t caa t t aagt aaac 3550
   at act cgcct ggat ct gaat cat t cat t t a at t act agat ct acccagct 3600
   gttatatcag gccaaaaaca gattcgtgtt tatataaaag agtaaacgat 3650
   ggt t gcaaat t t t ggct at t t agagt t gct act t cact at gaagagt cac 3700
   t t caaaacac t t cgct t gt c t t t agggat g at t t t t gcca t t t ccagt cc 3750
   acggt at gat act aaagct g t caagagagg t t t ct t ct t t t ct gaaact g 3800
   ccagct ct t t ccagccct gt t gat cact gg acat aaagct t ct t t t cccc 3850
   aat aat t ct t ct t t act t aa aat agt cagg at ct t t at ct acagat gt ac 3900
   tctccaggtt acctgtgatg atagccccct aatgtcctgc tagaaaagtc 3950
   t ccaagcaga gat gacat t a ct t ct gaat g ct cat aaacc acaccat gaa 4000
   at aaaagct c t t t gt t gt t t t aagat t gt g aagt gt cgt t aat gggt ccc 4050
   cacagat ggt ccct gct gga ct cacct gga at ct ct ccac agccat accc 4100
   act cat cact at cat t gaga cct gcacat c t t aat agaaa t at t at aaac 4150
   at cgaaaat c at gact t acc t agaagt t cg ct t gt aact a at gaaat t aa 4200
   acaaat gt gt t gcct t t t gt cat gt gt t t c t ct cct gt ga cat t t caaaa 4250
   t at cacat ct t gat aaat aa t gt gt t t cat ct t gaat agc t gaact aat t 4300
   gct t t ggaaa cagagt cct a gaaaagt gac t t caacagaa t t gt t act aa 4350
   aat t t gcact cacaacat ga aat aaat t t t ct t cct at gg aat aat cgt g 4400
<210> 19
   <211> 1715
   <212> DNA
   <213> Homo sapiens
<400> 19
   gaattccagc ggcggcgccg ttgccgctgc cgggaaacac aaggaaaggg 50
   aaccagcgca gcgtggcgat gggcgggggt agagccccgc cggagaggct 100
   gggcggctgc cggtgacaga ctgtgccctg tccacggtgc ctcctgcatg 150
   tcctgctgcc ctgagctgtc ccgagctagg tgacagcgta ccacgctgcc 200
   accatgaatg aggtgtctgt catcaaagaa ggctggctcc acaagcgtgg 250
   tgaatacatc aagacctgga ggccacggta cttcctgctg aagagcgacg 300
   gctccttcat tgggtacaag gagaggcccg aggcccctga tcagactcta 350
   ccccccttaa acaacttctc cgtagcagaa tgccagctga tgaagaccga 400
   gaggccgcga cccaacacct ttgtcatacg ctgcctgcag tggaccacag 450
   tcatcgagag gaccttccac gtggattctc cagacgagag ggaggagtgg 500
   atgcgggcca tccagatggt cgccaacagc ctcaagcagc gggccccagg 550
   cgaggacccc atggactaca agtgtggctc ccccagtgac tcctccacga 600
   ctgaggagat ggaagtggcg gtcagcaagg cacgggctaa agtgaccatg 650
   aatgacttcg actatctcaa actccttggc aagggaacct ttggcaaagt 700
   catcctggtg cgggagaagg ccactggccg ctactacgcc atgaagatcc 750
   tgcgaaagga agtcatcatt gccaaggatg aagtcgctca cacagtcacc 800
   gagagccggg tcctccagaa caccaggcac ccgttcctca ctgcgctgaa 850
   gtatgccttc cagacccacg accgcctgtg ctttgtgatg gagtatgcca 900
   acgggggtga gctgttcttc cacctgtccc gggagcgtgt cttcacagag 950
   gagcgggccc ggttttatgg tgcagagatt gtctcggctc ttgagtactt 1000
   gcactcgcgg gacgtggtat accgcgacat caagctggaa aacctcatgc 1050
   tggacaaaga tggccacatc aagatcactg actttggcct ctgcaaagag 1100
   ggcatcagtg acggggccac catgaaaacc ttctgtggga ccccggagta 1150
   cctggcgcct gaggtgctgg aggacaatga ctatggccgg gccgtggact 1200
   ggtgggggct gggtgtggtc atgtacgaga tgatgtgcgg ccgcctgccc 1250
   ttctacaacc aggaccacga gcgcctcttc gagctcatcc tcatggaaga 1300
   gatccgcttc ccgcgcacgc tcagccccga ggccaagtcc ctgcttgctg 1350
   ggctgcttaa gaaggacccc aagcagaggc ttggtggggg gcccagcgat 1400
   gccaaggagg tcatggagca caggttcttc ctcagcatca actggcagga 1450
   cgtggtccag aagaagctcc tgccaccctt caaacctcag gtcacgtccg 1500
   aggtcgacac aaggtacttc gatgatgaat ttaccgccca gtccatcaca 1550
   atcacacccc ctgaccgcta tgacagcctg ggcttactgg agctggacca 1600
   gcggacccac ttcccccagt tctcctactc ggccagcatc cgcgagtgag 1650
   cagtctgccc acgcagagga cgcacgctcg ctgccatcac cgctgggtgg 1700
   ttttttaccc ctgcc 1715
<210> 20
   <211> 1327
   <212> DNA
   <213> Homo sapiens
<400> 20
   cacaacggcc atcaccacct ctccgcaccc tctatctcct tccttctcgg 50
   gcctcacccc tggccctcgc tttcagtgcc cagcccggac cgcagcactc 100
   cagttccgcc ccaacccggg aaccctggac tgaggctccc gtttctgtcc 150
   t t t ct at t gg gcgcact t cc gat ggcgt ca ggaat t t cag ccaat aggag 200
   ccagccagga agtacctctc tgagcggttg gtgccgggta taaaagaagg 250
   ccagcacggc tgctcacgac gccgcgaagc ctgtgtagca ctgagacatc 300
   agtgagttgg ctacagcaga ccaaacagcc cagcagccca gcagcccacg 350
   cat gcggcgc ct cacagt cg at gact t t ga aat cgggcgt cccct gggca 400
   aggggaaat t t gggaat gt g t acct ggct c ggct caagga aagccat t t c 450
   attgtggccc tgaaggttct cttcaagtcg cagatagaga aggaaggact 500
   ggagcaccag ctgcgccggg aaattgagat ccaggctcat ctacaacacc 550
   ccaat at cct gcgcct gt at aact at t t cc at gat gcacg ccgggt gt ac 600
   ct gat t ct gg aat at gct cc aaggggt gag ct ct acaagg agct gcagaa 650
   aagcgagaaa ttagatgaac agcgcacagc cacgataata gaggaagttg 700
   cagat gccct gacct act gc cat gacaaga aagt gat t ca cagagat at t 750
   aagccagaga acctgctgct ggggttcagg ggtgaggtga agattgcaga 800
   ttttggctgg tctgtgcaca cccccctccc tgagaggaag acaatgtgtg 850
   ggacact gga ct act t gccg ccagaaat ga t t gaggggag aacat at gat 900
   gaaaaggt gg at t t gt ggt g cat t ggagt g ct ct gct at g agct gct ggt 950
   gggat at cca ccct t t gaga gcgcct ccca cagt gagact t acagacgca 1000
   tcctcaaggt agatgtgagg tttccactat caatgcctct gggggcccgg 1050
   gact t gat t t ccaggct t ct cagat accag ccct t ggaga gact gcccct 1100
   ggcccagat c ct gaagcacc cct gggt t ca ggcccact cc cgaagggt gc 1150
   t gcct ccct g t gct cagat g gct t cct gag ccct gt ct gc ct ct gt t ccc 1200
   t t t gt gt gt g t t cagggagc t ct cct gct c t gccacct ca t t t gt ct t t a 1250
   t t t t t t t ct c t t t t aagat g t aagat gct a at t aat aaaa gct gaat cat 1300
   ttcataccaa aaaaaaaaaa aaaaaaa 1327
<210> 21
   <211> 2575
   <212> DNA
   <213> Homo sapiens
<400> 21
   ggaggaggaa gcaagcgagg gggctggttc ctgagcttcg caattcctgt 50
   gtcgccttct gggctcccag cctgccgggt cgcatgatcc ctccggccgg 100
   agctggtttt tttgccagcc accgcgaggc cggctgagtt accggcatcc 150
   ccgcagccac ctcctctccc gacctgtgat acaaaagatc ttccgggggc 200
   tgcacctgcc tgcctttgcc taaggcggat ttgaatctct ttctctccct 250
   t cagaat ct t  at ct t ggct t  t ggat ct t ag  aagagaat ca  ct aaccagag  300
   acgagact ca  gt gagt gagc  aggt gt t t t g  gacaat ggac  t ggt t gagcc  350
   cat ccct at t  at aaaaat gt  ct cagagcaa  ccgggagct g  gt ggt t gact  400
   ttctctccta caagctttcc cagaaaggat acagctggag tcagtttagt 450
   gatgtggaag agaacaggac tgaggcccca gaagggactg aatcggagat 500
   ggagaccccc agtgccatca atggcaaccc atcctggcac ctggcagaca 550
   gccccgcggt gaatggagcc actggccaca gcagcagttt ggatgcccgg 600
   gaggtgatcc ccatggcagc agtaaagcaa gcgctgaggg aggcaggcga 650
   cgagt t t gaa  ct gcggt acc  ggcgggcat t  cagt gacct g  acat cccagc  700
   tccacatcac cccagggaca gcatatcaga gctttgaaca ggtagtgaat 750
   gaact ct t cc  gggat ggggt  aaact ggggt  cgcat t gt gg  cct t t t t ct c  800
   cttcggcggg gcactgtgcg tggaaagcgt agacaaggag atgcaggtat 850
   t ggt gagt cg  gat cgcagct  t ggat ggcca  ct t acct gaa  t gaccacct a  900
   gagcct t gga  t ccaggagaa  cggcggct gg  gat act t t t g  t ggaact ct a  950
   tgggaacaat gcagcagccg agagccgaaa gggccaggaa cgcttcaacc 1000
   gct ggt t cct  gacgggcat g  act gt ggccg  gcgt ggt t ct  gct gggct ca  1050
   ct ct t cagt c  ggaaat gacc  agacact gac  cat ccact ct  accct cccac  1100
   ccccttctct gctccaccac atcctccgtc cagccgccat tgccaccagg 1150
   agaaccact a  cat gcagccc  at gcccacct  gcccat caca  gggt t gggcc  1200
   cagatctggt cccttgcagc tagttttcta gaatttatca cacttctgtg 1250
   agacccccac acctcagttc ccttggcctc agaattcaca aaatttccac 1300
   aaaatctgtc caaaggaggc tggcaggtat ggaagggttt gtggctgggg 1350
   gcaggagggc cctacctgat tggtgcaacc cttacccctt agcctccctg 1400
   aaaat gt t t t  t ct gccaggg  agct t gaaag  t t t t cagaac  ct ct t cccca  1450
   gaaaggagac tagattgcct ttgttttgat gtttgtggcc tcagaattga 1500
   t cat t t t ccc  cccact ct cc  ccacact aac  ct gggt t ccc  t t t cct t cca  1550
   t ccct acccc  ct aagagcca  t t t aggggcc  act t t t gact  agggat t cag  1600
   gctgcttggg ataaagatgc aaggaccagg actccctcct cacctctgga 1650
   ct ggct agag  t cct cact cc  cagt ccaaat  gt cct ccaga  agcct ct ggc  1700
   tagaggccag ccccacccag gagggagggg gctatagcta caggaagcac 1750
   cccat gccaa  agct agggt g  gccct t gcag  t t cagcacca  ccct agt ccc  1800
   ttcccctccc tggctcccat gaccatactg agggaccaac tgggcccaag 1850
   acagat gccc  cagagct gt t  t at ggcct ca  gct gcct cac  t t cct acaag  1900
   agcagcct gt  ggcat ct t t g  cct t gggct g  ct cct cat gg  t gggt t cagg  1950
   ggactcagcc ctgaggtgaa agggagctat caggaacagc tatgggagcc 2000
   ccagggtctt ccctacctca ggcaggaagg gcaggaagga gagcctgctg 2050
   catggggtgg ggtagggctg actagaaggg ccagtcctgc ctggccaggc 2100
   agatctgtgc cccatgcctg tccagcctgg gcagccaggc tgccaaggcc 2150
   agagtggcct ggccaggagc tcttcaggcc tccctctctc ttctgctcca 2200
   cccttggcct gtctcatccc caggggtccc agccaccccg ggctctctgc 2250
   tgtacatatt tgagactagt ttttattcct tgtgaagatg atatactatt 2300
   tttgttaagc gtgtctgtat ttatgtgtga ggagctgctg gcttgcagtg 2350
   cgcgtgcacg tggagagctg gtgcccggag attggacggc ctgatgctcc 2400
   ctcccctgcc ctggtccagg gaagctggcc gagggtcctg gctcctgagg 2450
   ggcatctgcc cctcccccaa cccccacccc acacttgttc cagctctttg 2500
   aaatagtctg tgtgaaggtg aaagtgcagt tcagtaataa actgtgttta 2550
   ctcagtgaaa aaaaaaaaaa aaaaa 2575
<210> 22
   <211> 1154
   <212> DNA
   <213> Homo sapiens
<400> 22
   cgctcaggga aggcgggtgc gcgcctgcgg ggcggagatg ggcagggggc 50
   ggt gcgt ggg t cccagt ct g cagt t aaggg ggcaggagt g gcgct gct ca 100
   cctctggtgc caaagggcgg cgcagcggct gccgagctcg gccctggagg 150
   cggcgagaac atggtgcgca ggttcttggt gaccctccgg attcggcgcg 200
   cgt gcggccc gccgcgagt g agggt t t t cg t ggt t cacat cccgcggct c 250
   acgggggagt gggcagcgcc aggggcgccc gccgctgtgg ccctcgtgct 300
   gatgctactg aggagccagc gtctagggca gcagccgctt cctagaagac 350
   caggt cat ga t gat gggcag cgcccgagt g gcggagct gc t gct gct cca 400
   cggcgcggag cccaactgcg ccgaccccgc cactctcacc cgacccgtgc 450
   acgacgctgc ccgggagggc ttcctggaca cgctggtggt gctgcaccgg 500
   gccggggcgc ggctggacgt gcgcgatgcc tggggccgtc tgcccgtgga 550
   cct ggct gag gagct gggcc at cgcgat gt cgcacggt ac ct gcgcgcgg 600
   ctgcgggggg caccagaggc agtaaccatg cccgcataga tgccgcggaa 650
   ggtccctcag acatccccga ttgaaagaac cagagaggct ctgagaaacc 700
   t cgggaaact t agat cat ca gt caccgaag gt cct acagg gccacaact g 750
   cccccgccac aacccacccc gctttcgtag ttttcattta gaaaatagag 800
   ct t t t aaaaa t gt cct gcct t t t aacgt ag at at at gcct t cccccact a 850
   ccgt aaat gt ccat t t at at cat t t t t t at at at t ct t at aaaaat gt aa 900
   aaaagaaaaa caccgcttct gccttttcac tgtgttggag ttttctggag 950
   t gagcact ca cgccct aagc gcacat t cat gt gggcat t t ct t gcgagcc 1000
   t cgcagcct c cggaagct gt cgact t cat g acaagcat t t t gt gaact ag 1050
   ggaagct cag gggggt t act ggct t ct ct t gagt cacact gct agcaaat 1100
   ggcagaacca aagctcaaat aaaaataaaa taattttcat tcattcactc 1150
   aaaa 1154
<210> 23
   <211> 5511
   <212> DNA
   <213> Homo sapiens
<400> 23
   acacacacac acccctcccc tgccatccct ccccggactc cggctccggc 50
   tccgattgca atttgcaacc tccgctgccg tcgccgcagc agccaccaat 100
   t cgccagcgg t t caggt ggc t ct t gcct cg at gt cct agc ct aggggccc 150
   ccgggccgga cttggctggg ctcccttcac cctctgcgga gtcatgaggg 200
   cgaacgacgc tctgcaggtg ctgggcttgc ttttcagcct ggcccggggc 250
   t ccgaggt gg gcaact ct ca ggcagt gt gt cct gggact c t gaat ggcct 300
   gagt gt gacc ggcgat gct g agaaccaat a ccagacact g t acaagct ct 350
   acgagaggt g t gaggt ggt g at ggggaacc t t gagat t gt gct cacggga 400
   cacaat gccg acct ct cct t cct gcagt gg at t cgagaag t gacaggct a 450
   t gt cct cgt g gccat gaat g aat t ct ct ac t ct accat t g cccaacct cc 500
   gcgtggtgcg agggacccag gtctacgatg ggaagtttgc catcttcgtc 550
   at gt t gaact at aacaccaa ct ccagccac gct ct gcgcc agct ccgct t 600
   gactcagctc accgagattc tgtcaggggg tgtttatatt gagaagaacg 650
   ataagctttg tcacatggac acaattgact ggagggacat cgtgagggac 700
   cgagatgctg agatagtggt gaaggacaat ggcagaagct gtcccccctg 750
   t cat gaggt t t gcaaggggc gat gct gggg t cct ggat ca gaagact gcc 800
   agacat t gac caagaccat c t gt gct cct c agt gt aat gg t cact gct t t 850
   gggcccaacc ccaaccagtg ctgccatgat gagtgtgccg ggggctgctc 900
   aggccctcag gacacagact gctttgcctg ccggcacttc aatgacagtg 950
   gagcct gt gt acct cgct gt ccacagcct c t t gt ct acaa caagct aact 1000
   ttccagctgg aacccaatcc ccacaccaag tatcagtatg gaggagtttg 1050
   t gt agccagc t gt ccccat a act t t gt ggt ggat caaaca t cct gt gt ca 1100
   gggcct gt cc t cct gacaag at ggaagt ag at aaaaat gg gct caagat g 1150
   t gt gagcct t gt gggggact at gt cccaaa gcct gt gagg gaacaggct c 1200
   t gggagccgc t t ccagact g t ggact cgag caacat t gat ggat t t gt ga 1250
   act gcaccaa gat cct gggc aacct ggact t t ct gat cac cggcct caat 1300
   ggagacccct ggcacaagat ccctgccctg gacccagaga agctcaatgt 1350
   cttccggaca gtacgggaga tcacaggtta cctgaacatc cagtcctggc 1400
   cgccccacat gcacaacttc agtgtttttt ccaatttgac aaccattgga 1450
   ggcagaagcc tctacaaccg gggcttctca ttgttgatca tgaagaactt 1500
   gaatgtcaca tctctgggct tccgatccct gaaggaaatt agtgctgggc 1550
   gtatctatat aagtgccaat aggcagctct gctaccacca ctctttgaac 1600
   tggaccaagg tgcttcgggg gcctacggaa gagcgactag acatcaagca 1650
   taatcggccg cgcagagact gcgtggcaga gggcaaagtg tgtgacccac 1700
   tgtgctcctc tgggggatgc tggggcccag gccctggtca gtgcttgtcc 1750
   tgtcgaaatt atagccgagg aggtgtctgt gtgacccact gcaactttct 1800
   gaatggggag cctcgagaat ttgcccatga ggccgaatgc ttctcctgcc 1850
   acccggaatg ccaacccatg gagggcactg ccacatgcaa tggctcgggc 1900
   tctgatactt gtgctcaatg tgcccatttt cgagatgggc cccactgtgt 1950
   gagcagctgc ccccatggag tcctaggtgc caagggccca atctacaagt 2000
   acccagatgt tcagaatgaa tgtcggccct gccatgagaa ctgcacccag 2050
   gggtgtaaag gaccagagct tcaagactgt ttaggacaaa cactggtgct 2100
   gatcggcaaa acccatctga caatggcttt gacagtgata gcaggattgg 2150
   tagtgatttt catgatgctg ggcggcactt ttctctactg gcgtgggcgc 2200
   cggattcaga ataaaagggc tatgaggcga tacttggaac ggggtgagag 2250
   catagagcct ctggacccca gtgagaaggc taacaaagtc ttggccagaa 2300
   tcttcaaaga gacagagcta aggaagctta aagtgcttgg ctcgggtgtc 2350
   tttggaactg tgcacaaagg agtgtggatc cctgagggtg aatcaatcaa 2400
   gattccagtc tgcattaaag tcattgagga caagagtgga cggcagagtt 2450
   ttcaagctgt gacagatcat atgctggcca ttggcagcct ggaccatgcc 2500
   cacattgtaa ggctgctggg actatgccca gggtcatctc tgcagcttgt 2550
   cactcaatat ttgcctctgg gttctctgct ggatcatgtg agacaacacc 2600
   ggggggcact ggggccacag ctgctgctca actggggagt acaaattgcc 2650
   aagggaatgt actaccttga ggaacatggt atggtgcata gaaacctggc 2700
   tgcccgaaac gtgctactca agtcacccag tcaggttcag gtggcagatt 2750
   ttggtgtggc tgacctgctg cctcctgatg ataagcagct gctatacagt 2800
   gaggccaaga ctccaattaa gtggatggcc cttgagagta tccactttgg 2850
   gaaatacaca caccagagtg atgtctggag ctatggtgtg acagtttggg 2900
   agttgatgac cttcggggca gagccctatg cagggctacg attggctgaa 2950
   gtaccagacc tgctagagaa gggggagcgg ttggcacagc cccagatctg 3000
   cacaattgat gtctacatgg tgatggtcaa gtgttggatg attgatgaga 3050
   acattcgccc aacctttaaa gaactagcca atgagttcac caggatggcc 3100
   cgagacccac cacggtatct ggtcataaag agagagagtg ggcctggaat 3150
   agcccctggg ccagagcccc atggtctgac aaacaagaag ctagaggaag 3200
   tagagctgga gccagaacta gacctagacc tagacttgga agcagaggag 3250
   gacaacctgg caaccaccac actgggctcc gccctcagcc taccagttgg 3300
   aacacttaat cggccacgtg ggagccagag ccttttaagt ccatcatctg 3350
   gatacatgcc catgaaccag ggtaatcttg gggagtcttg ccaggagtct 3400
   gcagtttctg ggagcagtga acggtgcccc cgtccagtct ctctacaccc 3450
   aatgccacgg ggatgcctgg catcagagtc atcagagggg catgtaacag 3500
   gctctgaggc tgagctccag gagaaagtgt caatgtgtag gagccggagc 3550
   aggagccgga gcccacggcc acgcggagat agcgcctacc attcccagcg 3600
   ccacagtctg ctgactcctg ttaccccact ctccccaccc gggttagagg 3650
   aagaggatgt caacggttat gtcatgccag atacacacct caaaggtact 3700
   ccctcctccc gggaaggcac cctttcttca gtgggtctca gttctgtcct 3750
   gggtactgaa gaagaagatg aagatgagga gtatgaatac atgaaccgga 3800
   ggagaaggca cagtccacct catcccccta ggccaagttc ccttgaggag 3850
   ctgggttatg agtacatgga tgtggggtca gacctcagtg cctctctggg 3900
   cagcacacag agttgcccac tccaccctgt acccatcatg cccactgcag 3950
   gcacaactcc agatgaagac tatgaatata tgaatcggca acgagatgga 4000
   ggtggtcctg ggggtgatta tgcagccatg ggggcctgcc cagcatctga 4050
   gcaagggtat gaagagatga gagcttttca ggggcctgga catcaggccc 4100
   cccatgtcca ttatgcccgc ctaaaaactc tacgtagctt agaggctaca 4150
   gactctgcct ttgataaccc tgattactgg catagcaggc ttttccccaa 4200
   ggctaatgcc cagagaacgt aactcctgct ccctgtggca ctcagggagc 4250
   atttaatggc agctagtgcc tttagagggt accgtcttct ccctattccc 4300
   tctctctccc aggtcccagc cccttttccc cagtcccaga caattccatt 4350
   caatctttgg aggcttttaa acattttgac acaaaattct tatggtatgt 4400
   agccagctgt gcactttctt ctctttccca accccaggaa aggttttcct 4450
   tattttgtgt gctttcccag tcccattcct cagcttcttc acaggcactc 4500
   ctggagatat gaaggattac tctccatatc ccttcctctc aggctcttga 4550
   ctacttggaa ctaggctctt atgtgtgcct ttgtttccca tcagactgtc 4600
   aagaagagga aagggaggaa acctagcaga ggaaagtgta attttggttt 4650
   atgactctta accccctaga aagacagaag cttaaaatct gtgaagaaag 4700
   aggttaggag tagatattga ttactatcat aattcagcac ttaactatga 4750
   gccaggcatc atactaaact tcacctacat tatctcactt agtcctttat 4800
   cat cct t aaa acaat t ct gt gacat acat a t t at ct cat t t t acacaaag 4850
   ggaagt cggg cat ggt ggct cat gcct gt a at ct cagcac t t t gggaggc 4900
   tgaggcagaa ggattacctg aggcaaggag tttgagacca gcttagccaa 4950
   catagtaaga cccccatctc tttaaaaaaa aaaaaaaaaa aaaaaaaaaa 5000
   act t t agaac t gggt gcagt ggct cat gcc t gt aat ccca gccagcact t 5050
   t gggaggct g agat gggaag at cact t gag cccagaat t a gagat aagcc 5100
   tatggaaaca tagcaagaca ctgtctctac aggggaaaaa aaaaaaagaa 5150
   act gagcct t aaagagat ga aat aaat t aa gcagt agat c caggat gcaa 5200
   aatcctccca attcctgtgc atgtgctctt attgtaaggt gccaagaaaa 5250
   act gat t t aa gt t acagccc t t gt t t aagg ggcact gt t t ct t gt t t t t g 5300
   cactgaatca agtctaaccc caacagccac atcctcctat acctagacat 5350
   ctcatctcag gaagtggtgg tgggggtagt cagaaggaaa aataactgga 5400
   cat ct t t gt g t aaaccat aa t ccacat gt g ccgt aaat ga t ct t cact cc 5450
   t t at ccgagg gcaaat t cac aaggat cccc aagat ccact t t t agaagcc 5500
   at t ct cat cc a 5511
<210> 24
   <211> 5484
   <212> DNA
   <213> Homo sapiens
<400> 24
   aattgtcagc acgggatctg agacttccaa aaaatgaagc cggcgacagg 50
   actttgggtc tgggtgagcc ttctcgtggc ggcggggacc gtccagccca 100
   gcgattctca gtcagtgtgt gcaggaacgg agaataaact gagctctctc 150
   tctgacctgg aacagcagta ccgagccttg cgcaagtact atgaaaactg 200
   tgaggttgtc atgggcaacc tggagataac cagcattgag cacaaccggg 250
   acctctcctt cctgcggtct gttcgagaag tcacaggcta cgtgttagtg 300
   gctcttaatc agtttcgtta cctgcctctg gagaatttac gcattattcg 350
   tgggacaaaa ctttatgagg atcgatatgc cttggcaata tttttaaact 400
   acagaaaaga tggaaacttt ggacttcaag aacttggatt aaagaacttg 450
   acagaaatcc taaatggtgg agtctatgta gaccagaaca aattcctttg 500
   ttatgcagac accattcatt ggcaagatat tgttcggaac ccatggcctt 550
   ccaacttgac tcttgtgtca acaaatggta gttcaggatg tggacgttgc 600
   cataagtcct gtactggccg ttgctgggga cccacagaaa atcattgcca 650
   gactttgaca aggacggtgt gtgcagaaca atgtgacggc agatgctacg 700
   gaccttacgt cagtgactgc tgccatcgag aatgtgctgg aggctgctca 750
   ggacctaagg acacagactg ctttgcctgc atgaatttca atgacagtgg 800
   agcatgtgtt actcagtgtc cccaaacctt tgtctacaat ccaaccacct 850
   ttcaactgga gcacaatttc aatgcaaagt acacatatgg agcattctgt 900
   gtcaagaaat gtccacataa ctttgtggta gattccagtt cttgtgtgcg 950
   tgcctgccct agttccaaga tggaagtaga agaaaatggg attaaaatgt 1000
   gtaaaccttg cactgacatt tgcccaaaag cttgtgatgg cattggcaca 1050
   ggatcattga tgtcagctca gactgtggat tccagtaaca ttgacaaatt 1100
   cataaactgt accaagatca atgggaattt gatctttcta gtcactggta 1150
   ttcatgggga cccttacaat gcaattgaag ccatagaccc agagaaactg 1200
   aacgtctttc ggacagtcag agagataaca ggtttcctga acatacagtc 1250
   atggccacca aacatgactg acttcagtgt tttttctaac ctggtgacca 1300
   ttggtggaag agtactctat agtggcctgt ccttgcttat cctcaagcaa 1350
   cagggcatca cctctctaca gttccagtcc ctgaaggaaa tcagcgcagg 1400
   aaacatctat attactgaca acagcaacct gtgttattat cataccatta 1450
   actggacaac actcttcagc acaatcaacc agagaatagt aatccgggac 1500
   aacagaaaag ctgaaaattg tactgctgaa ggaatggtgt gcaaccatct 1550
   gtgttccagt gatggctgtt ggggacctgg gccagaccaa tgtctgtcgt 1600
   gtcgccgctt cagtagagga aggatctgca tagagtcttg taacctctat 1650
   gatggtgaat ttcgggagtt tgagaatggc tccatctgtg tggagtgtga 1700
   cccccagtgt gagaagatgg aagatggcct cctcacatgc catggaccgg 1750
   gtcctgacaa ctgtacaaag tgctctcatt ttaaagatgg cccaaactgt 1800
   gtggaaaaat gtccagatgg cttacagggg gcaaacagtt tcattttcaa 1850
   gtatgctgat ccagatcggg agtgccaccc atgccatcca aactgcaccc 1900
   aagggtgtaa cggtcccact agtcatgact gcatttacta cccatggacg 1950
   ggccattcca ctttaccaca acatgctaga actcccctga ttgcagctgg 2000
   agtaattggt gggctcttca ttctggtcat tgtgggtctg acatttgctg 2050
   tttatgttag aaggaagagc atcaaaaaga aaagagcctt gagaagattc 2100
   ttggaaacag agttggtgga accattaact cccagtggca cagcacccaa 2150
   tcaagctcaa cttcgtattt tgaaagaaac tgagctgaag agggtaaaag 2200
   tccttggctc aggtgctttt ggaacggttt ataaaggtat ttgggtacct 2250
   gaaggagaaa ctgtgaagat tcctgtggct attaagattc ttaatgagac 2300
   aactggtccc aaggcaaatg tggagttcat ggatgaagct ctgatcatgg 2350
   caagtatgga tcatccacac ctagtccggt tgctgggtgt gtgtctgagc 2400
   ccaaccatcc agctggttac tcaacttatg ccccatggct gcctgttgga 2450
   gtatgtccac gagcacaagg ataacattgg atcacaactg ctgcttaact 2500
   ggtgtgtcca gatagctaag ggaatgatgt acctggaaga aagacgactc 2550
   gt t cat cggg at t t ggcagc ccgt aat gt c t t agt gaaat ct ccaaacca 2600
   t gt gaaaat c acagat t t t g ggct agccag act ct t ggaa ggagat gaaa 2650
   aagagt acaa t gct gat gga ggaaagat gc caat t aaat g gat ggct ct g 2700
   gagt gt at ac at t acaggaa at t caccc at cagagt gacg t t t ggagct a 2750
   t ggagt t act at at gggaac t gat gacct t t ggaggaaaa ccct at gat g 2800
   gaat t ccaac gcgagaaat c cct gat t t at t agagaaagg agaacgt t t g 2850
   cct cagcct c ccat ct gcac t at t gacgt t t acat ggt ca t ggt caaat g 2900
   t t ggat gat t gat gct gaca gt agacct aa at t t aaggaa ct ggct gct g 2950
   agt t t t caag gat ggct cga gaccct caaa gat acct agt t at t cagggt 3000
   gatgatcgta tgaagcttcc cagtccaaat gacagcaagt tctttcagaa 3050
   t ct ct t ggat gaagaggat t t ggaagat at gat ggat gct gaggagt act 3100
   t ggt ccct ca ggct t t caac at cccacct c ccat ct at ac t t ccagagca 3150
   agaat t gact cgaat aggag t gaaat t gga cacagccct c ct cct gcct a 3200
   cacccccat g t caggaaacc agt t t gt at a ccgagat gga ggt t t t gct g 3250
   ct gaacaagg agt gt ct gt g ccct acagag ccccaact ag cacaat t cca 3300
   gaagct cct g t ggcacaggg t gct act gct gagat t t t t g at gact cct g 3350
   ctgtaatggc accctacgca agccagtggc accccatgtc caagaggaca 3400
   gtagcaccca gaggtacagt gctgacccca ccgtgtttgc cccagaacgg 3450
   agcccacgag gagagctgga tgaggaaggt tacatgactc ctatgcgaga 3500
   caaacccaaa caagaatacc tgaatccagt ggaggagaac ccttttgttt 3550
   ct cggagaaa aaat ggagac ct t caagcat t ggat aat cc cgaat at cac 3600
   aatgcatcca atggtccacc caaggccgag gatgagtatg tgaatgagcc 3650
   actgtacctc aacacctttg ccaacacctt gggaaaagct gagtacctga 3700
   agaacaacat actgtcaatg ccagagaagg ccaagaaagc gtttgacaac 3750
   cctgactact ggaaccacag cctgccacct cggagcaccc ttcagcaccc 3800
   agact acct g caggagt aca gcacaaaat a t t t t t at aaa cagaat gggc 3850
   ggat ccggcc t at t gt ggca gagaat cct g aat acct ct c t gagt t ct cc 3900
   ct gaagccag gcact gt gct gccgcct cca cct t acagac accggaat ac 3950
   t gt ggt gt aa gct cagt t gt ggt t t t t t ag gt ggagagac acacct gct c 4000
   caat t t cccc acccccct ct ct t t ct ct gg t ggt ct t cct t ct accccaa 4050
   ggccagt agt t t t gacact t cccagt ggaa gat acagaga t gcaat gat a 4100
   gttatgtgct tacctaactt gaacattaga gggaaagact gaaagagaaa 4150
   gat aggagga accacaat gt t t ct t cat t t ct ct gcat gg gt t ggt cagg 4200
   agaatgaaac agctagagaa ggaccagaaa atgtaaggca atgctgccta 4250
   ct at caaact agct gt cact t t t t t t ct t t t t ct t t t t ct t t ct t t gt t t 4300
   ctttcttcct cttctttttt tttttttttt taaagcagat ggttgaaaca 4350
   cccat gct at ct gt t cct at ct gcaggaac t gat gt gt gc at at t t agca 4400
   tccctggaaa tcataataaa gtttccatta gaacaaaaga ataacatttt 4450
   ct at aacat a t gat agt gt c t gaaat t gag aat ccagt t t ct t t ccccag 4500
   cagt t t ct gt cct agcaagt aagaat ggcc aact caact t t cat aat t t a 4550
   aaaat ct cca t t aaagt t at aact agt aat t at gt t t t ca acact t t t t g 4600
   gt t t t t t t ca t t t t gt t t t g ct ct gaccga t t cct t t at a t t t gct cccc 4650
   t at t t t t ggc t t t aat t t ct aat t gcaaag at gt t t acat caaagct t ct 4700
   t cacagaat t t aagcaagaa at at t t t aat at agt gaaat ggccact act 4750
   t t aagt at ac aat ct t t aaa at aagaaagg gaggct aat a t t t t t cat gc 4800
   tatcaattta tcttcaccct catcctttac atttttcaac attttttttt 4850
   ct ccat aaat gacact act t gat aggccgt t ggt t gt ct g aagagt agaa 4900
   gggaaact aa gagacagt t c t ct gt ggt t c aggaaaact a ct gat act t t 4950
   caggggt ggc ccaat gaggg aat ccat t ga act ggaagaa acacact gga 5000
   t t gggt at gt ct acct ggca gat act caga aat gt agt t t gcact t aagc 5050
   t gt aat t t t a t t t gt t ct t t t t ct gaact c cat t t t ggat t t t gaat caa 5100
   gcaatatgga agcaaccagc aaattaacta atttaagtac atttttaaaa 5150
   aaagagctaa gataaagact gtggaaatgc caaaccaagc aaattaggaa 5200
   ccttgcaacg gtatccaggg actatgatga gaggccagca cattatcttc 5250
   at at gt cacc t t t gct acgc aaggaaat t t gt t cagt t cg t at act t cgt 5300
   aagaaggaat gcgagtaagg attggcttga attccatgga atttctagta 5350
   t gagact at t t at at gaagt agaaggt aac t ct t t gcaca t aaat t ggt a 5400
   taataaaaag aaaaacacaa acattcaaag cttagggata ggtccttggg 5450
   t caaaagt t g t aaat aaat g t gaaacat ct t ct c 5484
<210> 25
   <211> 4093
   <212> DNA
   <213> Homo sapiens
<400> 25
<210> 26
   <211> 8680
   <212> DNA
   <213> Homo sapiens
<400> 26
<210> 27
   <211> 5830
   <212> DNA
   <213> Homo sapiens
<400> 27
   actgagtccc gggaccccgg gagagcggtc agtgtgtggt cgctgcgttt 50
   cctctgcctg cgccgggcat cacttgcgcg ccgcagaaag tccgtctggc 100
   agcctggata tcctctccta ccggcacccg cagacgcccc tgcagccgcc 150
   ggtcggcgcc cgggctccct agccctgtgc gctcaactgt cctgcgctgc 200
   ggggtgccgc gagttccacc tccgcgcctc cttctctaga caggcgctgg 250
   gagaaagaac cggctcccga gttctgggca tttcgcccgg ctcgaggtgc 300
   aggatgcaga gcaaggtgct gctggccgtc gccctgtggc tctgcgtgga 350
   gacccgggcc gcctctgtgg gtttgcctag tgtttctctt gatctgccca 400
   ggctcagcat acaaaaagac atacttacaa ttaaggctaa tacaactctt 450
   caaattactt gcaggggaca gagggacttg gactggcttt ggcccaataa 500
   tcagagtggc agtgagcaaa gggtggaggt gactgagtgc agcgatggcc 550
   tcttctgtaa gacactcaca attccaaaag tgatcggaaa tgacactgga 600
   gcctacaagt gcttctaccg ggaaactgac ttggcctcgg tcatttatgt 650
   ctatgttcaa gattacagat ctccatttat tgcttctgtt agtgaccaac 700
   atggagtcgt gtacattact gagaacaaaa acaaaactgt ggtgattcca 750
   tgtctcgggt ccatttcaaa tctcaacgtg tcactttgtg caagataccc 800
   agaaaagaga tttgttcctg atggtaacag aatttcctgg gacagcaaga 850
   agggctttac tattcccagc tacatgatca gctatgctgg catggtcttc 900
   tgtgaagcaa aaattaatga tgaaagttac cagtctatta tgtacatagt 950
   tgtcgttgta gggtatagga tttatgatgt ggttctgagt ccgtctcatg 1000
   gaattgaact atctgttgga gaaaagcttg tcttaaattg tacagcaaga 1050
   act gaact aa at gt ggggat t gact t caac t gggaat acc ct t ct t cgaa 1100
   gcat cagcat aagaaact t g t aaaccgaga cct aaaaacc cagt ct ggga 1150
   gt gagat gaa gaaat t t t t g agcacct t aa ct at agat gg t gt aacccgg 1200
   agt gaccaag gat t gt acac ct gt gcagca t ccagt gggc t gat gaccaa 1250
   gaagaacagc acatttgtca gggtccatga aaaccttttt gttgcttttg 1300
   gaagt ggcat ggaat ct ct g gt ggaagcca cggt ggggga gcgt gt caga 1350
   at ccct gcga agt acct t gg t t acccaccc ccagaaat aa aat ggt at aa 1400
   aaat ggaat a cccct t gagt ccaat cacac aat t aaagcg gggcat gt ac 1450
   tgacgattat ggaagtgagt gaaagagaca caggaaatta cactgtcatc 1500
   cttaccaatc ccatttcaaa ggagaagcag agccatgtgg tctctctggt 1550
   tgtgtatgtc ccaccccaga ttggtgagaa atctctaatc tctcctgtgg 1600
   at t cct acca gt acggcacc act caaacgc t gacat gt ac ggt ct at gcc 1650
   at t cct cccc cgcat cacat ccact ggt at t ggcagt t gg aggaagagt g 1700
   cgccaacgag cccagccaag ctgtctcagt gacaaaccca tacccttgtg 1750
   aagaat ggag aagt gt ggag gact t ccagg gaggaaat aa aat t gaagt t 1800
   aataaaaatc aatttgctct aattgaagga aaaaacaaaa ctgtaagtac 1850
   ccttgttatc caagcggcaa atgtgtcagc tttgtacaaa tgtgaagcgg 1900
   tcaacaaagt cgggagagga gagagggtga tctccttcca cgtgaccagg 1950
   ggtcctgaaa ttactttgca acctgacatg cagcccactg agcaggagag 2000
   cgt gt ct t t g t ggt gcact g cagacagat c t acgt t t gag aacct cacat 2050
   ggt acaagct t ggcccacag cct ct gccaa t ccat gt ggg agagt t gccc 2100
   acacct gt t t gcaagaact t ggat act ct t t ggaaat t ga at gccaccat 2150
   gt t ct ct aat agcacaaat g acat t t t gat cat ggagct t aagaat gcat 2200
   ccttgcagga ccaaggagac tatgtctgcc ttgctcaaga caggaagacc 2250
   aagaaaagac attgcgtggt caggcagctc acagtcctag agcgtgtggc 2300
   acccacgatc acaggaaacc tggagaatca gacgacaagt attggggaaa 2350
   gcat cgaagt ct cat gcacg gcat ct ggga at ccccct cc acagat cat g 2400
   t ggt t t aaag at aat gagac cct t gt agaa gact caggca t t gt at t gaa 2450
   ggatgggaac cggaacctca ctatccgcag agtgaggaag gaggacgaag 2500
   gcct ct acac ct gccaggca t gcagt gt t c t t ggct gt gc aaaagt ggag 2550
   gcatttttca taatagaagg tgcccaggaa aagacgaact tggaaatcat 2600
   tattctagta ggcacggcgg tgattgccat gttcttctgg ctacttcttg 2650
   tcatcatcct acggaccgtt aagcgggcca atggagggga actgaagaca 2700
   ggct act t gt ccat cgt cat ggat ccagat gaact cccat t ggat gaaca 2750
   ttgtgaacga ctgccttatg atgccagcaa atgggaattc cccagagacc 2800
   ggctgaagct aggtaagcct cttggccgtg gtgcctttgg ccaagtgatt 2850
   gaagcagatg cctttggaat tgacaagaca gcaacttgca ggacagtagc 2900
   agtcaaaatg ttgaaagaag gagcaacaca cagtgagcat cgagctctca 2950
   tgtctgaact caagatcctc attcatattg gtcaccatct caatgtggtc 3000
   aaccttctag gtgcctgtac caagccagga gggccactca tggtgattgt 3050
   ggaattctgc aaatttggaa acctgtccac ttacctgagg agcaagagaa 3100
   atgaatttgt cccctacaag accaaagggg cacgattccg tcaagggaaa 3150
   gactacgttg gagcaatccc tgtggatctg aaacggcgct tggacagcat 3200
   caccagtagc cagagctcag ccagctctgg atttgtggag gagaagtccc 3250
   tcagtgatgt agaagaagag gaagctcctg aagatctgta taaggacttc 3300
   ctgaccttgg agcatctcat ctgttacagc ttccaagtgg ctaagggcat 3350
   ggagttcttg gcatcgcgaa agtgtatcca cagggacctg gcggcacgaa 3400
   atatcctctt atcggagaag aacgtggtta aaatctgtga ctttggcttg 3450
   gcccgggata tttataaaga tccagattat gtcagaaaag gagatgctcg 3500
   cctccctttg aaatggatgg ccccagaaac aatttttgac agagtgtaca 3550
   caatccagag tgacgtctgg tcttttggtg ttttgctgtg ggaaatattt 3600
   tccttaggtg cttctccata tcctggggta aagattgatg aagaattttg 3650
   taggcgattg aaagaaggaa ctagaatgag ggcccctgat tatactacac 3700
   cagaaatgta ccagaccatg ctggactgct ggcacgggga gcccagtcag 3750
   agacccacgt tttcagagtt ggtggaacat ttgggaaatc tcttgcaagc 3800
   taatgctcag caggatggca aagactacat tgttcttccg atatcagaga 3850
   ctttgagcat ggaagaggat tctggactct ctctgcctac ctcacctgtt 3900
   tcctgtatgg aggaggagga agtatgtgac cccaaattcc attatgacaa 3950
   cacagcagga atcagtcagt atctgcagaa cagtaagcga aagagccggc 4000
   ctgtgagtgt aaaaacattt gaagatatcc cgttagaaga accagaagta 4050
   aaagtaatcc cagatgacaa ccagacggac agtggtatgg ttcttgcctc 4100
   agaagagctg aaaactttgg aagacagaac caaattatct ccatcttttg 4150
   gtggaatggt gcccagcaaa agcagggagt ctgtggcatc tgaaggctca 4200
   aaccagacaa gcggctacca gtccggatat cactccgatg acacagacac 4250
   caccgtgtac tccagtgagg aagcagaact tttaaagctg atagagattg 4300
   gagtgcaaac cggtagcaca gcccagattc tccagcctga ctcggggacc 4350
   acactgagct ctcctcctgt ttaaaaggaa gcatccacac cccaactccc 4400
   ggacatcaca tgagaggtct gctcagattt tgaagtgttg ttctttccac 4450
   cagcaggaag tagccgcatt tgattttcat ttcgacaaca gaaaaaggac 4500
   ctcggactgc agggagccag tcttctaggc atatcctgga agaggcttgt 4550
   gacccaagaa tgtgtctgtg tcttctccca gtgttgacct gatcctcttt 4600
   tttcattcat ttaaaaagca ttatcatgcc cctgctgcgg gtctcaccat 4650
   gggtttagaa caaagagctt caagcaatgg ccccatcctc aaagaagtag 4700
   cagtacctgg ggagctgaca cttctgtaaa actagaagat aaaccaggca 4750
   acgtaagtgt tcgaggtgtt gaagatggga aggatttgca gggctgagtc 4800
   tatccaagag gctttgttta ggacgtgggt cccaagccaa gccttaagtg 4850
   tggaattcgg attgatagaa aggaagacta acgttacctt gctttggaga 4900
   gtactggagc ctgcaaatgc attgtgtttg ctctggtgga ggtgggcatg 4950
   gggtctgttc tgaaatgtaa agggttcaga cggggtttct ggttttagaa 5000
   ggttgcgtgt tcttcgagtt gggctaaagt agagttcgtt gtgctgtttc 5050
   tgactcctaa tgagagttcc ttccagaccg ttagctgtct ccttgccaag 5100
   ccccaggaag aaaatgatgc agctctggct ccttgtctcc caggctgatc 5150
   ctttattcag aataccacaa agaaaggaca ttcagctcaa ggctccctgc 5200
   cgtgttgaag agttctgact gcacaaacca gcttctggtt tcttctggaa 5250
   tgaataccct catatctgtc ctgatgtgat atgtctgaga ctgaatgcgg 5300
   gaggttcaat gtgaagctgt gtgtggtgtc aaagtttcag gaaggatttt 5350
   acccttttgt tcttccccct gtccccaacc cactctcacc ccgcaaccca 5400
   tcagtatttt agttatttgg cctctactcc agtaaacctg attgggtttg 5450
   ttcactctct gaatgattat tagccagact tcaaaattat tttatagccc 5500
   aaattataac atctattgta ttatttagac ttttaacata tagagctatt 5550
   tctactgatt tttgcccttg ttctgtcctt tttttcaaaa aagaaaatgt 5600
   gttttttgtt tggtaccata gtgtgaaatg ctgggaacaa tgactataag 5650
   acatgctatg gcacatatat ttatagtctg tttatgtaga aacaaatgta 5700
   atatattaaa gccttatata taatgaactt tgtactattc acattttgta 5750
   tcagtattat gtagcataac aaaggtcata atgctttcag caattgatgt 5800
   catttattta aagaacattg aaaaacttga 5830
<210> 28
   <211> 1759
   <212> DNA
   <213> Homo sapiens
<400> 28
   cccctgcctc tcggactcgg gctgcggcgt cagccttctt cgggcctcgg 50
   cagcggtagc ggctcgctcg cctcagcccc agcgcccctc ggctaccctc 100
   ggcccaggcc cgcagcgccg cccgccctcg gccgccccga cgccggcctg 150
   ggccgcggcc gcagccccgg gctcgcgtag gcgccgaccg ctcccggccc 200
   gccccctatg ggccccggct agaggcgccg ccgccgccgg cccgcggagc 250
   cccgatgctg gcccggagga agccggtgct gccggcgctc accatcaacc 300
   ctaccatcgc cgagggccca tcccctacca gcgagggcgc ctccgaggca 350
   aacct ggt gg acct gcagaa gaagct ggag gagct ggaac t t gacgagca 400
   gcagaagaag cggctggaag cctttctcac ccagaaagcc aaggtcggcg 450
   aactcaaaga cgatgacttc gaaaggatct cagagctggg cgcgggcaac 500
   ggcggggtgg tcaccaaagt ccagcacaga ccctcgggcc tcatcatggc 550
   caggaagct g at ccacct t g agat caagcc ggccat ccgg aaccagat ca 600
   t ccgcgagct gcaggt cct g cacgaat gca act cgccgt a cat cgt gggc 650
   t t ct acgggg cct t ct acag t gacggggag at cagcat t t gcat ggaaca 700
   catggacggc ggctccctgg accaggtgct gaaagaggcc aagaggattc 750
   ccgaggagat cctggggaaa gtcagcatcg cggttctccg gggcttggcg 800
   tacctccgag agaagcacca gatcatgcac cgagatgtga agccctccaa 850
   cat cct cgt g aact ct agag gggagat caa gct gt gt gac t t cggggt ga 900
   gcggccagct catagactcc atggccaact ccttcgtggg cacgcgctcc 950
   tacatggctc cggagcggtt gcagggcaca cattactcgg tgcagtcgga 1000
   cat ct ggagc at gggcct gt ccct ggt gga gct ggccgt c ggaaggt acc 1050
   ccatcccccc gcccgacgcc aaagagctgg aggccatctt tggccggccc 1100
   gtggtcgacg gggaagaagg agagcctcac agcatctcgc ctcggccgag 1150
   gccccccggg cgccccgtca gcggtcacgg gatggatagc cggcctgcca 1200
   t ggccat ct t t gaact cct g gact at at t g t gaacgagcc acct cct aag 1250
   ct gcccaacg gt gt gt t cac ccccgact t c caggagt t t g t caat aaat g 1300
   cctcatcaag aacccagcgg agcgggcgga cctgaagatg ctcacaaacc 1350
   acacct t cat caagcggt cc gaggt ggaag aagt ggat t t t gccggct gg 1400
   ttgtgtaaaa ccctgcggct gaaccagccc ggcacaccca cgcgcaccgc 1450
   cgt gt gacag t ggccgggct ccct gcgt cc cgct ggt gac ct gcccaccg 1500
   t ccct gt cca t gccccgccc t t ccagct ga ggacaggct g gcgcct ccac 1550
   ccaccctcct gcctcacccc tgcggagagc accgtggcgg ggcgacagcg 1600
   cat gcaggaa cgggggt ct c ct ct cct gcc cgt cct ggcc ggggt gcct c 1650
   t ggggacggg cgacgct gct gt gt gt ggt c t cagaggct c t gct t cct t a 1700
   ggttacaaaa caaaacaggg agagaaaaag caaaaaaaaa aaaaaaaaaa 1750
   aaaaaaaaa 1759
<210> 29
   <211> 6641
   <212> DNA
   <213> Homo sapiens
<400> 29
   gccctcgccg cccgcggcgc cccgagcgct ttgtgagcag atgcggagcc 50
   gagtggaggg cgcgagccag atgcggggcg acagctgact tgctgagagg 100
   aggcggggag gcgcggagcg cgcgtgtggt ccttgcgccg ctgacttctc 150
   cactggttcc tgggcaccga aagataaacc tctcataatg aaggcccccg 200
   ctgtgcttgc acctggcatc ctcgtgctcc tgtttacctt ggtgcagagg 250
   agcaatgggg agtgtaaaga ggcactagca aagtccgaga tgaatgtgaa 300
   tatgaagtat cagcttccca acttcaccgc ggaaacaccc atccagaatg 350
   tcattctaca tgagcatcac attttccttg gtgccactaa ctacatttat 400
   gttttaaatg aggaagacct tcagaaggtt gctgagtaca agactgggcc 450
   tgtgctggaa cacccagatt gtttcccatg tcaggactgc agcagcaaag 500
   ccaatttatc aggaggtgtt tggaaagata acatcaacat ggctctagtt 550
   gtcgacacct actatgatga tcaactcatt agctgtggca gcgtcaacag 600
   agggacctgc cagcgacatg tctttcccca caatcatact gctgacatac 650
   agtcggaggt tcactgcata ttctccccac agatagaaga gcccagccag 700
   tgtcctgact gtgtggtgag cgccctggga gccaaagtcc tttcatctgt 750
   aaaggaccgg ttcatcaact tctttgtagg caataccata aattcttctt 800
   atttcccaga tcatccattg cattcgatat cagtgagaag gctaaaggaa 850
   acgaaagatg gttttatgtt tttgacggac cagtcctaca ttgatgtttt 900
   acctgagttc agagattctt accccattaa gtatgtccat gcctttgaaa 950
   gcaacaattt tatttacttc ttgacggtcc aaagggaaac tctagatgct 1000
   cagacttttc acacaagaat aatcaggttc tgttccataa actctggatt 1050
   gcattcctac atggaaatgc ctctggagtg tattctcaca gaaaagagaa 1100
   aaaagagatc cacaaagaag gaagtgttta atatacttca ggctgcgtat 1150
   gtcagcaagc ctggggccca gcttgctaga caaataggag ccagcctgaa 1200
   tgatgacatt cttttcgggg tgttcgcaca aagcaagcca gattctgccg 1250
   aaccaatgga tcgatctgcc atgtgtgcat tccctatcaa atatgtcaac 1300
   gacttcttca acaagatcgt caacaaaaac aatgtgagat gtctccagca 1350
   tttttacgga cccaatcatg agcactgctt taataggaca cttctgagaa 1400
   attcatcagg ctgtgaagcg cgccgtgatg aatatcgaac agagtttacc 1450
   acagctttgc agcgcgttga cttattcatg ggtcaattca gcgaagtcct 1500
   cttaacatct atatccacct tcattaaagg agacctcacc atagctaatc 1550
   ttgggacatc agagggtcgc ttcatgcagg ttgtggtttc tcgatcagga 1600
   ccatcaaccc ctcatgtgaa ttttctcctg gactcccatc cagtgtctcc 1650
   agaagtgatt gtggagcata cattaaacca aaatggctac acactggtta 1700
   tcactgggaa gaagatcacg aagatcccat tgaatggctt gggctgcaga 1750
   catttccagt cctgcagtca atgcctctct gccccaccct ttgttcagtg 1800
   tggctggtgc cacgacaaat gtgtgcgatc ggaggaatgc ctgagcggga 1850
   catggactca acagatctgt ctgcctgcaa tctacaaggt tttcccaaat 1900
   agtgcacccc ttgaaggagg gacaaggctg accatatgtg gctgggactt 1950
   tggatttcgg aggaataata aatttgattt aaagaaaact agagttctcc 2000
   ttggaaatga gagctgcacc ttgactttaa gtgagagcac gatgaataca 2050
   ttgaaatgca cagttggtcc tgccatgaat aagcatttca atatgtccat 2100
   aattatttca aatggccacg ggacaacaca atacagtaca ttctcctatg 2150
   tggatcctgt aataacaagt atttcgccga aatacggtcc tatggctggt 2200
   ggcactttac ttactttaac tggaaattac ctaaacagtg ggaattctag 2250
   acacatttca attggtggaa aaacatgtac tttaaaaagt gtgtcaaaca 2300
   gtattcttga atgttatacc ccagcccaaa ccatttcaac tgagtttgct 2350
   gttaaattga aaattgactt agccaaccga gagacaagca tcttcagtta 2400
   ccgtgaagat cccattgtct atgaaattca tccaaccaaa tcttttatta 2450
   gtggtgggag cacaataaca ggtgttggga aaaacctgaa ttcagttagt 2500
   gtcccgagaa tggtcataaa tgtgcatgaa gcaggaagga actttacagt 2550
   ggcatgtcaa catcgctcta attcagagat aatctgttgt accactcctt 2600
   ccctgcaaca gctgaatctg caactccccc tgaaaaccaa agcctttttc 2650
   atgttagatg ggatcctttc caaatacttt gatctcattt atgtacataa 2700
   tcctgtgttt aagccttttg aaaagccagt gatgatctca atgggcaatg 2750
   aaaatgtact ggaaattaag ggaaatgata ttgaccctga agcagttaaa 2800
   ggtgaagtgt taaaagttgg aaataagagc tgtgagaata tacacttaca 2850
   ttctgaagcc gttttatgca cggtccccaa tgacctgctg aaattgaaca 2900
   gcgagctaaa tatagagtgg aagcaagcaa tttcttcaac cgtccttgga 2950
   aaagtaatag ttcaaccaga tcagaatttc acaggattga ttgctggtgt 3000
   tgtctcaata tcaacagcac tgttattact acttgggttt ttcctgtggc 3050
   tgaaaaagag aaagcaaatt aaagatctgg gcagtgaatt agttcgctac 3100
   gatgcaagag tacacactcc tcatttggat aggcttgtaa gtgcccgaag 3150
   tgtaagccca actacagaaa tggtttcaaa tgaatctgta gactaccgag 3200
   ctacttttcc agaagatcag tttcctaatt catctcagaa cggttcatgc 3250
   cgacaagtgc agtatcctct gacagacatg tcccccatcc taactagtgg 3300
   ggactctgat atatccagtc cattactgca aaatactgtc cacattgacc 3350
   tcagtgctct aaatccagag ctggtccagg cagtgcagca tgtagtgatt 3400
   gggcccagta gcctgattgt gcatttcaat gaagtcatag gaagagggca 3450
   ttttggttgt gtatatcatg ggactttgtt ggacaatgat ggcaagaaaa 3500
   ttcactgtgc tgtgaaatcc ttgaacagaa tcactgacat aggagaagtt 3550
   tcccaatttc tgaccgaggg aatcatcatg aaagatttta gtcatcccaa 3600
   tgtcctctcg ctcctgggaa tctgcctgcg aagtgaaggg tctccgctgg 3650
   tggtcctacc atacatgaaa catggagatc ttcgaaattt cattcgaaat 3700
   gagactcata atccaactgt aaaagatctt attggctttg gtcttcaagt 3750
   agccaaaggc atgaaatatc ttgcaagcaa aaagtttgtc cacagagact 3800
   tggctgcaag aaactgtatg ctggatgaaa aattcacagt caaggttgct 3850
   gattttggtc ttgccagaga catgtatgat aaagaatact atagtgtaca 3900
   caacaaaaca ggtgcaaagc tgccagtgaa gtggatggct ttggaaagtc 3950
   tgcaaactca aaagtttacc accaagtcag atgtgtggtc ctttggcgtg 4000
   ctcctctggg agctgatgac aagaggagcc ccaccttatc ctgacgtaaa 4050
   cacctttgat ataactgttt acttgttgca agggagaaga ctcctacaac 4100
   ccgaatactg cccagacccc ttatatgaag taatgctaaa atgctggcac 4150
   cctaaagccg aaatgcgccc atccttttct gaactggtgt cccggatatc 4200
   agcgatcttc tctactttca ttggggagca ctatgtccat gtgaacgcta 4250
   cttatgtgaa cgtaaaatgt gtcgctccgt atccttctct gttgtcatca 4300
   gaagataacg ctgatgatga ggtggacaca cgaccagcct ccttctggga 4350
   gacatcatag tgctagtact atgtcaaagc aacagtccac actttgtcca 4400
   atggtttttt cactgcctga cctttaaaag gccatcgata ttctttgctc 4450
   ttgccaaaat tgcactatta taggacttgt attgttattt aaattactgg 4500
   attctaagga atttcttatc tgacagagca tcagaaccag aggcttggtc 4550
   ccacaggcca cggaccaatg gcctgcagcc gtgacaacac tcctgtcata 4600
   ttggagtcca aaacttgaat tctgggttga attttttaaa aatcaggtac 4650
   cacttgattt catatgggaa attgaagcag gaaatattga gggcttcttg 4700
   atcacagaaa actcagaaga gatagtaatg ctcaggacag gagcggcagc 4750
   cccagaacag gccactcatt tagaattcta gtgtttcaaa acacttttgt 4800
   gtgttgtatg gtcaataaca tttttcatta ctgatggtgt cattcaccca 4850
   ttaggtaaac attccctttt aaatgtttgt ttgttttttg agacaggatc 4900
   tcactctgtt gccagggctg tagtgcagtg gtgtgatcat agctcactgc 4950
   aacctccacc tcccaggctc aagcctcccg aatagctggg actacaggcg 5000
   cacaccacca tccccggcta atttttgtat tttttgtaga gacggggttt 5050
   tgccatgttg ccaaggctgg tttcaaactc ctggactcaa gaaatccacc 5100
   cacctcagcc tcccaaagtg ctaggattac aggcatgagc cactgcgccc 5150
   agcccttata aatttttgta tagacattcc tttggttgga agaatattta 5200
   taggcaatac agtcaaagtt tcaaaatagc atcacacaaa acatgtttat 5250
   aaatgaacag gatgtaatgt acatagatga cattaagaaa atttgtatga 5300
   aataatttag tcatcatgaa atatttagtt gtcatataaa aacccactgt 5350
   ttgagaatga tgctactctg atctaatgaa tgtgaacatg tagatgtttt 5400
   gtgtgtattt ttttaaatga aaactcaaaa taagacaagt aatttgttga 5450
   taaatatttt taaagataac tcagcatgtt tgtaaagcag gatacatttt 5500
   actaaaaggt tcattggttc caatcacagc tcataggtag agcaaagaaa 5550
   gggtggatgg attgaaaaga ttagcctctg tctcggtggc aggttcccac 5600
   ctcgcaagca attggaaaca aaacttttgg ggagttttat tttgcattag 5650
   ggtgtgtttt atgttaagca aaacatactt tagaaacaaa tgaaaaaggc 5700
   aattgaaaat cccagctatt tcacctagat ggaatagcca ccctgagcag 5750
   aactttgtga tgcttcattc tgtggaattt tgtgcttgct actgtatagt 5800
   gcatgtggtg taggttactc taactggttt tgtcgacgta aacatttaaa 5850
   gtgttatatt ttttataaaa atgtttattt ttaatgatat gagaaaaatt 5900
   ttgttaggcc acaaaaacac tgcactgtga acattttaga aaaggtatgt 5950
   cagactggga ttaatgacag catgattttc aatgactgta aattgcgata 6000
   aggaaatgta ctgattgcca atacacccca ccctcattac atcatcagga 6050
   cttgaagcca agggttaacc cagcaagcta caaagagggt gtgtcacact 6100
   gaaactcaat agttgagttt ggctgttgtt gcaggaaaat gattataact 6150
   aaaagctctc tgatagtgca gagacttacc agaagacaca aggaattgta 6200
   ctgaagagct attacaatcc aaatattgcc gtttcataaa tgtaataagt 6250
   aatactaatt cacagagtat tgtaaatggt ggatgacaaa agaaaatctg 6300
   ctctgtggaa agaaagaact gtctctacca gggtcaagag catgaacgca 6350
   tcaatagaaa gaactcgggg aaacatccca tcaacaggac tacacacttg 6400
   tatatacatt cttgagaaca ctgcaatgtg aaaatcacgt ttgctattta 6450
   taaacttgtc cttagattaa tgtgtctgga cagattgtgg gagtaagtga 6500
   ttcttctaag aattagatac ttgtcactgc ctatacctgc agctgaactg 6550
   aatggtactt cgtatgttaa tagttgttct gataaatcat gcaattaaag 6600
   taaagtgatg caacatcttg taaaaaaaaa aaaaaaaaaa a 6641
<210> 30
   <211> 3724
   <212> DNA
   <213> Homo sapiens
<400> 30
   tctccctcgg cgccgccgcc gccgcccgcg gggctgggac ccgatgcggt 50
   tagagccgcg gagcctggaa gagccccgag cgtttctgct ttgggacaac 100
   catacatcta attccttaaa gtagttttat atgtaaaact tgcaaagaat 150
   cagaacaatg cctccacgac catcatcagg tgaactgtgg ggcatccact 200
   tgatgccccc aagaatccta gtagaatgtt tactaccaaa tggaatgata 250
   gtgactttag aatgcctccg tgaggctaca ttaataacca taaagcatga 300
   actatttaaa gaagcaagaa aataccccct ccatcaactt cttcaagatg 350
   aatcttctta cattttcgta agtgttactc aagaagcaga aagggaagaa 400
   ttttttgatg aaacaagacg actttgtgac cttcggcttt ttcaaccctt 450
   tttaaaagta attgaaccag taggcaaccg tgaagaaaag atcctcaatc 500
   gagaaattgg ttttgctatc ggcatgccag tgtgtgaatt tgatatggtt 550
   aaagatccag aagtacagga cttccgaaga aatattctga acgtttgtaa 600
   agaagctgtg gatcttaggg acctcaattc acctcatagt agagcaatgt 650
   atgtctatcc tccaaatgta gaatcttcac cagaattgcc aaagcacata 700
   tataataaat tagataaagg gcaaataata gtggtgatct gggtaatagt 750
   ttctccaaat aatgacaagc agaagtatac tctgaaaatc aaccatgact 800
   gtgtaccaga acaagtaatt gctgaagcaa tcaggaaaaa aactcgaagt 850
   atgttgctat cctctgaaca actaaaactc tgtgttttag aatatcaggg 900
   caagtatatt ttaaaagtgt gtggatgtga tgaatacttc ctagaaaaat 950
   atcctctgag tcagtataag tatataagaa gctgtataat gcttgggagg 1000
   atgcccaatt tgatgttgat ggctaaagaa agcctttatt ctcaactgcc 1050
   aatggactgt tttacaatgc catcttattc cagacgcatt tccacagcta 1100
   caccatatat gaatggagaa acatctacaa aatccctttg ggttataaat 1150
   agtgcactca gaataaaaat tctttgtgca acctacgtga atgtaaatat 1200
   tcgagacatt gataagatct atgttcgaac aggtatctac catggaggag 1250
   aacccttatg tgacaatgtg aacactcaaa gagtaccttg ttccaatccc 1300
   aggtggaatg aatggctgaa ttatgatata tacattcctg atcttcctcg 1350
   tgctgctcga ctttgccttt ccatttgctc tgttaaaggc cgaaagggtg 1400
   ctaaagagga acactgtcca ttggcatggg gaaatataaa cttgtttgat 1450
   tacacagaca ctctagtatc tggaaaaatg gctttgaatc tttggccagt 1500
   acctcatgga ttagaagatt tgctgaaccc tattggtgtt actggatcaa 1550
   atccaaataa agaaactcca tgcttagagt tggagtttga ctggttcagc 1600
   agtgtggtaa agttcccaga tatgtcagtg attgaagagc atgccaattg 1650
   gtctgtatcc cgagaagcag gatttagcta ttcccacgca ggactgagta 1700
   acagactagc tagagacaat gaattaaggg aaaatgacaa agaacagctc 1750
   aaagcaattt ctacacgaga tcctctctct gaaatcactg agcaggagaa 1800
   agattttcta tggagtcaca gacactattg tgtaactatc cccgaaattc 1850
   tacccaaatt gcttctgtct gttaaatgga attctagaga tgaagtagcc 1900
   cagatgtatt gcttggtaaa agattggcct ccaatcaaac ctgaacaggc 1950
   tatggaactt ctggactgta attacccaga tcctatggtt cgaggttttg 2000
   ctgttcggtg cttggaaaaa tatttaacag atgacaaact ttctcagtat 2050
   ttaattcagc tagtacaggt cctaaaatat gaacaatatt tggataactt 2100
   gcttgtgaga tttttactga agaaagcatt gactaatcaa aggattgggc 2150
   actttttctt ttggcattta aaatctgaga tgcacaataa aacagttagc 2200
   cagaggtttg gcctgctttt ggagtcctat tgtcgtgcat gtgggatgta 2250
   tttgaagcac ctgaataggc aagtcgaggc aatggaaaag ctcattaact 2300
   taactgacat tctcaaacag gagaagaagg atgaaacaca aaaggtacag 2350
   atgaagtttt tagttgagca aatgaggcga ccagatttca tggatgctct 2400
   acagggcttt ctgtctcctc taaaccctgc tcatcaacta ggaaacctca 2450
   ggcttgaaga gtgtcgaatt atgtcctctg caaaaaggcc actgtggttg 2500
   aattgggaga acccagacat catgtcagag ttactgtttc agaacaatga 2550
   gatcatcttt aaaaatgggg atgatttacg gcaagatatg ctaacacttc 2600
   aaattattcg tattatggaa aatatctggc aaaatcaagg tcttgatctt 2650
   cgaatgttac cttatggttg tctgtcaatc ggtgactgtg tgggacttat 2700
   tgaggtggtg cgaaattctc acactattat gcaaattcag tgcaaaggcg 2750
   gcttgaaagg tgcactgcag ttcaacagcc acacactaca tcagtggctc 2800
   aaagacaaga acaaaggaga aatatatgat gcagccattg acctgtttac 2850
   acgttcatgt gctggatact gtgtagctac cttcattttg ggaattggag 2900
   atcgtcacaa tagtaacatc atggtgaaag acgatggaca actgtttcat 2950
   atagattttg gacacttttt ggatcacaag aagaaaaaat ttggttataa 3000
   acgagaacgt gtgccatttg ttttgacaca ggatttctta atagtgatta 3050
   gtaaaggagc ccaagaatgc acaaagacaa gagaatttga gaggtttcag 3100
   gagatgtgtt acaaggctta tctagctatt cgacagcatg ccaatctctt 3150
   cataaatctt ttctcaatga tgcttggctc tggaatgcca gaactacaat 3200
   cttttgatga cattgcatac attcgaaaga ccctagcctt agataaaact 3250
   gagcaagagg ctttggagta tttcatgaaa caaatgaatg atgcacatca 3300
   tggtggctgg acaacaaaaa tggattggat cttccacaca attaaacagc 3350
   atgcattgaa ctgaaaagat aactgagaaa atgaaagctc actctggatt 3400
   ccacactgca ctgttaataa ctctcagcag gcaaagaccg attgcatagg 3450
   aattgcacaa tccatgaaca gcattagaat ttacagcaag aacagaaata 3500
   aaatactata taatttaaat aatgtaaacg caaacagggt ttgatagcac 3550
   ttaaactagt tcatttcaaa attaagcttt agaataatgc gcaatttcat 3600
   gttatgcctt aagtccaaaa aggtaaactt tgaagattgt ttgtatcttt 3650
   ttttaaaaaa caaaacaaaa caaaaatccc caaaatatat agaaatgatg 3700
   gagaaggaaa aaaaaaaaaa aaaa 3724
<210> 31
   <211> 2204
   <212> DNA
   <213> Homo sapiens
<400> 31
   gagcggtgcg gaggctctgc tcggatcgag gtctgcagcg cagcttcggg 50
   agcatgagtg ctgcagtgac tgcagggaag ctggcacggg caccggccga 100
   ccctgggaaa gccggggtcc ccggagttgc agctcccgga gctccggcgg 150
   cggctccacc ggcgaaagag atcccggagg tcctagtgga cccacgcagc 200
   cggcggcgct atgtgcgggg ccgctttttg ggcaagggcg gctttgccaa 250
   gtgcttcgag atctcggacg cggacaccaa ggaggtgttc gcgggcaaga 300
   ttgtgcctaa gtctctgctg ctcaagccgc accagaggga gaagatgtcc 350
   atggaaatat ccattcaccg cagcctcgcc caccagcacg tcgtaggatt 400
   ccacggcttt ttcgaggaca acgacttcgt gttcgtggtg ttggagctct 450
   gccgccggag gtctctcctg gagctgcaca agaggaggaa agccctgact 500
   gagcctgagg cccgatacta cctacggcaa attgtgcttg gctgccagta 550
   cctgcaccga aaccgagtta ttcatcgaga cctcaagctg ggcaaccttt 600
   tcctgaatga agatctggag gtgaaaatag gggattttgg actggcaacc 650
   aaagtcgaat atgacgggga gaggaagaag accctgtgtg ggactcctaa 700
   ttacatagct cccgaggtgc tgagcaagaa agggcacagt ttcgaggtgg 750
   atgtgtggtc cattgggtgt atcatgtata ccttgttagt gggcaaacca 800
   ccttttgaga cttcttgcct aaaagagacc tacctccgga tcaagaagaa 850
   tgaatacagt attcccaagc acatcaaccc cgtggccgcc tccctcatcc 900
   agaagatgct tcagacagat cccactgccc gcccaaccat taacgagctg 950
   cttaatgacg agttctttac ttctggctat atccctgccc gtctccccat 1000
   cacctgcctg accattccac caaggttttc gattgctccc agcagcctgg 1050
   accccagcaa ccggaagccc ctcacagtcc tcaataaagg cttggagaac 1100
   cccctgcctg agcgtccccg ggaaaaagaa gaaccagtgg ttcgagagac 1150
   aggtgaggtg gtcgactgcc acctcagtga catgctgcag cagctgcaca 1200
   gtgtcaatgc ctccaagccc tcggagcgtg ggctggtcag gcaagaggag 1250
   gctgaggatc ctgcctgcat ccccatcttc tgggtcagca agtgggtgga 1300
   ctattcggac aagtacggcc ttgggtatca gctctgtgat aacagcgtgg 1350
   gggtgctctt caatgactca acacgcctca tcctctacaa tgatggtgac 1400
   agcctgcagt acatagagcg tgacggcact gagtcctacc tcaccgtgag 1450
   ttcccatccc aactccttga tgaagaagat caccctcctt aaatatttcc 1500
   gcaattacat gagcgagcac ttgctgaagg caggtgccaa catcacgccg 1550
   cgcgaaggtg atgagctcgc ccggctgccc tacctacgga cctggttccg 1600
   cacccgcagc gccatcatcc tgcacctcag caacggcagc gtgcagatca 1650
   acttcttcca ggatcacacc aagctcatct tgtgcccact gatggcagcc 1700
   gtgacctaca tcgacgagaa gcgggacttc cgcacatacc gcctgagtct 1750
   cctggaggag tacggctgct gcaaggagct ggccagccgg ctccgctacg 1800
   cccgcactat ggtggacaag ctgctgagct cacgctcggc cagcaaccgt 1850
   ctcaaggcct cctaatagct gccctcccct ccggactggt gccctcctca 1900
   ctcccacctg catctggggc ccatactggt tggctcccgc ggtgccatgt 1950
   ctgcagtgtg ccccccagcc ccggtggctg ggcagagctg catcatcctt 2000
   gcaggtgggg gttgctgtgt aagttatttt tgtacatgtt cgggtgtggg 2050
   ttctacagcc ttgtccccct ccccctcaac cccaccatat gaattgtaca 2100
   gaatatttct attgaattcg gaactgtcct ttccttggct ttatgcacat 2150
   taaacagatg tgaatattca aaaaaaaaaa aaaaaaaaaa aaaaaaaaaa 2200
   aaaa 2204
<210> 32
   <211> 6825
   <212> DNA
   <213> Homo sapiens
<400> 32
   gcgcccgccg tgtgagcagc agcagcggct ggtctgtcaa ccggagcccg 50
   agcccgagca gcctgcggcc agcagcgtcc tcgcaagccg agcgcccagg 100
   cgcgccagga gcccgcagca gcggcagcag cgcgccgggc cgcccgggaa 150
   gcctccgtcc ccgcggcggc ggcggcggcg gcggcaacat ggcctcggct 200
   ggtaacgccg ccgagcccca ggaccgcggc ggcggcggca gcggctgtat 250
   cggtgccccg ggacggccgg ctggaggcgg gaggcgcaga cggacggggg 300
   ggctgcgccg tgctgccgcg ccggaccggg actatctgca ccggcccagc 350
   tactgcgacg ccgccttcgc tctggagcag atttccaagg ggaaggctac 400
   tggccggaaa gcgccgctgt ggctgagagc gaagtttcag agactcttat 450
   ttaaactggg ttgttacatt caaaaaaact gcggcaagtt cttggttgtg 500
   ggcctcctca tatttggggc cttcgcggtg ggattaaaag cagcgaacct 550
   cgagaccaac gtggaggagc tgtgggtgga agttggagga cgagtaagtc 600
   gtgaattaaa ttatactcgc cagaagattg gagaagaggc tatgtttaat 650
   cctcaactca tgatacagac ccctaaagaa gaaggtgcta atgtcctgac 700
   cacagaagcg ctcctacaac acctggactc ggcactccag gccagccgtg 750
   t ccat gt at a cat gt acaac aggcagt gga aat t ggaaca t t t gt gt t ac 800
   aaatcaggag agcttatcac agaaacaggt tacatggatc agataataga 850
   at at ct t t ac cct t gt t t ga t t at t acacc t t t ggact gc t t ct gggaag 900
   gggcgaaat t acagt ct ggg acagcat acc t cct aggt aa acct cct t t g 950
   cggt ggacaa act t cgaccc t t t ggaat t c ct ggaagagt t aaagaaaat 1000
   aaactatcaa gtggacagct gggaggaaat gctgaataag gctgaggttg 1050
   gt cat ggt t a cat ggaccgc ccct gcct ca at ccggccga t ccagact gc 1100
   cccgccacag cccccaacaa aaattcaacc aaacctcttg atatggccct 1150
   t gt t t t gaat ggt ggat gt c at ggct t at c cagaaagt at at gcact ggc 1200
   aggaggagtt gattgtgggt ggcacagtca agaacagcac tggaaaactc 1250
   gt cagcgccc at gccct gca gaccat gt t c cagt t aat ga ct cccaagca 1300
   aat gt acgag cact t caagg ggt acgagt a t gt ct cacac at caact gga 1350
   acgaggacaa agcggcagcc atcctggagg cctggcagag gacatatgtg 1400
   gaggtggttc atcagagtgt cgcacagaac tccactcaaa aggtgctttc 1450
   ct t caccacc acgaccct gg acgacat cct gaaat cct t c t ct gacgt ca 1500
   gt gt cat ccg cgt ggccagc ggct act t ac t cat gct cgc ct at gcct gt 1550
   ct aaccat gc t gcgct ggga ct gct ccaag t cccagggt g ccgt ggggct 1600
   ggct ggcgt c ct gct ggt t g cact gt cagt ggct gcagga ct gggcct gt 1650
   gct cat t gat cggaat t t cc t t t aacgct g caacaact ca ggt t t t gcca 1700
   t t t ct cgct c t t ggt gt t gg t gt ggat gat gt t t t t ct t c t ggcccacgc 1750
   cttcagtgaa acaggacaga ataaaagaat cccttttgag gacaggaccg 1800
   gggagtgcct gaagcgcaca ggagccagcg tggccctcac gtccatcagc 1850
   aat gt cacag cct t ct t cat ggccgcgt t a at cccaat t c ccgct ct gcg 1900
   ggcgt t ct cc ct ccaggcag cggt agt agt ggt gt t caat t t t gccat gg 1950
   t t ct gct cat t t t t cct gca at t ct cagca t ggat t t at a t cgacgcgag 2000
   gacaggagac tggatatttt ctgctgtttt acaagcccct gcgtcagcag 2050
   agtgattcag gttgaacctc aggcctacac cgacacacac gacaataccc 2100
   gct acagccc cccacct ccc t acagcagcc acagct t t gc ccat gaaacg 2150
   cagattacca tgcagtccac tgtccagctc cgcacggagt acgaccccca 2200
   cacgcacgt g t act acacca ccgct gagcc gcgct ccgag at ct ct gt gc 2250
   agcccgtcac cgtgacacag gacaccctca gctgccagag cccagagagc 2300
   accagctcca caagggacct gctctcccag ttctccgact ccagcctcca 2350
   ct gcct cgag cccccct gt a cgaagt ggac act ct cat ct t t t gct gaga 2400
   agcactatgc tcctttcctc ttgaaaccaa aagccaaggt agtggtgatc 2450
   ttcctttttc tgggcttgct gggggtcagc ctttatggca ccacccgagt 2500
   gagagacggg ctggacctta cggacattgt acctcgggaa accagagaat 2550
   atgactttat tgctgcacaa ttcaaatact tttctttcta caacatgtat 2600
   atagtcaccc agaaagcaga ctacccgaat atccagcact tactttacga 2650
   cctacacagg agtttcagta acgtgaagta tgtcatgttg gaagaaaaca 2700
   aacagcttcc caaaatgtgg ctgcactact tcagagactg gcttcaggga 2750
   cttcaggatg catttgacag tgactgggaa accgggaaaa tcatgccaaa 2800
   caattacaag aatggatcag acgatggagt ccttgcctac aaactcctgg 2850
   tgcaaaccgg cagccgcgat aagcccatcg acatcagcca gttgactaaa 2900
   cagcgtctgg tggatgcaga tggcatcatt aatcccagcg ctttctacat 2950
   ctacctgacg gcttgggtca gcaacgaccc cgtcgcgtat gctgcctccc 3000
   aggccaacat ccggccacac cgaccagaat gggtccacga caaagccgac 3050
   tacatgcctg aaacaaggct gagaatcccg gcagcagagc ccatcgagta 3100
   tgcccagttc cctttctacc tcaacggctt gcgggacacc tcagactttg 3150
   tggaggcaat tgaaaaagta aggaccatct gcagcaacta tacgagcctg 3200
   gggctgtcca gttaccccaa cggctacccc ttcctcttct gggagcagta 3250
   catcggcctc cgccactggc tgctgctgtt catcagcgtg gtgttggcct 3300
   gcacattcct cgtgtgcgct gtcttccttc tgaacccctg gacggccggg 3350
   atcattgtga tggtcctggc gctgatgacg gtcgagctgt tcggcatgat 3400
   gggcctcatc ggaatcaagc tcagtgccgt gcccgtggtc atcctgatcg 3450
   cttctgttgg cataggagtg gagttcaccg ttcacgttgc tttggccttt 3500
   ctgacggcca tcggcgacaa gaaccgcagg gctgtgcttg ccctggagca 3550
   catgtttgca cccgtcctgg atggcgccgt gtccactctg ctgggagtgc 3600
   tgatgctggc gggatctgag ttcgacttca ttgtcaggta tttctttgct 3650
   gtgctggcga tcctcaccat cctcggcgtt ctcaatgggc tggttttgct 3700
   tcccgtgctt ttgtctttct ttggaccata tcctgaggtg tctccagcca 3750
   acggcttgaa ccgcctgccc acaccctccc ctgagccacc ccccagcgtg 3800
   gtccgcttcg ccatgccgcc cggccacacg cacagcgggt ctgattcctc 3850
   cgactcggag tatagttccc agacgacagt gtcaggcctc agcgaggagc 3900
   ttcggcacta cgaggcccag cagggcgcgg gaggccctgc ccaccaagtg 3950
   atcgtggaag ccacagaaaa ccccgtcttc gcccactcca ctgtggtcca 4000
   tcccgaatcc aggcatcacc caccctcgaa cccgagacag cagccccacc 4050
   tggactcagg gtccctgcct cccggacggc aaggccagca gccccgcagg 4100
   gaccccccca gagaaggctt gtggccaccc ctctacagac cgcgcagaga 4150
   cgcttttgaa atttctactg aagggcattc tggccctagc aatagggccc 4200
   gctggggccc tcgcggggcc cgttctcaca accctcggaa cccagcgtcc 4250
   actgccatgg gcagctccgt gcccggctac tgccagccca tcaccactgt 4300
   gacggcttct gcctccgtga ctgtcgccgt gcacccgccg cctgtccctg 4350
   ggcctgggcg gaacccccga gggggactct gcccaggcta ccctgagact 4400
   gaccacggcc tgtttgagga cccccacgtg cctttccacg tccggtgtga 4450
   gaggagggat tcgaaggtgg aagtcattga gctgcaggac gtggaatgcg 4500
   aggagaggcc ccggggaagc agctccaact gagggtgatt aaaatctgaa 4550
   gcaaagaggc caaagattgg aaacccccca cccccacctc tttccagaac 4600
   tgcttgaaga gaactggttg gagttatgga aaagatgccc tgtgccagga 4650
   cagcagttca ttgttactgt aaccgattgt attattttgt taaatatttc 4700
   tataaatatt taagagatgt acacatgtgt aatataggaa ggaaggatgt 4750
   aaagtggtat gatctggggc ttctccactc ctgccccaga gtgtggaggc 4800
   cacagtgggg cctctccgta tttgtgcatt gggctccgtg ccacaaccaa 4850
   gcttcattag tcttaaattt cagcatatgt tgctgctgct taaatattgt 4900
   ataatttact tgtataattc tatgcaaata ttgcttatgt aataggatta 4950
   ttttgtaaag gtttctgttt aaaatatttt aaatttgcat atcacaaccc 5000
   tgtggtagta tgaaatgtta ctgttaactt tcaaacacgc tatgcgtgat 5050
   aatttttttg tttaatgagc agatatgaag aaagcacgtt aatcctggtg 5100
   gcttctctag gtgtcgttgt gtgcggtcct cttgtttggc tgtgcgtgtg 5150
   aacacgtgtg tgagttcacc atgtactgta ctgtgatttt ttttttgtct 5200
   tgttttgttt ctctacactg tctgtaacct gtagtaggct ctgacctagt 5250
   caggctggaa gcgtcaggat atctttcttc cgtgctggtg agggctggcc 5300
   ctaaacatcc acctaatcct ttcaaatcag cccggcaaaa gctagactct 5350
   cctcgtgtct acggcatctc ttatgatcat tggctgccat ccaggacccc 5400
   aatttgtgct tcagggggat aatctccttc tctcggatca ttgtgatgga 5450
   tgctggaacc tcagggtatg gagctcacat cagttcatca tggtgggtgt 5500
   tagagaattc ggtgacatgc ctagtgctga gccttggctg ggccatgaga 5550
   gtctgtatac tctaaaaagc atgcagcatg gtgcccctct tctgaccaac 5600
   acacacacga cccctccccc aacaccccca aattcaagag tggatgtggc 5650
   cctgtcacag gtagaaaaac ctatttagtt aattctttct tggcccacag 5700
   tctcccagaa atgatgtttt gagtccctat agtttaaact ccctctctta 5750
   aatggagcag ctggttgagg ctttctagat ctgtttgcat cttctttaaa 5800
   actaagtggt gagcatgcat tgtggtgtag aggcaggcat tatgtaggat 5850
   aagagct ccg gggggat t ct t cat gcacca gt gt t t aggg t acgt gct t c 5900
   ct aagt aaat ccaaacat t g t ct ccat cct ccccgt cat t agt gct ct t t 5950
   caatgtgatg tgggaaagca ggaggatgga cacaccccac tgaaagatgt 6000
   aggcaggggc aggtctctca accaggcata tttttaaaag ttgcttctgt 6050
   act ggt t ct c t t ct t t t gct ct gaggt gt g ggct ccct ca t ct cgt aacc 6100
   agagaccagc acatgtcagg gaagcaccca gtgtcggctc cccatccaaa 6150
   tccacaccag caccttgtta cagacaagaa gtcagaggaa agggcggggt 6200
   ccct gcaggg ct gaagcct a agct act gt g aggcgct cac gagt ggcagc 6250
   t cct gt t act ccct t t t aaa t t acct ggga aat ct t aaca gaaaggt aat 6300
   gggcccccag aaatacccac agcatagtga cctcagaccc tgatactcac 6350
   cacaaaact t t t aagat gct gat t gggagc cgct t gt ggc t gct gggt gt 6400
   gt gt gt gt gt gt gt gcgt gc gt gcgt gt gt gt gt gt ct ct gct ggggacc 6450
   ct ggccaccc ccct gct gct gt ct t ggt gc ct gt caccca cat ggt ct gc 6500
   catcctaaca cccagctctg ctcagaaaac gtcctgcgtg gaggagggat 6550
   gat gcagaat t ct gaagt cg act t ccct ct ggct cct ggc gt gccct cgc 6600
   t ccct t cct g agcccagct c gt gt t gcgcc ggaggct gcg cggcccct ga 6650
   tttctgcatg gtgtagaact ttctccaata gtcacattgg caaagggaga 6700
   act ggggt gg gcggggggt g gggct ggcag ggaat t agaa t t t ct ct ct c 6750
   t ct t t t aat a gt t t t at t t t gt ct gt cct g t t t gt t cat t t ggat gt t t t 6800
   aat t t t t aaa aaaaaaaaaa aaaaa 6825
<210> 33
   <211> 4740
   <212> DNA
   <213> Homo sapiens
<400> 33
   ttccggtttt tctcagggga cgttgaaatt atttttgtaa cgggagtcgg 50
   gagaggacgg ggcgtgcccc gcgtgcgcgc gcgtcgtcct ccccggcgct 100
   cctccacagc tcgctggctc ccgccgcgga aaggcgtcat gccgcccaaa 150
   accccccgaa aaacggccgc caccgccgcc gctgccgccg cggaaccccc 200
   ggcaccgccg ccgccgcccc ctcctgagga ggacccagag caggacagcg 250
   gcccggagga cctgcctctc gtcaggcttg agtttgaaga aacagaagaa 300
   cctgatttta ctgcattatg tcagaaatta aagataccag atcatgtcag 350
   agagagagct tggttaactt gggagaaagt ttcatctgtg gatggagtat 400
   tgggaggtta tattcaaaag aaaaaggaac tgtggggaat ctgtatcttt 450
   attgcacgag ttgacctaga tgagatgtcg ttcactttac tgagctacag 500
   aaaaacatac gaaatcagtg tccataaatt ctttaactta ctaaaagaaa 550
   ttgataccag taccaaagtt gataatgcta tgtcaagact gttgaagaag 600
   tatgatgtat tgtttgcact cttcagcaaa ttggaaagga catgtgaact 650
   tatatatttg acacaaccca gcagttcgat atctactgaa ataaattctg 700
   cattggtgct aaaagtttct tggatcacat ttttattagc taaaggggaa 750
   gtattacaaa tggaagatga tctggtgatt tcatttcagt taatgctatg 800
   tgtccttgac tattttatta aactctcacc tcccatgttg ctcaaagaac 850
   catataaaac agctgttata cccattaatg gttcacctcg aacacccagg 900
   cgaggtcaga acaggagtgc acggatagca aaacaactag aaaatgatac 950
   aagaattatt gaagttctct gtaaagaaca tgaatgtaat atagatgagg 1000
   tgaaaaatgt ttatttcaaa aattttatac cttttatgaa ttctcttgga 1050
   cttgtaacat ctaatggact tccagaggtt gaaaatcttt ctaaacgata 1100
   cgaagaaatt tatcttaaaa ataaagatct agatcgaaga ttatttttgg 1150
   atcatgataa aactcttcag actgattcta tagacagttt tgaaacacag 1200
   agaacaccac gaaaaagtaa ccttgatgaa gaggtgaata taattcctcc 1250
   acacactcca gttaggactg ttatgaacac tatccaacaa ttaatgatga 1300
   ttttaaattc tgcaagtgat caaccttcag aaaatctgat ttcctatttt 1350
   aacaactgca cagtgaatcc aaaagaaagt atactgaaaa gagtgaagga 1400
   tataggatac atctttaaag agaaatttgc taaagctgtg ggacagggtt 1450
   gtgtcgaaat tggatcacag cgatacaaac ttggagttcg cttgtattac 1500
   cgagtaatgg aatccatgct taaatcagaa gaagaacgat tatccattca 1550
   aaattttagc aaacttctga atgacaacat ttttcatatg tctttattgg 1600
   cgtgcgctct tgaggttgta atggccacat atagcagaag tacatctcag 1650
   aatcttgatt ctggaacaga tttgtctttc ccatggattc tgaatgtgct 1700
   taatttaaaa gcctttgatt tttacaaagt gatcgaaagt tttatcaaag 1750
   cagaaggcaa cttgacaaga gaaatgataa aacatttaga acgatgtgaa 1800
   catcgaatca tggaatccct tgcatggctc tcagattcac ctttatttga 1850
   tcttattaaa caatcaaagg accgagaagg accaactgat caccttgaat 1900
   ctgcttgtcc tcttaatctt cctctccaga ataatcacac tgcagcagat 1950
   atgtatcttt ctcctgtaag atctccaaag aaaaaaggtt caactacgcg 2000
   tgtaaattct actgcaaatg cagagacaca agcaacctca gccttccaga 2050
   cccagaagcc attgaaatct acctctcttt cactgtttta taaaaaagtg 2100
   tatcggctag cctatctccg gctaaataca ctttgtgaac gccttctgtc 2150
   tgagcaccca gaattagaac atatcatctg gacccttttc cagcacaccc 2200
   tgcagaatga gtatgaactc atgagagaca ggcatttgga ccaaattatg 2250
   atgtgttcca tgtatggcat atgcaaagtg aagaatatag accttaaatt 2300
   caaaatcatt gtaacagcat acaaggatct tcctcatgct gttcaggaga 2350
   cattcaaacg tgttttgatc aaagaagagg agtatgattc tattatagta 2400
   ttctataact cggtcttcat gcagagactg aaaacaaata ttttgcagta 2450
   tgcttccacc aggcccccta ccttgtcacc aatacctcac attcctcgaa 2500
   gcccttacaa gtttcctagt tcacccttac ggattcctgg agggaacatc 2550
   tatatttcac ccctgaagag tccatataaa atttcagaag gtctgccaac 2600
   accaacaaaa atgactccaa gatcaagaat cttagtatca attggtgaat 2650
   cattcgggac ttctgagaag ttccagaaaa taaatcagat ggtatgtaac 2700
   agcgaccgtg tgctcaaaag aagtgctgaa ggaagcaacc ctcctaaacc 2750
   actgaaaaaa ctacgctttg atattgaagg atcagatgaa gcagatggaa 2800
   gtaaacatct cccaggagag tccaaatttc agcagaaact ggcagaaatg 2850
   acttctactc gaacacgaat gcaaaagcag aaaatgaatg atagcatgga 2900
   tacctcaaac aaggaagaga aatgaggatc tcaggacctt ggtggacact 2950
   gtgtacacct ctggattcat tgtctctcac agatgtgact gtataacttt 3000
   cccaggttct gtttatggcc acatttaata tcttcagctc tttttgtgga 3050
   tataaaatgt gcagatgcaa ttgtttgggt gagtcctaag ccacttgaaa 3100
   tgttagtcat tgttatttat acaagattga aaatcttgtg taaatcctgc 3150
   catttaaaaa gttgtagcag attgtttcct cttccaaagt aaaattgctg 3200
   tgctttatgg atagtaagaa tggccctaga gtgggagtcc tgataaccca 3250
   ggcctgtctg actactttgc cttcttttgt agcatatagg tgatgtttgc 3300
   tcttgttttt attaatttat atgtatattt ttttaattta acatgaacac 3350
   ccttagaaaa tgtgtcctat ctatcttcca aatgcaattt gattgactgc 3400
   ccattcacca aaattatcct gaactcttct gcaaaaatgg atattattag 3450
   aaattagaaa aaaattacta attttacaca ttagatttta ttttactatt 3500
   ggaatctgat atactgtgtg cttgttttat aaaattttgc ttttaattaa 3550
   ataaaagctg gaagcaaagt ataaccatat gatactatca tactactgaa 3600
   acagatttca tacctcagaa tgtaaaagaa cttactgatt attttcttca 3650
   tccaacttat gtttttaaat gaggattatt gatagtactc ttggttttta 3700
   taccattcag atcactgaat ttataaagta cccatctagt acttgaaaaa 3750
   gtaaagtgtt ctgccagatc ttaggtatag aggaccctaa cacagtatat 3800
   cccaagtgca ctttctaatg tttctgggtc ctgaagaatt aagatacaaa 3850
   ttaattttac tccataaaca gactgttaat tataggagcc ttaatttttt 3900
   tttcatagag atttgtctaa ttgcatctca aaattattct gccctcctta 3950
   atttgggaag gtttgtgttt tctctggaat ggtacatgtc ttccatgtat 4000
   cttttgaact ggcaattgtc tatttatctt ttattttttt aagtcagtat 4050
   ggtctaacac tggcatgttc aaagccacat tatttctagt ccaaaattac 4100
   aagtaatcaa gggtcattat gggttaggca ttaatgtttc tatctgattt 4150
   tgtgcaaaag cttcaaatta aaacagctgc attagaaaaa gaggcgcttc 4200
   tcccctcccc tacacctaaa ggtgtattta aactatcttg tgtgattaac 4250
   ttatttagag atgctgtaac ttaaaatagg ggatatttaa ggtagcttca 4300
   gctagctttt aggaaaatca ctttgtctaa ctcagaatta tttttaaaaa 4350
   gaaatctggt cttgttagaa aacaaaattt tattttgtgc tcatttaagt 4400
   ttcaaactta ctattttgac agttattttg ataacaatga cactagaaaa 4450
   cttgactcca tttcatcatt gtttctgcat gaatatcata caaatcagtt 4500
   agtttttagg tcaagggctt actatttctg ggtcttttgc tactaagttc 4550
   acattagaat tagtgccaga attttaggaa cttcagagat cgtgtattga 4600
   gatttcttaa ataatgcttc agatattatt gctttattgc ttttttgtat 4650
   tggttaaaac tgtacattta aaattgctat gttactattt tctacaatta 4700
   atagtttgtc tattttaaaa taaattagtt gttaagagtc 4740
<210> 34
   <211> 4161
   <212> DNA
   <213> Homo sapiens
<400> 34
   ccgaagcagg gcgcgcagca gcgctgagtg ccccggaacg tgcgtcgcgc 50
   ccccagtgtc cgtcgcgtcc gccgcgcccc gggcggggat ggggcggcca 100
   gactgagcgc cgcacccgcc atccagaccc gccggcccta gccgcagtcc 150
   ctccagccgt ggccccagcg cgcacgggcg atggcgaagg cgacgtccgg 200
   t gccgcgggg ct gcgt ct gc t gt t gct gct gct gct gccg ct gct aggca 250
   aagt ggcat t gggcct ct ac t t ct cgaggg at gct t act g ggagaagct g 300
   tatgtggacc aggcggccgg cacgcccttg ctgtacgtcc atgccctgcg 350
   ggacgcccct gaggaggtgc ccagcttccg cctgggccag catctctacg 400
   gcacgt accg cacacggct g cat gagaaca act ggat ct g cat ccaggag 450
   gacaccggcc tcctctacct taaccggagc ctggaccata gctcctggga 500
   gaagct cagt gt ccgcaacc gcggct t t cc cct gct cacc gt ct acct ca 550
   aggt ct t cct gt cacccaca t ccct t cgt g agggcgagt g ccagt ggcca 600
   ggct gt gccc gcgt at act t ct cct t ct t c aacacct cct t t ccagcct g 650
   cagctccctc aagccccggg agctctgctt cccagagaca aggccctcct 700
   tccgcattcg ggagaaccga cccccaggca ccttccacca gttccgcctg 750
   ct gcct gt gc agt t ct t gt g ccccaacat c agcgt ggcct acaggct cct 800
   ggagggtgag ggtctgccct tccgctgcgc cccggacagc ctggaggtga 850
   gcacgcgctg ggccctggac cgcgagcagc gggagaagta cgagctggtg 900
   gccgtgtgca ccgtgcacgc cggcgcgcgc gaggaggtgg tgatggtgcc 950
   cttcccggtg accgtgtacg acgaggacga ctcggcgccc accttccccg 1000
   cgggcgtcga caccgccagc gccgtggtgg agttcaagcg gaaggaggac 1050
   accgtggtgg ccacgctgcg tgtcttcgat gcagacgtgg tacctgcatc 1100
   aggggagctg gtgaggcggt acacaagcac gctgctcccc ggggacacct 1150
   gggcccagca gaccttccgg gtggaacact ggcccaacga gacctcggtc 1200
   caggccaacg gcagcttcgt gcgggcgacc gtacatgact ataggctggt 1250
   tctcaaccgg aacctctcca tctcggagaa ccgcaccatg cagctggcgg 1300
   tgctggtcaa tgactcagac ttccagggcc caggagcggg cgtcctcttg 1350
   ctccacttca acgtgtcggt gctgccggtc agcctgcacc tgcccagtac 1400
   ctactccctc tccgtgagca ggagggctcg ccgatttgcc cagatcggga 1450
   aagtctgtgt ggaaaactgc caggcattca gtggcatcaa cgtccagtac 1500
   aagctgcatt cctctggtgc caactgcagc acgctagggg tggtcacctc 1550
   agccgaggac acctcgggga tcctgtttgt gaatgacacc aaggccctgc 1600
   ggcggcccaa gtgtgccgaa cttcactaca tggtggtggc caccgaccag 1650
   cagacctcta ggcaggccca ggcccagctg cttgtaacag tggaggggtc 1700
   atatgtggcc gaggaggcgg gctgccccct gtcctgtgca gtcagcaaga 1750
   gacggctgga gtgtgaggag tgtggcggcc tgggctcccc aacaggcagg 1800
   tgtgagtgga ggcaaggaga tggcaaaggg atcaccagga acttctccac 1850
   ctgctctccc agcaccaaga cctgccccga cggccactgc gatgttgtgg 1900
   agacccaaga catcaacatt tgccctcagg actgcctccg gggcagcatt 1950
   gttgggggac acgagcctgg ggagccccgg gggattaaag ctggctatgg 2000
   cacctgcaac tgcttccctg aggaggagaa gtgcttctgc gagcccgaag 2050
   acatccagga tccactgtgc gacgagctgt gccgcacggt gatcgcagcc 2100
   gctgtcctct tctccttcat cgtctcggtg ctgctgtctg ccttctgcat 2150
   ccactgctac cacaagtttg cccacaagcc acccatctcc tcagctgaga 2200
   tgaccttccg gaggcccgcc caggccttcc cggtcagcta ctcctcttcc 2250
   ggtgcccgcc ggccctcgct ggactccatg gagaaccagg tctccgtgga 2300
   tgccttcaag atcctggagg atccaaagtg ggaattccct cggaagaact 2350
   tggttcttgg aaaaactcta ggagaaggcg aatttggaaa agtggtcaag 2400
   gcaacggcct tccatctgaa aggcagagca gggtacacca cggtggccgt 2450
   gaagatgctg aaagagaacg cctccccgag tgagctgcga gacctgctgt 2500
   cagagttcaa cgtcctgaag caggtcaacc acccacatgt catcaaattg 2550
   tatggggcct gcagccagga tggcccgctc ctcctcatcg tggagtacgc 2600
   caaatacggc tccctgcggg gcttcctccg cgagagccgc aaagtggggc 2650
   ctggctacct gggcagtgga ggcagccgca actccagctc cctggaccac 2700
   ccggatgagc gggccctcac catgggcgac ctcatctcat ttgcctggca 2750
   gatctcacag gggatgcagt atctggccga gatgaagctc gttcatcggg 2800
   acttggcagc cagaaacatc ctggtagctg aggggcggaa gatgaagatt 2850
   tcggatttcg gcttgtcccg agatgtttat gaagaggatt cctacgtgaa 2900
   gaggagccag ggtcggattc cagttaaatg gatggcaatt gaatcccttt 2950
   ttgatcatat ctacaccacg caaagtgatg tatggtcttt tggtgtcctg 3000
   ctgtgggaga tcgtgaccct agggggaaac ccctatcctg ggattcctcc 3050
   tgagcggctc ttcaaccttc tgaagaccgg ccaccggatg gagaggccag 3100
   acaactgcag cgaggagatg taccgcctga tgctgcaatg ctggaagcag 3150
   gagccggaca aaaggccggt gtttgcggac atcagcaaag acctggagaa 3200
   gatgatggtt aagaggagag actacttgga ccttgcggcg tccactccat 3250
   ctgactccct gatttatgac gacggcctct cagaggagga gacaccgctg 3300
   gtggactgta ataatgcccc cctccctcga gccctccctt ccacatggat 3350
   tgaaaacaaa ctctatggta gaatttccca tgcatttact agattctagc 3400
   accgctgtcc cctctgcact atccttcctc tctgtgatgc tttttaaaaa 3450
   tgtttctggt ctgaacaaaa ccaaagtctg ctctgaacct ttttatttgt 3500
   aaatgtctga ctttgcatcc agtttacatt taggcattat tgcaactatg 3550
   tttttctaaa ggaagtgaaa aataagtgta attaccacat tgcccagcaa 3600
   cttaggatgg tagaggaaaa aacagatcag ggcggaactc tcaggggaga 3650
   ccaagaacag gttgaataag gcgcttctgg ggtgggaatc aagtcatagt 3700
   acttctactt taactaagtg gataaatata caaatctggg gaggtattca 3750
   gttgagaaag gagccaccag caccactcag cctgcactgg gagcacagcc 3800
   aggttccccc agacccctcc tgggcaggca ggtgcctctc agaggccacc 3850
   cggcactggc gagcagccac tggccaagcc tcagccccag tcccagccac 3900
   atgtcctcca tcaggggtag cgaggttgca ggagctggct ggccctggga 3950
   ggacgcaccc ccactgctgt tttcacatcc tttcccttac ccaccttcag 4000
   gacggttgtc acttatgaag tcagtgctaa agctggagca gttgcttttt 4050
   gaaagaacat ggtctgtggt gctgtggtct tacaatggac agtaaatatg 4100
   gttcttgcca aaactccttc ttttgtcttt gattaaatac tagaaattta 4150
   aaaaaaaaaa a 4161
<210> 35
   <211> 2346
   <212> DNA
   <213> Homo sapiens
<400> 35
   acaaggcagc ctcgctcgag cgcaggccaa tcggctttct agctagaggg 50
   t t t aact cct at t t aaaaag aagaacct t t gaat t ct aac ggct gagct c 100
   ttggaagact tgggtccttg ggtcgcaggt gggagccgac gggtgggtag 150
   accgtggggg atatctcagt ggcggacgag gacggcgggg acaaggggcg 200
   gct ggt cgga gt ggcggagc gt caagt ccc ct gt cggt t c ct ccgt ccct 250
   gagt gt cct t ggcgct gcct t gt gcccgcc cagcgcct t t gcat ccgct c 300
   ct gggcaccg aggcgccct g t aggat act g ct t gt t act t at t acagct a 350
   gaggcatcat ggaccgatct aaagaaaact gcatttcagg acctgttaag 400
   gct acagct c cagt t ggagg t ccaaaacgt gt t ct cgt ga ct cagcaat t 450
   t cct t gt cag aat ccat t ac ct gt aaat ag t ggccaggct cagcgggt ct 500
   t gt gt cct t c aaat t ct t cc cagcgcat t c ct t t gcaagc acaaaagct t 550
   gtctccagtc acaagccggt tcagaatcag aagcagaagc aattgcaggc 600
   aaccagtgta cctcatcctg tctccaggcc actgaataac acccaaaaga 650
   gcaagcagcc cctgccatcg gcacctgaaa ataatcctga ggaggaactg 700
   gcatcaaaac agaaaaatga agaatcaaaa aagaggcagt gggctttgga 750
   agact t t gaa at t ggt cgcc ct ct gggt aa aggaaagt t t ggt aat gt t t 800
   atttggcaag agaaaagcaa agcaagttta ttctggctct taaagtgtta 850
   t t t aaagct c agct ggagaa agccggagt g gagcat cagc t cagaagaga 900
   agt agaaat a cagt cccacc t t cggcat cc t aat at t ct t agact gt at g 950
   gt t at t t cca t gat gct acc agagt ct acc t aat t ct gga at at gcacca 1000
   ct t ggaacag t t t at agaga act t cagaaa ct t t caaagt t t gat gagca 1050
   gagaact gct act t at at aa cagaat t ggc aaat gccct g t ct t act gt c 1100
   attcgaagag agttattcat agagacatta agccagagaa cttacttctt 1150
   ggat cagct g gagagct t aa aat t gcagat t t t gggt ggt cagt acat gc 1200
   tccatcttcc aggaggacca ctctctgtgg caccctggac tacctgcccc 1250
   ct gaaat gat t gaaggt cgg at gcat gat g agaaggt gga t ct ct ggagc 1300
   ct t ggagt t c t t t gct at ga at t t t t agt t gggaagcct c ct t t t gaggc 1350
   aaacacatac caagagacct acaaaagaat atcacgggtt gaattcacat 1400
   tccctgactt tgtaacagag ggagccaggg acctcatttc aagactgttg 1450
   aagcataatc ccagccagag gccaatgctc agagaagtac ttgaacaccc 1500
   ctggatcaca gcaaattcat caaaaccatc aaattgccaa aacaaagaat 1550
   cagctagcaa acagtcttag gaatcgtgca gggggagaaa tccttgagcc 1600
   agggct gcca t at aacct ga caggaacat g ct act gaagt t t at t t t acc 1650
   attgactgct gccctcaatc tagaacgcta cacaagaaat atttgtttta 1700
   ctcagcaggt gtgccttaac ctccctattc agaaagctcc acatcaataa 1750
   acatgacact ctgaagtgaa agtagccacg agaattgtgc tacttatact 1800
   ggttcataat ctggaggcaa ggttcgactg cagccgcccc gtcagcctgt 1850
   gctaggcatg gtgtcttcac aggaggcaaa tccagagcct ggctgtgggg 1900
   aaagtgacca ctctgccctg accccgatca gttaaggagc tgtgcaataa 1950
   ccttcctagt acctgagtga gtgtgtaact tattgggttg gcgaagcctg 2000
   gtaaagctgt tggaatgagt atgtgattct ttttaagtat gaaaataaag 2050
   atatatgtac agacttgtat tttttctctg gtggcattcc tttaggaatg 2100
   ctgtgtgtct gtccggcacc ccggtaggcc tgattgggtt tctagtcctc 2150
   cttaaccact tatctcccat atgagagtgt gaaaaatagg aacacgtgct 2200
   ctacctccat ttagggattt gcttgggata cagaagaggc catgtgtctc 2250
   agagctgtta agggcttatt tttttaaaac attggagtca tagcatgtgt 2300
   gtaaacttta aatatgcaaa taaataagta tctatgtcta aaaaaa 2346
<210> 36
   <211> 2090
   <212> DNA
   <213> Homo sapiens
<400> 36
   gttggcgagg agtttcctgt ttcccccgca gcgctgagtt gaagttgagt 50
   gagtcactcg cgcgcacgga gcgacgacac ccccgcgcgt gcacccgctc 100
   gggacaggag ccggactcct gtgcagcttc cctcggccgc cgggggcctc 150
   cccgcgcctc gccggcctcc aggcccctcc tggctggcga gcgggcgcca 200
   catctggccc gcacatctgc gctgccggcc cggcgcgggg tccggagagg 250
   gcgcggcgcg gagcgcagcc aggggtccgg gaaggcgccg tccgtgcgct 300
   gggggctcgg tctatgacga gcagcggggt ctgccatggg tcgggggctg 350
   ctcaggggcc tgtggccgct gcacatcgtc ctgtggacgc gtatcgccag 400
   cacgatccca ccgcacgttc agaagtcggt taataacgac atgatagtca 450
   ctgacaacaa cggtgcagtc aagtttccac aactgtgtaa attttgtgat 500
   gtgagatttt ccacctgtga caaccagaaa tcctgcatga gcaactgcag 550
   catcacctcc atctgtgaga agccacagga agtctgtgtg gctgtatgga 600
   gaaagaatga cgagaacata acactagaga cagtttgcca tgaccccaag 650
   ctcccctacc atgactttat tctggaagat gctgcttctc caaagtgcat 700
   tatgaaggaa aaaaaaaagc ctggtgagac tttcttcatg tgttcctgta 750
   gctctgatga gtgcaatgac aacatcatct tctcagaaga atataacacc 800
   agcaatcctg acttgttgct agtcatattt caagtgacag gcatcagcct 850
   cctgccacca ctgggagttg ccatatctgt catcatcatc ttctactgct 900
   accgcgttaa ccggcagcag aagctgagtt caacctggga aaccggcaag 950
   acgcggaagc tcatggagtt cagcgagcac tgtgccatca tcctggaaga 1000
   tgaccgctct gacatcagct ccacgtgtgc caacaacatc aaccacaaca 1050
   cagagct gct gcccat t gag ct ggacaccc t ggt ggggaa aggt cgct t t 1100
   gct gaggt ct at aaggccaa gct gaagcag aacact t cag agcagt t t ga 1150
   gacagt ggca gt caagat ct t t ccct at ga ggagt at gcc t ct t ggaaga 1200
   cagagaagga catcttctca gacatcaatc tgaagcatga gaacatactc 1250
   cagttcctga cggctgagga gcggaagacg gagttgggga aacaatactg 1300
   gctgatcacc gccttccacg ccaagggcaa cctacaggag tacctgacgc 1350
   ggcat gt cat cagct gggag gacct gcgca agct gggcag ct ccct cgcc 1400
   cgggggattg ctcacctcca cagtgatcac actccatgtg ggaggcccaa 1450
   gat gcccat c gt gcacaggg acct caagag ct ccaat at c ct cgt gaaga 1500
   acgacct aac ct gct gcct g t gt gact t t g ggct t t ccct gcgt ct ggac 1550
   cct act ct gt ct gt ggat ga cct ggct aac agt gggcagg t gggaact gc 1600
   aagat acat g gct ccagaag t cct agaat c caggat gaat t t ggagaat g 1650
   ctgagtcctt caagcagacc gatgtctact ccatggctct ggtgctctgg 1700
   gaaat gacat ct cgct gt aa t gcagt ggga gaagt aaaag at t at gagcc 1750
   tccatttggt tccaaggtgc gggagcaccc ctgtgtcgaa agcatgaagg 1800
   acaacgtgtt gagagatcga gggcgaccag aaattcccag cttctggctc 1850
   aaccaccagg gcatccagat ggtgtgtgag acgttgactg agtgctggga 1900
   ccacgaccca gaggcccgtc tcacagccca gtgtgtggca gaacgcttca 1950
   gt gagct gga gcat ct ggac aggct ct cgg ggaggagct g ct cggaggag 2000
   aagat t cct g aagacggct c cct aaacact accaaat agc t ct t at gggg 2050
   caggct gggc at gt ccaaag aggct gcccc t ct caccaaa 2090
<210> 37
   <211> 2629
   <212> DNA
   <213> Homo sapiens
<400> 37
   act t gt cat g gcgact gt cc agct t t gt gc caggagcct c gcaggggt t g 50
   at gggat t gg ggt t t t cccc t cccat gt gc t caagact gg cgct aaaagt 100
   tttgagcttc tcaaaagtct agagccaccg tccagggagc aggtagctgc 150
   t gggct ccgg ggacact t t g cgt t cgggct gggagcgt gc t t t ccacgac 200
   ggt gacacgc t t ccct ggat t ggcagccag act gcct t cc gggt cact gc 250
   catggaggag ccgcagtcag atcctagcgt cgagccccct ctgagtcagg 300
   aaacat t t t c agacct at gg aaact act t c ct gaaaacaa cgt t ct gt cc 350
   cccttgccgt cccaagcaat ggatgatttg atgctgtccc cggacgatat 400
   tgaacaatgg ttcactgaag acccaggtcc agatgaagct cccagaatgc 450
   cagaggctgc tccccgcgtg gcccctgcac cagcagctcc tacaccggcg 500
   gcccctgcac cagccccctc ctggcccctg tcatcttctg tcccttccca 550
   gaaaacctac cagggcagct acggtttccg tctgggcttc ttgcattctg 600
   ggacagccaa gtctgtgact tgcacgtact cccctgccct caacaagatg 650
   ttttgccaac tggccaagac ctgccctgtg cagctgtggg ttgattccac 700
   acccccgccc ggcacccgcg tccgcgccat ggccatctac aagcagtcac 750
   agcacatgac ggaggttgtg aggcgctgcc cccaccatga gcgctgctca 800
   gatagcgatg gtctggcccc tcctcagcat cttatccgag tggaaggaaa 850
   tttgcgtgtg gagtatttgg atgacagaaa cacttttcga catagtgtgg 900
   tggtgcccta tgagccgcct gaggttggct ctgactgtac caccatccac 950
   tacaactaca tgtgtaacag ttcctgcatg ggcggcatga accggaggcc 1000
   catcctcacc atcatcacac tggaagactc cagtggtaat ctactgggac 1050
   ggaacagctt tgaggtgcgt gtttgtgcct gtcctgggag agaccggcgc 1100
   acagaggaag agaatctccg caagaaaggg gagcctcacc acgagctgcc 1150
   cccagggagc actaagcgag cactgcccaa caacaccagc tcctctcccc 1200
   agccaaagaa gaaaccactg gatggagaat atttcaccct tcagatccgt 1250
   gggcgtgagc gcttcgagat gttccgagag ctgaatgagg ccttggaact 1300
   caaggatgcc caggctggga aggagccagg ggggagcagg gctcactcca 1350
   gccacctgaa gtccaaaaag ggtcagtcta cctcccgcca taaaaaactc 1400
   atgttcaaga cagaagggcc tgactcagac tgacattctc cacttcttgt 1450
   tccccactga cagcctccca cccccatctc tccctcccct gccattttgg 1500
   gttttgggtc tttgaaccct tgcttgcaat aggtgtgcgt cagaagcacc 1550
   caggacttcc atttgctttg tcccggggct ccactgaaca agttggcctg 1600
   cactggtgtt ttgttgtggg gaggaggatg gggagtagga cataccagct 1650
   tagattttaa ggtttttact gtgagggatg tttgggagat gtaagaaatg 1700
   ttcttgcagt taagggttag tttacaatca gccacattct aggtaggtag 1750
   gggcccactt caccgtacta accagggaag ctgtccctca tgttgaattt 1800
   tctctaactt caaggcccat atctgtgaaa tgctggcatt tgcacctacc 1850
   tcacagagtg cattgtgagg gttaatgaaa taatgtacat ctggccttga 1900
   aaccaccttt tattacatgg ggtctaaaac ttgaccccct tgagggtgcc 1950
   tgttccctct ccctctccct gttggctggt gggttggtag tttctacagt 2000
   tgggcagctg gttaggtaga gggagttgtc aagtcttgct ggcccagcca 2050
   aaccctgtct gacaacctct tggtcgacct tagtacctaa aaggaaatct 2100
   caccccat cc cacaccct gg aggat t t cat ct ct t gt at a t gat gat ct g 2150
   gat ccaccaa gact t gt t t t at gct caggg t caat t t ct t t t t t ct t t t t 2200
   t t t t t t t t t t t t t ct t t t t c t t t gagact g ggt ct cgct t t gt t gcccag 2250
   gct ggagt gg agt ggcgt ga t ct t ggct t a ct gcagcct t t gcct ccccg 2300
   gct cgagcag t cct gcct ca gcct ccggag t agct gggac cacaggt t ca 2350
   tgccaccatg gccagccaac ttttgcatgt tttgtagaga tggggtctca 2400
   cagt gt t gcc caggct ggt c t caaact cct gggct caggc gat ccacct g 2450
   t ct cagcct c ccagagt gct gggat t acaa t t gt gagcca ccacgt ggag 2500
   ct ggaagggt caacat ct t t t acat t ct gc aagcacat ct gcat t t t cac 2550
   cccaccct t c ccct cct t ct ccct t t t t at at cccat t t t t at at cgat c 2600
   t ct t at t t t a caat aaaact t t gct gcca 2629
<210> 38
   <211> 1130
   <212> PRT
   <213> Homo sapiens
<400> 38
   Met Leu Glu Ile Cys Leu Lys Leu Val Gly Cys Lys Ser Lys Lys
   1 5 10 15
   Gly Leu Ser Ser Ser Ser Ser Cys Tyr Leu Glu Glu Ala Leu Gln
   20 25 30
   Arg Pro Val Ala Ser Asp Phe Glu Pro Gln Gly Leu Ser Glu Ala
   35 40 45
   Ala Arg Trp Asn Ser Lys Glu Asn Leu Leu Ala Gly Pro Ser Glu
   50 55 60
   Asn Asp Pro Asn Leu Phe Val Ala Leu Tyr Asp Phe Val Ala Ser
   65 70 75
   Gly Asp Asn Thr Leu Ser Ile Thr Lys Gly Glu Lys Leu Arg Val
   80 85 90
   Leu Gly Tyr Asn His Asn Gly Glu Trp Cys Glu Ala Gln Thr Lys
   95 100 105
   Asn Gly Gln Gly Trp Val Pro Ser Asn Tyr Ile Thr Pro Val Asn
   110 115 120
   Ser Leu Glu Lys His Ser Trp Tyr His Gly Pro Val Ser Arg Asn
   125 130 135
   Ala Ala Glu Tyr Leu Leu Ser Ser Gly Ile Asn Gly Ser Phe Leu
   140 145 150
   Val Arg Glu Ser Glu Ser Ser Pro Gly Gln Arg Ser Ile Ser Leu
   155 160 165
   Arg Tyr Glu Gly Arg Val Tyr His Tyr Arg Ile Asn Thr Ala Ser
   170 175 180
   Asp Gly Lys Leu Tyr Val Ser Ser Glu Ser Arg Phe Asn Thr Leu
   185 190 195
   Ala Glu Leu Val His His His Ser Thr Val Ala Asp Gly Leu Ile
   200 205 210
   Thr Thr Leu His Tyr Pro Ala Pro Lys Arg Asn Lys Pro Thr Val
   215 220 225
   Tyr Gly Val Ser Pro Asn Tyr Asp Lys Trp Glu Met Glu Arg Thr
   230 235 240
   Asp Ile Thr Met Lys His Lys Leu Gly Gly Gly Gln Tyr Gly Glu
   245 250 255
   Val Tyr Glu Gly Val Trp Lys Lys Tyr Ser Leu Thr Val Ala Val
   260 265 270
   Lys Thr Leu Lys Glu Asp Thr Met Glu Val Glu Glu Phe Leu Lys
   275 280 285
   Glu Ala Ala Val Met Lys Glu Ile Lys His Pro Asn Leu Val Gln
   290 295 300
   Leu Leu Gly Val Cys Thr Arg Glu Pro Pro Phe Tyr Ile Ile Thr
   305 310 315
   Glu Phe Met Thr Tyr Gly Asn Leu Leu Asp Tyr Leu Arg Glu Cys
   320 325 330
   Asn Arg Gln Glu Val Asn Ala Val Val Leu Leu Tyr Met Ala Thr
   335 340 345
   Gln Ile Ser Ser Ala Met Glu Tyr Leu Glu Lys Lys Asn Phe Ile
   350 355 360
   His Arg Asp Leu Ala Ala Arg Asn Cys Leu Val Gly Glu Asn His
   365 370 375
   Leu Val Lys Val Ala Asp Phe Gly Leu Ser Arg Leu Met Thr Gly
   380 385 390
   Asp Thr Tyr Thr Ala His Ala Gly Ala Lys Phe Pro Ile Lys Trp
   395 400 405
   Thr Ala Pro Glu Ser Leu Ala Tyr Asn Lys Phe Ser Ile Lys Ser
   410 415 420
   Asp Val Trp Ala Phe Gly Val Leu Leu Trp Glu Ile Ala Thr Tyr
   425 430 435
   Gly Met Ser Pro Tyr Pro Gly Ile Asp Leu Ser Gln Val Tyr Glu
   440 445 450
   Leu Leu Glu Lys Asp Tyr Arg Met Glu Arg Pro Glu Gly Cys Pro
   455 460 465
   Glu Lys Val Tyr Glu Leu Met Arg Ala Cys Trp Gln Trp Asn Pro
   470 475 480
   Ser Asp Arg Pro Ser Phe Ala Glu Ile His Gln Ala Phe Glu Thr
   485 490 495
   Met Phe Gln Glu Ser Ser Ile Ser Asp Glu Val Glu Lys Glu Leu
   500 505 510
   Gly Lys Gln Gly Val Arg Gly Ala Val Ser Thr Leu Leu Gln Ala
   515 520 525
   Pro Glu Leu Pro Thr Lys Thr Arg Thr Ser Arg Arg Ala Ala Glu
   530 535 540
   His Arg Asp Thr Thr Asp Val Pro Glu Met Pro His Ser Lys Gly
   545 550 555
   Gln Gly Glu Ser Asp Pro Leu Asp His Glu Pro Ala Val Ser Pro
   560 565 570
   Leu Leu Pro Arg Lys Glu Arg Gly Pro Pro Glu Gly Gly Leu Asn
   575 580 585
   Glu Asp Glu Arg Leu Leu Pro Lys Asp Lys Lys Thr Asn Leu Phe
   590 595 600
   Ser Ala Leu Ile Lys Lys Lys Lys Lys Thr Ala Pro Thr Pro Pro
   605 610 615
   Lys Arg Ser Ser Ser Phe Arg Glu Met Asp Gly Gln Pro Glu Arg
   620 625 630
   Arg Gly Ala Gly Glu Glu Glu Gly Arg Asp Ile Ser Asn Gly Ala
   635 640 645
   Leu Ala Phe Thr Pro Leu Asp Thr Ala Asp Pro Ala Lys Ser Pro
   650 655 660
   Lys Pro Ser Asn Gly Ala Gly Val Pro Asn Gly Ala Leu Arg Glu
   665 670 675
   Ser Gly Gly Ser Gly Phe Arg Ser Pro His Leu Trp Lys Lys Ser
   680 685 690
   Ser Thr Leu Thr Ser Ser Arg Leu Ala Thr Gly Glu Glu Glu Gly
   695 700 705
   Gly Gly Ser Ser Ser Lys Arg Phe Leu Arg Ser Cys Ser Ala Ser
   710 715 720
   Cys Val Pro His Gly Ala Lys Asp Thr Glu Trp Arg Ser Val Thr
   725 730 735
   Leu Pro Arg Asp Leu Gln Ser Thr Gly Arg Gln Phe Asp Ser Ser
   740 745 750
   Thr Phe Gly Gly His Lys Ser Glu Lys Pro Ala Leu Pro Arg Lys
   755 760 765
   Arg Ala Gly Glu Asn Arg Ser Asp Gln Val Thr Arg Gly Thr Val
   770 775 780
   Thr Pro Pro Pro Arg Leu Val Lys Lys Asn Glu Glu Ala Ala Asp
   785 790 795
   Glu Val Phe Lys Asp Ile Met Glu Ser Ser Pro Gly Ser Ser Pro
   800 805 810
   Pro Asn Leu Thr Pro Lys Pro Leu Arg Arg Gln Val Thr Val Ala
   815 820 825
   Pro Ala Ser Gly Leu Pro His Lys Glu Glu Ala Gly Lys Gly Ser
   830 835 840
   Ala Leu Gly Thr Pro Ala Ala Ala Glu Pro Val Thr Pro Thr Ser
   845 850 855
   Lys Ala Gly Ser Gly Ala Pro Gly Gly Thr Ser Lys Gly Pro Ala
   860 865 870
   Glu Glu Ser Arg Val Arg Arg His Lys His Ser Ser Glu Ser Pro
   875 880 885
   Gly Arg Asp Lys Gly Lys Leu Ser Arg Leu Lys Pro Ala Pro Pro
   890 895 900
   Pro Pro Pro Ala Ala Ser Ala Gly Lys Ala Gly Gly Lys Pro Ser
   905 910 915
   Gln Ser Pro Ser Gln Glu Ala Ala Gly Glu Ala Val Leu Gly Ala
   920 925 930
   Lys Thr Lys Ala Thr Ser Leu Val Asp Ala Val Asn Ser Asp Ala
   935 940 945
   Ala Lys Pro Ser Gln Pro Gly Glu Gly Leu Lys Lys Pro Val Leu
   950 955 960
   Pro Ala Thr Pro Lys Pro Gln Ser Ala Lys Pro Ser Gly Thr Pro
   965 970 975
   Ile Ser Pro Ala Pro Val Pro Ser Thr Leu Pro Ser Ala Ser Ser
   980 985 990
   Ala Leu Ala Gly Asp Gln Pro Ser Ser Thr Ala Phe Ile Pro Leu
   995 1000 1005
   Ile Ser Thr Arg Val Ser Leu Arg Lys Thr Arg Gln Pro Pro Glu
   1010 1015 1020
   Arg Ile Ala Ser Gly Ala Ile Thr Lys Gly Val Val Leu Asp Ser
   1025 1030 1035
   Thr Glu Ala Leu Cys Leu Ala Ile Ser Arg Asn Ser Glu Gln Met
   1040 1045 1050
   Ala Ser His Ser Ala Val Leu Glu Ala Gly Lys Asn Leu Tyr Thr
   1055 1060 1065
   Phe Cys Val Ser Tyr Val Asp Ser Ile Gln Gln Met Arg Asn Lys
   1070 1075 1080
   Phe Ala Phe Arg Glu Ala Ile Asn Lys Leu Glu Asn Asn Leu Arg
   1085 1090 1095
   Glu Leu Gln Ile Cys Pro Ala Thr Ala Gly Ser Gly Pro Ala Ala
   1100 1105 1110
   Thr Gln Asp Phe Ser Lys Leu Leu Ser Ser Val Lys Glu Ile Ser
   1115 1120 1125
   Asp Ile Val Gln Arg
   1130
<210> 39
   <211> 2843
   <212> PRT
   <213> Homo sapiens
<400> 39
   Met Ala Ala Ala Ser Tyr Asp Gln Leu Leu Lys Gln Val Glu Ala
   1 5 10 15
   Leu Lys Met Glu Asn Ser Asn Leu Arg Gln Glu Leu Glu Asp Asn
   20 25 30
   Ser Asn His Leu Thr Lys Leu Glu Thr Glu Ala Ser Asn Met Lys
   35 40 45
   Glu Val Leu Lys Gln Leu Gln Gly Ser Ile Glu Asp Glu Ala Met
   50 55 60
   Ala Ser Ser Gly Gln Ile Asp Leu Leu Glu Arg Leu Lys Glu Leu
   65 70 75
   Asn Leu Asp Ser Ser Asn Phe Pro Gly Val Lys Leu Arg Ser Lys
                   80                  85                  90
   Met Ser Leu Arg Ser Tyr Gly Ser Arg Glu Gly Ser Val Ser Ser
                   95                  100                 105
   Arg Ser Gly Glu Cys Ser Pro Val Pro Met Gly Ser Phe Pro Arg
                   110                 115                 120
   Arg Gly Phe Val Asn Gly Ser Arg Glu Ser Thr Gly Tyr Leu Glu
                   125                 130                 135
   Glu Leu Glu Lys Glu Arg Ser Leu Leu Leu Ala Asp Leu Asp Lys
                   140                 145                 150
   Glu Glu Lys Glu Lys Asp Trp Tyr Tyr Ala Gln Leu Gln Asn Leu
                   155                 160                 165
   Thr Lys Arg Ile Asp Ser Leu Pro Leu Thr Glu Asn Phe Ser Leu
                   170                 175                 180
   Gln Thr Asp Met Thr Arg Arg Gln Leu Glu Tyr Glu Ala Arg Gln
                   185                 190                 195
   Ile Arg Val Ala Met Glu Glu Gln Leu Gly Thr Cys Gln Asp Met
                   200                 205                 210
   Glu Lys Arg Ala Gln Arg Arg Ile Ala Arg Ile Gln Gln Ile Glu
                   215                 220                 225
   Lys Asp Ile Leu Arg Ile Arg Gln Leu Leu Gln Ser Gln Ala Thr
                   230                 235                 240
   Glu Ala Glu Arg Ser Ser Gln Asn Lys His Glu Thr Gly Ser His
                   245                 250                 255
   Asp Ala Glu Arg Gln Asn Glu Gly Gln Gly Val Gly Glu Ile Asn
                   260                 265                 270
   Met Ala Thr Ser Gly Asn Gly Gln Gly Ser Thr Thr Arg Met Asp
                   275                 280                 285
   His Glu Thr Ala Ser Val Leu Ser Ser Ser Ser Thr His Ser Ala
                   290                 295                 300
   Pro Arg Arg Leu Thr Ser His Leu Gly Thr Lys Val Glu Met Val
                   305                 310                 315
   Tyr Ser Leu Leu Ser Met Leu Gly Thr His Asp Lys Asp Asp Met
                   320                 325                 330
   Ser Arg Thr Leu Leu Ala Met Ser Ser Ser Gln Asp Ser Cys Ile
                   335                 340                 345
   Ser Met Arg Gln Ser Gly Cys Leu Pro Leu Leu Ile Gln Leu Leu
                   350                 355                 360
   His Gly Asn Asp Lys Asp Ser Val Leu Leu Gly Asn Ser Arg Gly
                   365                 370                 375
   Ser Lys Glu Ala Arg Ala Arg Ala Ser Ala Ala Leu His Asn Ile
                   380                 385                 390
   Ile His Ser Gln Pro Asp Asp Lys Arg Gly Arg Arg Glu Ile Arg
                   395                 400                 405
   Val Leu His Leu Leu Glu Gln Ile Arg Ala Tyr Cys Glu Thr Cys
                   410                 415                 420
   Trp Glu Trp Gln Glu Ala His Glu Pro Gly Met Asp Gln Asp Lys
   425 430 435
   Asn Pro Met Pro Ala Pro Val Glu His Gln Ile Cys Pro Ala Val
   440 445 450
   Cys Val Leu Met Lys Leu Ser Phe Asp Glu Glu His Arg His Ala
   455 460 465
   Met Asn Glu Leu Gly Gly Leu Gln Ala Ile Ala Glu Leu Leu Gln
   470 475 480
   Val Asp Cys Glu Met Tyr Gly Leu Thr Asn Asp His Tyr Ser Ile
   485 490 495
   Thr Leu Arg Arg Tyr Ala Gly Met Ala Leu Thr Asn Leu Thr Phe
   500 505 510
   Gly Asp Val Ala Asn Lys Ala Thr Leu Cys Ser Met Lys Gly Cys
   515 520 525
   Met Arg Ala Leu Val Ala Gln Leu Lys Ser Glu Ser Glu Asp Leu
   530 535 540
   Gln Gln Val Ile Ala Ser Val Leu Arg Asn Leu Ser Trp Arg Ala
   545 550 555
   Asp Val Asn Ser Lys Lys Thr Leu Arg Glu Val Gly Ser Val Lys
   560 565 570
   Ala Leu Met Glu Cys Ala Leu Glu Val Lys Lys Glu Ser Thr Leu
   575 580 585
   Lys Ser Val Leu Ser Ala Leu Trp Asn Leu Ser Ala His Cys Thr
   590 595 600
   Glu Asn Lys Ala Asp Ile Cys Ala Val Asp Gly Ala Leu Ala Phe
   605 610 615
   Leu Val Gly Thr Leu Thr Tyr Arg Ser Gln Thr Asn Thr Leu Ala
   620 625 630
   Ile Ile Glu Ser Gly Gly Gly Ile Leu Arg Asn Val Ser Ser Leu
   635 640 645
   Ile Ala Thr Asn Glu Asp His Arg Gln Ile Leu Arg Glu Asn Asn
   650 655 660
   Cys Leu Gln Thr Leu Leu Gln His Leu Lys Ser His Ser Leu Thr
   665 670 675
   Ile Val Ser Asn Ala Cys Gly Thr Leu Trp Asn Leu Ser Ala Arg
   680 685 690
   Asn Pro Lys Asp Gln Glu Ala Leu Trp Asp Met Gly Ala Val Ser
   695 700 705
   Met Leu Lys Asn Leu Ile His Ser Lys His Lys Met Ile Ala Met
   710 715 720
   Gly Ser Ala Ala Ala Leu Arg Asn Leu Met Ala Asn Arg Pro Ala
   725 730 735
   Lys Tyr Lys Asp Ala Asn Ile Met Ser Pro Gly Ser Ser Leu Pro
   740 745 750
   Ser Leu His Val Arg Lys Gln Lys Ala Leu Glu Ala Glu Leu Asp
   755 760 765
   Ala Gln His Leu Ser Glu Thr Phe Asp Asn Ile Asp Asn Leu Ser
   770 775 780
   Pro Lys Ala Ser His Arg Ser Lys Gln Arg His Lys Gln Ser Leu
   785 790 795
   Tyr Gly Asp Tyr Val Phe Asp Thr Asn Arg His Asp Asp Asn Arg
   800 805 810
   Ser Asp Asn Phe Asn Thr Gly Asn Met Thr Val Leu Ser Pro Tyr
   815 820 825
   Leu Asn Thr Thr Val Leu Pro Ser Ser Ser Ser Ser Arg Gly Ser
   830 835 840
   Leu Asp Ser Ser Arg Ser Glu Lys Asp Arg Ser Leu Glu Arg Glu
   845 850 855
   Arg Gly Ile Gly Leu Gly Asn Tyr His Pro Ala Thr Glu Asn Pro
   860 865 870
   Gly Thr Ser Ser Lys Arg Gly Leu Gln Ile Ser Thr Thr Ala Ala
   875 880 885
   Gln Ile Ala Lys Val Met Glu Glu Val Ser Ala Ile His Thr Ser
   890 895 900
   Gln Glu Asp Arg Ser Ser Gly Ser Thr Thr Glu Leu His Cys Val
   905 910 915
   Thr Asp Glu Arg Asn Ala Leu Arg Arg Ser Ser Ala Ala His Thr
   920 925 930
   His Ser Asn Thr Tyr Asn Phe Thr Lys Ser Glu Asn Ser Asn Arg
   935 940 945
   Thr Cys Ser Met Pro Tyr Ala Lys Leu Glu Tyr Lys Arg Ser Ser
   950 955 960
   Asn Asp Ser Leu Asn Ser Val Ser Ser Ser Asp Gly Tyr Gly Lys
   965 970 975
   Arg Gly Gln Met Lys Pro Ser Ile Glu Ser Tyr Ser Glu Asp Asp
   980 985 990
   Glu Ser Lys Phe Cys Ser Tyr Gly Gln Tyr Pro Ala Asp Leu Ala
   995 1000 1005
   His Lys Ile His Ser Ala Asn His Met Asp Asp Asn Asp Gly Glu
   1010 1015 1020
   Leu Asp Thr Pro Ile Asn Tyr Ser Leu Lys Tyr Ser Asp Glu Gln
   1025 1030 1035
   Leu Asn Ser Gly Arg Gln Ser Pro Ser Gln Asn Glu Arg Trp Ala
   1040 1045 1050
   Arg Pro Lys His Ile Ile Glu Asp Glu Ile Lys Gln Ser Glu Gln
   1055 1060 1065
   Arg Gln Ser Arg Asn Gln Ser Thr Thr Tyr Pro Val Tyr Thr Glu
   1070 1075 1080
   Ser Thr Asp Asp Lys His Leu Lys Phe Gln Pro His Phe Gly Gln
   1085 1090 1095
   Gln Glu Cys Val Ser Pro Tyr Arg Ser Arg Gly Ala Asn Gly Ser
   1100 1105 1110
   Glu Thr Asn Arg Val Gly Ser Asn His Gly Ile Asn Gln Asn Val
   1115 1120 1125
   Ser Gln Ser Leu Cys Gln Glu Asp Asp Tyr Glu Asp Asp Lys Pro
   1130 1135 1140
   Thr Asn Tyr Ser Glu Arg Tyr Ser Glu Glu Glu Gln His Glu Glu
   1145 1150 1155
   Glu Glu Arg Pro Thr Asn Tyr Ser Ile Lys Tyr Asn Glu Glu Lys
   1160 1165 1170
   Arg His Val Asp Gln Pro Ile Asp Tyr Ser Leu Lys Tyr Ala Thr
   1175 1180 1185
   Asp Ile Pro Ser Ser Gln Lys Gln Ser Phe Ser Phe Ser Lys Ser
   1190 1195 1200
   Ser Ser Gly Gln Ser Ser Lys Thr Glu His Met Ser Ser Ser Ser
   1205 1210 1215
   Glu Asn Thr Ser Thr Pro Ser Ser Asn Ala Lys Arg Gln Asn Gln
   1220 1225 1230
   Leu His Pro Ser Ser Ala Gln Ser Arg Ser Gly Gln Pro Gln Lys
   1235 1240 1245
   Ala Ala Thr Cys Lys Val Ser Ser Ile Asn Gln Glu Thr Ile Gln
   1250 1255 1260
   Thr Tyr Cys Val Glu Asp Thr Pro Ile Cys Phe Ser Arg Cys Ser
   1265 1270 1275
   Ser Leu Ser Ser Leu Ser Ser Ala Glu Asp Glu Ile Gly Cys Asn
   1280 1285 1290
   Gln Thr Thr Gln Glu Ala Asp Ser Ala Asn Thr Leu Gln Ile Ala
   1295 1300 1305
   Glu Ile Lys Glu Lys Ile Gly Thr Arg Ser Ala Glu Asp Pro Val
   1310 1315 1320
   Ser Glu Val Pro Ala Val Ser Gln His Pro Arg Thr Lys Ser Ser
   1325 1330 1335
   Arg Leu Gln Gly Ser Ser Leu Ser Ser Glu Ser Ala Arg His Lys
   1340 1345 1350
   Ala Val Glu Phe Ser Ser Gly Ala Lys Ser Pro Ser Lys Ser Gly
   1355 1360 1365
   Ala Gln Thr Pro Lys Ser Pro Pro Glu His Tyr Val Gln Glu Thr
   1370 1375 1380
   Pro Leu Met Phe Ser Arg Cys Thr Ser Val Ser Ser Leu Asp Ser
   1385 1390 1395
   Phe Glu Ser Arg Ser Ile Ala Ser Ser Val Gln Ser Glu Pro Cys
   1400 1405 1410
   Ser Gly Met Val Ser Gly Ile Ile Ser Pro Ser Asp Leu Pro Asp
   1415 1420 1425
   Ser Pro Gly Gln Thr Met Pro Pro Ser Arg Ser Lys Thr Pro Pro
   1430 1435 1440
   Pro Pro Pro Gln Thr Ala Gln Thr Lys Arg Glu Val Pro Lys Asn
   1445 1450 1455
   Lys Ala Pro Thr Ala Glu Lys Arg Glu Ser Gly Pro Lys Gln Ala
   1460 1465 1470
   Ala Val Asn Ala Ala Val Gln Arg Val Gln Val Leu Pro Asp Ala
   1475 1480 1485
   Asp Thr Leu Leu His Phe Ala Thr Glu Ser Thr Pro Asp Gly Phe
   1490 1495 1500
   Ser Cys Ser Ser Ser Leu Ser Ala Leu Ser Leu Asp Glu Pro Phe
   1505 1510 1515
   Ile Gln Lys Asp Val Glu Leu Arg Ile Met Pro Pro Val Gln Glu
   1520 1525 1530
   Asn Asp Asn Gly Asn Glu Thr Glu Ser Glu Gln Pro Lys Glu Ser
   1535 1540 1545
   Asn Glu Asn Gln Glu Lys Glu Ala Glu Lys Thr Ile Asp Ser Glu
   1550 1555 1560
   Lys Asp Leu Leu Asp Asp Ser Asp Asp Asp Asp Ile Glu Ile Leu
   1565 1570 1575
   Glu Glu Cys Ile Ile Ser Ala Met Pro Thr Lys Ser Ser Arg Lys
   1580 1585 1590
   Ala Lys Lys Pro Ala Gln Thr Ala Ser Lys Leu Pro Pro Pro Val
   1595 1600 1605
   Ala Arg Lys Pro Ser Gln Leu Pro Val Tyr Lys Leu Leu Pro Ser
   1610 1615 1620
   Gln Asn Arg Leu Gln Pro Gln Lys His Val Ser Phe Thr Pro Gly
   1625 1630 1635
   Asp Asp Met Pro Arg Val Tyr Cys Val Glu Gly Thr Pro Ile Asn
   1640 1645 1650
   Phe Ser Thr Ala Thr Ser Leu Ser Asp Leu Thr Ile Glu Ser Pro
   1655 1660 1665
   Pro Asn Glu Leu Ala Ala Gly Glu Gly Val Arg Gly Gly Ala Gln
   1670 1675 1680
   Ser Gly Glu Phe Glu Lys Arg Asp Thr Ile Pro Thr Glu Gly Arg
   1685 1690 1695
   Ser Thr Asp Glu Ala Gln Gly Gly Lys Thr Ser Ser Val Thr Ile
   1700 1705 1710
   Pro Glu Leu Asp Asp Asn Lys Ala Glu Glu Gly Asp Ile Leu Ala
   1715 1720 1725
   Glu Cys Ile Asn Ser Ala Met Pro Lys Gly Lys Ser His Lys Pro
   1730 1735 1740
   Phe Arg Val Lys Lys Ile Met Asp Gln Val Gln Gln Ala Ser Ala
   1745 1750 1755
   Ser Ser Ser Ala Pro Asn Lys Asn Gln Leu Asp Gly Lys Lys Lys
   1760 1765 1770
   Lys Pro Thr Ser Pro Val Lys Pro Ile Pro Gln Asn Thr Glu Tyr
   1775 1780 1785
   Arg Thr Arg Val Arg Lys Asn Ala Asp Ser Lys Asn Asn Leu Asn
   1790 1795 1800
   Ala Glu Arg Val Phe Ser Asp Asn Lys Asp Ser Lys Lys Gln Asn
   1805 1810 1815
   Leu Lys Asn Asn Ser Lys Val Phe Asn Asp Lys Leu Pro Asn Asn
   1820 1825 1830
   Glu Asp Arg Val Arg Gly Ser Phe Ala Phe Asp Ser Pro His His
   1835 1840 1845
   Tyr Thr Pro Ile Glu Gly Thr Pro Tyr Cys Phe Ser Arg Asn Asp
   1850 1855 1860
   Ser Leu Ser Ser Leu Asp Phe Asp Asp Asp Asp Val Asp Leu Ser
   1865 1870 1875
   Arg Glu Lys Ala Glu Leu Arg Lys Ala Lys Glu Asn Lys Glu Ser
   1880 1885 1890
   Glu Ala Lys Val Thr Ser His Thr Glu Leu Thr Ser Asn Gln Gln
   1895 1900 1905
   Ser Ala Asn Lys Thr Gln Ala Ile Ala Lys Gln Pro Ile Asn Arg
   1910 1915 1920
   Gly Gln Pro Lys Pro Ile Leu Gln Lys Gln Ser Thr Phe Pro Gln
   1925 1930 1935
   Ser Ser Lys Asp Ile Pro Asp Arg Gly Ala Ala Thr Asp Glu Lys
   1940 1945 1950
   Leu Gln Asn Phe Ala Ile Glu Asn Thr Pro Val Cys Phe Ser His
   1955 1960 1965
   Asn Ser Ser Leu Ser Ser Leu Ser Asp Ile Asp Gln Glu Asn Asn
   1970 1975 1980
   Asn Lys Glu Asn Glu Pro Ile Lys Glu Thr Glu Pro Pro Asp Ser
   1985 1990 1995
   Gln Gly Glu Pro Ser Lys Pro Gln Ala Ser Gly Tyr Ala Pro Lys
   2000 2005 2010
   Ser Phe His Val Glu Asp Thr Pro Val Cys Phe Ser Arg Asn Ser
   2015 2020 2025
   Ser Leu Ser Ser Leu Ser Ile Asp Ser Glu Asp Asp Leu Leu Gln
   2030 2035 2040
   Glu Cys Ile Ser Ser Ala Met Pro Lys Lys Lys Lys Pro Ser Arg
   2045 2050 2055
   Leu Lys Gly Asp Asn Glu Lys His Ser Pro Arg Asn Met Gly Gly
   2060 2065 2070
   Ile Leu Gly Glu Asp Leu Thr Leu Asp Leu Lys Asp Ile Gln Arg
   2075 2080 2085
   Pro Asp Ser Glu His Gly Leu Ser Pro Asp Ser Glu Asn Phe Asp
   2090 2095 2100
   Trp Lys Ala Ile Gln Glu Gly Ala Asn Ser Ile Val Ser Ser Leu
   2105 2110 2115
   His Gln Ala Ala Ala Ala Ala Cys Leu Ser Arg Gln Ala Ser Ser
   2120 2125 2130
   Asp Ser Asp Ser Ile Leu Ser Leu Lys Ser Gly Ile Ser Leu Gly
   2135 2140 2145
   Ser Pro Phe His Leu Thr Pro Asp Gln Glu Glu Lys Pro Phe Thr
   2150 2155 2160
   Ser Asn Lys Gly Pro Arg Ile Leu Lys Pro Gly Glu Lys Ser Thr
   2165 2170 2175
   Leu Glu Thr Lys Lys Ile Glu Ser Glu Ser Lys Gly Ile Lys Gly
   2180 2185 2190
   Gly Lys Lys Val Tyr Lys Ser Leu Ile Thr Gly Lys Val Arg Ser
   2195 2200 2205
   Asn Ser Glu Ile Ser Gly Gln Met Lys Gln Pro Leu Gln Ala Asn
   2210 2215 2220
   Met Pro Ser Ile Ser Arg Gly Arg Thr Met Ile His Ile Pro Gly
   2225 2230 2235
   Val Arg Asn Ser Ser Ser Ser Thr Ser Pro Val Ser Lys Lys Gly
   2240 2245 2250
   Pro Pro Leu Lys Thr Pro Ala Ser Lys Ser Pro Ser Glu Gly Gln
   2255 2260 2265
   Thr Ala Thr Thr Ser Pro Arg Gly Ala Lys Pro Ser Val Lys Ser
   2270 2275 2280
   Glu Leu Ser Pro Val Ala Arg Gln Thr Ser Gln Ile Gly Gly Ser
   2285 2290 2295
   Ser Lys Ala Pro Ser Arg Ser Gly Ser Arg Asp Ser Thr Pro Ser
   2300 2305 2310
   Arg Pro Ala Gln Gln Pro Leu Ser Arg Pro Ile Gln Ser Pro Gly
   2315 2320 2325
   Arg Asn Ser Ile Ser Pro Gly Arg Asn Gly Ile Ser Pro Pro Asn
   2330 2335 2340
   Lys Leu Ser Gln Leu Pro Arg Thr Ser Ser Pro Ser Thr Ala Ser
   2345 2350 2355
   Thr Lys Ser Ser Gly Ser Gly Lys Met Ser Tyr Thr Ser Pro Gly
   2360 2365 2370
   Arg Gln Met Ser Gln Gln Asn Leu Thr Lys Gln Thr Gly Leu Ser
   2375 2380 2385
   Lys Asn Ala Ser Ser Ile Pro Arg Ser Glu Ser Ala Ser Lys Gly
   2390 2395 2400
   Leu Asn Gln Met Asn Asn Gly Asn Gly Ala Asn Lys Lys Val Glu
   2405 2410 2415
   Leu Ser Arg Met Ser Ser Thr Lys Ser Ser Gly Ser Glu Ser Asp
   2420 2425 2430
   Arg Ser Glu Arg Pro Val Leu Val Arg Gln Ser Thr Phe Ile Lys
   2435 2440 2445
   Glu Ala Pro Ser Pro Thr Leu Arg Arg Lys Leu Glu Glu Ser Ala
   2450 2455 2460
   Ser Phe Glu Ser Leu Ser Pro Ser Ser Arg Pro Ala Ser Pro Thr
   2465 2470 2475
   Arg Ser Gln Ala Gln Thr Pro Val Leu Ser Pro Ser Leu Pro Asp
   2480 2485 2490
   Met Ser Leu Ser Thr His Ser Ser Val Gln Ala Gly Gly Trp Arg
   2495 2500 2505
   Lys Leu Pro Pro Asn Leu Ser Pro Thr Ile Glu Tyr Asn Asp Gly
   2510 2515 2520
   Arg Pro Ala Lys Arg His Asp Ile Ala Arg Ser His Ser Glu Ser
   2525 2530 2535
   Pro Ser Arg Leu Pro Ile Asn Arg Ser Gly Thr Trp Lys Arg Glu
   2540 2545 2550
   His Ser Lys His Ser Ser Ser Leu Pro Arg Val Ser Thr Trp Arg
   2555 2560 2565
   Arg Thr Gly Ser Ser Ser Ser Ile Leu Ser Ala Ser Ser Glu Ser
   2570 2575 2580
   Ser Glu Lys Ala Lys Ser Glu Asp Glu Lys His Val Asn Ser Ile
   2585 2590 2595
   Ser Gly Thr Lys Gln Ser Lys Glu Asn Gln Val Ser Ala Lys Gly
   2600 2605 2610
   Thr Trp Arg Lys Ile Lys Glu Asn Glu Phe Ser Pro Thr Asn Ser
   2615 2620 2625
   Thr Ser Gln Thr Val Ser Ser Gly Ala Thr Asn Gly Ala Glu Ser
   2630 2635 2640
   Lys Thr Leu Ile Tyr Gln Met Ala Pro Ala Val Ser Lys Thr Glu
   2645 2650 2655
   Asp Val Trp Val Arg Ile Glu Asp Cys Pro Ile Asn Asn Pro Arg
   2660 2665 2670
   Ser Gly Arg Ser Pro Thr Gly Asn Thr Pro Pro Val Ile Asp Ser
   2675 2680 2685
   Val Ser Glu Lys Ala Asn Pro Asn Ile Lys Asp Ser Lys Asp Asn
   2690 2695 2700
   Gln Ala Lys Gln Asn Val Gly Asn Gly Ser Val Pro Met Arg Thr
   2705 2710 2715
   Val Gly Leu Glu Asn Arg Leu Asn Ser Phe Ile Gln Val Asp Ala
   2720 2725 2730
   Pro Asp Gln Lys Gly Thr Glu Ile Lys Pro Gly Gln Asn Asn Pro
   2735 2740 2745
   Val Pro Val Ser Glu Thr Asn Glu Ser Ser Ile Val Glu Arg Thr
   2750 2755 2760
   Pro Phe Ser Ser Ser Ser Ser Ser Lys His Ser Ser Pro Ser Gly
   2765 2770 2775
   Thr Val Ala Ala Arg Val Thr Pro Phe Asn Tyr Asn Pro Ser Pro
   2780 2785 2790
   Arg Lys Ser Ser Ala Asp Ser Thr Ser Ala Arg Pro Ser Gln Ile
   2795 2800 2805
   Pro Thr Pro Val Asn Asn Asn Thr Lys Lys Arg Asp Ser Lys Thr
   2810 2815 2820
   Asp Ser Thr Glu Ser Ser Gly Thr Gln Ser Pro Lys Arg His Ser
   2825 2830 2835
   Gly Ser Tyr Leu Val Thr Ser Val
   2840
<210> 40
   <211> 3056
   <212> PRT
   <213> Homo sapiens
<400> 40
   Met Ser Leu Val Leu Asn Asp Leu Leu Ile Cys Cys Arg Gln Leu
   1 5 10 15
   Glu His Asp Arg Ala Thr Glu Arg Lys Lys Glu Val Glu Lys Phe
   20 25 30
   Lys Arg Leu Ile Arg Asp Pro Glu Thr Ile Lys His Leu Asp Arg
   35 40 45
   His Ser Asp Ser Lys Gln Gly Lys Tyr Leu Asn Trp Asp Ala Val
   50 55 60
   Phe Arg Phe Leu Gln Lys Tyr Ile Gln Lys Glu Thr Glu Cys Leu
   65 70 75
   Arg Ile Ala Lys Pro Asn Val Ser Ala Ser Thr Gln Ala Ser Arg
   80 85 90
   Gln Lys Lys Met Gln Glu Ile Ser Ser Leu Val Lys Tyr Phe Ile
   95 100 105
   Lys Cys Ala Asn Arg Arg Ala Pro Arg Leu Lys Cys Gln Glu Leu
   110 115 120
   Leu Asn Tyr Ile Met Asp Thr Val Lys Asp Ser Ser Asn Gly Ala
   125 130 135
   Ile Tyr Gly Ala Asp Cys Ser Asn Ile Leu Leu Lys Asp Ile Leu
   140 145 150
   Ser Val Arg Lys Tyr Trp Cys Glu Ile Ser Gln Gln Gln Trp Leu
   155 160 165
   Glu Leu Phe Ser Val Tyr Phe Arg Leu Tyr Leu Lys Pro Ser Gln
   170 175 180
   Asp Val His Arg Val Leu Val Ala Arg Ile Ile His Ala Val Thr
   185 190 195
   Lys Gly Cys Cys Ser Gln Thr Asp Gly Leu Asn Ser Lys Phe Leu
   200 205 210
   Asp Phe Phe Ser Lys Ala Ile Gln Cys Ala Arg Gln Glu Lys Ser
   215 220 225
   Ser Ser Gly Leu Asn His Ile Leu Ala Ala Leu Thr Ile Phe Leu
   230 235 240
   Lys Thr Leu Ala Val Asn Phe Arg Ile Arg Val Cys Glu Leu Gly
   245 250 255
   Asp Glu Ile Leu Pro Thr Leu Leu Tyr Ile Trp Thr Gln His Arg
   260 265 270
   Leu Asn Asp Ser Leu Lys Glu Val Ile Ile Glu Leu Phe Gln Leu
   275 280 285
   Gln Ile Tyr Ile His His Pro Lys Gly Ala Lys Thr Gln Glu Lys
   290 295 300
   Gly Ala Tyr Glu Ser Thr Lys Trp Arg Ser Ile Leu Tyr Asn Leu
   305 310 315
   Tyr Asp Leu Leu Val Asn Glu Ile Ser His Ile Gly Ser Arg Gly
   320 325 330
   Lys Tyr Ser Ser Gly Phe Arg Asn Ile Ala Val Lys Glu Asn Leu
   335 340 345
   Ile Glu Leu Met Ala Asp Ile Cys His Gln Val Phe Asn Glu Asp
   350 355 360
   Thr Arg Ser Leu Glu Ile Ser Gln Ser Tyr Thr Thr Thr Gln Arg
   365 370 375
   Glu Ser Ser Asp Tyr Ser Val Pro Cys Lys Arg Lys Lys Ile Glu
   380 385 390
   Leu Gly Trp Glu Val Ile Lys Asp His Leu Gln Lys Ser Gln Asn
   395 400 405
   Asp Phe Asp Leu Val Pro Trp Leu Gln Ile Ala Thr Gln Leu Ile
   410 415 420
   Ser Lys Tyr Pro Ala Ser Leu Pro Asn Cys Glu Leu Ser Pro Leu
   425 430 435
   Leu Met Ile Leu Ser Gln Leu Leu Pro Gln Gln Arg His Gly Glu
   440 445 450
   Arg Thr Pro Tyr Val Leu Arg Cys Leu Thr Glu Val Ala Leu Cys
   455 460 465
   Gln Asp Lys Arg Ser Asn Leu Glu Ser Ser Gln Lys Ser Asp Leu
   470 475 480
   Leu Lys Leu Trp Asn Lys Ile Trp Cys Ile Thr Phe Arg Gly Ile
   485 490 495
   Ser Ser Glu Gln Ile Gln Ala Glu Asn Phe Gly Leu Leu Gly Ala
   500 505 510
   Ile Ile Gln Gly Ser Leu Val Glu Val Asp Arg Glu Phe Trp Lys
   515 520 525
   Leu Phe Thr Gly Ser Ala Cys Arg Pro Ser Cys Pro Ala Val Cys
   530 535 540
   Cys Leu Thr Leu Ala Leu Thr Thr Ser Ile Val Pro Gly Thr Val
   545 550 555
   Lys Met Gly Ile Glu Gln Asn Met Cys Glu Val Asn Arg Ser Phe
   560 565 570
   Ser Leu Lys Glu Ser Ile Met Lys Trp Leu Leu Phe Tyr Gln Leu
   575 580 585
   Glu Gly Asp Leu Glu Asn Ser Thr Glu Val Pro Pro Ile Leu His
   590 595 600
   Ser Asn Phe Pro His Leu Val Leu Glu Lys Ile Leu Val Ser Leu
   605 610 615
   Thr Met Lys Asn Cys Lys Ala Ala Met Asn Phe Phe Gln Ser Val
   620 625 630
   Pro Glu Cys Glu His His Gln Lys Asp Lys Glu Glu Leu Ser Phe
   635 640 645
   Ser Glu Val Glu Glu Leu Phe Leu Gln Thr Thr Phe Asp Lys Met
   650 655 660
   Asp Phe Leu Thr Ile Val Arg Glu Cys Gly Ile Glu Lys His Gln
   665 670 675
   Ser Ser Ile Gly Phe Ser Val His Gln Asn Leu Lys Glu Ser Leu
   680 685 690
   Asp Arg Cys Leu Leu Gly Leu Ser Glu Gln Leu Leu Asn Asn Tyr
   695 700 705
   Ser Ser Glu Ile Thr Asn Ser Glu Thr Leu Val Arg Cys Ser Arg
   710 715 720
   Leu Leu Val Gly Val Leu Gly Cys Tyr Cys Tyr Met Gly Val Ile
   725 730 735
   Ala Glu Glu Glu Ala Tyr Lys Ser Glu Leu Phe Gln Lys Ala Lys
   740 745 750
   Ser Leu Met Gln Cys Ala Gly Glu Ser Ile Thr Leu Phe Lys Asn
   755 760 765
   Lys Thr Asn Glu Glu Phe Arg Ile Gly Ser Leu Arg Asn Met Met
   770 775 780
   Gln Leu Cys Thr Arg Cys Leu Ser Asn Cys Thr Lys Lys Ser Pro
   785 790 795
   Asn Lys Ile Ala Ser Gly Phe Phe Leu Arg Leu Leu Thr Ser Lys
   800 805 810
   Leu Met Asn Asp Ile Ala Asp Ile Cys Lys Ser Leu Ala Ser Phe
   815 820 825
   Ile Lys Lys Pro Phe Asp Arg Gly Glu Val Glu Ser Met Glu Asp
   830 835 840
   Asp Thr Asn Gly Asn Leu Met Glu Val Glu Asp Gln Ser Ser Met
   845 850 855
   Asn Leu Phe Asn Asp Tyr Pro Asp Ser Ser Val Ser Asp Ala Asn
   860 865 870
   Glu Pro Gly Glu Ser Gln Ser Thr Ile Gly Ala Ile Asn Pro Leu
   875 880 885
   Ala Glu Glu Tyr Leu Ser Lys Gln Asp Leu Leu Phe Leu Asp Met
   890 895 900
   Leu Lys Phe Leu Cys Leu Cys Val Thr Thr Ala Gln Thr Asn Thr
   905 910 915
   Val Ser Phe Arg Ala Ala Asp Ile Arg Arg Lys Leu Leu Met Leu
   920 925 930
   Ile Asp Ser Ser Thr Leu Glu Pro Thr Lys Ser Leu His Leu His
   935 940 945
   Met Tyr Leu Met Leu Leu Lys Glu Leu Pro Gly Glu Glu Tyr Pro
   950 955 960
   Leu Pro Met Glu Asp Val Leu Glu Leu Leu Lys Pro Leu Ser Asn
   965 970 975
   Val Cys Ser Leu Tyr Arg Arg Asp Gln Asp Val Cys Lys Thr Ile
   980 985 990
   Leu Asn His Val Leu His Val Val Lys Asn Leu Gly Gln Ser Asn
   995 1000 1005
   Met Asp Ser Glu Asn Thr Arg Asp Ala Gln Gly Gln Phe Leu Thr
   1010 1015 1020
   Val Ile Gly Ala Phe Trp His Leu Thr Lys Glu Arg Lys Tyr Ile
   1025 1030 1035
   Phe Ser Val Arg Met Ala Leu Val Asn Cys Leu Lys Thr Leu Leu
   1040 1045 1050
   Glu Ala Asp Pro Tyr Ser Lys Trp Ala Ile Leu Asn Val Met Gly
   1055 1060 1065
   Lys Asp Phe Pro Val Asn Glu Val Phe Thr Gln Phe Leu Ala Asp
   1070 1075 1080
   Asn His His Gln Val Arg Met Leu Ala Ala Glu Ser Ile Asn Arg
   1085 1090 1095
   Leu Phe Gln Asp Thr Lys Gly Asp Ser Ser Arg Leu Leu Lys Ala
   1100 1105 1110
   Leu Pro Leu Lys Leu Gln Gln Thr Ala Phe Glu Asn Ala Tyr Leu
   1115 1120 1125
   Lys Ala Gln Glu Gly Met Arg Glu Met Ser His Ser Ala Glu Asn
   1130 1135 1140
   Pro Glu Thr Leu Asp Glu Ile Tyr Asn Arg Lys Ser Val Leu Leu
   1145 1150 1155
   Thr Leu Ile Ala Val Val Leu Ser Cys Ser Pro Ile Cys Glu Lys
   1160 1165 1170
   Gln Ala Leu Phe Ala Leu Cys Lys Ser Val Lys Glu Asn Gly Leu
   1175 1180 1185
   Glu Pro His Leu Val Lys Lys Val Leu Glu Lys Val Ser Glu Thr
   1190 1195 1200
   Phe Gly Tyr Arg Arg Leu Glu Asp Phe Met Ala Ser His Leu Asp
   1205 1210 1215
   Tyr Leu Val Leu Glu Trp Leu Asn Leu Gln Asp Thr Glu Tyr Asn
   1220 1225 1230
   Leu Ser Ser Phe Pro Phe Ile Leu Leu Asn Tyr Thr Asn Ile Glu
   1235 1240 1245
   Asp Phe Tyr Arg Ser Cys Tyr Lys Val Leu Ile Pro His Leu Val
   1250 1255 1260
   Ile Arg Ser His Phe Asp Glu Val Lys Ser Ile Ala Asn Gln Ile
   1265 1270 1275
   Gln Glu Asp Trp Lys Ser Leu Leu Thr Asp Cys Phe Pro Lys Ile
   1280 1285 1290
   Leu Val Asn Ile Leu Pro Tyr Phe Ala Tyr Glu Gly Thr Arg Asp
   1295 1300 1305
   Ser Gly Met Ala Gln Gln Arg Glu Thr Ala Thr Lys Val Tyr Asp
   1310 1315 1320
   Met Leu Lys Ser Glu Asn Leu Leu Gly Lys Gln Ile Asp His Leu
   1325 1330 1335
   Phe Ile Ser Asn Leu Pro Glu Ile Val Val Glu Leu Leu Met Thr
   1340 1345 1350
   Leu His Glu Pro Ala Asn Ser Ser Ala Ser Gln Ser Thr Asp Leu
   1355 1360 1365
   Cys Asp Phe Ser Gly Asp Leu Asp Pro Ala Pro Asn Pro Pro His
   1370 1375 1380
   Phe Pro Ser His Val Ile Lys Ala Thr Phe Ala Tyr Ile Ser Asn
   1385 1390 1395
   Cys His Lys Thr Lys Leu Lys Ser Ile Leu Glu Ile Leu Ser Lys
   1400 1405 1410
   Ser Pro Asp Ser Tyr Gln Lys Ile Leu Leu Ala Ile Cys Glu Gln
   1415 1420 1425
   Ala Ala Glu Thr Asn Asn Val Tyr Lys Lys His Arg Ile Leu Lys
   1430 1435 1440
   Ile Tyr His Leu Phe Val Ser Leu Leu Leu Lys Asp Ile Lys Ser
   1445 1450 1455
   Gly Leu Gly Gly Ala Trp Ala Phe Val Leu Arg Asp Val Ile Tyr
   1460 1465 1470
   Thr Leu Ile His Tyr Ile Asn Gln Arg Pro Ser Cys Ile Met Asp
   1475 1480 1485
   Val Ser Leu Arg Ser Phe Ser Leu Cys Cys Asp Leu Leu Ser Gln
   1490 1495 1500
   Val Cys Gln Thr Ala Val Thr Tyr Cys Lys Asp Ala Leu Glu Asn
   1505 1510 1515
   His Leu His Val Ile Val Gly Thr Leu Ile Pro Leu Val Tyr Glu
   1520 1525 1530
   Gln Val Glu Val Gln Lys Gln Val Leu Asp Leu Leu Lys Tyr Leu
   1535 1540 1545
   Val Ile Asp Asn Lys Asp Asn Glu Asn Leu Tyr Ile Thr Ile Lys
   1550 1555 1560
   Leu Leu Asp Pro Phe Pro Asp His Val Val Phe Lys Asp Leu Arg
   1565 1570 1575
   Ile Thr Gln Gln Lys Ile Lys Tyr Ser Arg Gly Pro Phe Ser Leu
   1580 1585 1590
   Leu Glu Glu Ile Asn His Phe Leu Ser Val Ser Val Tyr Asp Ala
   1595 1600 1605
   Leu Pro Leu Thr Arg Leu Glu Gly Leu Lys Asp Leu Arg Arg Gln
   1610 1615 1620
   Leu Glu Leu His Lys Asp Gln Met Val Asp Ile Met Arg Ala Ser
   1625 1630 1635
   Gln Asp Asn Pro Gln Asp Gly Ile Met Val Lys Leu Val Val Asn
   1640 1645 1650
   Leu Leu Gln Leu Ser Lys Met Ala Ile Asn His Thr Gly Glu Lys
   1655 1660 1665
   Glu Val Leu Glu Ala Val Gly Ser Cys Leu Gly Glu Val Gly Pro
   1670 1675 1680
   Ile Asp Phe Ser Thr Ile Ala Ile Gln His Ser Lys Asp Ala Ser
   1685 1690 1695
   Tyr Thr Lys Ala Leu Lys Leu Phe Glu Asp Lys Glu Leu Gln Trp
   1700 1705 1710
   Thr Phe Ile Met Leu Thr Tyr Leu Asn Asn Thr Leu Val Glu Asp
   1715 1720 1725
   Cys Val Lys Val Arg Ser Ala Ala Val Thr Cys Leu Lys Asn Ile
   1730 1735 1740
   Leu Ala Thr Lys Thr Gly His Ser Phe Trp Glu Ile Tyr Lys Met
   1745 1750 1755
   Thr Thr Asp Pro Met Leu Ala Tyr Leu Gln Pro Phe Arg Thr Ser
   1760 1765 1770
   Arg Lys Lys Phe Leu Glu Val Pro Arg Phe Asp Lys Glu Asn Pro
   1775 1780 1785
   Phe Glu Gly Leu Asp Asp Ile Asn Leu Trp Ile Pro Leu Ser Glu
   1790 1795 1800
   Asn His Asp Ile Trp Ile Lys Thr Leu Thr Cys Ala Phe Leu Asp
   1805 1810 1815
   Ser Gly Gly Thr Lys Cys Glu Ile Leu Gln Leu Leu Lys Pro Met
   1820 1825 1830
   Cys Glu Val Lys Thr Asp Phe Cys Gln Thr Val Leu Pro Tyr Leu
   1835 1840 1845
   Ile His Asp Ile Leu Leu Gln Asp Thr Asn Glu Ser Trp Arg Asn
   1850 1855 1860
   Leu Leu Ser Thr His Val Gln Gly Phe Phe Thr Ser Cys Leu Arg
   1865 1870 1875
   His Phe Ser Gln Thr Ser Arg Ser Thr Thr Pro Ala Asn Leu Asp
   1880 1885 1890
   Ser Glu Ser Glu His Phe Phe Arg Cys Cys Leu Asp Lys Lys Ser
   1895 1900 1905
   Gln Arg Thr Met Leu Ala Val Val Asp Tyr Met Arg Arg Gln Lys
   1910 1915 1920
   Arg Pro Ser Ser Gly Thr Ile Phe Asn Asp Ala Phe Trp Leu Asp
   1925 1930 1935
   Leu Asn Tyr Leu Glu Val Ala Lys Val Ala Gln Ser Cys Ala Ala
   1940 1945 1950
   His Phe Thr Ala Leu Leu Tyr Ala Glu Ile Tyr Ala Asp Lys Lys
   1955 1960 1965
   Ser Met Asp Asp Gln Glu Lys Arg Ser Leu Ala Phe Glu Glu Gly
   1970 1975 1980
   Ser Gln Asn Thr Thr Ile Ser Ser Leu Ser Glu Lys Ser Lys Glu
   1985 1990 1995
   Glu Thr Gly Ile Ser Leu Gln Asp Leu Leu Leu Glu Ile Tyr Arg
   2000 2005 2010
   Ser Ile Gly Glu Pro Asp Ser Leu Tyr Gly Cys Gly Gly Gly Lys
   2015 2020 2025
   Met Leu Gln Pro Ile Thr Arg Leu Arg Thr Tyr Glu His Glu Ala
   2030 2035 2040
   Met Trp Gly Lys Ala Leu Val Thr Tyr Asp Leu Glu Thr Ala Ile
   2045 2050 2055
   Pro Ser Ser Thr Arg Gln Ala Gly Ile Ile Gln Ala Leu Gln Asn
   2060 2065 2070
   Leu Gly Leu Cys His Ile Leu Ser Val Tyr Leu Lys Gly Leu Asp
   2075 2080 2085
   Tyr Glu Asn Lys Asp Trp Cys Pro Glu Leu Glu Glu Leu His Tyr
   2090 2095 2100
   Gln Ala Ala Trp Arg Asn Met Gln Trp Asp His Cys Thr Ser Val
   2105 2110 2115
   Ser Lys Glu Val Glu Gly Thr Ser Tyr His Glu Ser Leu Tyr Asn
   2120 2125 2130
   Ala Leu Gln Ser Leu Arg Asp Arg Glu Phe Ser Thr Phe Tyr Glu
   2135 2140 2145
   Ser Leu Lys Tyr Ala Arg Val Lys Glu Val Glu Glu Met Cys Lys
   2150 2155 2160
   Arg Ser Leu Glu Ser Val Tyr Ser Leu Tyr Pro Thr Leu Ser Arg
   2165 2170 2175
   Leu Gln Ala Ile Gly Glu Leu Glu Ser Ile Gly Glu Leu Phe Ser
   2180 2185 2190
   Arg Ser Val Thr His Arg Gln Leu Ser Glu Val Tyr Ile Lys Trp
   2195 2200 2205
   Gln Lys His Ser Gln Leu Leu Lys Asp Ser Asp Phe Ser Phe Gln
   2210 2215 2220
   Glu Pro Ile Met Ala Leu Arg Thr Val Ile Leu Glu Ile Leu Met
   2225 2230 2235
   Glu Lys Glu Met Asp Asn Ser Gln Arg Glu Cys Ile Lys Asp Ile
   2240 2245 2250
   Leu Thr Lys His Leu Val Glu Leu Ser Ile Leu Ala Arg Thr Phe
   2255 2260 2265
   Lys Asn Thr Gln Leu Pro Glu Arg Ala Ile Phe Gln Ile Lys Gln
   2270 2275 2280
   Tyr Asn Ser Val Ser Cys Gly Val Ser Glu Trp Gln Leu Glu Glu
   2285 2290 2295
   Ala Gln Val Phe Trp Ala Lys Lys Glu Gln Ser Leu Ala Leu Ser
   2300 2305 2310
   Ile Leu Lys Gln Met Ile Lys Lys Leu Asp Ala Ser Cys Ala Ala
   2315 2320 2325
   Asn Asn Pro Ser Leu Lys Leu Thr Tyr Thr Glu Cys Leu Arg Val
   2330 2335 2340
   Cys Gly Asn Trp Leu Ala Glu Thr Cys Leu Glu Asn Pro Ala Val
   2345 2350 2355
   Ile Met Gln Thr Tyr Leu Glu Lys Ala Val Glu Val Ala Gly Asn
   2360 2365 2370
   Tyr Asp Gly Glu Ser Ser Asp Glu Leu Arg Asn Gly Lys Met Lys
   2375 2380 2385
   Ala Phe Leu Ser Leu Ala Arg Phe Ser Asp Thr Gln Tyr Gln Arg
   2390 2395 2400
   Ile Glu Asn Tyr Met Lys Ser Ser Glu Phe Glu Asn Lys Gln Ala
   2405 2410 2415
   Leu Leu Lys Arg Ala Lys Glu Glu Val Gly Leu Leu Arg Glu His
   2420 2425 2430
   Lys Ile Gln Thr Asn Arg Tyr Thr Val Lys Val Gln Arg Glu Leu
   2435 2440 2445
   Glu Leu Asp Glu Leu Ala Leu Arg Ala Leu Lys Glu Asp Arg Lys
   2450 2455 2460
   Arg Phe Leu Cys Lys Ala Val Glu Asn Tyr Ile Asn Cys Leu Leu
   2465 2470 2475
   Ser Gly Glu Glu His Asp Met Trp Val Phe Arg Leu Cys Ser Leu
   2480 2485 2490
   Trp Leu Glu Asn Ser Gly Val Ser Glu Val Asn Gly Met Met Lys
   2495 2500 2505
   Ala Asn Gly Met Lys Ile Pro Thr Tyr Lys Phe Leu Pro Leu Met
   2510 2515 2520
   Tyr Gln Leu Ala Ala Arg Met Gly Thr Lys Met Met Gly Gly Leu
   2525 2530 2535
   Gly Phe His Glu Val Leu Asn Asn Leu Ile Ser Arg Ile Ser Met
   2540 2545 2550
   Asp His Pro His His Thr Leu Phe Ile Ile Leu Ala Leu Ala Asn
   2555 2560 2565
   Ala Asn Arg Asp Glu Phe Leu Thr Lys Pro Glu Val Ala Arg Arg
   2570 2575 2580
   Ser Arg Ile Thr Lys Asn Val Pro Lys Gln Ser Ser Gln Leu Asp
   2585 2590 2595
   Glu Asp Arg Thr Glu Ala Ala Asn Arg Ile Ile Cys Thr Ile Arg
   2600 2605 2610
   Ser Arg Arg Pro Gln Met Val Arg Ser Val Glu Ala Leu Cys Asp
   2615 2620 2625
   Ala Tyr Ile Ile Leu Ala Asn Leu Asp Ala Thr Gln Trp Lys Thr
   2630 2635 2640
   Gln Arg Lys Gly Ile Asn Ile Pro Ala Asp Gln Pro Ile Thr Lys
   2645 2650 2655
   Leu Lys Asn Leu Glu Asp Val Val Val Pro Thr Met Glu Ile Lys
   2660 2665 2670
   Val Asp His Thr Gly Glu Tyr Gly Asn Leu Val Thr Ile Gln Ser
   2675 2680 2685
   Phe Lys Ala Glu Phe Arg Leu Ala Gly Gly Val Asn Leu Pro Lys
   2690 2695 2700
   Ile Ile Asp Cys Val Gly Ser Asp Gly Lys Glu Arg Arg Gln Leu
   2705 2710 2715
   Val Lys Gly Arg Asp Asp Leu Arg Gln Asp Ala Val Met Gln Gln
   2720 2725 2730
   Val Phe Gln Met Cys Asn Thr Leu Leu Gln Arg Asn Thr Glu Thr
   2735 2740 2745
   Arg Lys Arg Lys Leu Thr Ile Cys Thr Tyr Lys Val Val Pro Leu
   2750 2755 2760
   Ser Gln Arg Ser Gly Val Leu Glu Trp Cys Thr Gly Thr Val Pro
   2765 2770 2775
   Ile Gly Glu Phe Leu Val Asn Asn Glu Asp Gly Ala His Lys Arg
   2780 2785 2790
   Tyr Arg Pro Asn Asp Phe Ser Ala Phe Gln Cys Gln Lys Lys Met
   2795 2800 2805
   Met Glu Val Gln Lys Lys Ser Phe Glu Glu Lys Tyr Glu Val Phe
   2810 2815 2820
   Met Asp Val Cys Gln Asn Phe Gln Pro Val Phe Arg Tyr Phe Cys
   2825 2830 2835
   Met Glu Lys Phe Leu Asp Pro Ala Ile Trp Phe Glu Lys Arg Leu
   2840 2845 2850
   Ala Tyr Thr Arg Ser Val Ala Thr Ser Ser Ile Val Gly Tyr Ile
   2855 2860 2865
   Leu Gly Leu Gly Asp Arg His Val Gln Asn Ile Leu Ile Asn Glu
   2870 2875 2880
   Gln Ser Ala Glu Leu Val His Ile Asp Leu Gly Val Ala Phe Glu
   2885 2890 2895
   Gln Gly Lys Ile Leu Pro Thr Pro Glu Thr Val Pro Phe Arg Leu
   2900 2905 2910
   Thr Arg Asp Ile Val Asp Gly Met Gly Ile Thr Gly Val Glu Gly
   2915 2920 2925
   Val Phe Arg Arg Cys Cys Glu Lys Thr Met Glu Val Met Arg Asn
   2930 2935 2940
   Ser Gln Glu Thr Leu Leu Thr Ile Val Glu Val Leu Leu Tyr Asp
   2945 2950 2955
   Pro Leu Phe Asp Trp Thr Met Asn Pro Leu Lys Ala Leu Tyr Leu
   2960 2965 2970
   Gln Gln Arg Pro Glu Asp Glu Thr Glu Leu His Pro Thr Leu Asn
   2975 2980 2985
   Ala Asp Asp Gln Glu Cys Lys Arg Asn Leu Ser Asp Ile Asp Gln
   2990 2995 3000
   Ser Phe Asn Lys Val Ala Glu Arg Val Leu Met Arg Leu Gln Glu
   3005 3010 3015
   Lys Leu Lys Gly Val Glu Glu Gly Thr Val Leu Ser Val Gly Gly
   3020 3025 3030
   Gln Val Asn Leu Leu Ile Gln Gln Ala Ile Asp Pro Lys Asn Leu
   3035 3040 3045
   Ser Arg Leu Phe Pro Gly Trp Lys Ala Trp Val
   3050 3055
<210> 41
   <211> 2644
   <212> PRT
   <213> Homo sapiens
<400> 41
   Met Gly Glu His Gly Leu Glu Leu Ala Ser Met Ile Pro Ala Leu
   1               5                   10                  15
   Arg Glu Leu Gly Ser Ala Thr Pro Glu Glu Tyr Asn Thr Val Val
                   20                  25                  30
   Gln Lys Pro Arg Gln Ile Leu Cys Gln Phe Ile Asp Arg Ile Leu
                   35                  40                  45
   Thr Asp Val Asn Val Val Ala Val Glu Leu Val Lys Lys Thr Asp
                   50                  55                  60
   Ser Gln Pro Thr Ser Val Met Leu Leu Asp Phe Ile Gln His Ile
                   65                  70                  75
   Met Lys Ser Ser Pro Leu Met Phe Val Asn Val Ser Gly Ser His
                   80                  85                  90
   Glu Ala Lys Gly Ser Cys Ile Glu Phe Ser Asn Trp Ile Ile Thr
                   95                  100                 105
   Arg Leu Leu Arg Ile Ala Ala Thr Pro Ser Cys His Leu Leu His
                   110                 115                 120
   Lys Lys Ile Cys Glu Val Ile Cys Ser Leu Leu Phe Leu Phe Lys
                   125                 130                 135
   Ser Lys Ser Pro Ala Ile Phe Gly Val Leu Thr Lys Glu Leu Leu
                   140                 145                 150
   Gln Leu Phe Glu Asp Leu Val Tyr Leu His Arg Arg Asn Val Met
                   155                 160                 165
   Gly His Ala Val Glu Trp Pro Val Val Met Ser Arg Phe Leu Ser
                   170                 175                 180
   Gln Leu Asp Glu His Met Gly Tyr Leu Gln Ser Ala Pro Leu Gln
                   185                 190                 195
   Leu Met Ser Met Gln Asn Leu Glu Phe Ile Glu Val Thr Leu Leu
                   200                 205                 210
   Met Val Leu Thr Arg Ile Ile Ala Ile Val Phe Phe Arg Arg Gln
                   215                 220                 225
   Glu Leu Leu Leu Trp Gln Ile Gly Cys Val Leu Leu Glu Tyr Gly
                   230                 235                 240
   Ser Pro Lys Ile Lys Ser Leu Ala Ile Ser Phe Leu Thr Glu Leu
                   245                 250                 255
   Phe Gln Leu Gly Gly Leu Pro Ala Gln Pro Ala Ser Thr Phe Phe
                   260                 265                 270
   Ser Ser Phe Leu Glu Leu Leu Lys His Leu Val Glu Met Asp Thr
                   275                 280                 285
   Asp Gln Leu Lys Leu Tyr Glu Glu Pro Leu Ser Lys Leu Ile Lys
                   290                 295                 300
   Thr Leu Phe Pro Phe Glu Ala Glu Ala Tyr Arg Asn Ile Glu Pro
                   305                 310                 315
   Val Tyr Leu Asn Met Leu Leu Glu Lys Leu Cys Val Met Phe Glu
   320 325 330
   Asp Gly Val Leu Met Arg Leu Lys Ser Asp Leu Leu Lys Ala Ala
   335 340 345
   Leu Cys His Leu Leu Gln Tyr Phe Leu Lys Phe Val Pro Ala Gly
   350 355 360
   Tyr Glu Ser Ala Leu Gln Val Arg Lys Val Tyr Val Arg Asn Ile
   365 370 375
   Cys Lys Ala Leu Leu Asp Val Leu Gly Ile Glu Val Asp Ala Glu
   380 385 390
   Tyr Leu Leu Gly Pro Leu Tyr Ala Ala Leu Lys Met Glu Ser Met
   395 400 405
   Glu Ile Ile Glu Glu Ile Gln Cys Gln Thr Gln Gln Glu Asn Leu
   410 415 420
   Ser Ser Asn Ser Asp Gly Ile Ser Pro Lys Arg Arg Arg Leu Ser
   425 430 435
   Ser Ser Leu Asn Pro Ser Lys Arg Ala Pro Lys Gln Thr Glu Glu
   440 445 450
   Ile Lys His Val Asp Met Asn Gln Lys Ser Ile Leu Trp Ser Ala
   455 460 465
   Leu Lys Gln Lys Ala Glu Ser Leu Gln Ile Ser Leu Glu Tyr Ser
   470 475 480
   Gly Leu Lys Asn Pro Val Ile Glu Met Leu Glu Gly Ile Ala Val
   485 490 495
   Val Leu Gln Leu Thr Ala Leu Cys Thr Val His Cys Ser His Gln
   500 505 510
   Asn Met Asn Cys Arg Thr Phe Lys Asp Cys Gln His Lys Ser Lys
   515 520 525
   Lys Lys Pro Ser Val Val Ile Thr Trp Met Ser Leu Asp Phe Tyr
   530 535 540
   Thr Lys Val Leu Lys Ser Cys Arg Ser Leu Leu Glu Ser Val Gln
   545 550 555
   Lys Leu Asp Leu Glu Ala Thr Ile Asp Lys Val Val Lys Ile Tyr
   560 565 570
   Asp Ala Leu Ile Tyr Met Gln Val Asn Ser Ser Phe Glu Asp His
   575 580 585
   Ile Leu Glu Asp Leu Cys Gly Met Leu Ser Leu Pro Trp Ile Tyr
   590 595 600
   Ser His Ser Asp Asp Gly Cys Leu Lys Leu Thr Thr Phe Ala Ala
   605 610 615
   Asn Leu Leu Thr Leu Ser Cys Arg Ile Ser Asp Ser Tyr Ser Pro
   620 625 630
   Gln Ala Gln Ser Arg Cys Val Phe Leu Leu Thr Leu Phe Pro Arg
   635 640 645
   Arg Ile Phe Leu Glu Trp Arg Thr Ala Val Tyr Asn Trp Ala Leu
   650 655 660
   Gln Ser Ser His Glu Val Ile Arg Ala Ser Cys Val Ser Gly Phe
   665 670 675
   Phe Ile Leu Leu Gln Gln Gln Asn Ser Cys Asn Arg Val Pro Lys
   680 685 690
   Ile Leu Ile Asp Lys Val Lys Asp Asp Ser Asp Ile Val Lys Lys
   695 700 705
   Glu Phe Ala Ser Ile Leu Gly Gln Leu Val Cys Thr Leu His Gly
   710 715 720
   Met Phe Tyr Leu Thr Ser Ser Leu Thr Glu Pro Phe Ser Glu His
   725 730 735
   Gly His Val Asp Leu Phe Cys Arg Asn Leu Lys Ala Thr Ser Gln
   740 745 750
   His Glu Cys Ser Ser Ser Gln Leu Lys Ala Ser Val Cys Lys Pro
   755 760 765
   Phe Leu Phe Leu Leu Lys Lys Lys Ile Pro Ser Pro Val Lys Leu
   770 775 780
   Ala Phe Ile Asp Asn Leu His His Leu Cys Lys His Leu Asp Phe
   785 790 795
   Arg Glu Asp Glu Thr Asp Val Lys Ala Val Leu Gly Thr Leu Leu
   800 805 810
   Asn Leu Met Glu Asp Pro Asp Lys Asp Val Arg Val Ala Phe Ser
   815 820 825
   Gly Asn Ile Lys His Ile Leu Glu Ser Leu Asp Ser Glu Asp Gly
   830 835 840
   Phe Ile Lys Glu Leu Phe Val Leu Arg Met Lys Glu Ala Tyr Thr
   845 850 855
   His Ala Gln Ile Ser Arg Asn Asn Glu Leu Lys Asp Thr Leu Ile
   860 865 870
   Leu Thr Thr Gly Asp Ile Gly Arg Ala Ala Lys Gly Asp Leu Val
   875 880 885
   Pro Phe Ala Leu Leu His Leu Leu His Cys Leu Leu Ser Lys Ser
   890 895 900
   Ala Ser Val Ser Gly Ala Ala Tyr Thr Glu Ile Arg Ala Leu Val
   905 910 915
   Ala Ala Lys Ser Val Lys Leu Gln Ser Phe Phe Ser Gln Tyr Lys
   920 925 930
   Lys Pro Ile Cys Gln Phe Leu Val Glu Ser Leu His Ser Ser Gln
   935 940 945
   Met Thr Ala Leu Pro Asn Thr Pro Cys Gln Asn Ala Asp Val Arg
   950 955 960
   Lys Gln Asp Val Ala His Gln Arg Glu Met Ala Leu Asn Thr Leu
   965 970 975
   Ser Glu Ile Ala Asn Val Phe Asp Phe Pro Asp Leu Asn Arg Phe
   980 985 990
   Leu Thr Arg Thr Leu Gln Val Leu Leu Pro Asp Leu Ala Ala Lys
   995 1000 1005
   Ala Ser Pro Ala Ala Ser Ala Leu Ile Arg Thr Leu Gly Lys Gln
   1010 1015 1020
   Leu Asn Val Asn Arg Arg Glu Ile Leu Ile Asn Asn Phe Lys Tyr
   1025 1030 1035
   Ile Phe Ser His Leu Val Cys Ser Cys Ser Lys Asp Glu Leu Glu
   1040 1045 1050
   Arg Ala Leu His Tyr Leu Lys Asn Glu Thr Glu Ile Glu Leu Gly
   1055 1060 1065
   Ser Leu Leu Arg Gln Asp Phe Gln Gly Leu His Asn Glu Leu Leu
   1070 1075 1080
   Leu Arg Ile Gly Glu His Tyr Gln Gln Val Phe Asn Gly Leu Ser
   1085 1090 1095
   Ile Leu Ala Ser Phe Ala Ser Ser Asp Asp Pro Tyr Gln Gly Pro
   1100 1105 1110
   Arg Asp Ile Ile Ser Pro Glu Leu Met Ala Asp Tyr Leu Gln Pro
   1115 1120 1125
   Lys Leu Leu Gly Ile Leu Ala Phe Phe Asn Met Gln Leu Leu Ser
   1130 1135 1140
   Ser Ser Val Gly Ile Glu Asp Lys Lys Met Ala Leu Asn Ser Leu
   1145 1150 1155
   Met Ser Leu Met Lys Leu Met Gly Pro Lys His Val Ser Ser Val
   1160 1165 1170
   Arg Val Lys Met Met Thr Thr Leu Arg Thr Gly Leu Arg Phe Lys
   1175 1180 1185
   Asp Asp Phe Pro Glu Leu Cys Cys Arg Ala Trp Asp Cys Phe Val
   1190 1195 1200
   Arg Cys Leu Asp His Ala Cys Leu Gly Ser Leu Leu Ser His Val
   1205 1210 1215
   Ile Val Ala Leu Leu Pro Leu Ile His Ile Gln Pro Lys Glu Thr
   1220 1225 1230
   Ala Ala Ile Phe His Tyr Leu Ile Ile Glu Asn Arg Asp Ala Val
   1235 1240 1245
   Gln Asp Phe Leu His Glu Ile Tyr Phe Leu Pro Asp His Pro Glu
   1250 1255 1260
   Leu Lys Lys Ile Lys Ala Val Leu Gln Glu Tyr Arg Lys Glu Thr
   1265 1270 1275
   Ser Glu Ser Thr Asp Leu Gln Thr Thr Leu Gln Leu Ser Met Lys
   1280 1285 1290
   Ala Ile Gln His Glu Asn Val Asp Val Arg Ile His Ala Leu Thr
   1295 1300 1305
   Ser Leu Lys Glu Thr Leu Tyr Lys Asn Gln Glu Lys Leu Ile Lys
   1310 1315 1320
   Tyr Ala Thr Asp Ser Glu Thr Val Glu Pro Ile Ile Ser Gln Leu
   1325 1330 1335
   Val Thr Val Leu Leu Lys Gly Cys Gln Asp Ala Asn Ser Gln Ala
   1340 1345 1350
   Arg Leu Leu Cys Gly Glu Cys Leu Gly Glu Leu Gly Ala Ile Asp
   1355 1360 1365
   Pro Gly Arg Leu Asp Phe Ser Thr Thr Glu Thr Gln Gly Lys Asp
   1370 1375 1380
   Phe Thr Phe Val Thr Gly Val Glu Asp Ser Ser Phe Ala Tyr Gly
   1385 1390 1395
   Leu Leu Met Glu Leu Thr Arg Ala Tyr Leu Ala Tyr Ala Asp Asn
   1400 1405 1410
   Ser Arg Ala Gln Asp Ser Ala Ala Tyr Ala Ile Gln Glu Leu Leu
   1415 1420 1425
   Ser Ile Tyr Asp Cys Arg Glu Met Glu Thr Asn Gly Pro Gly His
   1430 1435 1440
   Gln Leu Trp Arg Arg Phe Pro Glu His Val Arg Glu Ile Leu Glu
   1445 1450 1455
   Pro His Leu Asn Thr Arg Tyr Lys Ser Ser Gln Lys Ser Thr Asp
   1460 1465 1470
   Trp Ser Gly Val Lys Lys Pro Ile Tyr Leu Ser Lys Leu Gly Ser
   1475 1480 1485
   Asn Phe Ala Glu Trp Ser Ala Ser Trp Ala Gly Tyr Leu Ile Thr
   1490 1495 1500
   Lys Val Arg His Asp Leu Ala Ser Lys Ile Phe Thr Cys Cys Ser
   1505 1510 1515
   Ile Met Met Lys His Asp Phe Lys Val Thr Ile Tyr Leu Leu Pro
   1520 1525 1530
   His Ile Leu Val Tyr Val Leu Leu Gly Cys Asn Gln Glu Asp Gln
   1535 1540 1545
   Gln Glu Val Tyr Ala Glu Ile Met Ala Val Leu Lys His Asp Asp
   1550 1555 1560
   Gln His Thr Ile Asn Thr Gln Asp Ile Ala Ser Asp Leu Cys Gln
   1565 1570 1575
   Leu Ser Thr Gln Thr Val Phe Ser Met Leu Asp His Leu Thr Gln
   1580 1585 1590
   Trp Ala Arg His Lys Phe Gln Ala Leu Lys Ala Glu Lys Cys Pro
   1595 1600 1605
   His Ser Lys Ser Asn Arg Asn Lys Val Asp Ser Met Val Ser Thr
   1610 1615 1620
   Val Asp Tyr Glu Asp Tyr Gln Ser Val Thr Arg Phe Leu Asp Leu
   1625 1630 1635
   Ile Pro Gln Asp Thr Leu Ala Val Ala Ser Phe Arg Ser Lys Ala
   1640 1645 1650
   Tyr Thr Arg Ala Val Met His Phe Glu Ser Phe Ile Thr Glu Lys
   1655 1660 1665
   Lys Gln Asn Ile Gln Glu His Leu Gly Phe Leu Gln Lys Leu Tyr
   1670 1675 1680
   Ala Ala Met His Glu Pro Asp Gly Val Ala Gly Val Ser Ala Ile
   1685 1690 1695
   Arg Lys Ala Glu Pro Ser Leu Lys Glu Gln Ile Leu Glu His Glu
   1700 1705 1710
   Ser Leu Gly Leu Leu Arg Asp Ala Thr Ala Cys Tyr Asp Arg Ala
   1715 1720 1725
   Ile Gln Leu Glu Pro Asp Gln Ile Ile His Tyr His Gly Val Val
   1730 1735 1740
   Lys Ser Met Leu Gly Leu Gly Gln Leu Ser Thr Val Ile Thr Gln
   1745 1750 1755
   Val Asn Gly Val His Ala Asn Arg Ser Glu Trp Thr Asp Glu Leu
   1760 1765 1770
   Asn Thr Tyr Arg Val Glu Ala Ala Trp Lys Leu Ser Gln Trp Asp
   1775 1780 1785
   Leu Val Glu Asn Tyr Leu Ala Ala Asp Gly Lys Ser Thr Thr Trp
   1790 1795 1800
   Ser Val Arg Leu Gly Gln Leu Leu Leu Ser Ala Lys Lys Arg Asp
   1805 1810 1815
   Ile Thr Ala Phe Tyr Asp Ser Leu Lys Leu Val Arg Ala Glu Gln
   1820 1825 1830
   Ile Val Pro Leu Ser Ala Ala Ser Phe Glu Arg Gly Ser Tyr Gln
   1835 1840 1845
   Arg Gly Tyr Glu Tyr Ile Val Arg Leu His Met Leu Cys Glu Leu
   1850 1855 1860
   Glu His Ser Ile Lys Pro Leu Phe Gln His Ser Pro Gly Asp Ser
   1865 1870 1875
   Ser Gln Glu Asp Ser Leu Asn Trp Val Ala Arg Leu Glu Met Thr
   1880 1885 1890
   Gln Asn Ser Tyr Arg Ala Lys Glu Pro Ile Leu Ala Leu Arg Arg
   1895 1900 1905
   Ala Leu Leu Ser Leu Asn Lys Arg Pro Asp Tyr Asn Glu Met Val
   1910 1915 1920
   Gly Glu Cys Trp Leu Gln Ser Ala Arg Val Ala Arg Lys Ala Gly
   1925 1930 1935
   His His Gln Thr Ala Tyr Asn Ala Leu Leu Asn Ala Gly Glu Ser
   1940 1945 1950
   Arg Leu Ala Glu Leu Tyr Val Glu Arg Ala Lys Trp Leu Trp Ser
   1955 1960 1965
   Lys Gly Asp Val His Gln Ala Leu Ile Val Leu Gln Lys Gly Val
   1970 1975 1980
   Glu Leu Cys Phe Pro Glu Asn Glu Thr Pro Pro Glu Gly Lys Asn
   1985 1990 1995
   Met Leu Ile His Gly Arg Ala Met Leu Leu Val Gly Arg Phe Met
   2000 2005 2010
   Glu Glu Thr Ala Asn Phe Glu Ser Asn Ala Ile Met Lys Lys Tyr
   2015 2020 2025
   Lys Asp Val Thr Ala Cys Leu Pro Glu Trp Glu Asp Gly His Phe
   2030 2035 2040
   Tyr Leu Ala Lys Tyr Tyr Asp Lys Leu Met Pro Met Val Thr Asp
   2045 2050 2055
   Asn Lys Met Glu Lys Gln Gly Asp Leu Ile Arg Tyr Ile Val Leu
   2060 2065 2070
   His Phe Gly Arg Ser Leu Gln Tyr Gly Asn Gln Phe Ile Tyr Gln
   2075 2080 2085
   Ser Met Pro Arg Met Leu Thr Leu Trp Leu Asp Tyr Gly Thr Lys
   2090 2095 2100
   Ala Tyr Glu Trp Glu Lys Ala Gly Arg Ser Asp Arg Val Gln Met
   2105 2110 2115
   Arg Asn Asp Leu Gly Lys Ile Asn Lys Val Ile Thr Glu His Thr
   2120 2125 2130
   Asn Tyr Leu Ala Pro Tyr Gln Phe Leu Thr Ala Phe Ser Gln Leu
   2135 2140 2145
   Ile Ser Arg Ile Cys His Ser His Asp Glu Val Phe Val Val Leu
   2150 2155 2160
   Met Glu Ile Ile Ala Lys Val Phe Leu Ala Tyr Pro Gln Gln Ala
   2165 2170 2175
   Met Trp Met Met Thr Ala Val Ser Lys Ser Ser Tyr Pro Met Arg
   2180 2185 2190
   Val Asn Arg Cys Lys Glu Ile Leu Asn Lys Ala Ile His Met Lys
   2195 2200 2205
   Lys Ser Leu Glu Lys Phe Val Gly Asp Ala Thr Arg Leu Thr Asp
   2210 2215 2220
   Lys Leu Leu Glu Leu Cys Asn Lys Pro Val Asp Gly Ser Ser Ser
   2225 2230 2235
   Thr Leu Ser Met Ser Thr His Phe Lys Met Leu Lys Lys Leu Val
   2240 2245 2250
   Glu Glu Ala Thr Phe Ser Glu Ile Leu Ile Pro Leu Gln Ser Val
   2255 2260 2265
   Met Ile Pro Thr Leu Pro Ser Ile Leu Gly Thr His Ala Asn His
   2270 2275 2280
   Ala Ser His Glu Pro Phe Pro Gly His Trp Ala Tyr Ile Ala Gly
   2285 2290 2295
   Phe Asp Asp Met Val Glu Ile Leu Ala Ser Leu Gln Lys Pro Lys
   2300 2305 2310
   Lys Ile Ser Leu Lys Gly Ser Asp Gly Lys Phe Tyr Ile Met Met
   2315 2320 2325
   Cys Lys Pro Lys Asp Asp Leu Arg Lys Asp Cys Arg Leu Met Glu
   2330 2335 2340
   Phe Asn Ser Leu Ile Asn Lys Cys Leu Arg Lys Asp Ala Glu Ser
   2345 2350 2355
   Arg Arg Arg Glu Leu His Ile Arg Thr Tyr Ala Val Ile Pro Leu
   2360 2365 2370
   Asn Asp Glu Cys Gly Ile Ile Glu Trp Val Asn Asn Thr Ala Gly
   2375 2380 2385
   Leu Arg Pro Ile Leu Thr Lys Leu Tyr Lys Glu Lys Gly Val Tyr
   2390 2395 2400
   Met Thr Gly Lys Glu Leu Arg Gln Cys Met Leu Pro Lys Ser Ala
   2405 2410 2415
   Ala Leu Ser Glu Lys Leu Lys Val Phe Arg Glu Phe Leu Leu Pro
   2420 2425 2430
   Arg His Pro Pro Ile Phe His Glu Trp Phe Leu Arg Thr Phe Pro
   2435 2440 2445
   Asp Pro Thr Ser Trp Tyr Ser Ser Arg Ser Ala Tyr Cys Arg Ser
   2450 2455 2460
   Thr Ala Val Met Ser Met Val Gly Tyr Ile Leu Gly Leu Gly Asp
   2465 2470 2475
   Arg His Gly Glu Asn Ile Leu Phe Asp Ser Leu Thr Gly Glu Cys
   2480 2485 2490
   Val His Val Asp Phe Asn Cys Leu Phe Asn Lys Gly Glu Thr Phe
   2495 2500 2505
   Glu Val Pro Glu Ile Val Pro Phe Arg Leu Thr His Asn Met Val
   2510 2515 2520
   Asn Gly Met Gly Pro Met Gly Thr Glu Gly Leu Phe Arg Arg Ala
   2525 2530 2535
   Cys Glu Val Thr Met Arg Leu Met Arg Asp Gln Arg Glu Pro Leu
   2540 2545 2550
   Met Ser Val Leu Lys Thr Phe Leu His Asp Pro Leu Val Glu Trp
   2555 2560 2565
   Ser Lys Pro Val Lys Gly His Ser Lys Ala Pro Leu Asn Glu Thr
   2570 2575 2580
   Gly Glu Val Val Asn Glu Lys Ala Lys Thr His Val Leu Asp Ile
   2585 2590 2595
   Glu Gln Arg Leu Gln Gly Val Ile Lys Thr Arg Asn Arg Val Thr
   2600 2605 2610
   Gly Leu Pro Leu Ser Ile Glu Gly His Val His Tyr Leu Ile Gln
   2615 2620 2625
   Glu Ala Thr Asp Glu Asn Leu Leu Cys Gln Met Tyr Leu Gly Trp
   2630 2635 2640
   Thr Pro Tyr Met
<210> 42
   <211> 627
   <212> PRT
   <213> Homo sapiens
<400> 42
   Met Cys Val Pro Arg Ser Gly Leu Ile Leu Ser Cys Ile Cys Leu
   1 5 10 15
   Tyr Cys Gly Val Leu Leu Pro Val Leu Leu Pro Asn Leu Pro Phe
   20 25 30
   Leu Thr Cys Leu Ser Met Ser Thr Leu Glu Ser Val Thr Tyr Leu
   35 40 45
   Pro Glu Lys Gly Leu Tyr Cys Gln Arg Leu Pro Ser Ser Arg Thr
   50 55 60
   His Gly Gly Thr Glu Ser Leu Lys Gly Lys Asn Thr Glu Asn Met
   65 70 75
   Gly Phe Tyr Gly Thr Leu Lys Met Ile Phe Tyr Lys Met Lys Arg
   80 85 90
   Lys Leu Asp His Gly Ser Glu Val Arg Ser Phe Ser Leu Gly Lys
   95 100 105
   Lys Pro Cys Lys Val Ser Glu Tyr Thr Ser Thr Thr Gly Leu Val
   110 115 120
   Pro Cys Ser Ala Thr Pro Thr Thr Phe Gly Asp Leu Arg Ala Ala
   125 130 135
   Asn Gly Gln Gly Gln Gln Arg Arg Arg Ile Thr Ser Val Gln Pro
   140 145 150
   Pro Thr Gly Leu Gln Glu Trp Leu Lys Met Phe Gln Ser Trp Ser
   155 160 165
   Gly Pro Glu Lys Leu Leu Ala Leu Asp Glu Leu Ile Asp Ser Cys
   170 175 180
   Glu Pro Thr Gln Val Lys His Met Met Gln Val Ile Glu Pro Gln
   185 190 195
   Phe Gln Arg Asp Phe Ile Ser Leu Leu Pro Lys Glu Leu Ala Leu
   200 205 210
   Tyr Val Leu Ser Phe Leu Glu Pro Lys Asp Leu Leu Gln Ala Ala
   215 220 225
   Gln Thr Cys Arg Tyr Trp Arg Ile Leu Ala Glu Asp Asn Leu Leu
   230 235 240
   Trp Arg Glu Lys Cys Lys Glu Glu Gly Ile Asp Glu Pro Leu His
   245 250 255
   Ile Lys Arg Arg Lys Val Ile Lys Pro Gly Phe Ile His Ser Pro
   260 265 270
   Trp Lys Ser Ala Tyr Ile Arg Gln His Arg Ile Asp Thr Asn Trp
   275 280 285
   Arg Arg Gly Glu Leu Lys Ser Pro Lys Val Leu Lys Gly His Asp
   290 295 300
   Asp His Val Ile Thr Cys Leu Gln Phe Cys Gly Asn Arg Ile Val
   305 310 315
   Ser Gly Ser Asp Asp Asn Thr Leu Lys Val Trp Ser Ala Val Thr
   320 325 330
   Gly Lys Cys Leu Arg Thr Leu Val Gly His Thr Gly Gly Val Trp
   335 340 345
   Ser Ser Gln Met Arg Asp Asn Ile Ile Ile Ser Gly Ser Thr Asp
   350 355 360
   Arg Thr Leu Lys Val Trp Asn Ala Glu Thr Gly Glu Cys Ile His
   365 370 375
   Thr Leu Tyr Gly His Thr Ser Thr Val Arg Cys Met His Leu His
   380 385 390
   Glu Lys Arg Val Val Ser Gly Ser Arg Asp Ala Thr Leu Arg Val
   395 400 405
   Trp Asp Ile Glu Thr Gly Gln Cys Leu His Val Leu Met Gly His
   410 415 420
   Val Ala Ala Val Arg Cys Val Gln Tyr Asp Gly Arg Arg Val Val
   425 430 435
   Ser Gly Ala Tyr Asp Phe Met Val Lys Val Trp Asp Pro Glu Thr
   440 445 450
   Glu Thr Cys Leu His Thr Leu Gln Gly His Thr Asn Arg Val Tyr
   455 460 465
   Ser Leu Gln Phe Asp Gly Ile His Val Val Ser Gly Ser Leu Asp
   470 475 480
   Thr Ser Ile Arg Val Trp Asp Val Glu Thr Gly Asn Cys Ile His
   485 490 495
   Thr Leu Thr Gly His Gln Ser Leu Thr Ser Gly Met Glu Leu Lys
   500 505 510
   Asp Asn Ile Leu Val Ser Gly Asn Ala Asp Ser Thr Val Lys Ile
   515 520 525
   Trp Asp Ile Lys Thr Gly Gln Cys Leu Gln Thr Leu Gln Gly Pro
   530 535 540
   Asn Lys His Gln Ser Ala Val Thr Cys Leu Gln Phe Asn Lys Asn
   545 550 555
   Phe Val Ile Thr Ser Ser Asp Asp Gly Thr Val Lys Leu Trp Asp
   560 565 570
   Leu Lys Thr Gly Glu Phe Ile Arg Asn Leu Val Thr Leu Glu Ser
   575 580 585
   Gly Gly Ser Gly Gly Val Val Trp Arg Ile Arg Ala Ser Asn Thr
   590 595 600
   Lys Leu Val Cys Ala Val Gly Ser Arg Asn Gly Thr Glu Glu Thr
   605 610 615
   Lys Leu Leu Val Leu Asp Phe Asp Val Asp Met Lys
   620 625
<210> 43
   <211> 1338
   <212> PRT
   <213> Homo sapiens
<400> 43
   Met Val Ser Tyr Trp Asp Thr Gly Val Leu Leu Cys Ala Leu Leu
   1 5 10 15
   Ser Cys Leu Leu Leu Thr Gly Ser Ser Ser Gly Ser Lys Leu Lys
   20 25 30
   Asp Pro Glu Leu Ser Leu Lys Gly Thr Gln His Ile Met Gln Ala
   35 40 45
   Gly Gln Thr Leu His Leu Gln Cys Arg Gly Glu Ala Ala His Lys
   50 55 60
   Trp Ser Leu Pro Glu Met Val Ser Lys Glu Ser Glu Arg Leu Ser
   65 70 75
   Ile Thr Lys Ser Ala Cys Gly Arg Asn Gly Lys Gln Phe Cys Ser
   80 85 90
   Thr Leu Thr Leu Asn Thr Ala Gln Ala Asn His Thr Gly Phe Tyr
   95 100 105
   Ser Cys Lys Tyr Leu Ala Val Pro Thr Ser Lys Lys Lys Glu Thr
   110 115 120
   Glu Ser Ala Ile Tyr Ile Phe Ile Ser Asp Thr Gly Arg Pro Phe
   125 130 135
   Val Glu Met Tyr Ser Glu Ile Pro Glu Ile Ile His Met Thr Glu
   140 145 150
   Gly Arg Glu Leu Val Ile Pro Cys Arg Val Thr Ser Pro Asn Ile
   155 160 165
   Thr Val Thr Leu Lys Lys Phe Pro Leu Asp Thr Leu Ile Pro Asp
   170 175 180
   Gly Lys Arg Ile Ile Trp Asp Ser Arg Lys Gly Phe Ile Ile Ser
   185 190 195
   Asn Ala Thr Tyr Lys Glu Ile Gly Leu Leu Thr Cys Glu Ala Thr
   200 205 210
   Val Asn Gly His Leu Tyr Lys Thr Asn Tyr Leu Thr His Arg Gln
   215 220 225
   Thr Asn Thr Ile Ile Asp Val Gln Ile Ser Thr Pro Arg Pro Val
   230 235 240
   Lys Leu Leu Arg Gly His Thr Leu Val Leu Asn Cys Thr Ala Thr
   245 250 255
   Thr Pro Leu Asn Thr Arg Val Gln Met Thr Trp Ser Tyr Pro Asp
   260 265 270
   Glu Lys Asn Lys Arg Ala Ser Val Arg Arg Arg Ile Asp Gln Ser
   275 280 285
   Asn Ser His Ala Asn Ile Phe Tyr Ser Val Leu Thr Ile Asp Lys
   290 295 300
   Met Gln Asn Lys Asp Lys Gly Leu Tyr Thr Cys Arg Val Arg Ser
   305 310 315
   Gly Pro Ser Phe Lys Ser Val Asn Thr Ser Val His Ile Tyr Asp
   320 325 330
   Lys Ala Phe Ile Thr Val Lys His Arg Lys Gln Gln Val Leu Glu
   335 340 345
   Thr Val Ala Gly Lys Arg Ser Tyr Arg Leu Ser Met Lys Val Lys
   350 355 360
   Ala Phe Pro Ser Pro Glu Val Val Trp Leu Lys Asp Gly Leu Pro
   365 370 375
   Ala Thr Glu Lys Ser Ala Arg Tyr Leu Thr Arg Gly Tyr Ser Leu
   380 385 390
   Ile Ile Lys Asp Val Thr Glu Glu Asp Ala Gly Asn Tyr Thr Ile
   395 400 405
   Leu Leu Ser Ile Lys Gln Ser Asn Val Phe Lys Asn Leu Thr Ala
   410 415 420
   Thr Leu Ile Val Asn Val Lys Pro Gln Ile Tyr Glu Lys Ala Val
   425 430 435
   Ser Ser Phe Pro Asp Pro Ala Leu Tyr Pro Leu Gly Ser Arg Gln
   440 445 450
   Ile Leu Thr Cys Thr Ala Tyr Gly Ile Pro Gln Pro Thr Ile Lys
   455 460 465
   Trp Phe Trp His Pro Cys Asn His Asn His Ser Glu Ala Arg Cys
   470 475 480
   Asp Phe Cys Ser Asn Asn Glu Glu Ser Phe Ile Leu Asp Ala Asp
   485 490 495
   Ser Asn Met Gly Asn Arg Ile Glu Ser Ile Thr Gln Arg Met Ala
   500 505 510
   Ile Ile Glu Gly Lys Asn Lys Met Ala Ser Thr Leu Val Val Ala
   515 520 525
   Asp Ser Arg Ile Ser Gly Ile Tyr Ile Cys Ile Ala Ser Asn Lys
   530 535 540
   Val Gly Thr Val Gly Arg Asn Ile Ser Phe Tyr Ile Thr Asp Val
   545 550 555
   Pro Asn Gly Phe His Val Asn Leu Glu Lys Met Pro Thr Glu Gly
   560 565 570
   Glu Asp Leu Lys Leu Ser Cys Thr Val Asn Lys Phe Leu Tyr Arg
   575 580 585
   Asp Val Thr Trp Ile Leu Leu Arg Thr Val Asn Asn Arg Thr Met
   590 595 600
   His Tyr Ser Ile Ser Lys Gln Lys Met Ala Ile Thr Lys Glu His
   605 610 615
   Ser Ile Thr Leu Asn Leu Thr Ile Met Asn Val Ser Leu Gln Asp
   620 625 630
   Ser Gly Thr Tyr Ala Cys Arg Ala Arg Asn Val Tyr Thr Gly Glu
   635 640 645
   Glu Ile Leu Gln Lys Lys Glu Ile Thr Ile Arg Asp Gln Glu Ala
   650 655 660
   Pro Tyr Leu Leu Arg Asn Leu Ser Asp His Thr Val Ala Ile Ser
   665 670 675
   Ser Ser Thr Thr Leu Asp Cys His Ala Asn Gly Val Pro Glu Pro
   680 685 690
   Gln Ile Thr Trp Phe Lys Asn Asn His Lys Ile Gln Gln Glu Pro
   695 700 705
   Gly Ile Ile Leu Gly Pro Gly Ser Ser Thr Leu Phe Ile Glu Arg
   710 715 720
   Val Thr Glu Glu Asp Glu Gly Val Tyr His Cys Lys Ala Thr Asn
   725 730 735
   Gln Lys Gly Ser Val Glu Ser Ser Ala Tyr Leu Thr Val Gln Gly
   740 745 750
   Thr Ser Asp Lys Ser Asn Leu Glu Leu Ile Thr Leu Thr Cys Thr
   755 760 765
   Cys Val Ala Ala Thr Leu Phe Trp Leu Leu Leu Thr Leu Phe Ile
                   770                 775                 780
   Arg Lys Met Lys Arg Ser Ser Ser Glu Ile Lys Thr Asp Tyr Leu
                   785                 790                 795
   Ser Ile Ile Met Asp Pro Asp Glu Val Pro Leu Asp Glu Gln Cys
                   800                 805                 810
   Glu Arg Leu Pro Tyr Asp Ala Ser Lys Trp Glu Phe Ala Arg Glu
                   815                 820                 825
   Arg Leu Lys Leu Gly Lys Ser Leu Gly Arg Gly Ala Phe Gly Lys
                   830                 835                 840
   Val Val Gln Ala Ser Ala Phe Gly Ile Lys Lys Ser Pro Thr Cys
                   845                 850                 855
   Arg Thr Val Ala Val Lys Met Leu Lys Glu Gly Ala Thr Ala Ser
                   860                 865                 870
   Glu Tyr Lys Ala Leu Met Thr Glu Leu Lys Ile Leu Thr His Ile
                   875                 880                 885
   Gly His His Leu Asn Val Val Asn Leu Leu Gly Ala Cys Thr Lys
                   890                 895                 900
   Gln Gly Gly Pro Leu Met Val Ile Val Glu Tyr Cys Lys Tyr Gly
                   905                 910                 915
   Asn Leu Ser Asn Tyr Leu Lys Ser Lys Arg Asp Leu Phe Phe Leu
                   920                 925                 930
   Asn Lys Asp Ala Ala Leu His Met Glu Pro Lys Lys Glu Lys Met
                   935                 940                 945
   Glu Pro Gly Leu Glu Gln Gly Lys Lys Pro Arg Leu Asp Ser Val
                   950                 955                 960
   Thr Ser Ser Glu Ser Phe Ala Ser Ser Gly Phe Gln Glu Asp Lys
                   965                 970                 975
   Ser Leu Ser Asp Val Glu Glu Glu Glu Asp Ser Asp Gly Phe Tyr
                   980                 985                 990
   Lys Glu Pro Ile Thr Met Glu Asp Leu Ile Ser Tyr Ser Phe Gln
                   995                 1000                1005
   Val Ala Arg Gly Met Glu Phe Leu Ser Ser Arg Lys Cys Ile His
                   1010                1015                1020
   Arg Asp Leu Ala Ala Arg Asn Ile Leu Leu Ser Glu Asn Asn Val
                   1025                1030                1035
   Val Lys Ile Cys Asp Phe Gly Leu Ala Arg Asp Ile Tyr Lys Asn
                   1040                1045                1050
   Pro Asp Tyr Val Arg Lys Gly Asp Thr Arg Leu Pro Leu Lys Trp
                   1055                1060                1065
   Met Ala Pro Glu Ser Ile Phe Asp Lys Ile Tyr Ser Thr Lys Ser
                   1070                1075                1080
   Asp Val Trp Ser Tyr Gly Val Leu Leu Trp Glu Ile Phe Ser Leu
                   1085                1090                1095
   Gly Gly Ser Pro Tyr Pro Gly Val Gln Met Asp Glu Asp Phe Cys
                   1100                1105                1110
   Ser Arg Leu Arg Glu Gly Met Arg Met Arg Ala Pro Glu Tyr Ser
   1115 1120 1125
   Thr Pro Glu Ile Tyr Gln Ile Met Leu Asp Cys Trp His Arg Asp
   1130 1135 1140
   Pro Lys Glu Arg Pro Arg Phe Ala Glu Leu Val Glu Lys Leu Gly
   1145 1150 1155
   Asp Leu Leu Gln Ala Asn Val Gln Gln Asp Gly Lys Asp Tyr Ile
   1160 1165 1170
   Pro Ile Asn Ala Ile Leu Thr Gly Asn Ser Gly Phe Thr Tyr Ser
   1175 1180 1185
   Thr Pro Ala Phe Ser Glu Asp Phe Phe Lys Glu Ser Ile Ser Ala
   1190 1195 1200
   Pro Lys Phe Asn Ser Gly Ser Ser Asp Asp Val Arg Tyr Val Asn
   1205 1210 1215
   Ala Phe Lys Phe Met Ser Leu Glu Arg Ile Lys Thr Phe Glu Glu
   1220 1225 1230
   Leu Leu Pro Asn Ala Thr Ser Met Phe Asp Asp Tyr Gln Gly Asp
   1235 1240 1245
   Ser Ser Thr Leu Leu Ala Ser Pro Met Leu Lys Arg Phe Thr Trp
   1250 1255 1260
   Thr Asp Ser Lys Pro Lys Ala Ser Leu Lys Ile Asp Leu Arg Val
   1265 1270 1275
   Thr Ser Lys Ser Lys Glu Ser Gly Leu Ser Asp Val Ser Arg Pro
   1280 1285 1290
   Ser Phe Cys His Ser Ser Cys Gly His Val Ser Glu Gly Lys Arg
   1295 1300 1305
   Arg Phe Thr Tyr Asp His Ala Glu Leu Glu Arg Lys Ile Ala Cys
   1310 1315 1320
   Cys Ser Pro Pro Pro Asp Tyr Asn Ser Val Val Leu Tyr Ser Thr
   1325 1330 1335
   Pro Pro Ile
<210> 44
   <211> 993
   <212> PRT
   <213> Homo sapiens
<400> 44
   Met Pro Ala Leu Ala Arg Asp Gly Gly Gln Leu Pro Leu Leu Val
   1 5 10 15
   Val Phe Ser Ala Met Ile Phe Gly Thr Ile Thr Asn Gln Asp Leu
   20 25 30
   Pro Val Ile Lys Cys Val Leu Ile Asn His Lys Asn Asn Asp Ser
   35 40 45
   Ser Val Gly Lys Ser Ser Ser Tyr Pro Met Val Ser Glu Ser Pro
   50 55 60
   Glu Asp Leu Gly Cys Ala Leu Arg Pro Gln Ser Ser Gly Thr Val
   65 70 75
   Tyr Glu Ala Ala Ala Val Glu Val Asp Val Ser Ala Ser Ile Thr
   80 85 90
   Leu Gln Val Leu Val Asp Ala Pro Gly Asn Ile Ser Cys Leu Trp
   95 100 105
   Val Phe Lys His Ser Ser Leu Asn Cys Gln Pro His Phe Asp Leu
   110 115 120
   Gln Asn Arg Gly Val Val Ser Met Val Ile Leu Lys Met Thr Glu
   125 130 135
   Thr Gln Ala Gly Glu Tyr Leu Leu Phe Ile Gln Ser Glu Ala Thr
   140 145 150
   Asn Tyr Thr Ile Leu Phe Thr Val Ser Ile Arg Asn Thr Leu Leu
   155 160 165
   Tyr Thr Leu Arg Arg Pro Tyr Phe Arg Lys Met Glu Asn Gln Asp
   170 175 180
   Ala Leu Val Cys Ile Ser Glu Ser Val Pro Glu Pro Ile Val Glu
   185 190 195
   Trp Val Leu Cys Asp Ser Gln Gly Glu Ser Cys Lys Glu Glu Ser
   200 205 210
   Pro Ala Val Val Lys Lys Glu Glu Lys Val Leu His Glu Leu Phe
   215 220 225
   Gly Thr Asp Ile Arg Cys Cys Ala Arg Asn Glu Leu Gly Arg Glu
   230 235 240
   Cys Thr Arg Leu Phe Thr Ile Asp Leu Asn Gln Thr Pro Gln Thr
   245 250 255
   Thr Leu Pro Gln Leu Phe Leu Lys Val Gly Glu Pro Leu Trp Ile
   260 265 270
   Arg Cys Lys Ala Val His Val Asn His Gly Phe Gly Leu Thr Trp
   275 280 285
   Glu Leu Glu Asn Lys Ala Leu Glu Glu Gly Asn Tyr Phe Glu Met
   290 295 300
   Ser Thr Tyr Ser Thr Asn Arg Thr Met Ile Arg Ile Leu Phe Ala
   305 310 315
   Phe Val Ser Ser Val Ala Arg Asn Asp Thr Gly Tyr Tyr Thr Cys
   320 325 330
   Ser Ser Ser Lys His Pro Ser Gln Ser Ala Leu Val Thr Ile Val
   335 340 345
   Glu Lys Gly Phe Ile Asn Ala Thr Asn Ser Ser Glu Asp Tyr Glu
   350 355 360
   Ile Asp Gln Tyr Glu Glu Phe Cys Phe Ser Val Arg Phe Lys Ala
   365 370 375
   Tyr Pro Gln Ile Arg Cys Thr Trp Thr Phe Ser Arg Lys Ser Phe
   380 385 390
   Pro Cys Glu Gln Lys Gly Leu Asp Asn Gly Tyr Ser Ile Ser Lys
   395 400 405
   Phe Cys Asn His Lys His Gln Pro Gly Glu Tyr Ile Phe His Ala
   410 415 420
   Glu Asn Asp Asp Ala Gln Phe Thr Lys Met Phe Thr Leu Asn Ile
   425 430 435
   Arg Arg Lys Pro Gln Val Leu Ala Glu Ala Ser Ala Ser Gln Ala
   440 445 450
   Ser Cys Phe Ser Asp Gly Tyr Pro Leu Pro Ser Trp Thr Trp Lys
   455 460 465
   Lys Cys Ser Asp Lys Ser Pro Asn Cys Thr Glu Glu Ile Thr Glu
   470 475 480
   Gly Val Trp Asn Arg Lys Ala Asn Arg Lys Val Phe Gly Gln Trp
   485 490 495
   Val Ser Ser Ser Thr Leu Asn Met Ser Glu Ala Ile Lys Gly Phe
   500 505 510
   Leu Val Lys Cys Cys Ala Tyr Asn Ser Leu Gly Thr Ser Cys Glu
   515 520 525
   Thr Ile Leu Leu Asn Ser Pro Gly Pro Phe Pro Phe Ile Gln Asp
   530 535 540
   Asn Ile Ser Phe Tyr Ala Thr Ile Gly Val Cys Leu Leu Phe Ile
   545 550 555
   Val Val Leu Thr Leu Leu Ile Cys His Lys Tyr Lys Lys Gln Phe
   560 565 570
   Arg Tyr Glu Ser Gln Leu Gln Met Val Gln Val Thr Gly Ser Ser
   575 580 585
   Asp Asn Glu Tyr Phe Tyr Val Asp Phe Arg Glu Tyr Glu Tyr Asp
   590 595 600
   Leu Lys Trp Glu Phe Pro Arg Glu Asn Leu Glu Phe Gly Lys Val
   605 610 615
   Leu Gly Ser Gly Ala Phe Gly Lys Val Met Asn Ala Thr Ala Tyr
   620 625 630
   Gly Ile Ser Lys Thr Gly Val Ser Ile Gln Val Ala Val Lys Met
   635 640 645
   Leu Lys Glu Lys Ala Asp Ser Ser Glu Arg Glu Ala Leu Met Ser
   650 655 660
   Glu Leu Lys Met Met Thr Gln Leu Gly Ser His Glu Asn Ile Val
   665 670 675
   Asn Leu Leu Gly Ala Cys Thr Leu Ser Gly Pro Ile Tyr Leu Ile
   680 685 690
   Phe Glu Tyr Cys Cys Tyr Gly Asp Leu Leu Asn Tyr Leu Arg Ser
   695 700 705
   Lys Arg Glu Lys Phe His Arg Thr Trp Thr Glu Ile Phe Lys Glu
   710 715 720
   His Asn Phe Ser Phe Tyr Pro Thr Phe Gln Ser His Pro Asn Ser
   725 730 735
   Ser Met Pro Gly Ser Arg Glu Val Gln Ile His Pro Asp Ser Asp
   740 745 750
   Gln Ile Ser Gly Leu His Gly Asn Ser Phe His Ser Glu Asp Glu
   755 760 765
   Ile Glu Tyr Glu Asn Gln Lys Arg Leu Glu Glu Glu Glu Asp Leu
   770 775 780
   Asn Val Leu Thr Phe Glu Asp Leu Leu Cys Phe Ala Tyr Gln Val
   785 790 795
   Ala Lys Gly Met Glu Phe Leu Glu Phe Lys Ser Cys Val His Arg
   800 805 810
   Asp Leu Ala Ala Arg Asn Val Leu Val Thr His Gly Lys Val Val
   815 820 825
   Lys Ile Cys Asp Phe Gly Leu Ala Arg Asp Ile Met Ser Asp Ser
   830 835 840
   Asn Tyr Val Val Arg Gly Asn Ala Arg Leu Pro Val Lys Trp Met
   845 850 855
   Ala Pro Glu Ser Leu Phe Glu Gly Ile Tyr Thr Ile Lys Ser Asp
   860 865 870
   Val Trp Ser Tyr Gly Ile Leu Leu Trp Glu Ile Phe Ser Leu Gly
   875 880 885
   Val Asn Pro Tyr Pro Gly Ile Pro Val Asp Ala Asn Phe Tyr Lys
   890 895 900
   Leu Ile Gln Asn Gly Phe Lys Met Asp Gln Pro Phe Tyr Ala Thr
   905 910 915
   Glu Glu Ile Tyr Ile Ile Met Gln Ser Cys Trp Ala Phe Asp Ser
   920 925 930
   Arg Lys Arg Pro Ser Phe Pro Asn Leu Thr Ser Phe Leu Gly Cys
   935 940 945
   Gln Leu Ala Asp Ala Glu Glu Ala Met Tyr Gln Asn Val Asp Gly
   950 955 960
   Arg Val Ser Glu Cys Pro His Thr Tyr Gln Asn Arg Arg Pro Phe
   965 970 975
   Ser Arg Glu Met Asp Leu Gly Leu Leu Ser Pro Gln Ala Gln Val
   980 985 990
   Glu Asp Ser
<210> 45
   <211> 2551
   <212> PRT
   <213> Homo sapiens
<400> 45
   Met Leu Gly Thr Gly Pro Ala Ala Ala Thr Thr Ala Ala Thr Thr
   1               5                   10                  15
   Ser Ser Asn Val Ser Val Leu Gln Gln Phe Ala Ser Gly Leu Lys
                   20                  25                  30
   Ser Arg Asn Glu Glu Thr Arg Ala Lys Ala Ala Lys Glu Leu Gln
                   35                  40                  45
   His Tyr Val Thr Met Glu Leu Arg Glu Met Ser Gln Glu Glu Ser
                   50                  55                  60
   Thr Arg Phe Tyr Asp Gln Leu Asn His His Ile Phe Glu Leu Val
                   65                  70                  75
   Ser Ser Ser Asp Ala Asn Glu Arg Lys Gly Gly Ile Leu Ala Ile
   80 85 90
   Ala Ser Leu Ile Gly Val Glu Gly Gly Asn Ala Thr Arg Ile Gly
   95 100 105
   Arg Phe Ala Asn Tyr Leu Arg Asn Leu Leu Pro Ser Asn Asp Pro
   110 115 120
   Val Val Met Glu Met Ala Ser Lys Ala Ile Gly Arg Leu Ala Met
   125 130 135
   Ala Gly Asp Thr Phe Thr Ala Glu Tyr Val Glu Phe Glu Val Lys
   140 145 150
   Arg Ala Leu Glu Trp Leu Gly Ala Asp Arg Asn Glu Gly Arg Arg
   155 160 165
   His Ala Ala Val Leu Val Leu Arg Glu Leu Ala Ile Ser Val Pro
   170 175 180
   Thr Phe Phe Phe Gln Gln Val Gln Pro Phe Phe Asp Asn Ile Phe
   185 190 195
   Val Ala Val Trp Asp Pro Lys Gln Ala Ile Arg Glu Gly Ala Val
   200 205 210
   Ala Ala Leu Arg Ala Cys Leu Ile Leu Thr Thr Gln Arg Glu Pro
   215 220 225
   Lys Glu Met Gln Lys Pro Gln Trp Tyr Arg His Thr Phe Glu Glu
   230 235 240
   Ala Glu Lys Gly Phe Asp Glu Thr Leu Ala Lys Glu Lys Gly Met
   245 250 255
   Asn Arg Asp Asp Arg Ile His Gly Ala Leu Leu Ile Leu Asn Glu
   260 265 270
   Leu Val Arg Ile Ser Ser Met Glu Gly Glu Arg Leu Arg Glu Glu
   275 280 285
   Met Glu Glu Ile Thr Gln Gln Gln Leu Val His Asp Lys Tyr Cys
   290 295 300
   Lys Asp Leu Met Gly Phe Gly Thr Lys Pro Arg His Ile Thr Pro
   305 310 315
   Phe Thr Ser Phe Gln Ala Val Gln Pro Gln Gln Ser Asn Ala Leu
   320 325 330
   Val Gly Leu Leu Gly Tyr Ser Ser His Gln Gly Leu Met Gly Phe
   335 340 345
   Gly Thr Ser Pro Ser Pro Ala Lys Ser Thr Leu Val Glu Ser Arg
   350 355 360
   Cys Cys Arg Asp Leu Met Glu Glu Lys Phe Asp Gln Val Cys Gln
   365 370 375
   Trp Val Leu Lys Cys Arg Asn Ser Lys Asn Ser Leu Ile Gln Met
   380 385 390
   Thr Ile Leu Asn Leu Leu Pro Arg Leu Ala Ala Phe Arg Pro Ser
   395 400 405
   Ala Phe Thr Asp Thr Gln Tyr Leu Gln Asp Thr Met Asn His Val
   410 415 420
   Leu Ser Cys Val Lys Lys Glu Lys Glu Arg Thr Ala Ala Phe Gln
   425 430 435
   Ala Leu Gly Leu Leu Ser Val Ala Val Arg Ser Glu Phe Lys Val
   440 445 450
   Tyr Leu Pro Arg Val Leu Asp Ile Ile Arg Ala Ala Leu Pro Pro
   455 460 465
   Lys Asp Phe Ala His Lys Arg Gln Lys Ala Met Gln Val Asp Ala
   470 475 480
   Thr Val Phe Thr Cys Ile Ser Met Leu Ala Arg Ala Met Gly Pro
   485 490 495
   Gly Ile Gln Gln Asp Ile Lys Glu Leu Leu Glu Pro Met Leu Ala
   500 505 510
   Val Gly Leu Ser Pro Ala Leu Thr Ala Val Leu Tyr Asp Leu Ser
   515 520 525
   Arg Gln Ile Pro Gln Leu Lys Lys Asp Ile Gln Asp Gly Leu Leu
   530 535 540
   Lys Met Leu Ser Leu Val Leu Met His Lys Pro Leu Arg His Pro
   545 550 555
   Gly Met Pro Lys Gly Leu Ala His Gln Leu Ala Ser Pro Gly Leu
   560 565 570
   Thr Thr Leu Pro Glu Ala Ser Asp Val Gly Ser Ile Thr Leu Ala
   575 580 585
   Leu Arg Thr Leu Gly Ser Phe Glu Phe Glu Gly His Ser Leu Thr
   590 595 600
   Gln Phe Val Arg His Cys Ala Asp His Phe Leu Asn Ser Glu His
   605 610 615
   Lys Glu Ile Arg Met Glu Ala Ala Arg Thr Cys Ser Arg Leu Leu
   620 625 630
   Thr Pro Ser Ile His Leu Ile Ser Gly His Ala His Val Val Ser
   635 640 645
   Gln Thr Ala Val Gln Val Val Ala Asp Val Leu Ser Lys Leu Leu
   650 655 660
   Val Val Gly Ile Thr Asp Pro Asp Pro Asp Ile Arg Tyr Cys Val
   665 670 675
   Leu Ala Ser Leu Asp Glu Arg Phe Asp Ala His Leu Ala Gln Ala
   680 685 690
   Glu Asn Leu Gln Ala Leu Phe Val Ala Leu Asn Asp Gln Val Phe
   695 700 705
   Glu Ile Arg Glu Leu Ala Ile Cys Thr Val Gly Arg Leu Ser Ser
   710 715 720
   Met Asn Pro Ala Phe Val Met Pro Phe Leu Arg Lys Met Leu Ile
   725 730 735
   Gln Ile Leu Thr Glu Leu Glu His Ser Gly Ile Gly Arg Ile Lys
   740 745 750
   Glu Gln Ser Ala Arg Met Leu Gly His Leu Val Ser Asn Ala Pro
   755 760 765
   Arg Leu Ile Arg Pro Tyr Met Glu Pro Ile Leu Lys Ala Leu Ile
   770 775 780
   Leu Lys Leu Lys Asp Pro Asp Pro Asp Pro Asn Pro Gly Val Ile
   785 790 795
   Asn Asn Val Leu Ala Thr Ile Gly Glu Leu Ala Gln Val Ser Gly
   800 805 810
   Leu Glu Met Arg Lys Trp Val Asp Glu Leu Phe Ile Ile Ile Met
   815 820 825
   Asp Met Leu Gln Asp Ser Ser Leu Leu Ala Lys Arg Gln Val Ala
   830 835 840
   Leu Trp Thr Leu Gly Gln Leu Val Ala Ser Thr Gly Tyr Val Val
   845 850 855
   Glu Pro Tyr Arg Lys Tyr Pro Thr Leu Leu Glu Val Leu Leu Asn
   860 865 870
   Phe Leu Lys Thr Glu Gln Asn Gln Gly Thr Arg Arg Glu Ala Ile
   875 880 885
   Arg Val Leu Gly Leu Leu Gly Ala Leu Asp Pro Tyr Lys His Lys
   890 895 900
   Val Asn Ile Gly Met Ile Asp Gln Ser Arg Asp Ala Ser Ala Val
   905 910 915
   Ser Leu Ser Glu Ser Lys Ser Ser Gln Asp Ser Ser Asp Tyr Ser
   920 925 930
   Thr Ser Glu Met Leu Val Asn Met Gly Asn Leu Pro Leu Asp Glu
   935 940 945
   Phe Tyr Pro Ala Val Ser Met Val Ala Leu Met Arg Ile Phe Arg
   950 955 960
   Asp Gln Ser Leu Ser His His His Thr Met Val Val Gln Ala Ile
   965 970 975
   Thr Phe Ile Phe Lys Ser Leu Gly Leu Lys Cys Val Gln Phe Leu
   980 985 990
   Pro Gln Val Met Pro Thr Phe Leu Asn Val Ile Arg Val Cys Asp
   995 1000 1005
   Gly Ala Ile Arg Glu Phe Leu Phe Gln Gln Leu Gly Met Leu Val
   1010 1015 1020
   Ser Phe Val Lys Ser His Ile Arg Pro Tyr Met Asp Glu Ile Val
   1025 1030 1035
   Thr Leu Met Arg Glu Phe Trp Val Met Asn Thr Ser Ile Gln Ser
   1040 1045 1050
   Thr Ile Ile Leu Leu Ile Glu Gln Ile Val Val Ala Leu Gly Gly
   1055 1060 1065
   Glu Phe Lys Leu Tyr Leu Pro Gln Leu Ile Pro His Met Leu Arg
   1070 1075 1080
   Val Phe Met His Asp Asn Ser Pro Gly Arg Ile Val Ser Ile Lys
   1085 1090 1095
   Leu Leu Ala Ala Ile Gln Leu Phe Gly Ala Asn Leu Asp Asp Tyr
   1100 1105 1110
   Leu His Leu Leu Leu Pro Pro Ile Val Lys Leu Phe Asp Ala Pro
   1115 1120 1125
   Glu Ala Pro Leu Pro Ser Arg Lys Ala Ala Leu Glu Thr Val Asp
   1130 1135 1140
   Arg Leu Thr Glu Ser Leu Asp Phe Thr Asp Tyr Ala Ser Arg Ile
   1145 1150 1155
   Ile His Pro Ile Val Arg Thr Leu Asp Gln Ser Pro Glu Leu Arg
   1160 1165 1170
   Ser Thr Ala Met Asp Thr Leu Ser Ser Leu Val Phe Gln Leu Gly
   1175 1180 1185
   Lys Lys Tyr Gln Ile Phe Ile Pro Met Val Asn Lys Val Leu Val
   1190 1195 1200
   Arg His Arg Ile Asn His Gln Arg Tyr Asp Val Leu Ile Cys Arg
   1205 1210 1215
   Ile Val Lys Gly Tyr Thr Leu Ala Asp Glu Glu Glu Asp Pro Leu
   1220 1225 1230
   Ile Tyr Gln His Arg Met Leu Arg Ser Gly Gln Gly Asp Ala Leu
   1235 1240 1245
   Ala Ser Gly Pro Val Glu Thr Gly Pro Met Lys Lys Leu His Val
   1250 1255 1260
   Ser Thr Ile Asn Leu Gln Lys Ala Thr Ala Trp Gly Ala Ala Arg
   1265 1270 1275
   Arg Val Ser Lys Asp Asp Trp Leu Glu Trp Leu Arg Arg Leu Ser
   1280 1285 1290
   Leu Glu Leu Leu Lys Asp Ser Ser Ser Pro Ser Leu Arg Ser Cys
   1295 1300 1305
   Trp Ala Leu Ala Gln Ala Tyr Asn Pro Met Ala Arg Asp Leu Phe
   1310 1315 1320
   Asn Ala Ala Phe Val Ser Cys Trp Ser Glu Leu Asn Glu Asp Gln
   1325 1330 1335
   Gln Asp Glu Leu Ile Arg Ser Ile Glu Leu Ala Leu Thr Ser Gln
   1340 1345 1350
   Asp Ile Ala Glu Val Thr Gln Thr Leu Leu Asn Leu Ala Glu Phe
   1355 1360 1365
   Met Glu His Ser Asp Lys Gly Pro Leu Pro Leu Arg Asp Asp Asn
   1370 1375 1380
   Gly Ile Val Leu Leu Gly Glu Arg Ala Ala Lys Cys Arg Ala Tyr
   1385 1390 1395
   Ala Lys Ala Leu His Tyr Lys Glu Leu Glu Phe Gln Lys Gly Pro
   1400 1405 1410
   Thr Pro Ala Ile Leu Glu Ser Leu Ile Ser Ile Asn Asn Lys Leu
   1415 1420 1425
   Gln Gln Pro Glu Ala Ala Ala Gly Val Leu Glu Tyr Ala Met Lys
   1430 1435 1440
   His Phe Gly Glu Leu Glu Ile Gln Ala Thr Trp Tyr Glu Lys Leu
   1445 1450 1455
   His Glu Trp Glu Asp Ala Leu Val Ala Tyr Asp Lys Lys Met Asp
   1460 1465 1470
   Thr Asn Lys Asp Asp Pro Glu Leu Met Leu Gly Arg Met Arg Cys
   1475 1480 1485
   Leu Glu Ala Leu Gly Glu Trp Gly Gln Leu His Gln Gln Cys Cys
   1490 1495 1500
   Glu Lys Trp Thr Leu Val Asn Asp Glu Thr Gln Ala Lys Met Ala
   1505 1510 1515
   Arg Met Ala Ala Ala Ala Ala Trp Gly Leu Gly Gln Trp Asp Ser
   1520 1525 1530
   Met Glu Glu Tyr Thr Cys Met Ile Pro Arg Asp Thr His Asp Gly
   1535 1540 1545
   Ala Phe Tyr Arg Ala Val Leu Ala Leu His Gln Asp Leu Phe Ser
   1550 1555 1560
   Leu Ala Gln Gln Cys Ile Asp Lys Ala Arg Asp Leu Leu Asp Ala
   1565 1570 1575
   Glu Leu Thr Ala Met Ala Gly Glu Ser Tyr Ser Arg Ala Tyr Gly
   1580 1585 1590
   Ala Met Val Ser Cys His Met Leu Ser Glu Leu Glu Glu Val Ile
   1595 1600 1605
   Gln Tyr Lys Leu Val Pro Glu Arg Arg Glu Ile Ile Arg Gln Ile
   1610 1615 1620
   Trp Trp Glu Arg Leu Gln Gly Cys Gln Arg Ile Val Glu Asp Trp
   1625 1630 1635
   Gln Lys Ile Leu Met Val Arg Ser Leu Val Val Ser Pro His Glu
   1640 1645 1650
   Asp Met Arg Thr Trp Leu Lys Tyr Ala Ser Leu Cys Gly Lys Ser
   1655 1660 1665
   Gly Arg Leu Ala Leu Ala His Lys Thr Leu Val Leu Leu Leu Gly
   1670 1675 1680
   Val Asp Pro Ser Arg Gln Leu Asp His Pro Leu Pro Thr Val His
   1685 1690 1695
   Pro Gln Val Thr Tyr Ala Tyr Met Lys Asn Met Trp Lys Ser Ala
   1700 1705 1710
   Arg Lys Ile Asp Ala Phe Gln His Met Gln His Phe Val Gln Thr
   1715 1720 1725
   Met Gln Gln Gln Ala Gln His Ala Ile Ala Thr Glu Asp Gln Gln
   1730 1735 1740
   His Lys Gln Glu Leu His Lys Leu Met Ala Arg Cys Phe Leu Lys
   1745 1750 1755
   Leu Gly Glu Trp Gln Leu Asn Leu Gln Gly Ile Asn Glu Ser Thr
   1760 1765 1770
   Ile Pro Lys Val Leu Gln Tyr Tyr Ser Ala Ala Thr Glu His Asp
   1775 1780 1785
   Arg Ser Trp Tyr Lys Ala Trp His Ala Trp Ala Val Met Asn Phe
   1790 1795 1800
   Glu Ala Val Leu His Tyr Lys His Gln Asn Gln Ala Arg Asp Glu
   1805 1810 1815
   Lys Lys Lys Leu Arg His Ala Ser Gly Ala Asn Ile Thr Asn Ala
   1820 1825 1830
   Thr Thr Ala Ala Thr Thr Ala Ala Thr Ala Thr Thr Thr Ala Ser
   1835 1840 1845
   Thr Glu Gly Ser Asn Ser Glu Ser Glu Ala Glu Ser Thr Glu Asn
   1850 1855 1860
   Ser Pro Thr Pro Ser Pro Leu Gln Lys Lys Val Thr Glu Asp Leu
   1865 1870 1875
   Ser Lys Thr Leu Leu Met Tyr Thr Val Pro Ala Val Gln Gly Phe
   1880 1885 1890
   Phe Arg Ser Ile Ser Leu Ser Arg Gly Asn Asn Leu Gln Asp Thr
   1895 1900 1905
   Leu Arg Val Leu Thr Leu Trp Phe Asp Tyr Gly His Trp Pro Asp
   1910 1915 1920
   Val Asn Glu Ala Leu Val Glu Gly Val Lys Ala Ile Gln Ile Asp
   1925 1930 1935
   Thr Trp Leu Gln Val Ile Pro Gln Leu Ile Ala Arg Ile Asp Thr
   1940 1945 1950
   Pro Arg Pro Leu Val Gly Arg Leu Ile His Gln Leu Leu Thr Asp
   1955 1960 1965
   Ile Gly Arg Tyr His Pro Gln Ala Leu Ile Tyr Pro Leu Thr Val
   1970 1975 1980
   Ala Ser Lys Ser Thr Thr Thr Ala Arg His Asn Ala Ala Asn Lys
   1985 1990 1995
   Ile Leu Lys Asn Met Cys Glu His Ser Asn Thr Leu Val Gln Gln
   2000 2005 2010
   Ala Met Met Val Ser Glu Glu Leu Ile Arg Val Ala Ile Leu Trp
   2015 2020 2025
   His Glu Met Trp His Glu Gly Leu Glu Glu Ala Ser Arg Leu Tyr
   2030 2035 2040
   Phe Gly Glu Arg Asn Val Lys Gly Met Phe Glu Val Leu Glu Pro
   2045 2050 2055
   Leu His Ala Met Met Glu Arg Gly Pro Gln Thr Leu Lys Glu Thr
   2060 2065 2070
   Ser Phe Asn Gln Ala Tyr Gly Arg Asp Leu Met Glu Ala Gln Glu
   2075 2080 2085
   Trp Cys Arg Lys Tyr Met Lys Ser Gly Asn Val Lys Asp Leu Thr
   2090 2095 2100
   Gln Ala Trp Asp Leu Tyr Tyr His Val Phe Arg Arg Ile Ser Lys
   2105 2110 2115
   Gln Leu Pro Gln Leu Thr Ser Leu Glu Leu Gln Tyr Val Ser Pro
   2120 2125 2130
   Lys Leu Leu Met Cys Arg Asp Leu Glu Leu Ala Val Pro Gly Thr
   2135 2140 2145
   Tyr Asp Pro Asn Gln Pro Ile Ile Arg Ile Gln Ser Ile Ala Pro
   2150 2155 2160
   Ser Leu Gln Val Ile Thr Ser Lys Gln Arg Pro Arg Lys Leu Thr
   2165 2170 2175
   Leu Met Gly Ser Asn Gly His Glu Phe Val Phe Leu Leu Lys Gly
   2180 2185 2190
   His Glu Asp Leu Arg Gln Asp Glu Arg Val Met Gln Leu Phe Gly
   2195 2200 2205
   Leu Val Asn Thr Leu Leu Ala Asn Asp Pro Thr Ser Leu Arg Lys
   2210 2215 2220
   Asn Leu Ser Ile Gln Arg Tyr Ala Val Ile Pro Leu Ser Thr Asn
   2225 2230 2235
   Ser Gly Leu Ile Gly Trp Val Pro His Cys Asp Thr Leu His Ala
   2240 2245 2250
   Leu Ile Arg Asp Tyr Arg Glu Lys Lys Lys Ile Leu Leu Asn Ile
   2255 2260 2265
   Glu His Arg Ile Met Leu Arg Met Ala Pro Asp Tyr Asp His Leu
   2270 2275 2280
   Thr Leu Met Gln Lys Val Glu Val Phe Glu His Ala Val Asn Asn
   2285 2290 2295
   Thr Ala Gly Asp Asp Leu Ala Lys Leu Leu Trp Leu Lys Ser Pro
   2300 2305 2310
   Ser Ser Glu Val Trp Phe Asp Arg Arg Thr Asn Tyr Thr Arg Ser
   2315 2320 2325
   Leu Ala Val Met Ser Met Val Gly Tyr Ile Leu Gly Leu Gly Asp
   2330 2335 2340
   Arg His Pro Ser Asn Leu Met Leu Asp Arg Leu Ser Gly Lys Ile
   2345 2350 2355
   Leu His Ile Asp Phe Gly Asp Cys Phe Glu Val Ala Met Thr Arg
   2360 2365 2370
   Glu Lys Phe Pro Glu Lys Ile Pro Phe Arg Leu Thr Arg Met Leu
   2375 2380 2385
   Thr Asn Ala Met Glu Val Thr Gly Leu Asp Gly Asn Tyr Arg Ile
   2390 2395 2400
   Thr Cys His Thr Val Met Glu Val Leu Arg Glu His Lys Asp Ser
   2405 2410 2415
   Val Met Ala Val Leu Glu Ala Phe Val Tyr Asp Pro Leu Leu Asn
   2420 2425 2430
   Trp Arg Leu Met Asp Thr Asn Thr Lys Gly Asn Lys Arg Ser Arg
   2435 2440 2445
   Thr Arg Thr Asp Ser Tyr Ser Ala Gly Gln Ser Val Glu Ile Leu
   2450 2455 2460
   Asp Gly Val Glu Leu Gly Glu Pro Ala His Lys Lys Thr Gly Thr
   2465 2470 2475
   Thr Val Pro Glu Ser Ile His Ser Phe Ile Gly Asp Gly Leu Val
   2480 2485 2490
   Lys Pro Glu Ala Leu Asn Lys Lys Ala Ile Gln Ile Ile Asn Arg
   2495 2500 2505
   Val Arg Asp Lys Leu Thr Gly Arg Asp Phe Ser His Asp Asp Thr
   2510 2515 2520
   Leu Asp Val Pro Thr Gln Val Glu Leu Leu Ile Lys Gln Ala Thr
   2525 2530 2535
   Ser His Glu Asn Leu Cys Gln Cys Tyr Ile Gly Trp Cys Pro Phe
   2540 2545 2550
   Trp
<210> 46
   <211> 1356
   <212> PRT
   <213> Homo sapiens
<400> 46
<210> 47
   <211> 1408
   <212> PRT
   <213> Homo sapiens
<400> 47
<210> 48
   <211> 512
   <212> PRT
   <213> Homo sapiens
<400> 48
   Met Ala His Ser Cys Arg Trp Arg Phe Pro Ala Arg Pro Gly Thr
   1               5                   10                  15
   Thr Gly Gly Gly Gly Gly Gly Gly Arg Arg Gly Leu Gly Gly Ala
                   20                  25                  30
   Pro Arg Gln Arg Val Pro Ala Leu Leu Leu Pro Pro Gly Pro Pro
                   35                  40                  45
   Val Gly Gly Gly Gly Pro Gly Ala Pro Pro Ser Pro Pro Ala Val
                   50                  55                  60
   Ala Ala Ala Ala Ala Ala Ala Gly Ser Ser Gly Ala Gly Val Pro
                   65                  70                  75
   Gly Gly Ala Ala Ala Ala Ser Ala Ala Ser Ser Ser Ser Ala Ser
                   80                  85                  90
   Ser Ser Ser Ser Ser Ser Ser Ser Ala Ser Ser Gly Pro Ala Leu
                   95                  100                 105
   Leu Arg Val Gly Pro Gly Phe Asp Ala Ala Leu Gln Val Ser Ala
                   110                 115                 120
   Ala Ile Gly Thr Asn Leu Arg Arg Phe Arg Ala Val Phe Gly Glu
                   125                 130                 135
   Ser Gly Gly Gly Gly Gly Ser Gly Glu Leu Thr Thr Gln Ile Pro
                   140                 145                 150
   Cys Ser Trp Arg Thr Lys Gly His Ile His Asp Lys Lys Thr Glu
                   155                 160                 165
   Pro Phe Arg Leu Leu Ala Trp Ser Trp Cys Leu Asn Asp Glu Gln
   170 175 180
   Phe Leu Gly Phe Gly Ser Asp Glu Glu Val Arg Val Arg Ser Pro
   185 190 195
   Thr Arg Ser Pro Ser Val Lys Thr Ser Pro Arg Lys Pro Arg Gly
   200 205 210
   Arg Pro Arg Ser Gly Ser Asp Arg Asn Ser Ala Ile Leu Ser Asp
   215 220 225
   Pro Ser Val Phe Ser Pro Leu Asn Lys Ser Glu Thr Lys Ser Gly
   230 235 240
   Asp Lys Ile Lys Lys Lys Asp Ser Lys Ser Ile Glu Lys Lys Arg
   245 250 255
   Gly Arg Pro Pro Thr Phe Pro Gly Val Lys Ile Lys Ile Thr His
   260 265 270
   Gly Lys Asp Ile Ser Glu Leu Pro Lys Gly Asn Lys Glu Asp Ser
   275 280 285
   Leu Lys Lys Ile Lys Arg Thr Pro Ser Ala Thr Phe Gln Gln Ala
   290 295 300
   Thr Lys Ile Lys Lys Leu Arg Ala Gly Lys Leu Ser Pro Leu Lys
   305 310 315
   Ser Lys Phe Lys Thr Gly Lys Leu Gln Ile Gly Arg Lys Gly Val
   320 325 330
   Gln Ile Val Arg Arg Arg Gly Arg Pro Pro Ser Thr Glu Arg Ile
   335 340 345
   Lys Thr Pro Ser Gly Leu Leu Ile Asn Ser Glu Leu Glu Lys Pro
   350 355 360
   Gln Lys Val Arg Lys Asp Lys Glu Gly Thr Pro Pro Leu Thr Lys
   365 370 375
   Glu Asp Lys Thr Val Val Arg Gln Ser Pro Arg Arg Ile Lys Pro
   380 385 390
   Val Arg Ile Ile Pro Ser Ser Lys Arg Thr Asp Ala Thr Ile Ala
   395 400 405
   Lys Gln Leu Leu Gln Arg Ala Lys Lys Gly Ala Gln Lys Lys Ile
   410 415 420
   Glu Lys Glu Ala Ala Gln Leu Gln Gly Arg Lys Val Lys Thr Gln
   425 430 435
   Val Lys Asn Ile Arg Gln Phe Ile Met Pro Val Val Ser Ala Ile
   440 445 450
   Ser Ser Arg Ile Ile Lys Thr Pro Arg Arg Phe Ile Glu Asp Glu
   455 460 465
   Asp Tyr Asp Pro Pro Ile Lys Ile Ala Arg Leu Glu Ser Thr Pro
   470 475 480
   Asn Ser Arg Phe Ser Ala Pro Ser Cys Gly Ser Ser Glu Lys Ser
   485 490 495
   Ser Ala Ala Ser Gln His Ser Ser Gln Met Ser Ser Asp Ser Ser
   500 505 510
   Arg Ser
<210> 49
   <211> 2839
   <212> PRT
   <213> Homo sapiens
<400> 49
   Met Ala Ala His Arg Pro Val Glu Trp Val Gln Ala Val Val Ser
   1 5 10 15
   Arg Phe Asp Glu Gln Leu Pro Ile Lys Thr Gly Gln Gln Asn Thr
   20 25 30
   His Thr Lys Val Ser Thr Glu His Asn Lys Glu Cys Leu Ile Asn
   35 40 45
   Ile Ser Lys Tyr Lys Phe Ser Leu Val Ile Ser Gly Leu Thr Thr
   50 55 60
   Ile Leu Lys Asn Val Asn Asn Met Arg Ile Phe Gly Glu Ala Ala
   65 70 75
   Glu Lys Asn Leu Tyr Leu Ser Gln Leu Ile Ile Leu Asp Thr Leu
   80 85 90
   Glu Lys Cys Leu Ala Gly Gln Pro Lys Asp Thr Met Arg Leu Asp
   95 100 105
   Glu Thr Met Leu Val Lys Gln Leu Leu Pro Glu Ile Cys His Phe
   110 115 120
   Leu His Thr Cys Arg Glu Gly Asn Gln His Ala Ala Glu Leu Arg
   125 130 135
   Asn Ser Ala Ser Gly Val Leu Phe Ser Leu Ser Cys Asn Asn Phe
   140 145 150
   Asn Ala Val Phe Ser Arg Ile Ser Thr Arg Leu Gln Glu Leu Thr
   155 160 165
   Val Cys Ser Glu Asp Asn Val Asp Val His Asp Ile Glu Leu Leu
   170 175 180
   Gln Tyr Ile Asn Val Asp Cys Ala Lys Leu Lys Arg Leu Leu Lys
   185 190 195
   Glu Thr Ala Phe Lys Phe Lys Ala Leu Lys Lys Val Ala Gln Leu
   200 205 210
   Ala Val Ile Asn Ser Leu Glu Lys Ala Phe Trp Asn Trp Val Glu
   215 220 225
   Asn Tyr Pro Asp Glu Phe Thr Lys Leu Tyr Gln Ile Pro Gln Thr
   230 235 240
   Asp Met Ala Glu Cys Ala Glu Lys Leu Phe Asp Leu Val Asp Gly
   245 250 255
   Phe Ala Glu Ser Thr Lys Arg Lys Ala Ala Val Trp Pro Leu Gln
   260 265 270
   Ile Ile Leu Leu Ile Leu Cys Pro Glu Ile Ile Gln Asp Ile Ser
   275 280 285
   Lys Asp Val Val Asp Glu Asn Asn Met Asn Lys Lys Leu Phe Leu
   290 295 300
   Asp Ser Leu Arg Lys Ala Leu Ala Gly His Gly Gly Ser Arg Gln
   305 310 315
   Leu Thr Glu Ser Ala Ala Ile Ala Cys Val Lys Leu Cys Lys Ala
   320 325 330
   Ser Thr Tyr Ile Asn Trp Glu Asp Asn Ser Val Ile Phe Leu Leu
   335 340 345
   Val Gln Ser Met Val Val Asp Leu Lys Asn Leu Leu Phe Asn Pro
   350 355 360
   Ser Lys Pro Phe Ser Arg Gly Ser Gln Pro Ala Asp Val Asp Leu
   365 370 375
   Met Ile Asp Cys Leu Val Ser Cys Phe Arg Ile Ser Pro His Asn
   380 385 390
   Asn Gln His Phe Lys Ile Cys Leu Ala Gln Asn Ser Pro Ser Thr
   395 400 405
   Phe His Tyr Val Leu Val Asn Ser Leu His Arg Ile Ile Thr Asn
   410 415 420
   Ser Ala Leu Asp Trp Trp Pro Lys Ile Asp Ala Val Tyr Cys His
   425 430 435
   Ser Val Glu Leu Arg Asn Met Phe Gly Glu Thr Leu His Lys Ala
   440 445 450
   Val Gln Gly Cys Gly Ala His Pro Ala Ile Arg Met Ala Pro Ser
   455 460 465
   Leu Thr Phe Lys Glu Lys Val Thr Ser Leu Lys Phe Lys Glu Lys
   470 475 480
   Pro Thr Asp Leu Glu Thr Arg Ser Tyr Lys Tyr Leu Leu Leu Ser
   485 490 495
   Met Val Lys Leu Ile His Ala Asp Pro Lys Leu Leu Leu Cys Asn
   500 505 510
   Pro Arg Lys Gln Gly Pro Glu Thr Gln Gly Ser Thr Ala Glu Leu
   515 520 525
   Ile Thr Gly Leu Val Gln Leu Val Pro Gln Ser His Met Pro Glu
   530 535 540
   Ile Ala Gln Glu Ala Met Glu Ala Leu Leu Val Leu His Gln Leu
   545 550 555
   Asp Ser Ile Asp Leu Trp Asn Pro Asp Ala Pro Val Glu Thr Phe
   560 565 570
   Trp Glu Ile Ser Ser Gln Met Leu Phe Tyr Ile Cys Lys Lys Leu
   575 580 585
   Thr Ser His Gln Met Leu Ser Ser Thr Glu Ile Leu Lys Trp Leu
   590 595 600
   Arg Glu Ile Leu Ile Cys Arg Asn Lys Phe Leu Leu Lys Asn Lys
   605 610 615
   Gln Ala Asp Arg Ser Ser Cys His Phe Leu Leu Phe Tyr Gly Val
   620 625 630
   Gly Cys Asp Ile Pro Ser Ser Gly Asn Thr Ser Gln Met Ser Met
   635 640 645
   Asp His Glu Glu Leu Leu Arg Thr Pro Gly Ala Ser Leu Arg Lys
   650 655 660
   Gly Lys Gly Asn Ser Ser Met Asp Ser Ala Ala Gly Cys Ser Gly
   665 670 675
   Thr Pro Pro Ile Cys Arg Gln Ala Gln Thr Lys Leu Glu Val Ala
   680 685 690
   Leu Tyr Met Phe Leu Trp Asn Pro Asp Thr Glu Ala Val Leu Val
   695 700 705
   Ala Met Ser Cys Phe Arg His Leu Cys Glu Glu Ala Asp Ile Arg
   710 715 720
   Cys Gly Val Asp Glu Val Ser Val His Asn Leu Leu Pro Asn Tyr
   725 730 735
   Asn Thr Phe Met Glu Phe Ala Ser Val Ser Asn Met Met Ser Thr
   740 745 750
   Gly Arg Ala Ala Leu Gln Lys Arg Val Met Ala Leu Leu Arg Arg
   755 760 765
   Ile Glu His Pro Thr Ala Gly Asn Thr Glu Ala Trp Glu Asp Thr
   770 775 780
   His Ala Lys Trp Glu Gln Ala Thr Lys Leu Ile Leu Asn Tyr Pro
   785 790 795
   Lys Ala Lys Met Glu Asp Gly Gln Ala Ala Glu Ser Leu His Lys
   800 805 810
   Thr Ile Val Lys Arg Arg Met Ser His Val Ser Gly Gly Gly Ser
   815 820 825
   Ile Asp Leu Ser Asp Thr Asp Ser Leu Gln Glu Trp Ile Asn Met
   830 835 840
   Thr Gly Phe Leu Cys Ala Leu Gly Gly Val Cys Leu Gln Gln Arg
   845 850 855
   Ser Asn Ser Gly Leu Ala Thr Tyr Ser Pro Pro Met Gly Pro Val
   860 865 870
   Ser Glu Arg Lys Gly Ser Met Ile Ser Val Met Ser Ser Glu Gly
   875 880 885
   Asn Ala Asp Thr Pro Val Ser Lys Phe Met Asp Arg Leu Leu Ser
   890 895 900
   Leu Met Val Cys Asn His Glu Lys Val Gly Leu Gln Ile Arg Thr
   905 910 915
   Asn Val Lys Asp Leu Val Gly Leu Glu Leu Ser Pro Ala Leu Tyr
   920 925 930
   Pro Met Leu Phe Asn Lys Leu Lys Asn Thr Ile Ser Lys Phe Phe
   935 940 945
   Asp Ser Gln Gly Gln Val Leu Leu Thr Asp Thr Asn Thr Gln Phe
   950 955 960
   Val Glu Gln Thr Ile Ala Ile Met Lys Asn Leu Leu Asp Asn His
   965 970 975
   Thr Glu Gly Ser Ser Glu His Leu Gly Gln Ala Ser Ile Glu Thr
   980 985 990
   Met Met Leu Asn Leu Val Arg Tyr Val Arg Val Leu Gly Asn Met
   995 1000 1005
   Val His Ala Ile Gln Ile Lys Thr Lys Leu Cys Gln Leu Val Glu
   1010 1015 1020
   Val Met Met Ala Arg Arg Asp Asp Leu Ser Phe Cys Gln Glu Met
   1025 1030 1035
   Lys Phe Arg Asn Lys Met Val Glu Tyr Leu Thr Asp Trp Val Met
   1040 1045 1050
   Gly Thr Ser Asn Gln Ala Ala Asp Asp Asp Val Lys Cys Leu Thr
   1055 1060 1065
   Arg Asp Leu Asp Gln Ala Ser Met Glu Ala Val Val Ser Leu Leu
   1070 1075 1080
   Ala Gly Leu Pro Leu Gln Pro Glu Glu Gly Asp Gly Val Glu Leu
   1085 1090 1095
   Met Glu Ala Lys Ser Gln Leu Phe Leu Lys Tyr Phe Thr Leu Phe
   1100 1105 1110
   Met Asn Leu Leu Asn Asp Cys Ser Glu Val Glu Asp Glu Ser Ala
   1115 1120 1125
   Gln Thr Gly Gly Arg Lys Arg Gly Met Ser Arg Arg Leu Ala Ser
   1130 1135 1140
   Leu Arg His Cys Thr Val Leu Ala Met Ser Asn Leu Leu Asn Ala
   1145 1150 1155
   Asn Val Asp Ser Gly Leu Met His Ser Ile Gly Leu Gly Tyr His
   1160 1165 1170
   Lys Asp Leu Gln Thr Arg Ala Thr Phe Met Glu Val Leu Thr Lys
   1175 1180 1185
   Ile Leu Gln Gln Gly Thr Glu Phe Asp Thr Leu Ala Glu Thr Val
   1190 1195 1200
   Leu Ala Asp Arg Phe Glu Arg Leu Val Glu Leu Val Thr Met Met
   1205 1210 1215
   Gly Asp Gln Gly Glu Leu Pro Ile Ala Met Ala Leu Ala Asn Val
   1220 1225 1230
   Val Pro Cys Ser Gln Trp Asp Glu Leu Ala Arg Val Leu Val Thr
   1235 1240 1245
   Leu Phe Asp Ser Arg His Leu Leu Tyr Gln Leu Leu Trp Asn Met
   1250 1255 1260
   Phe Ser Lys Glu Val Glu Leu Ala Asp Ser Met Gln Thr Leu Phe
   1265 1270 1275
   Arg Gly Asn Ser Leu Ala Ser Lys Ile Met Thr Phe Cys Phe Lys
   1280 1285 1290
   Val Tyr Gly Ala Thr Tyr Leu Gln Lys Leu Leu Asp Pro Leu Leu
   1295 1300 1305
   Arg Ile Val Ile Thr Ser Ser Asp Trp Gln His Val Ser Phe Glu
   1310 1315 1320
   Val Asp Pro Thr Arg Leu Glu Pro Ser Glu Ser Leu Glu Glu Asn
   1325 1330 1335
   Gln Arg Asn Leu Leu Gln Met Thr Glu Lys Phe Phe His Ala Ile
   1340 1345 1350
   Ile Ser Ser Ser Ser Glu Phe Pro Pro Gln Leu Arg Ser Val Cys
   1355 1360 1365
   His Cys Leu Tyr Gln Ala Thr Cys His Ser Leu Leu Asn Lys Ala
   1370 1375 1380
   Thr Val Lys Glu Lys Lys Glu Asn Lys Lys Ser Val Val Ser Gln
   1385 1390 1395
   Arg Phe Pro Gln Asn Ser Ile Gly Ala Val Gly Ser Ala Met Phe
   1400 1405 1410
   Leu Arg Phe Ile Asn Pro Ala Ile Val Ser Pro Tyr Glu Ala Gly
   1415 1420 1425
   Ile Leu Asp Lys Lys Pro Pro Pro Arg Ile Glu Arg Gly Leu Lys
   1430 1435 1440
   Leu Met Ser Lys Ile Leu Gln Ser Ile Ala Asn His Val Leu Phe
   1445 1450 1455
   Thr Lys Glu Glu His Met Arg Pro Phe Asn Asp Phe Val Lys Ser
   1460 1465 1470
   Asn Phe Asp Ala Ala Arg Arg Phe Phe Leu Asp Ile Ala Ser Asp
   1475 1480 1485
   Cys Pro Thr Ser Asp Ala Val Asn His Ser Leu Ser Phe Ile Ser
   1490 1495 1500
   Asp Gly Asn Val Leu Ala Leu His Arg Leu Leu Trp Asn Asn Gln
   1505 1510 1515
   Glu Lys Ile Gly Gln Tyr Leu Ser Ser Asn Arg Asp His Lys Ala
   1520 1525 1530
   Val Gly Arg Arg Pro Phe Asp Lys Met Ala Thr Leu Leu Ala Tyr
   1535 1540 1545
   Leu Gly Pro Pro Glu His Lys Pro Val Ala Asp Thr His Trp Ser
   1550 1555 1560
   Ser Leu Asn Leu Thr Ser Ser Lys Phe Glu Glu Phe Met Thr Arg
   1565 1570 1575
   His Gln Val His Glu Lys Glu Glu Phe Lys Ala Leu Lys Thr Leu
   1580 1585 1590
   Ser Ile Phe Tyr Gln Ala Gly Thr Ser Lys Ala Gly Asn Pro Ile
   1595 1600 1605
   Phe Tyr Tyr Val Ala Arg Arg Phe Lys Thr Gly Gln Ile Asn Gly
   1610 1615 1620
   Asp Leu Leu Ile Tyr His Val Leu Leu Thr Leu Lys Pro Tyr Tyr
   1625 1630 1635
   Ala Lys Pro Tyr Glu Ile Val Val Asp Leu Thr His Thr Gly Pro
   1640 1645 1650
   Ser Asn Arg Phe Lys Thr Asp Phe Leu Ser Lys Trp Phe Val Val
   1655 1660 1665
   Phe Pro Gly Phe Ala Tyr Asp Asn Val Ser Ala Val Tyr Ile Tyr
   1670 1675 1680
   Asn Cys Asn Ser Trp Val Arg Glu Tyr Thr Lys Tyr His Glu Arg
   1685 1690 1695
   Leu Leu Thr Gly Leu Lys Gly Ser Lys Arg Leu Val Phe Ile Asp
   1700 1705 1710
   Cys Pro Gly Lys Leu Ala Glu His Ile Glu His Glu Gln Gln Lys
   1715 1720 1725
   Leu Pro Ala Ala Thr Leu Ala Leu Glu Glu Asp Leu Lys Val Phe
   1730 1735 1740
   His Asn Ala Leu Lys Leu Ala His Lys Asp Thr Lys Val Ser Ile
   1745 1750 1755
   Lys Val Gly Ser Thr Ala Val Gln Val Thr Ser Ala Glu Arg Thr
   1760 1765 1770
   Lys Val Leu Gly Gln Ser Val Phe Leu Asn Asp Ile Tyr Tyr Ala
   1775 1780 1785
   Ser Glu Ile Glu Glu Ile Cys Leu Val Asp Glu Asn Gln Phe Thr
   1790 1795 1800
   Leu Thr Ile Ala Asn Gln Gly Thr Pro Leu Thr Phe Met His Gln
   1805 1810 1815
   Glu Cys Glu Ala Ile Val Gln Ser Ile Ile His Ile Arg Thr Arg
   1820 1825 1830
   Trp Glu Leu Ser Gln Pro Asp Ser Ile Pro Gln His Thr Lys Ile
   1835 1840 1845
   Arg Pro Lys Asp Val Pro Gly Thr Leu Leu Asn Ile Ala Leu Leu
   1850 1855 1860
   Asn Leu Gly Ser Ser Asp Pro Ser Leu Arg Ser Ala Ala Tyr Asn
   1865 1870 1875
   Leu Leu Cys Ala Leu Thr Cys Thr Phe Asn Leu Lys Ile Glu Gly
   1880 1885 1890
   Gln Leu Leu Glu Thr Ser Gly Leu Cys Ile Pro Ala Asn Asn Thr
   1895 1900 1905
   Leu Phe Ile Val Ser Ile Ser Lys Thr Leu Ala Ala Asn Glu Pro
   1910 1915 1920
   His Leu Thr Leu Glu Phe Leu Glu Glu Cys Ile Ser Gly Phe Ser
   1925 1930 1935
   Lys Ser Ser Ile Glu Leu Lys His Leu Cys Leu Glu Tyr Met Thr
   1940 1945 1950
   Pro Trp Leu Ser Asn Leu Val Arg Phe Cys Lys His Asn Asp Asp
   1955 1960 1965
   Ala Lys Arg Gln Arg Val Thr Ala Ile Leu Asp Lys Leu Ile Thr
   1970 1975 1980
   Met Thr Ile Asn Glu Lys Gln Met Tyr Pro Ser Ile Gln Ala Lys
   1985 1990 1995
   Ile Trp Gly Ser Leu Gly Gln Ile Thr Asp Leu Leu Asp Val Val
   2000 2005 2010
   Leu Asp Ser Phe Ile Lys Thr Ser Ala Thr Gly Gly Leu Gly Ser
   2015 2020 2025
   Ile Lys Ala Glu Val Met Ala Asp Thr Ala Val Ala Leu Ala Ser
   2030 2035 2040
   Gly Asn Val Lys Leu Val Ser Ser Lys Val Ile Gly Arg Met Cys
   2045 2050 2055
   Lys Ile Ile Asp Lys Thr Cys Leu Ser Pro Thr Pro Thr Leu Glu
   2060 2065 2070
   Gln His Leu Met Trp Asp Asp Ile Ala Ile Leu Ala Arg Tyr Met
   2075 2080 2085
   Leu Met Leu Ser Phe Asn Asn Ser Leu Asp Val Ala Ala His Leu
   2090 2095 2100
   Pro Tyr Leu Phe His Val Val Thr Phe Leu Val Ala Thr Gly Pro
   2105 2110 2115
   Leu Ser Leu Arg Ala Ser Thr His Gly Leu Val Ile Asn Ile Ile
   2120 2125 2130
   His Ser Leu Cys Thr Cys Ser Gln Leu His Phe Ser Glu Glu Thr
   2135 2140 2145
   Lys Gln Val Leu Arg Leu Ser Leu Thr Glu Phe Ser Leu Pro Lys
   2150 2155 2160
   Phe Tyr Leu Leu Phe Gly Ile Ser Lys Val Lys Ser Ala Ala Val
   2165 2170 2175
   Ile Ala Phe Arg Ser Ser Tyr Arg Asp Arg Ser Phe Ser Pro Gly
   2180 2185 2190
   Ser Tyr Glu Arg Glu Thr Phe Ala Leu Thr Ser Leu Glu Thr Val
   2195 2200 2205
   Thr Glu Ala Leu Leu Glu Ile Met Glu Ala Cys Met Arg Asp Ile
   2210 2215 2220
   Pro Thr Cys Lys Trp Leu Asp Gln Trp Thr Glu Leu Ala Gln Arg
   2225 2230 2235
   Phe Ala Phe Gln Tyr Asn Pro Ser Leu Gln Pro Arg Ala Leu Val
   2240 2245 2250
   Val Phe Gly Cys Ile Ser Lys Arg Val Ser His Gly Gln Ile Lys
   2255 2260 2265
   Gln Ile Ile Arg Ile Leu Ser Lys Ala Leu Glu Ser Cys Leu Lys
   2270 2275 2280
   Gly Pro Asp Thr Tyr Asn Ser Gln Val Leu Ile Glu Ala Thr Val
   2285 2290 2295
   Ile Ala Leu Thr Lys Leu Gln Pro Leu Leu Asn Lys Asp Ser Pro
   2300 2305 2310
   Leu His Lys Ala Leu Phe Trp Val Ala Val Ala Val Leu Gln Leu
   2315 2320 2325
   Asp Glu Val Asn Leu Tyr Ser Ala Gly Thr Ala Leu Leu Glu Gln
   2330 2335 2340
   Asn Leu His Thr Leu Asp Ser Leu Arg Ile Phe Asn Asp Lys Ser
   2345 2350 2355
   Pro Glu Glu Val Phe Met Ala Ile Arg Asn Pro Leu Glu Trp His
   2360 2365 2370
   Cys Lys Gln Met Asp His Phe Val Gly Leu Asn Phe Asn Ser Asn
   2375 2380 2385
   Phe Asn Phe Ala Leu Val Gly His Leu Leu Lys Gly Tyr Arg His
   2390 2395 2400
   Pro Ser Pro Ala Ile Val Ala Arg Thr Val Arg Ile Leu His Thr
   2405 2410 2415
   Leu Leu Thr Leu Val Asn Lys His Arg Asn Cys Asp Lys Phe Glu
   2420 2425 2430
   Val Asn Thr Gln Ser Val Ala Tyr Leu Ala Ala Leu Leu Thr Val
   2435 2440 2445
   Ser Glu Glu Val Arg Ser Arg Cys Ser Leu Lys His Arg Lys Ser
   2450 2455 2460
   Leu Leu Leu Thr Asp Ile Ser Met Glu Asn Val Pro Met Asp Thr
   2465 2470 2475
   Tyr Pro Ile His His Gly Asp Pro Ser Tyr Arg Thr Leu Lys Glu
   2480 2485 2490
   Thr Gln Pro Trp Ser Ser Pro Lys Gly Ser Glu Gly Tyr Leu Ala
   2495 2500 2505
   Ala Thr Tyr Pro Thr Val Gly Gln Thr Ser Pro Arg Ala Arg Lys
   2510 2515 2520
   Ser Met Ser Leu Asp Met Gly Gln Pro Ser Gln Ala Asn Thr Lys
   2525 2530 2535
   Lys Leu Leu Gly Thr Arg Lys Ser Phe Asp His Leu Ile Ser Asp
   2540 2545 2550
   Thr Lys Ala Pro Lys Arg Gln Glu Met Glu Ser Gly Ile Thr Thr
   2555 2560 2565
   Pro Pro Lys Met Arg Arg Val Ala Glu Thr Asp Tyr Glu Met Glu
   2570 2575 2580
   Thr Gln Arg Ile Ser Ser Ser Gln Gln His Pro His Leu Arg Lys
   2585 2590 2595
   Val Ser Val Ser Glu Ser Asn Val Leu Leu Asp Glu Glu Val Leu
   2600 2605 2610
   Thr Asp Pro Lys Ile Gln Ala Leu Leu Leu Thr Val Leu Ala Thr
   2615 2620 2625
   Leu Val Lys Tyr Thr Thr Asp Glu Phe Asp Gln Arg Ile Leu Tyr
   2630 2635 2640
   Glu Tyr Leu Ala Glu Ala Ser Val Val Phe Pro Lys Val Phe Pro
   2645 2650 2655
   Val Val His Asn Leu Leu Asp Ser Lys Ile Asn Thr Leu Leu Ser
   2660 2665 2670
   Leu Cys Gln Asp Pro Asn Leu Leu Asn Pro Ile His Gly Ile Val
   2675 2680 2685
   Gln Ser Val Val Tyr His Glu Glu Ser Pro Pro Gln Tyr Gln Thr
   2690 2695 2700
   Ser Tyr Leu Gln Ser Phe Gly Phe Asn Gly Leu Trp Arg Phe Ala
   2705 2710 2715
   Gly Pro Phe Ser Lys Gln Thr Gln Ile Pro Asp Tyr Ala Glu Leu
   2720 2725 2730
   Ile Val Lys Phe Leu Asp Ala Leu Ile Asp Thr Tyr Leu Pro Gly
   2735 2740 2745
   Ile Asp Glu Glu Thr Ser Glu Glu Ser Leu Leu Thr Pro Thr Ser
   2750 2755 2760
   Pro Tyr Pro Pro Ala Leu Gln Ser Gln Leu Ser Ile Thr Ala Asn
   2765 2770 2775
   Leu Asn Leu Ser Asn Ser Met Thr Ser Leu Ala Thr Ser Gln His
   2780 2785 2790
   Ser Pro Gly Ile Asp Lys Glu Asn Val Glu Leu Ser Pro Thr Thr
   2795 2800 2805
   Gly His Cys Asn Ser Gly Arg Thr Arg His Gly Ser Ala Ser Gln
   2810 2815 2820
   Val Gln Lys Gln Arg Ser Ala Gly Ser Phe Lys Arg Asn Ser Ile
   2825 2830 2835
   Lys Lys Ile Val
<210> 50
   <211> 1089
   <212> PRT
   <213> Homo sapiens
<400> 50
<210> 51
   <211> 1106
   <212> PRT
   <213> Homo sapiens
<400> 51
<210> 52
   <211> 603
   <212> PRT
   <213> Homo sapiens
<400> 52
   Met Ser Ala Ala Val Thr Ala Gly Lys Leu Ala Arg Ala Pro Ala
   1               5                   10                  15
   Asp Pro Gly Lys Ala Gly Val Pro Gly Val Ala Ala Pro Gly Ala
                   20                  25                  30
   Pro Ala Ala Ala Pro Pro Ala Lys Glu Ile Pro Glu Val Leu Val
                   35                  40                  45
   Asp Pro Arg Ser Arg Arg Arg Tyr Val Arg Gly Arg Phe Leu Gly
                   50                  55                  60
   Lys Gly Gly Phe Ala Lys Cys Phe Glu Ile Ser Asp Ala Asp Thr
                   65                  70                  75
   Lys Glu Val Phe Ala Gly Lys Ile Val Pro Lys Ser Leu Leu Leu
                   80                  85                  90
   Lys Pro His Gln Arg Glu Lys Met Ser Met Glu Ile Ser Ile His
                   95                  100                 105
   Arg Ser Leu Ala His Gln His Val Val Gly Phe His Gly Phe Phe
                   110                 115                 120
   Glu Asp Asn Asp Phe Val Phe Val Val Leu Glu Leu Cys Arg Arg
                   125                 130                 135
   Arg Ser Leu Leu Glu Leu His Lys Arg Arg Lys Ala Leu Thr Glu
                   140                 145                 150
   Pro Glu Ala Arg Tyr Tyr Leu Arg Gln Ile Val Leu Gly Cys Gln
                   155                 160                 165
   Tyr Leu His Arg Asn Arg Val Ile His Arg Asp Leu Lys Leu Gly
                   170                 175                 180
   Asn Leu Phe Leu Asn Glu Asp Leu Glu Val Lys Ile Gly Asp Phe
                   185                 190                 195
   Gly Leu Ala Thr Lys Val Glu Tyr Asp Gly Glu Arg Lys Lys Thr
                   200                 205                 210
   Leu Cys Gly Thr Pro Asn Tyr Ile Ala Pro Glu Val Leu Ser Lys
                   215                 220                 225
   Lys Gly His Ser Phe Glu Val Asp Val Trp Ser Ile Gly Cys Ile
                   230                 235                 240
   Met Tyr Thr Leu Leu Val Gly Lys Pro Pro Phe Glu Thr Ser Cys
                   245                 250                 255
   Leu Lys Glu Thr Tyr Leu Arg Ile Lys Lys Asn Glu Tyr Ser Ile
                   260                 265                 270
   Pro Lys His Ile Asn Pro Val Ala Ala Ser Leu Ile Gln Lys Met
                   275                 280                 285
   Leu Gln Thr Asp Pro Thr Ala Arg Pro Thr Ile Asn Glu Leu Leu
                   290                 295                 300
   Asn Asp Glu Phe Phe Thr Ser Gly Tyr Ile Pro Ala Arg Leu Pro
                   305                 310                 315
   Ile Thr Cys Leu Thr Ile Pro Pro Arg Phe Ser Ile Ala Pro Ser
                   320                 325                 330
   Ser Leu Asp Pro Ser Asn Arg Lys Pro Leu Thr Val Leu Asn Lys
   335 340 345
   Gly Leu Glu Asn Pro Leu Pro Glu Arg Pro Arg Glu Lys Glu Glu
   350 355 360
   Pro Val Val Arg Glu Thr Gly Glu Val Val Asp Cys His Leu Ser
   365 370 375
   Asp Met Leu Gln Gln Leu His Ser Val Asn Ala Ser Lys Pro Ser
   380 385 390
   Glu Arg Gly Leu Val Arg Gln Glu Glu Ala Glu Asp Pro Ala Cys
   395 400 405
   Ile Pro Ile Phe Trp Val Ser Lys Trp Val Asp Tyr Ser Asp Lys
   410 415 420
   Tyr Gly Leu Gly Tyr Gln Leu Cys Asp Asn Ser Val Gly Val Leu
   425 430 435
   Phe Asn Asp Ser Thr Arg Leu Ile Leu Tyr Asn Asp Gly Asp Ser
   440 445 450
   Leu Gln Tyr Ile Glu Arg Asp Gly Thr Glu Ser Tyr Leu Thr Val
   455 460 465
   Ser Ser His Pro Asn Ser Leu Met Lys Lys Ile Thr Leu Leu Lys
   470 475 480
   Tyr Phe Arg Asn Tyr Met Ser Glu His Leu Leu Lys Ala Gly Ala
   485 490 495
   Asn Ile Thr Pro Arg Glu Gly Asp Glu Leu Ala Arg Leu Pro Tyr
   500 505 510
   Leu Arg Thr Trp Phe Arg Thr Arg Ser Ala Ile Ile Leu His Leu
   515 520 525
   Ser Asn Gly Ser Val Gln Ile Asn Phe Phe Gln Asp His Thr Lys
   530 535 540
   Leu Ile Leu Cys Pro Leu Met Ala Ala Val Thr Tyr Ile Asp Glu
   545 550 555
   Lys Arg Asp Phe Arg Thr Tyr Arg Leu Ser Leu Leu Glu Glu Tyr
   560 565 570
   Gly Cys Cys Lys Glu Leu Ala Ser Arg Leu Arg Tyr Ala Arg Thr
   575 580 585
   Met Val Asp Lys Leu Leu Ser Ser Arg Ser Ala Ser Asn Arg Leu
   590 595 600
   Lys Ala Ser
<210> 53
   <211> 631
   <212> PRT
   <213> Homo sapiens
<400> 53
<210> 54
   <211> 393
   <212> PRT
   <213> Homo sapiens
<400> 54
<210> 55
   <211> 764
   <212> PRT
   <213> Homo sapiens
<400> 55
<210> 56
   <211> 481
   <212> PRT
   <213> Homo sapiens
<400> 56
<210> 57
   <211> 275
   <212> PRT
   <213> Homo sapiens
<400> 57
   Met Arg Arg Leu Thr Val Asp Asp Phe Glu Ile Gly Arg Pro Leu
   1 5 10 15
   Gly Lys Gly Lys Phe Gly Asn Val Tyr Leu Ala Arg Leu Lys Glu
   20 25 30
   Ser His Phe Ile Val Ala Leu Lys Val Leu Phe Lys Ser Gln Ile
   35 40 45
   Glu Lys Glu Gly Leu Glu His Gln Leu Arg Arg Glu Ile Glu Ile
   50 55 60
   Gln Ala His Leu Gln His Pro Asn Ile Leu Arg Leu Tyr Asn Tyr
   65 70 75
   Phe His Asp Ala Arg Arg Val Tyr Leu Ile Leu Glu Tyr Ala Pro
   80 85 90
   Arg Gly Glu Leu Tyr Lys Glu Leu Gln Lys Ser Glu Lys Leu Asp
   95 100 105
   Glu Gln Arg Thr Ala Thr Ile Ile Glu Glu Val Ala Asp Ala Leu
   110 115 120
   Thr Tyr Cys His Asp Lys Lys Val Ile His Arg Asp Ile Lys Pro
   125 130 135
   Glu Asn Leu Leu Leu Gly Phe Arg Gly Glu Val Lys Ile Ala Asp
   140 145 150
   Phe Gly Trp Ser Val His Thr Pro Leu Pro Glu Arg Lys Thr Met
   155 160 165
   Cys Gly Thr Leu Asp Tyr Leu Pro Pro Glu Met Ile Glu Gly Arg
   170 175 180
   Thr Tyr Asp Glu Lys Val Asp Leu Trp Cys Ile Gly Val Leu Cys
   185 190 195
   Tyr Glu Leu Leu Val Gly Tyr Pro Pro Phe Glu Ser Ala Ser His
   200 205 210
   Ser Glu Thr Tyr Arg Arg Ile Leu Lys Val Asp Val Arg Phe Pro
   215 220 225
   Leu Ser Met Pro Leu Gly Ala Arg Asp Leu Ile Ser Arg Leu Leu
   230 235 240
   Arg Tyr Gln Pro Leu Glu Arg Leu Pro Leu Ala Gln Ile Leu Lys
   245 250 255
   His Pro Trp Val Gln Ala His Ser Arg Arg Val Leu Pro Pro Cys
   260 265 270
   Ala Gln Met Ala Ser
   275
<210> 58
   <211> 233
   <212> PRT
   <213> Homo sapiens
<400> 58
   Met Ser Gln Ser Asn Arg Glu Leu Val Val Asp Phe Leu Ser Tyr
   1 5 10 15
   Lys Leu Ser Gln Lys Gly Tyr Ser Trp Ser Gln Phe Ser Asp Val
   20 25 30
   Glu Glu Asn Arg Thr Glu Ala Pro Glu Gly Thr Glu Ser Glu Met
   35 40 45
   Glu Thr Pro Ser Ala Ile Asn Gly Asn Pro Ser Trp His Leu Ala
   50 55 60
   Asp Ser Pro Ala Val Asn Gly Ala Thr Gly His Ser Ser Ser Leu
   65 70 75
   Asp Ala Arg Glu Val Ile Pro Met Ala Ala Val Lys Gln Ala Leu
   80 85 90
   Arg Glu Ala Gly Asp Glu Phe Glu Leu Arg Tyr Arg Arg Ala Phe
   95 100 105
   Ser Asp Leu Thr Ser Gln Leu His Ile Thr Pro Gly Thr Ala Tyr
   110 115 120
   Gln Ser Phe Glu Gln Val Val Asn Glu Leu Phe Arg Asp Gly Val
   125 130 135
   Asn Trp Gly Arg Ile Val Ala Phe Phe Ser Phe Gly Gly Ala Leu
   140 145 150
   Cys Val Glu Ser Val Asp Lys Glu Met Gln Val Leu Val Ser Arg
   155 160 165
   Ile Ala Ala Trp Met Ala Thr Tyr Leu Asn Asp His Leu Glu Pro
   170 175 180
   Trp Ile Gln Glu Asn Gly Gly Trp Asp Thr Phe Val Glu Leu Tyr
   185 190 195
   Gly Asn Asn Ala Ala Ala Glu Ser Arg Lys Gly Gln Glu Arg Phe
   200 205 210
   Asn Arg Trp Phe Leu Thr Gly Met Thr Val Ala Gly Val Val Leu
   215 220 225
   Leu Gly Ser Leu Phe Ser Arg Lys
   230
<210> 59
   <211> 173
   <212> PRT
   <213> Homo sapiens
<400> 59
   Met Gly Arg Gly Arg Cys Val Gly Pro Ser Leu Gln Leu Arg Gly
   1 5 10 15
   Gln Glu Trp Arg Cys Ser Pro Leu Val Pro Lys Gly Gly Ala Ala
   20 25 30
   Ala Ala Glu Leu Gly Pro Gly Gly Gly Glu Asn Met Val Arg Arg
   35 40 45
   Phe Leu Val Thr Leu Arg Ile Arg Arg Ala Cys Gly Pro Pro Arg
   50 55 60
   Val Arg Val Phe Val Val His Ile Pro Arg Leu Thr Gly Glu Trp
   65 70 75
   Ala Ala Pro Gly Ala Pro Ala Ala Val Ala Leu Val Leu Met Leu
   80 85 90
   Leu Arg Ser Gln Arg Leu Gly Gln Gln Pro Leu Pro Arg Arg Pro
   95 100 105
   Gly His Asp Asp Gly Gln Arg Pro Ser Gly Gly Ala Ala Ala Ala
   110 115 120
   Pro Arg Arg Gly Ala Gln Leu Arg Arg Pro Arg His Ser His Pro
   125 130 135
   Thr Arg Ala Arg Arg Cys Pro Gly Gly Leu Pro Gly His Ala Gly
   140 145 150
   Gly Ala Ala Pro Gly Arg Gly Ala Ala Gly Arg Ala Arg Cys Leu
   155 160 165
   Gly Pro Ser Ala Arg Gly Pro Gly
   170
<210> 60
   <211> 1342
   <212> PRT
   <213> Homo sapiens
<400> 60
<210> 61
   <211> 1308
   <212> PRT
   <213> Homo sapiens
<400> 61
<210> 62
   <211> 806
   <212> PRT
   <213> Homo sapiens
<400> 62
   Met Gly Ala Pro Ala Cys Ala Leu Ala Leu Cys Val Ala Val Ala
   1 5 10 15
   Ile Val Ala Gly Ala Ser Ser Glu Ser Leu Gly Thr Glu Gln Arg
   20 25 30
   Val Val Gly Arg Ala Ala Glu Val Pro Gly Pro Glu Pro Gly Gln
   35 40 45
   Gln Glu Gln Leu Val Phe Gly Ser Gly Asp Ala Val Glu Leu Ser
   50 55 60
   Cys Pro Pro Pro Gly Gly Gly Pro Met Gly Pro Thr Val Trp Val
   65 70 75
   Lys Asp Gly Thr Gly Leu Val Pro Ser Glu Arg Val Leu Val Gly
   80 85 90
   Pro Gln Arg Leu Gln Val Leu Asn Ala Ser His Glu Asp Ser Gly
   95 100 105
   Ala Tyr Ser Cys Arg Gln Arg Leu Thr Gln Arg Val Leu Cys His
   110 115 120
   Phe Ser Val Arg Val Thr Asp Ala Pro Ser Ser Gly Asp Asp Glu
   125 130 135
   Asp Gly Glu Asp Glu Ala Glu Asp Thr Gly Val Asp Thr Gly Ala
   140 145 150
   Pro Tyr Trp Thr Arg Pro Glu Arg Met Asp Lys Lys Leu Leu Ala
   155 160 165
   Val Pro Ala Ala Asn Thr Val Arg Phe Arg Cys Pro Ala Ala Gly
   170 175 180
   Asn Pro Thr Pro Ser Ile Ser Trp Leu Lys Asn Gly Arg Glu Phe
   185 190 195
   Arg Gly Glu His Arg Ile Gly Gly Ile Lys Leu Arg His Gln Gln
   200 205 210
   Trp Ser Leu Val Met Glu Ser Val Val Pro Ser Asp Arg Gly Asn
   215 220 225
   Tyr Thr Cys Val Val Glu Asn Lys Phe Gly Ser Ile Arg Gln Thr
   230 235 240
   Tyr Thr Leu Asp Val Leu Glu Arg Ser Pro His Arg Pro Ile Leu
   245 250 255
   Gln Ala Gly Leu Pro Ala Asn Gln Thr Ala Val Leu Gly Ser Asp
   260 265 270
   Val Glu Phe His Cys Lys Val Tyr Ser Asp Ala Gln Pro His Ile
   275 280 285
   Gln Trp Leu Lys His Val Glu Val Asn Gly Ser Lys Val Gly Pro
   290 295 300
   Asp Gly Thr Pro Tyr Val Thr Val Leu Lys Thr Ala Gly Ala Asn
   305 310 315
   Thr Thr Asp Lys Glu Leu Glu Val Leu Ser Leu His Asn Val Thr
   320 325 330
   Phe Glu Asp Ala Gly Glu Tyr Thr Cys Leu Ala Gly Asn Ser Ile
   335 340 345
   Gly Phe Ser His His Ser Ala Trp Leu Val Val Leu Pro Ala Glu
   350 355 360
   Glu Glu Leu Val Glu Ala Asp Glu Ala Gly Ser Val Tyr Ala Gly
   365 370 375
   Ile Leu Ser Tyr Gly Val Gly Phe Phe Leu Phe Ile Leu Val Val
   380 385 390
   Ala Ala Val Thr Leu Cys Arg Leu Arg Ser Pro Pro Lys Lys Gly
   395 400 405
   Leu Gly Ser Pro Thr Val His Lys Ile Ser Arg Phe Pro Leu Lys
   410 415 420
   Arg Gln Val Ser Leu Glu Ser Asn Ala Ser Met Ser Ser Asn Thr
   425 430 435
   Pro Leu Val Arg Ile Ala Arg Leu Ser Ser Gly Glu Gly Pro Thr
   440 445 450
   Leu Ala Asn Val Ser Glu Leu Glu Leu Pro Ala Asp Pro Lys Trp
   455 460 465
   Glu Leu Ser Arg Ala Arg Leu Thr Leu Gly Lys Pro Leu Gly Glu
   470 475 480
   Gly Cys Phe Gly Gln Val Val Met Ala Glu Ala Ile Gly Ile Asp
   485 490 495
   Lys Asp Arg Ala Ala Lys Pro Val Thr Val Ala Val Lys Met Leu
   500 505 510
   Lys Asp Asp Ala Thr Asp Lys Asp Leu Ser Asp Leu Val Ser Glu
   515 520 525
   Met Glu Met Met Lys Met Ile Gly Lys His Lys Asn Ile Ile Asn
   530 535 540
   Leu Leu Gly Ala Cys Thr Gln Gly Gly Pro Leu Tyr Val Leu Val
   545 550 555
   Glu Tyr Ala Ala Lys Gly Asn Leu Arg Glu Phe Leu Arg Ala Arg
   560 565 570
   Arg Pro Pro Gly Leu Asp Tyr Ser Phe Asp Thr Cys Lys Pro Pro
   575 580 585
   Glu Glu Gln Leu Thr Phe Lys Asp Leu Val Ser Cys Ala Tyr Gln
   590 595 600
   Val Ala Arg Gly Met Glu Tyr Leu Ala Ser Gln Lys Cys Ile His
   605 610 615
   Arg Asp Leu Ala Ala Arg Asn Val Leu Val Thr Glu Asp Asn Val
   620 625 630
   Met Lys Ile Ala Asp Phe Gly Leu Ala Arg Asp Val His Asn Leu
   635 640 645
   Asp Tyr Tyr Lys Lys Thr Thr Asn Gly Arg Leu Pro Val Lys Trp
   650 655 660
   Met Ala Pro Glu Ala Leu Phe Asp Arg Val Tyr Thr His Gln Ser
   665 670 675
   Asp Val Trp Ser Phe Gly Val Leu Leu Trp Glu Ile Phe Thr Leu
   680 685 690
   Gly Gly Ser Pro Tyr Pro Gly Ile Pro Val Glu Glu Leu Phe Lys
   695 700 705
   Leu Leu Lys Glu Gly His Arg Met Asp Lys Pro Ala Asn Cys Thr
   710 715 720
   His Asp Leu Tyr Met Ile Met Arg Glu Cys Trp His Ala Ala Pro
   725 730 735
   Ser Gln Arg Pro Thr Phe Lys Gln Leu Val Glu Asp Leu Asp Arg
   740 745 750
   Val Leu Thr Val Thr Ser Thr Asp Glu Tyr Leu Asp Leu Ser Ala
   755 760 765
   Pro Phe Glu Gln Tyr Ser Pro Gly Gly Gln Asp Thr Pro Ser Ser
   770 775 780
   Ser Ser Ser Gly Asp Asp Ser Val Phe Ala His Asp Leu Leu Pro
   785 790 795
   Pro Ala Pro Pro Ser Ser Gly Gly Ser Arg Thr
   800 805
<210> 63
   <211> 2549
   <212> PRT
   <213> Homo sapiens
<400> 63
   Met Leu Gly Thr Gly Pro Ala Ala Ala Thr Thr Ala Ala Thr Thr
   1 5 10 15
   Ser Ser Asn Val Ser Val Leu Gln Gln Phe Ala Ser Gly Leu Lys
   20 25 30
   Ser Arg Asn Glu Glu Thr Arg Ala Lys Ala Ala Lys Glu Leu Gln
   35 40 45
   His Tyr Val Thr Met Glu Leu Arg Glu Met Ser Gln Glu Glu Ser
   50 55 60
   Thr Arg Phe Tyr Asp Gln Leu Asn His His Ile Phe Glu Leu Val
   65 70 75
   Ser Ser Ser Asp Ala Asn Glu Arg Lys Gly Gly Ile Leu Ala Ile
   80 85 90
   Ala Ser Leu Ile Gly Val Glu Gly Gly Asn Ala Thr Arg Ile Gly
   95 100 105
   Arg Phe Ala Asn Tyr Leu Arg Asn Leu Leu Pro Ser Asn Asp Pro
   110 115 120
   Val Val Met Glu Met Ala Ser Lys Ala Ile Gly Arg Leu Ala Met
   125 130 135
   Ala Gly Asp Thr Phe Thr Ala Glu Tyr Val Glu Phe Glu Val Lys
   140 145 150
   Arg Ala Leu Glu Trp Leu Gly Ala Asp Arg Asn Glu Gly Arg Arg
   155 160 165
   His Ala Ala Val Leu Val Leu Arg Glu Leu Ala Ile Ser Val Pro
   170 175 180
   Thr Phe Phe Phe Gln Gln Val Gln Pro Phe Phe Asp Asn Ile Phe
   185 190 195
   Val Ala Val Trp Asp Pro Lys Gln Ala Ile Arg Glu Gly Ala Val
   200 205 210
   Ala Ala Leu Arg Ala Cys Leu Ile Leu Thr Thr Gln Arg Glu Pro
   215 220 225
   Lys Glu Met Gln Lys Pro Gln Trp Tyr Arg His Thr Phe Glu Glu
   230 235 240
   Ala Glu Lys Gly Phe Asp Glu Thr Leu Ala Lys Glu Lys Gly Met
   245 250 255
   Asn Arg Asp Asp Arg Ile His Gly Ala Leu Leu Ile Leu Asn Glu
   260 265 270
   Leu Val Arg Ile Ser Ser Met Glu Gly Glu Arg Leu Arg Glu Glu
   275 280 285
   Met Glu Glu Ile Thr Gln Gln Gln Leu Val His Asp Lys Tyr Cys
   290 295 300
   Lys Asp Leu Met Gly Phe Gly Thr Lys Pro Arg His Ile Thr Pro
   305 310 315
   Phe Thr Ser Phe Gln Ala Val Gln Pro Gln Gln Ser Asn Ala Leu
   320 325 330
   Val Gly Leu Leu Gly Tyr Ser Ser His Gln Gly Leu Met Gly Phe
   335 340 345
   Gly Thr Ser Pro Ser Pro Ala Lys Ser Thr Leu Val Glu Ser Arg
   350 355 360
   Cys Cys Arg Asp Leu Met Glu Glu Lys Phe Asp Gln Val Cys Gln
   365 370 375
   Trp Val Leu Lys Cys Arg Asn Ser Lys Asn Ser Leu Ile Gln Met
   380 385 390
   Thr Ile Leu Asn Leu Leu Pro Arg Leu Ala Ala Phe Arg Pro Ser
   395 400 405
   Ala Phe Thr Asp Thr Gln Tyr Leu Gln Asp Thr Met Asn His Val
   410 415 420
   Leu Ser Cys Val Lys Lys Glu Lys Glu Arg Thr Ala Ala Phe Gln
   425 430 435
   Ala Leu Gly Leu Leu Ser Val Ala Val Arg Ser Glu Phe Lys Val
   440 445 450
   Tyr Leu Pro Arg Val Leu Asp Ile Ile Arg Ala Ala Leu Pro Pro
   455 460 465
   Lys Asp Phe Ala His Lys Arg Gln Lys Ala Met Gln Val Asp Ala
   470 475 480
   Thr Val Phe Thr Cys Ile Ser Met Leu Ala Arg Ala Met Gly Pro
   485 490 495
   Gly Ile Gln Gln Asp Ile Lys Glu Leu Leu Glu Pro Met Leu Ala
   500 505 510
   Val Gly Leu Ser Pro Ala Leu Thr Ala Val Leu Tyr Asp Leu Ser
   515 520 525
   Arg Gln Ile Pro Gln Leu Lys Lys Asp Ile Gln Asp Gly Leu Leu
   530 535 540
   Lys Met Leu Ser Leu Val Leu Met His Lys Pro Leu Arg His Pro
   545 550 555
   Gly Met Pro Lys Gly Leu Ala His Gln Leu Ala Ser Pro Gly Leu
   560 565 570
   Thr Thr Leu Pro Glu Ala Ser Asp Val Gly Ser Ile Thr Leu Ala
   575 580 585
   Leu Arg Thr Leu Gly Ser Phe Glu Phe Glu Gly His Ser Leu Thr
   590 595 600
   Gln Phe Val Arg His Cys Ala Asp His Phe Leu Asn Ser Glu His
   605 610 615
   Lys Glu Ile Arg Met Glu Ala Ala Arg Thr Cys Ser Arg Leu Leu
   620 625 630
   Thr Pro Ser Ile His Leu Ile Ser Gly His Ala His Val Val Ser
   635 640 645
   Gln Thr Ala Val Gln Val Val Ala Asp Val Leu Ser Lys Leu Leu
   650 655 660
   Val Val Gly Ile Thr Asp Pro Asp Pro Asp Ile Arg Tyr Cys Val
   665 670 675
   Leu Ala Ser Leu Asp Glu Arg Phe Asp Ala His Leu Ala Gln Ala
   680 685 690
   Glu Asn Leu Gln Ala Leu Phe Val Ala Leu Asn Asp Gln Val Phe
   695 700 705
   Glu Ile Arg Glu Leu Ala Ile Cys Thr Val Gly Arg Leu Ser Ser
   710 715 720
   Met Asn Pro Ala Phe Val Met Pro Phe Leu Arg Lys Met Leu Ile
   725 730 735
   Gln Ile Leu Thr Glu Leu Glu His Ser Gly Ile Gly Arg Ile Lys
   740 745 750
   Glu Gln Ser Ala Arg Met Leu Gly His Leu Val Ser Asn Ala Pro
   755 760 765
   Arg Leu Ile Arg Pro Tyr Met Glu Pro Ile Leu Lys Ala Leu Ile
   770 775 780
   Leu Lys Leu Lys Asp Pro Asp Pro Asp Pro Asn Pro Gly Val Ile
   785 790 795
   Asn Asn Val Leu Ala Thr Ile Gly Glu Leu Ala Gln Val Ser Gly
   800 805 810
   Leu Glu Met Arg Lys Trp Val Asp Glu Leu Phe Ile Ile Ile Met
   815 820 825
   Asp Met Leu Gln Asp Ser Ser Leu Leu Ala Lys Arg Gln Val Ala
   830 835 840
   Leu Trp Thr Leu Gly Gln Leu Val Ala Ser Thr Gly Tyr Val Val
   845 850 855
   Glu Pro Tyr Arg Lys Tyr Pro Thr Leu Leu Glu Val Leu Leu Asn
   860 865 870
   Phe Leu Lys Thr Glu Gln Asn Gln Gly Thr Arg Arg Glu Ala Ile
   875 880 885
   Arg Val Leu Gly Leu Leu Gly Ala Leu Asp Pro Tyr Lys His Lys
   890 895 900
   Val Asn Ile Gly Met Ile Asp Gln Ser Arg Asp Ala Ser Ala Val
   905 910 915
   Ser Leu Ser Glu Ser Lys Ser Ser Gln Asp Ser Ser Asp Tyr Ser
   920 925 930
   Thr Ser Glu Met Leu Val Asn Met Gly Asn Leu Pro Leu Asp Glu
   935 940 945
   Phe Tyr Pro Ala Val Ser Met Val Ala Leu Met Arg Ile Phe Arg
   950 955 960
   Asp Gln Ser Leu Ser His His His Thr Met Val Val Gln Ala Ile
   965 970 975
   Thr Phe Ile Phe Lys Ser Leu Gly Leu Lys Cys Val Gln Phe Leu
   980 985 990
   Pro Gln Val Met Pro Thr Phe Leu Asn Val Ile Arg Val Cys Asp
   995 1000 1005
   Gly Ala Ile Arg Glu Phe Leu Phe Gln Gln Leu Gly Met Leu Val
   1010 1015 1020
   Ser Phe Val Lys Ser His Ile Arg Pro Tyr Met Asp Glu Ile Val
   1025 1030 1035
   Thr Leu Met Arg Glu Phe Trp Val Met Asn Thr Ser Ile Gln Ser
   1040 1045 1050
   Thr Ile Ile Leu Leu Ile Glu Gln Ile Val Val Ala Leu Gly Gly
   1055 1060 1065
   Glu Phe Lys Leu Tyr Leu Pro Gln Leu Ile Pro His Met Leu Arg
   1070 1075 1080
   Val Phe Met His Asp Asn Ser Pro Gly Arg Ile Val Ser Ile Lys
   1085 1090 1095
   Leu Leu Ala Ala Ile Gln Leu Phe Gly Ala Asn Leu Asp Asp Tyr
   1100 1105 1110
   Leu His Leu Leu Leu Pro Pro Ile Val Lys Leu Phe Asp Ala Pro
   1115 1120 1125
   Glu Ala Pro Leu Pro Ser Arg Lys Ala Ala Leu Glu Thr Val Asp
   1130 1135 1140
   Arg Leu Thr Glu Ser Leu Asp Phe Thr Asp Tyr Ala Ser Arg Ile
   1145 1150 1155
   Ile His Pro Ile Val Arg Thr Leu Asp Gln Ser Pro Glu Leu Arg
   1160 1165 1170
   Ser Thr Ala Met Asp Thr Leu Ser Ser Leu Val Phe Gln Leu Gly
   1175 1180 1185
   Lys Lys Tyr Gln Ile Phe Ile Pro Met Val Asn Lys Val Leu Val
   1190 1195 1200
   Arg His Arg Ile Asn His Gln Arg Tyr Asp Val Leu Ile Cys Arg
   1205 1210 1215
   Ile Val Lys Gly Tyr Thr Leu Ala Asp Glu Glu Glu Asp Pro Leu
   1220 1225 1230
   Ile Tyr Gln His Arg Met Leu Arg Ser Gly Gln Gly Asp Ala Leu
   1235 1240 1245
   Ala Ser Gly Pro Val Glu Thr Gly Pro Met Lys Lys Leu His Val
   1250 1255 1260
   Ser Thr Ile Asn Leu Gln Lys Ala Trp Gly Ala Ala Arg Arg Val
   1265 1270 1275
   Ser Lys Asp Asp Trp Leu Glu Trp Leu Arg Arg Leu Ser Leu Glu
   1280 1285 1290
   Leu Leu Lys Asp Ser Ser Ser Pro Ser Leu Arg Ser Cys Trp Ala
   1295 1300 1305
   Leu Ala Gln Ala Tyr Asn Pro Met Ala Arg Asp Leu Phe Asn Ala
   1310 1315 1320
   Ala Phe Val Ser Cys Trp Ser Glu Leu Asn Glu Asp Gln Gln Asp
   1325 1330 1335
   Glu Leu Ile Arg Ser Ile Glu Leu Ala Leu Thr Ser Gln Asp Ile
   1340 1345 1350
   Ala Glu Val Thr Gln Thr Leu Leu Asn Leu Ala Glu Phe Met Glu
   1355 1360 1365
   His Ser Asp Lys Gly Pro Leu Pro Leu Arg Asp Asp Asn Gly Ile
   1370 1375 1380
   Val Leu Leu Gly Glu Arg Ala Ala Lys Cys Arg Ala Tyr Ala Lys
   1385 1390 1395
   Ala Leu His Tyr Lys Glu Leu Glu Phe Gln Lys Gly Pro Thr Pro
   1400 1405 1410
   Ala Ile Leu Glu Ser Leu Ile Ser Ile Asn Asn Lys Leu Gln Gln
   1415 1420 1425
   Pro Glu Ala Ala Ala Gly Val Leu Glu Tyr Ala Met Lys His Phe
   1430 1435 1440
   Gly Glu Leu Glu Ile Gln Ala Thr Trp Tyr Glu Lys Leu His Glu
   1445 1450 1455
   Trp Glu Asp Ala Leu Val Ala Tyr Asp Lys Lys Met Asp Thr Asn
   1460 1465 1470
   Lys Asp Asp Pro Glu Leu Met Leu Gly Arg Met Arg Cys Leu Glu
   1475 1480 1485
   Ala Leu Gly Glu Trp Gly Gln Leu His Gln Gln Cys Cys Glu Lys
   1490 1495 1500
   Trp Thr Leu Val Asn Asp Glu Thr Gln Ala Lys Met Ala Arg Met
   1505 1510 1515
   Ala Ala Ala Ala Ala Trp Gly Leu Gly Gln Trp Asp Ser Met Glu
   1520 1525 1530
   Glu Tyr Thr Cys Met Ile Pro Arg Asp Thr His Asp Gly Ala Phe
   1535 1540 1545
   Tyr Arg Ala Val Leu Ala Leu His Gln Asp Leu Phe Ser Leu Ala
   1550 1555 1560
   Gln Gln Cys Ile Asp Lys Ala Arg Asp Leu Leu Asp Ala Glu Leu
   1565 1570 1575
   Thr Ala Met Ala Gly Glu Ser Tyr Ser Arg Ala Tyr Gly Ala Met
   1580 1585 1590
   Val Ser Cys His Met Leu Ser Glu Leu Glu Glu Val Ile Gln Tyr
   1595 1600 1605
   Lys Leu Val Pro Glu Arg Arg Glu Ile Ile Arg Gln Ile Trp Trp
   1610 1615 1620
   Glu Arg Leu Gln Gly Cys Gln Arg Ile Val Glu Asp Trp Gln Lys
   1625 1630 1635
   Ile Leu Met Val Arg Ser Leu Val Val Ser Pro His Glu Asp Met
   1640 1645 1650
   Arg Thr Trp Leu Lys Tyr Ala Ser Leu Cys Gly Lys Ser Gly Arg
   1655 1660 1665
   Leu Ala Leu Ala His Lys Thr Leu Val Leu Leu Leu Gly Val Asp
   1670 1675 1680
   Pro Ser Arg Gln Leu Asp His Pro Leu Pro Thr Val His Pro Gln
   1685 1690 1695
   Val Thr Tyr Ala Tyr Met Lys Asn Met Trp Lys Ser Ala Arg Lys
   1700 1705 1710
   Ile Asp Ala Phe Gln His Met Gln His Phe Val Gln Thr Met Gln
   1715 1720 1725
   Gln Gln Ala Gln His Ala Ile Ala Thr Glu Asp Gln Gln His Lys
   1730 1735 1740
   Gln Glu Leu His Lys Leu Met Ala Arg Cys Phe Leu Lys Leu Gly
   1745 1750 1755
   Glu Trp Gln Leu Asn Leu Gln Gly Ile Asn Glu Ser Thr Ile Pro
   1760 1765 1770
   Lys Val Leu Gln Tyr Tyr Ser Ala Ala Thr Glu His Asp Arg Ser
   1775 1780 1785
   Trp Tyr Lys Ala Trp His Ala Trp Ala Val Met Asn Phe Glu Ala
   1790 1795 1800
   Val Leu His Tyr Lys His Gln Asn Gln Ala Arg Asp Glu Lys Lys
   1805 1810 1815
   Lys Leu Arg His Ala Ser Gly Ala Asn Ile Thr Asn Ala Thr Thr
   1820 1825 1830
   Ala Ala Thr Thr Ala Ala Thr Ala Thr Thr Thr Ala Ser Thr Glu
   1835 1840 1845
   Gly Ser Asn Ser Glu Ser Glu Ala Glu Ser Thr Glu Asn Ser Pro
   1850 1855 1860
   Thr Pro Ser Pro Leu Gln Lys Lys Val Thr Glu Asp Leu Ser Lys
   1865 1870 1875
   Thr Leu Leu Met Tyr Thr Val Pro Ala Val Gln Gly Phe Phe Arg
   1880 1885 1890
   Ser Ile Ser Leu Ser Arg Gly Asn Asn Leu Gln Asp Thr Leu Arg
   1895 1900 1905
   Val Leu Thr Leu Trp Phe Asp Tyr Gly His Trp Pro Asp Val Asn
   1910 1915 1920
   Glu Ala Leu Val Glu Gly Val Lys Ala Ile Gln Ile Asp Thr Trp
   1925 1930 1935
   Leu Gln Val Ile Pro Gln Leu Ile Ala Arg Ile Asp Thr Pro Arg
   1940 1945 1950
   Pro Leu Val Gly Arg Leu Ile His Gln Leu Leu Thr Asp Ile Gly
   1955 1960 1965
   Arg Tyr His Pro Gln Ala Leu Ile Tyr Pro Leu Thr Val Ala Ser
   1970 1975 1980
   Lys Ser Thr Thr Thr Ala Arg His Asn Ala Ala Asn Lys Ile Leu
   1985 1990 1995
   Lys Asn Met Cys Glu His Ser Asn Thr Leu Val Gln Gln Ala Met
   2000 2005 2010
   Met Val Ser Glu Glu Leu Ile Arg Val Ala Ile Leu Trp His Glu
   2015 2020 2025
   Met Trp His Glu Gly Leu Glu Glu Ala Ser Arg Leu Tyr Phe Gly
   2030 2035 2040
   Glu Arg Asn Val Lys Gly Met Phe Glu Val Leu Glu Pro Leu His
   2045 2050 2055
   Ala Met Met Glu Arg Gly Pro Gln Thr Leu Lys Glu Thr Ser Phe
   2060 2065 2070
   Asn Gln Ala Tyr Gly Arg Asp Leu Met Glu Ala Gln Glu Trp Cys
   2075 2080 2085
   Arg Lys Tyr Met Lys Ser Gly Asn Val Lys Asp Leu Thr Gln Ala
   2090 2095 2100
   Trp Asp Leu Tyr Tyr His Val Phe Arg Arg Ile Ser Lys Gln Leu
   2105 2110 2115
   Pro Gln Leu Thr Ser Leu Glu Leu Gln Tyr Val Ser Pro Lys Leu
   2120 2125 2130
   Leu Met Cys Arg Asp Leu Glu Leu Ala Val Pro Gly Thr Tyr Asp
   2135 2140 2145
   Pro Asn Gln Pro Ile Ile Arg Ile Gln Ser Ile Ala Pro Ser Leu
   2150 2155 2160
   Gln Val Ile Thr Ser Lys Gln Arg Pro Arg Lys Leu Thr Leu Met
   2165 2170 2175
   Gly Ser Asn Gly His Glu Phe Val Phe Leu Leu Lys Gly His Glu
   2180 2185 2190
   Asp Leu Arg Gln Asp Glu Arg Val Met Gln Leu Phe Gly Leu Val
   2195 2200 2205
   Asn Thr Leu Leu Ala Asn Asp Pro Thr Ser Leu Arg Lys Asn Leu
   2210 2215 2220
   Ser Ile Gln Arg Tyr Ala Val Ile Pro Leu Ser Thr Asn Ser Gly
   2225 2230 2235
   Leu Ile Gly Trp Val Pro His Cys Asp Thr Leu His Ala Leu Ile
   2240 2245 2250
   Arg Asp Tyr Arg Glu Lys Lys Lys Ile Leu Leu Asn Ile Glu His
   2255 2260 2265
   Arg Ile Met Leu Arg Met Ala Pro Asp Tyr Asp His Leu Thr Leu
   2270 2275 2280
   Met Gln Lys Val Glu Val Phe Glu His Ala Val Asn Asn Thr Ala
   2285 2290 2295
   Gly Asp Asp Leu Ala Lys Leu Leu Trp Leu Lys Ser Pro Ser Ser
   2300 2305 2310
   Glu Val Trp Phe Asp Arg Arg Thr Asn Tyr Thr Arg Ser Leu Ala
   2315 2320 2325
   Val Met Ser Met Val Gly Tyr Ile Leu Gly Leu Gly Asp Arg His
   2330 2335 2340
   Pro Ser Asn Leu Met Leu Asp Arg Leu Ser Gly Lys Ile Leu His
   2345 2350 2355
   Ile Asp Phe Gly Asp Cys Phe Glu Val Ala Met Thr Arg Glu Lys
   2360 2365 2370
   Phe Pro Glu Lys Ile Pro Phe Arg Leu Thr Arg Met Leu Thr Asn
   2375 2380 2385
   Ala Met Glu Val Thr Gly Leu Asp Gly Asn Tyr Arg Ile Thr Cys
   2390 2395 2400
   His Thr Val Met Glu Val Leu Arg Glu His Lys Asp Ser Val Met
   2405 2410 2415
   Ala Val Leu Glu Ala Phe Val Tyr Asp Pro Leu Leu Asn Trp Arg
   2420 2425 2430
   Leu Met Asp Thr Asn Thr Lys Gly Asn Lys Arg Ser Arg Thr Arg
   2435 2440 2445
   Thr Asp Ser Tyr Ser Ala Gly Gln Ser Val Glu Ile Leu Asp Gly
   2450 2455 2460
   Val Glu Leu Gly Glu Pro Ala His Lys Lys Thr Gly Thr Thr Val
   2465 2470 2475
   Pro Glu Ser Ile His Ser Phe Ile Gly Asp Gly Leu Val Lys Pro
   2480 2485 2490
   Glu Ala Leu Asn Lys Lys Ala Ile Gln Ile Ile Asn Arg Val Arg
   2495 2500 2505
   Asp Lys Leu Thr Gly Arg Asp Phe Ser His Asp Asp Thr Leu Asp
   2510 2515 2520
   Val Pro Thr Gln Val Glu Leu Leu Ile Lys Gln Ala Thr Ser His
   2525 2530 2535
   Glu Asn Leu Cys Gln Cys Tyr Ile Gly Trp Cys Pro Phe Trp
   2540 2545
<210> 64
   <211> 1356
   <212> PRT
   <213> Homo sapiens
<400> 64
<210> 65
   <211> 400
   <212> PRT
   <213> Homo sapiens
<400> 65
<210> 66
   <211> 1390
   <212> PRT
   <213> Homo sapiens
<400> 66
   Met Lys Ala Pro Ala Val Leu Ala Pro Gly Ile Leu Val Leu Leu
   1 5 10 15
   Phe Thr Leu Val Gln Arg Ser Asn Gly Glu Cys Lys Glu Ala Leu
   20 25 30
   Ala Lys Ser Glu Met Asn Val Asn Met Lys Tyr Gln Leu Pro Asn
   35 40 45
   Phe Thr Ala Glu Thr Pro Ile Gln Asn Val Ile Leu His Glu His
   50 55 60
   His Ile Phe Leu Gly Ala Thr Asn Tyr Ile Tyr Val Leu Asn Glu
   65 70 75
   Glu Asp Leu Gln Lys Val Ala Glu Tyr Lys Thr Gly Pro Val Leu
   80 85 90
   Glu His Pro Asp Cys Phe Pro Cys Gln Asp Cys Ser Ser Lys Ala
   95 100 105
   Asn Leu Ser Gly Gly Val Trp Lys Asp Asn Ile Asn Met Ala Leu
   110 115 120
   Val Val Asp Thr Tyr Tyr Asp Asp Gln Leu Ile Ser Cys Gly Ser
   125 130 135
   Val Asn Arg Gly Thr Cys Gln Arg His Val Phe Pro His Asn His
   140 145 150
   Thr Ala Asp Ile Gln Ser Glu Val His Cys Ile Phe Ser Pro Gln
   155 160 165
   Ile Glu Glu Pro Ser Gln Cys Pro Asp Cys Val Val Ser Ala Leu
   170 175 180
   Gly Ala Lys Val Leu Ser Ser Val Lys Asp Arg Phe Ile Asn Phe
   185 190 195
   Phe Val Gly Asn Thr Ile Asn Ser Ser Tyr Phe Pro Asp His Pro
   200 205 210
   Leu His Ser Ile Ser Val Arg Arg Leu Lys Glu Thr Lys Asp Gly
   215 220 225
   Phe Met Phe Leu Thr Asp Gln Ser Tyr Ile Asp Val Leu Pro Glu
   230 235 240
   Phe Arg Asp Ser Tyr Pro Ile Lys Tyr Val His Ala Phe Glu Ser
   245 250 255
   Asn Asn Phe Ile Tyr Phe Leu Thr Val Gln Arg Glu Thr Leu Asp
   260 265 270
   Ala Gln Thr Phe His Thr Arg Ile Ile Arg Phe Cys Ser Ile Asn
   275 280 285
   Ser Gly Leu His Ser Tyr Met Glu Met Pro Leu Glu Cys Ile Leu
   290 295 300
   Thr Glu Lys Arg Lys Lys Arg Ser Thr Lys Lys Glu Val Phe Asn
   305 310 315
   Ile Leu Gln Ala Ala Tyr Val Ser Lys Pro Gly Ala Gln Leu Ala
   320 325 330
   Arg Gln Ile Gly Ala Ser Leu Asn Asp Asp Ile Leu Phe Gly Val
   335 340 345
   Phe Ala Gln Ser Lys Pro Asp Ser Ala Glu Pro Met Asp Arg Ser
   350 355 360
   Ala Met Cys Ala Phe Pro Ile Lys Tyr Val Asn Asp Phe Phe Asn
   365 370 375
   Lys Ile Val Asn Lys Asn Asn Val Arg Cys Leu Gln His Phe Tyr
   380 385 390
   Gly Pro Asn His Glu His Cys Phe Asn Arg Thr Leu Leu Arg Asn
   395 400 405
   Ser Ser Gly Cys Glu Ala Arg Arg Asp Glu Tyr Arg Thr Glu Phe
   410 415 420
   Thr Thr Ala Leu Gln Arg Val Asp Leu Phe Met Gly Gln Phe Ser
   425 430 435
   Glu Val Leu Leu Thr Ser Ile Ser Thr Phe Ile Lys Gly Asp Leu
   440 445 450
   Thr Ile Ala Asn Leu Gly Thr Ser Glu Gly Arg Phe Met Gln Val
   455 460 465
   Val Val Ser Arg Ser Gly Pro Ser Thr Pro His Val Asn Phe Leu
   470 475 480
   Leu Asp Ser His Pro Val Ser Pro Glu Val Ile Val Glu His Thr
   485 490 495
   Leu Asn Gln Asn Gly Tyr Thr Leu Val Ile Thr Gly Lys Lys Ile
   500 505 510
   Thr Lys Ile Pro Leu Asn Gly Leu Gly Cys Arg His Phe Gln Ser
   515 520 525
   Cys Ser Gln Cys Leu Ser Ala Pro Pro Phe Val Gln Cys Gly Trp
   530 535 540
   Cys His Asp Lys Cys Val Arg Ser Glu Glu Cys Leu Ser Gly Thr
   545 550 555
   Trp Thr Gln Gln Ile Cys Leu Pro Ala Ile Tyr Lys Val Phe Pro
   560 565 570
   Asn Ser Ala Pro Leu Glu Gly Gly Thr Arg Leu Thr Ile Cys Gly
   575 580 585
   Trp Asp Phe Gly Phe Arg Arg Asn Asn Lys Phe Asp Leu Lys Lys
   590 595 600
   Thr Arg Val Leu Leu Gly Asn Glu Ser Cys Thr Leu Thr Leu Ser
   605 610 615
   Glu Ser Thr Met Asn Thr Leu Lys Cys Thr Val Gly Pro Ala Met
   620 625 630
   Asn Lys His Phe Asn Met Ser Ile Ile Ile Ser Asn Gly His Gly
   635 640 645
   Thr Thr Gln Tyr Ser Thr Phe Ser Tyr Val Asp Pro Val Ile Thr
   650 655 660
   Ser Ile Ser Pro Lys Tyr Gly Pro Met Ala Gly Gly Thr Leu Leu
   665 670 675
   Thr Leu Thr Gly Asn Tyr Leu Asn Ser Gly Asn Ser Arg His Ile
   680 685 690
   Ser Ile Gly Gly Lys Thr Cys Thr Leu Lys Ser Val Ser Asn Ser
   695 700 705
   Ile Leu Glu Cys Tyr Thr Pro Ala Gln Thr Ile Ser Thr Glu Phe
   710 715 720
   Ala Val Lys Leu Lys Ile Asp Leu Ala Asn Arg Glu Thr Ser Ile
   725 730 735
   Phe Ser Tyr Arg Glu Asp Pro Ile Val Tyr Glu Ile His Pro Thr
   740 745 750
   Lys Ser Phe Ile Ser Gly Gly Ser Thr Ile Thr Gly Val Gly Lys
   755 760 765
   Asn Leu Asn Ser Val Ser Val Pro Arg Met Val Ile Asn Val His
   770 775 780
   Glu Ala Gly Arg Asn Phe Thr Val Ala Cys Gln His Arg Ser Asn
   785 790 795
   Ser Glu Ile Ile Cys Cys Thr Thr Pro Ser Leu Gln Gln Leu Asn
   800 805 810
   Leu Gln Leu Pro Leu Lys Thr Lys Ala Phe Phe Met Leu Asp Gly
   815 820 825
   Ile Leu Ser Lys Tyr Phe Asp Leu Ile Tyr Val His Asn Pro Val
   830 835 840
   Phe Lys Pro Phe Glu Lys Pro Val Met Ile Ser Met Gly Asn Glu
   845 850 855
   Asn Val Leu Glu Ile Lys Gly Asn Asp Ile Asp Pro Glu Ala Val
   860 865 870
   Lys Gly Glu Val Leu Lys Val Gly Asn Lys Ser Cys Glu Asn Ile
   875 880 885
   His Leu His Ser Glu Ala Val Leu Cys Thr Val Pro Asn Asp Leu
   890 895 900
   Leu Lys Leu Asn Ser Glu Leu Asn Ile Glu Trp Lys Gln Ala Ile
   905 910 915
   Ser Ser Thr Val Leu Gly Lys Val Ile Val Gln Pro Asp Gln Asn
   920 925 930
   Phe Thr Gly Leu Ile Ala Gly Val Val Ser Ile Ser Thr Ala Leu
   935 940 945
   Leu Leu Leu Leu Gly Phe Phe Leu Trp Leu Lys Lys Arg Lys Gln
   950 955 960
   Ile Lys Asp Leu Gly Ser Glu Leu Val Arg Tyr Asp Ala Arg Val
   965 970 975
   His Thr Pro His Leu Asp Arg Leu Val Ser Ala Arg Ser Val Ser
   980 985 990
   Pro Thr Thr Glu Met Val Ser Asn Glu Ser Val Asp Tyr Arg Ala
   995 1000 1005
   Thr Phe Pro Glu Asp Gln Phe Pro Asn Ser Ser Gln Asn Gly Ser
   1010 1015 1020
   Cys Arg Gln Val Gln Tyr Pro Leu Thr Asp Met Ser Pro Ile Leu
   1025 1030 1035
   Thr Ser Gly Asp Ser Asp Ile Ser Ser Pro Leu Leu Gln Asn Thr
   1040 1045 1050
   Val His Ile Asp Leu Ser Ala Leu Asn Pro Glu Leu Val Gln Ala
   1055 1060 1065
   Val Gln His Val Val Ile Gly Pro Ser Ser Leu Ile Val His Phe
   1070 1075 1080
   Asn Glu Val Ile Gly Arg Gly His Phe Gly Cys Val Tyr His Gly
   1085 1090 1095
   Thr Leu Leu Asp Asn Asp Gly Lys Lys Ile His Cys Ala Val Lys
   1100 1105 1110
   Ser Leu Asn Arg Ile Thr Asp Ile Gly Glu Val Ser Gln Phe Leu
   1115 1120 1125
   Thr Glu Gly Ile Ile Met Lys Asp Phe Ser His Pro Asn Val Leu
   1130 1135 1140
   Ser Leu Leu Gly Ile Cys Leu Arg Ser Glu Gly Ser Pro Leu Val
   1145 1150 1155
   Val Leu Pro Tyr Met Lys His Gly Asp Leu Arg Asn Phe Ile Arg
   1160 1165 1170
   Asn Glu Thr His Asn Pro Thr Val Lys Asp Leu Ile Gly Phe Gly
   1175 1180 1185
   Leu Gln Val Ala Lys Gly Met Lys Tyr Leu Ala Ser Lys Lys Phe
   1190 1195 1200
   Val His Arg Asp Leu Ala Ala Arg Asn Cys Met Leu Asp Glu Lys
   1205 1210 1215
   Phe Thr Val Lys Val Ala Asp Phe Gly Leu Ala Arg Asp Met Tyr
   1220 1225 1230
   Asp Lys Glu Tyr Tyr Ser Val His Asn Lys Thr Gly Ala Lys Leu
   1235 1240 1245
   Pro Val Lys Trp Met Ala Leu Glu Ser Leu Gln Thr Gln Lys Phe
   1250 1255 1260
   Thr Thr Lys Ser Asp Val Trp Ser Phe Gly Val Leu Leu Trp Glu
   1265 1270 1275
   Leu Met Thr Arg Gly Ala Pro Pro Tyr Pro Asp Val Asn Thr Phe
   1280 1285 1290
   Asp Ile Thr Val Tyr Leu Leu Gln Gly Arg Arg Leu Leu Gln Pro
   1295 1300 1305
   Glu Tyr Cys Pro Asp Pro Leu Tyr Glu Val Met Leu Lys Cys Trp
   1310 1315 1320
   His Pro Lys Ala Glu Met Arg Pro Ser Phe Ser Glu Leu Val Ser
   1325 1330 1335
   Arg Ile Ser Ala Ile Phe Ser Thr Phe Ile Gly Glu His Tyr Val
   1340 1345 1350
   His Val Asn Ala Thr Tyr Val Asn Val Lys Cys Val Ala Pro Tyr
   1355 1360 1365
   Pro Ser Leu Leu Ser Ser Glu Asp Asn Ala Asp Asp Glu Val Asp
   1370 1375 1380
   Thr Arg Pro Ala Ser Phe Trp Glu Thr Ser
   1385 1390
<210> 67
   <211> 1068
   <212> PRT
   <213> Homo sapiens
<400> 67
<210> 68
   <211> 603
   <212> PRT
   <213> Homo sapiens
<400> 68
   Met Ser Ala Ala Val Thr Ala Gly Lys Leu Ala Arg Ala Pro Ala
   1 5 10 15
   Asp Pro Gly Lys Ala Gly Val Pro Gly Val Ala Ala Pro Gly Ala
   20 25 30
   Pro Ala Ala Ala Pro Pro Ala Lys Glu Ile Pro Glu Val Leu Val
   35 40 45
   Asp Pro Arg Ser Arg Arg Arg Tyr Val Arg Gly Arg Phe Leu Gly
   50 55 60
   Lys Gly Gly Phe Ala Lys Cys Phe Glu Ile Ser Asp Ala Asp Thr
   65 70 75
   Lys Glu Val Phe Ala Gly Lys Ile Val Pro Lys Ser Leu Leu Leu
   80 85 90
   Lys Pro His Gln Arg Glu Lys Met Ser Met Glu Ile Ser Ile His
   95 100 105
   Arg Ser Leu Ala His Gln His Val Val Gly Phe His Gly Phe Phe
   110 115 120
   Glu Asp Asn Asp Phe Val Phe Val Val Leu Glu Leu Cys Arg Arg
   125 130 135
   Arg Ser Leu Leu Glu Leu His Lys Arg Arg Lys Ala Leu Thr Glu
   140 145 150
   Pro Glu Ala Arg Tyr Tyr Leu Arg Gln Ile Val Leu Gly Cys Gln
   155 160 165
   Tyr Leu His Arg Asn Arg Val Ile His Arg Asp Leu Lys Leu Gly
   170 175 180
   Asn Leu Phe Leu Asn Glu Asp Leu Glu Val Lys Ile Gly Asp Phe
   185 190 195
   Gly Leu Ala Thr Lys Val Glu Tyr Asp Gly Glu Arg Lys Lys Thr
   200 205 210
   Leu Cys Gly Thr Pro Asn Tyr Ile Ala Pro Glu Val Leu Ser Lys
   215 220 225
   Lys Gly His Ser Phe Glu Val Asp Val Trp Ser Ile Gly Cys Ile
   230 235 240
   Met Tyr Thr Leu Leu Val Gly Lys Pro Pro Phe Glu Thr Ser Cys
   245 250 255
   Leu Lys Glu Thr Tyr Leu Arg Ile Lys Lys Asn Glu Tyr Ser Ile
   260 265 270
   Pro Lys His Ile Asn Pro Val Ala Ala Ser Leu Ile Gln Lys Met
   275 280 285
   Leu Gln Thr Asp Pro Thr Ala Arg Pro Thr Ile Asn Glu Leu Leu
   290 295 300
   Asn Asp Glu Phe Phe Thr Ser Gly Tyr Ile Pro Ala Arg Leu Pro
   305 310 315
   Ile Thr Cys Leu Thr Ile Pro Pro Arg Phe Ser Ile Ala Pro Ser
   320 325 330
   Ser Leu Asp Pro Ser Asn Arg Lys Pro Leu Thr Val Leu Asn Lys
   335 340 345
   Gly Leu Glu Asn Pro Leu Pro Glu Arg Pro Arg Glu Lys Glu Glu
   350 355 360
   Pro Val Val Arg Glu Thr Gly Glu Val Val Asp Cys His Leu Ser
   365 370 375
   Asp Met Leu Gln Gln Leu His Ser Val Asn Ala Ser Lys Pro Ser
   380 385 390
   Glu Arg Gly Leu Val Arg Gln Glu Glu Ala Glu Asp Pro Ala Cys
   395 400 405
   Ile Pro Ile Phe Trp Val Ser Lys Trp Val Asp Tyr Ser Asp Lys
   410 415 420
   Tyr Gly Leu Gly Tyr Gln Leu Cys Asp Asn Ser Val Gly Val Leu
   425 430 435
   Phe Asn Asp Ser Thr Arg Leu Ile Leu Tyr Asn Asp Gly Asp Ser
   440 445 450
   Leu Gln Tyr Ile Glu Arg Asp Gly Thr Glu Ser Tyr Leu Thr Val
   455 460 465
   Ser Ser His Pro Asn Ser Leu Met Lys Lys Ile Thr Leu Leu Lys
   470 475 480
   Tyr Phe Arg Asn Tyr Met Ser Glu His Leu Leu Lys Ala Gly Ala
   485 490 495
   Asn Ile Thr Pro Arg Glu Gly Asp Glu Leu Ala Arg Leu Pro Tyr
   500 505 510
   Leu Arg Thr Trp Phe Arg Thr Arg Ser Ala Ile Ile Leu His Leu
   515 520 525
   Ser Asn Gly Ser Val Gln Ile Asn Phe Phe Gln Asp His Thr Lys
   530 535 540
   Leu Ile Leu Cys Pro Leu Met Ala Ala Val Thr Tyr Ile Asp Glu
   545 550 555
   Lys Arg Asp Phe Arg Thr Tyr Arg Leu Ser Leu Leu Glu Glu Tyr
   560 565 570
   Gly Cys Cys Lys Glu Leu Ala Ser Arg Leu Arg Tyr Ala Arg Thr
   575 580 585
   Met Val Asp Lys Leu Leu Ser Ser Arg Ser Ala Ser Asn Arg Leu
   590 595 600
   Lys Ala Ser
<210> 69
   <211> 1447
   <212> PRT
   <213> Homo sapiens
<400> 69
   Met Ala Ser Ala Gly Asn Ala Ala Glu Pro Gln Asp Arg Gly Gly
   1               5                   10                  15
   Gly Gly Ser Gly Cys Ile Gly Ala Pro Gly Arg Pro Ala Gly Gly
                   20                  25                  30
   Gly Arg Arg Arg Arg Thr Gly Gly Leu Arg Arg Ala Ala Ala Pro
                   35                  40                  45
   Asp Arg Asp Tyr Leu His Arg Pro Ser Tyr Cys Asp Ala Ala Phe
                   50                  55                  60
   Ala Leu Glu Gln Ile Ser Lys Gly Lys Ala Thr Gly Arg Lys Ala
                   65                  70                  75
   Pro Leu Trp Leu Arg Ala Lys Phe Gln Arg Leu Leu Phe Lys Leu
                   80                  85                  90
   Gly Cys Tyr Ile Gln Lys Asn Cys Gly Lys Phe Leu Val Val Gly
                   95                  100                 105
   Leu Leu Ile Phe Gly Ala Phe Ala Val Gly Leu Lys Ala Ala Asn
                   110                 115                 120
   Leu Glu Thr Asn Val Glu Glu Leu Trp Val Glu Val Gly Gly Arg
                   125                 130                 135
   Val Ser Arg Glu Leu Asn Tyr Thr Arg Gln Lys Ile Gly Glu Glu
   140 145 150
   Ala Met Phe Asn Pro Gln Leu Met Ile Gln Thr Pro Lys Glu Glu
   155 160 165
   Gly Ala Asn Val Leu Thr Thr Glu Ala Leu Leu Gln His Leu Asp
   170 175 180
   Ser Ala Leu Gln Ala Ser Arg Val His Val Tyr Met Tyr Asn Arg
   185 190 195
   Gln Trp Lys Leu Glu His Leu Cys Tyr Lys Ser Gly Glu Leu Ile
   200 205 210
   Thr Glu Thr Gly Tyr Met Asp Gln Ile Ile Glu Tyr Leu Tyr Pro
   215 220 225
   Cys Leu Ile Ile Thr Pro Leu Asp Cys Phe Trp Glu Gly Ala Lys
   230 235 240
   Leu Gln Ser Gly Thr Ala Tyr Leu Leu Gly Lys Pro Pro Leu Arg
   245 250 255
   Trp Thr Asn Phe Asp Pro Leu Glu Phe Leu Glu Glu Leu Lys Lys
   260 265 270
   Ile Asn Tyr Gln Val Asp Ser Trp Glu Glu Met Leu Asn Lys Ala
   275 280 285
   Glu Val Gly His Gly Tyr Met Asp Arg Pro Cys Leu Asn Pro Ala
   290 295 300
   Asp Pro Asp Cys Pro Ala Thr Ala Pro Asn Lys Asn Ser Thr Lys
   305 310 315
   Pro Leu Asp Met Ala Leu Val Leu Asn Gly Gly Cys His Gly Leu
   320 325 330
   Ser Arg Lys Tyr Met His Trp Gln Glu Glu Leu Ile Val Gly Gly
   335 340 345
   Thr Val Lys Asn Ser Thr Gly Lys Leu Val Ser Ala His Ala Leu
   350 355 360
   Gln Thr Met Phe Gln Leu Met Thr Pro Lys Gln Met Tyr Glu His
   365 370 375
   Phe Lys Gly Tyr Glu Tyr Val Ser His Ile Asn Trp Asn Glu Asp
   380 385 390
   Lys Ala Ala Ala Ile Leu Glu Ala Trp Gln Arg Thr Tyr Val Glu
   395 400 405
   Val Val His Gln Ser Val Ala Gln Asn Ser Thr Gln Lys Val Leu
   410 415 420
   Ser Phe Thr Thr Thr Thr Leu Asp Asp Ile Leu Lys Ser Phe Ser
   425 430 435
   Asp Val Ser Val Ile Arg Val Ala Ser Gly Tyr Leu Leu Met Leu
   440 445 450
   Ala Tyr Ala Cys Leu Thr Met Leu Arg Trp Asp Cys Ser Lys Ser
   455 460 465
   Gln Gly Ala Val Gly Leu Ala Gly Val Leu Leu Val Ala Leu Ser
   470 475 480
   Val Ala Ala Gly Leu Gly Leu Cys Ser Leu Ile Gly Ile Ser Phe
   485 490 495
   Asn Ala Ala Thr Thr Gln Val Leu Pro Phe Leu Ala Leu Gly Val
   500 505 510
   Gly Val Asp Asp Val Phe Leu Leu Ala His Ala Phe Ser Glu Thr
   515 520 525
   Gly Gln Asn Lys Arg Ile Pro Phe Glu Asp Arg Thr Gly Glu Cys
   530 535 540
   Leu Lys Arg Thr Gly Ala Ser Val Ala Leu Thr Ser Ile Ser Asn
   545 550 555
   Val Thr Ala Phe Phe Met Ala Ala Leu Ile Pro Ile Pro Ala Leu
   560 565 570
   Arg Ala Phe Ser Leu Gln Ala Ala Val Val Val Val Phe Asn Phe
   575 580 585
   Ala Met Val Leu Leu Ile Phe Pro Ala Ile Leu Ser Met Asp Leu
   590 595 600
   Tyr Arg Arg Glu Asp Arg Arg Leu Asp Ile Phe Cys Cys Phe Thr
   605 610 615
   Ser Pro Cys Val Ser Arg Val Ile Gln Val Glu Pro Gln Ala Tyr
   620 625 630
   Thr Asp Thr His Asp Asn Thr Arg Tyr Ser Pro Pro Pro Pro Tyr
   635 640 645
   Ser Ser His Ser Phe Ala His Glu Thr Gln Ile Thr Met Gln Ser
   650 655 660
   Thr Val Gln Leu Arg Thr Glu Tyr Asp Pro His Thr His Val Tyr
   665 670 675
   Tyr Thr Thr Ala Glu Pro Arg Ser Glu Ile Ser Val Gln Pro Val
   680 685 690
   Thr Val Thr Gln Asp Thr Leu Ser Cys Gln Ser Pro Glu Ser Thr
   695 700 705
   Ser Ser Thr Arg Asp Leu Leu Ser Gln Phe Ser Asp Ser Ser Leu
   710 715 720
   His Cys Leu Glu Pro Pro Cys Thr Lys Trp Thr Leu Ser Ser Phe
   725 730 735
   Ala Glu Lys His Tyr Ala Pro Phe Leu Leu Lys Pro Lys Ala Lys
   740 745 750
   Val Val Val Ile Phe Leu Phe Leu Gly Leu Leu Gly Val Ser Leu
   755 760 765
   Tyr Gly Thr Thr Arg Val Arg Asp Gly Leu Asp Leu Thr Asp Ile
   770 775 780
   Val Pro Arg Glu Thr Arg Glu Tyr Asp Phe Ile Ala Ala Gln Phe
   785 790 795
   Lys Tyr Phe Ser Phe Tyr Asn Met Tyr Ile Val Thr Gln Lys Ala
   800 805 810
   Asp Tyr Pro Asn Ile Gln His Leu Leu Tyr Asp Leu His Arg Ser
   815 820 825
   Phe Ser Asn Val Lys Tyr Val Met Leu Glu Glu Asn Lys Gln Leu
   830 835 840
   Pro Lys Met Trp Leu His Tyr Phe Arg Asp Trp Leu Gln Gly Leu
   845 850 855
   Gln Asp Ala Phe Asp Ser Asp Trp Glu Thr Gly Lys Ile Met Pro
   860 865 870
   Asn Asn Tyr Lys Asn Gly Ser Asp Asp Gly Val Leu Ala Tyr Lys
   875 880 885
   Leu Leu Val Gln Thr Gly Ser Arg Asp Lys Pro Ile Asp Ile Ser
   890 895 900
   Gln Leu Thr Lys Gln Arg Leu Val Asp Ala Asp Gly Ile Ile Asn
   905 910 915
   Pro Ser Ala Phe Tyr Ile Tyr Leu Thr Ala Trp Val Ser Asn Asp
   920 925 930
   Pro Val Ala Tyr Ala Ala Ser Gln Ala Asn Ile Arg Pro His Arg
   935 940 945
   Pro Glu Trp Val His Asp Lys Ala Asp Tyr Met Pro Glu Thr Arg
   950 955 960
   Leu Arg Ile Pro Ala Ala Glu Pro Ile Glu Tyr Ala Gln Phe Pro
   965 970 975
   Phe Tyr Leu Asn Gly Leu Arg Asp Thr Ser Asp Phe Val Glu Ala
   980 985 990
   Ile Glu Lys Val Arg Thr Ile Cys Ser Asn Tyr Thr Ser Leu Gly
   995 1000 1005
   Leu Ser Ser Tyr Pro Asn Gly Tyr Pro Phe Leu Phe Trp Glu Gln
   1010 1015 1020
   Tyr Ile Gly Leu Arg His Trp Leu Leu Leu Phe Ile Ser Val Val
   1025 1030 1035
   Leu Ala Cys Thr Phe Leu Val Cys Ala Val Phe Leu Leu Asn Pro
   1040 1045 1050
   Trp Thr Ala Gly Ile Ile Val Met Val Leu Ala Leu Met Thr Val
   1055 1060 1065
   Glu Leu Phe Gly Met Met Gly Leu Ile Gly Ile Lys Leu Ser Ala
   1070 1075 1080
   Val Pro Val Val Ile Leu Ile Ala Ser Val Gly Ile Gly Val Glu
   1085 1090 1095
   Phe Thr Val His Val Ala Leu Ala Phe Leu Thr Ala Ile Gly Asp
   1100 1105 1110
   Lys Asn Arg Arg Ala Val Leu Ala Leu Glu His Met Phe Ala Pro
   1115 1120 1125
   Val Leu Asp Gly Ala Val Ser Thr Leu Leu Gly Val Leu Met Leu
   1130 1135 1140
   Ala Gly Ser Glu Phe Asp Phe Ile Val Arg Tyr Phe Phe Ala Val
   1145 1150 1155
   Leu Ala Ile Leu Thr Ile Leu Gly Val Leu Asn Gly Leu Val Leu
   1160 1165 1170
   Leu Pro Val Leu Leu Ser Phe Phe Gly Pro Tyr Pro Glu Val Ser
                   1175                1180                1185
   Pro Ala Asn Gly Leu Asn Arg Leu Pro Thr Pro Ser Pro Glu Pro
                   1190                1195                1200
   Pro Pro Ser Val Val Arg Phe Ala Met Pro Pro Gly His Thr His
                   1205                1210                1215
   Ser Gly Ser Asp Ser Ser Asp Ser Glu Tyr Ser Ser Gln Thr Thr
                   1220                1225                1230
   Val Ser Gly Leu Ser Glu Glu Leu Arg His Tyr Glu Ala Gln Gln
                   1235                1240                1245
   Gly Ala Gly Gly Pro Ala His Gln Val Ile Val Glu Ala Thr Glu
                   1250                1255                1260
   Asn Pro Val Phe Ala His Ser Thr Val Val His Pro Glu Ser Arg
                   1265                1270                1275
   His His Pro Pro Ser Asn Pro Arg Gln Gln Pro His Leu Asp Ser
                   1280                1285                1290
   Gly Ser Leu Pro Pro Gly Arg Gln Gly Gln Gln Pro Arg Arg Asp
                   1295                1300                1305
   Pro Pro Arg Glu Gly Leu Trp Pro Pro Leu Tyr Arg Pro Arg Arg
                   1310                1315                1320
   Asp Ala Phe Glu Ile Ser Thr Glu Gly His Ser Gly Pro Ser Asn
                   1325                1330                1335
   Arg Ala Arg Trp Gly Pro Arg Gly Ala Arg Ser His Asn Pro Arg
                   1340                1345                1350
   Asn Pro Ala Ser Thr Ala Met Gly Ser Ser Val Pro Gly Tyr Cys
                   1355                1360                1365
   Gln Pro Ile Thr Thr Val Thr Ala Ser Ala Ser Val Thr Val Ala
                   1370                1375                1380
   Val His Pro Pro Pro Val Pro Gly Pro Gly Arg Asn Pro Arg Gly
                   1385                1390                1395
   Gly Leu Cys Pro Gly Tyr Pro Glu Thr Asp His Gly Leu Phe Glu
                   1400                1405                1410
   Asp Pro His Val Pro Phe His Val Arg Cys Glu Arg Arg Asp Ser
                   1415                1420                1425
   Lys Val Glu Val Ile Glu Leu Gln Asp Val Glu Cys Glu Glu Arg
                   1430                1435                1440
   Pro Arg Gly Ser Ser Ser Asn
                   1445
<210> 70
   <211> 928
   <212> PRT
   <213> Homo sapiens
<400> 70
   Met Pro Pro Lys Thr Pro Arg Lys Thr Ala Ala Thr Ala Ala Ala
   1 5 10 15
   Ala Ala Ala Glu Pro Pro Ala Pro Pro Pro Pro Pro Pro Pro Glu
   20 25 30
   Glu Asp Pro Glu Gln Asp Ser Gly Pro Glu Asp Leu Pro Leu Val
                   35                  40                  45
   Arg Leu Glu Phe Glu Glu Thr Glu Glu Pro Asp Phe Thr Ala Leu
                   50                  55                  60
   Cys Gln Lys Leu Lys Ile Pro Asp His Val Arg Glu Arg Ala Trp
                   65                  70                  75
   Leu Thr Trp Glu Lys Val Ser Ser Val Asp Gly Val Leu Gly Gly
                   80                  85                  90
   Tyr Ile Gln Lys Lys Lys Glu Leu Trp Gly Ile Cys Ile Phe Ile
                   95                  100                 105
   Ala Arg Val Asp Leu Asp Glu Met Ser Phe Thr Leu Leu Ser Tyr
                   110                 115                 120
   Arg Lys Thr Tyr Glu Ile Ser Val His Lys Phe Phe Asn Leu Leu
                   125                 130                 135
   Lys Glu Ile Asp Thr Ser Thr Lys Val Asp Asn Ala Met Ser Arg
                   140                 145                 150
   Leu Leu Lys Lys Tyr Asp Val Leu Phe Ala Leu Phe Ser Lys Leu
                   155                 160                 165
   Glu Arg Thr Cys Glu Leu Ile Tyr Leu Thr Gln Pro Ser Ser Ser
                   170                 175                 180
   Ile Ser Thr Glu Ile Asn Ser Ala Leu Val Leu Lys Val Ser Trp
                   185                 190                 195
   Ile Thr Phe Leu Leu Ala Lys Gly Glu Val Leu Gln Met Glu Asp
                   200                 205                 210
   Asp Leu Val Ile Ser Phe Gln Leu Met Leu Cys Val Leu Asp Tyr
                   215                 220                 225
   Phe Ile Lys Leu Ser Pro Pro Met Leu Leu Lys Glu Pro Tyr Lys
                   230                 235                 240
   Thr Ala Val Ile Pro Ile Asn Gly Ser Pro Arg Thr Pro Arg Arg
                   245                 250                 255
   Gly Gln Asn Arg Ser Ala Arg Ile Ala Lys Gln Leu Glu Asn Asp
                   260                 265                 270
   Thr Arg Ile Ile Glu Val Leu Cys Lys Glu His Glu Cys Asn Ile
                   275                 280                 285
   Asp Glu Val Lys Asn Val Tyr Phe Lys Asn Phe Ile Pro Phe Met
                   290                 295                 300
   Asn Ser Leu Gly Leu Val Thr Ser Asn Gly Leu Pro Glu Val Glu
                   305                 310                 315
   Asn Leu Ser Lys Arg Tyr Glu Glu Ile Tyr Leu Lys Asn Lys Asp
                   320                 325                 330
   Leu Asp Arg Arg Leu Phe Leu Asp His Asp Lys Thr Leu Gln Thr
                   335                 340                 345
   Asp Ser Ile Asp Ser Phe Glu Thr Gln Arg Thr Pro Arg Lys Ser
                   350                 355                 360
   Asn Leu Asp Glu Glu Val Asn Ile Ile Pro Pro His Thr Pro Val
                   365                 370                 375
   Arg Thr Val Met Asn Thr Ile Gln Gln Leu Met Met Ile Leu Asn
   380 385 390
   Ser Ala Ser Asp Gln Pro Ser Glu Asn Leu Ile Ser Tyr Phe Asn
   395 400 405
   Asn Cys Thr Val Asn Pro Lys Glu Ser Ile Leu Lys Arg Val Lys
   410 415 420
   Asp Ile Gly Tyr Ile Phe Lys Glu Lys Phe Ala Lys Ala Val Gly
   425 430 435
   Gln Gly Cys Val Glu Ile Gly Ser Gln Arg Tyr Lys Leu Gly Val
   440 445 450
   Arg Leu Tyr Tyr Arg Val Met Glu Ser Met Leu Lys Ser Glu Glu
   455 460 465
   Glu Arg Leu Ser Ile Gln Asn Phe Ser Lys Leu Leu Asn Asp Asn
   470 475 480
   Ile Phe His Met Ser Leu Leu Ala Cys Ala Leu Glu Val Val Met
   485 490 495
   Ala Thr Tyr Ser Arg Ser Thr Ser Gln Asn Leu Asp Ser Gly Thr
   500 505 510
   Asp Leu Ser Phe Pro Trp Ile Leu Asn Val Leu Asn Leu Lys Ala
   515 520 525
   Phe Asp Phe Tyr Lys Val Ile Glu Ser Phe Ile Lys Ala Glu Gly
   530 535 540
   Asn Leu Thr Arg Glu Met Ile Lys His Leu Glu Arg Cys Glu His
   545 550 555
   Arg Ile Met Glu Ser Leu Ala Trp Leu Ser Asp Ser Pro Leu Phe
   560 565 570
   Asp Leu Ile Lys Gln Ser Lys Asp Arg Glu Gly Pro Thr Asp His
   575 580 585
   Leu Glu Ser Ala Cys Pro Leu Asn Leu Pro Leu Gln Asn Asn His
   590 595 600
   Thr Ala Ala Asp Met Tyr Leu Ser Pro Val Arg Ser Pro Lys Lys
   605 610 615
   Lys Gly Ser Thr Thr Arg Val Asn Ser Thr Ala Asn Ala Glu Thr
   620 625 630
   Gln Ala Thr Ser Ala Phe Gln Thr Gln Lys Pro Leu Lys Ser Thr
   635 640 645
   Ser Leu Ser Leu Phe Tyr Lys Lys Val Tyr Arg Leu Ala Tyr Leu
   650 655 660
   Arg Leu Asn Thr Leu Cys Glu Arg Leu Leu Ser Glu His Pro Glu
   665 670 675
   Leu Glu His Ile Ile Trp Thr Leu Phe Gln His Thr Leu Gln Asn
   680 685 690
   Glu Tyr Glu Leu Met Arg Asp Arg His Leu Asp Gln Ile Met Met
   695 700 705
   Cys Ser Met Tyr Gly Ile Cys Lys Val Lys Asn Ile Asp Leu Lys
   710 715 720
   Phe Lys Ile Ile Val Thr Ala Tyr Lys Asp Leu Pro His Ala Val
   725 730 735
   Gln Glu Thr Phe Lys Arg Val Leu Ile Lys Glu Glu Glu Tyr Asp
   740 745 750
   Ser Ile Ile Val Phe Tyr Asn Ser Val Phe Met Gln Arg Leu Lys
   755 760 765
   Thr Asn Ile Leu Gln Tyr Ala Ser Thr Arg Pro Pro Thr Leu Ser
   770 775 780
   Pro Ile Pro His Ile Pro Arg Ser Pro Tyr Lys Phe Pro Ser Ser
   785 790 795
   Pro Leu Arg Ile Pro Gly Gly Asn Ile Tyr Ile Ser Pro Leu Lys
   800 805 810
   Ser Pro Tyr Lys Ile Ser Glu Gly Leu Pro Thr Pro Thr Lys Met
   815 820 825
   Thr Pro Arg Ser Arg Ile Leu Val Ser Ile Gly Glu Ser Phe Gly
   830 835 840
   Thr Ser Glu Lys Phe Gln Lys Ile Asn Gln Met Val Cys Asn Ser
   845 850 855
   Asp Arg Val Leu Lys Arg Ser Ala Glu Gly Ser Asn Pro Pro Lys
   860 865 870
   Pro Leu Lys Lys Leu Arg Phe Asp Ile Glu Gly Ser Asp Glu Ala
   875 880 885
   Asp Gly Ser Lys His Leu Pro Gly Glu Ser Lys Phe Gln Gln Lys
   890 895 900
   Leu Ala Glu Met Thr Ser Thr Arg Thr Arg Met Gln Lys Gln Lys
   905 910 915
   Met Asn Asp Ser Met Asp Thr Ser Asn Lys Glu Glu Lys
   920 925
<210> 71
   <211> 1072
   <212> PRT
   <213> Homo sapiens
<400> 71
   Met Ala Lys Ala Thr Ser Gly Ala Ala Gly Leu Arg Leu Leu Leu
   1               5                   10                  15
   Leu Leu Leu Leu Pro Leu Leu Gly Lys Val Ala Leu Gly Leu Tyr
                   20                  25                  30
   Phe Ser Arg Asp Ala Tyr Trp Glu Lys Leu Tyr Val Asp Gln Ala
                   35                  40                  45
   Ala Gly Thr Pro Leu Leu Tyr Val His Ala Leu Arg Asp Ala Pro
                   50                  55                  60
   Glu Glu Val Pro Ser Phe Arg Leu Gly Gln His Leu Tyr Gly Thr
                   65                  70                  75
   Tyr Arg Thr Arg Leu His Glu Asn Asn Trp Ile Cys Ile Gln Glu
                   80                  85                  90
   Asp Thr Gly Leu Leu Tyr Leu Asn Arg Ser Leu Asp His Ser Ser
                   95                  100                 105
   Trp Glu Lys Leu Ser Val Arg Asn Arg Gly Phe Pro Leu Leu Thr
   110 115 120
   Val Tyr Leu Lys Val Phe Leu Ser Pro Thr Ser Leu Arg Glu Gly
   125 130 135
   Glu Cys Gln Trp Pro Gly Cys Ala Arg Val Tyr Phe Ser Phe Phe
   140 145 150
   Asn Thr Ser Phe Pro Ala Cys Ser Ser Leu Lys Pro Arg Glu Leu
   155 160 165
   Cys Phe Pro Glu Thr Arg Pro Ser Phe Arg Ile Arg Glu Asn Arg
   170 175 180
   Pro Pro Gly Thr Phe His Gln Phe Arg Leu Leu Pro Val Gln Phe
   185 190 195
   Leu Cys Pro Asn Ile Ser Val Ala Tyr Arg Leu Leu Glu Gly Glu
   200 205 210
   Gly Leu Pro Phe Arg Cys Ala Pro Asp Ser Leu Glu Val Ser Thr
   215 220 225
   Arg Trp Ala Leu Asp Arg Glu Gln Arg Glu Lys Tyr Glu Leu Val
   230 235 240
   Ala Val Cys Thr Val His Ala Gly Ala Arg Glu Glu Val Val Met
   245 250 255
   Val Pro Phe Pro Val Thr Val Tyr Asp Glu Asp Asp Ser Ala Pro
   260 265 270
   Thr Phe Pro Ala Gly Val Asp Thr Ala Ser Ala Val Val Glu Phe
   275 280 285
   Lys Arg Lys Glu Asp Thr Val Val Ala Thr Leu Arg Val Phe Asp
   290 295 300
   Ala Asp Val Val Pro Ala Ser Gly Glu Leu Val Arg Arg Tyr Thr
   305 310 315
   Ser Thr Leu Leu Pro Gly Asp Thr Trp Ala Gln Gln Thr Phe Arg
   320 325 330
   Val Glu His Trp Pro Asn Glu Thr Ser Val Gln Ala Asn Gly Ser
   335 340 345
   Phe Val Arg Ala Thr Val His Asp Tyr Arg Leu Val Leu Asn Arg
   350 355 360
   Asn Leu Ser Ile Ser Glu Asn Arg Thr Met Gln Leu Ala Val Leu
   365 370 375
   Val Asn Asp Ser Asp Phe Gln Gly Pro Gly Ala Gly Val Leu Leu
   380 385 390
   Leu His Phe Asn Val Ser Val Leu Pro Val Ser Leu His Leu Pro
   395 400 405
   Ser Thr Tyr Ser Leu Ser Val Ser Arg Arg Ala Arg Arg Phe Ala
   410 415 420
   Gln Ile Gly Lys Val Cys Val Glu Asn Cys Gln Ala Phe Ser Gly
   425 430 435
   Ile Asn Val Gln Tyr Lys Leu His Ser Ser Gly Ala Asn Cys Ser
   440 445 450
   Thr Leu Gly Val Val Thr Ser Ala Glu Asp Thr Ser Gly Ile Leu
   455 460 465
   Phe Val Asn Asp Thr Lys Ala Leu Arg Arg Pro Lys Cys Ala Glu
   470 475 480
   Leu His Tyr Met Val Val Ala Thr Asp Gln Gln Thr Ser Arg Gln
   485 490 495
   Ala Gln Ala Gln Leu Leu Val Thr Val Glu Gly Ser Tyr Val Ala
   500 505 510
   Glu Glu Ala Gly Cys Pro Leu Ser Cys Ala Val Ser Lys Arg Arg
   515 520 525
   Leu Glu Cys Glu Glu Cys Gly Gly Leu Gly Ser Pro Thr Gly Arg
   530 535 540
   Cys Glu Trp Arg Gln Gly Asp Gly Lys Gly Ile Thr Arg Asn Phe
   545 550 555
   Ser Thr Cys Ser Pro Ser Thr Lys Thr Cys Pro Asp Gly His Cys
   560 565 570
   Asp Val Val Glu Thr Gln Asp Ile Asn Ile Cys Pro Gln Asp Cys
   575 580 585
   Leu Arg Gly Ser Ile Val Gly Gly His Glu Pro Gly Glu Pro Arg
   590 595 600
   Gly Ile Lys Ala Gly Tyr Gly Thr Cys Asn Cys Phe Pro Glu Glu
   605 610 615
   Glu Lys Cys Phe Cys Glu Pro Glu Asp Ile Gln Asp Pro Leu Cys
   620 625 630
   Asp Glu Leu Cys Arg Thr Val Ile Ala Ala Ala Val Leu Phe Ser
   635 640 645
   Phe Ile Val Ser Val Leu Leu Ser Ala Phe Cys Ile His Cys Tyr
   650 655 660
   His Lys Phe Ala His Lys Pro Pro Ile Ser Ser Ala Glu Met Thr
   665 670 675
   Phe Arg Arg Pro Ala Gln Ala Phe Pro Val Ser Tyr Ser Ser Ser
   680 685 690
   Gly Ala Arg Arg Pro Ser Leu Asp Ser Met Glu Asn Gln Val Ser
   695 700 705
   Val Asp Ala Phe Lys Ile Leu Glu Asp Pro Lys Trp Glu Phe Pro
   710 715 720
   Arg Lys Asn Leu Val Leu Gly Lys Thr Leu Gly Glu Gly Glu Phe
   725 730 735
   Gly Lys Val Val Lys Ala Thr Ala Phe His Leu Lys Gly Arg Ala
   740 745 750
   Gly Tyr Thr Thr Val Ala Val Lys Met Leu Lys Glu Asn Ala Ser
   755 760 765
   Pro Ser Glu Leu Arg Asp Leu Leu Ser Glu Phe Asn Val Leu Lys
   770 775 780
   Gln Val Asn His Pro His Val Ile Lys Leu Tyr Gly Ala Cys Ser
   785 790 795
   Gln Asp Gly Pro Leu Leu Leu Ile Val Glu Tyr Ala Lys Tyr Gly
   800 805 810
   Ser Leu Arg Gly Phe Leu Arg Glu Ser Arg Lys Val Gly Pro Gly
   815 820 825
   Tyr Leu Gly Ser Gly Gly Ser Arg Asn Ser Ser Ser Leu Asp His
   830 835 840
   Pro Asp Glu Arg Ala Leu Thr Met Gly Asp Leu Ile Ser Phe Ala
   845 850 855
   Trp Gln Ile Ser Gln Gly Met Gln Tyr Leu Ala Glu Met Lys Leu
   860 865 870
   Val His Arg Asp Leu Ala Ala Arg Asn Ile Leu Val Ala Glu Gly
   875 880 885
   Arg Lys Met Lys Ile Ser Asp Phe Gly Leu Ser Arg Asp Val Tyr
   890 895 900
   Glu Glu Asp Ser Tyr Val Lys Arg Ser Gln Gly Arg Ile Pro Val
   905 910 915
   Lys Trp Met Ala Ile Glu Ser Leu Phe Asp His Ile Tyr Thr Thr
   920 925 930
   Gln Ser Asp Val Trp Ser Phe Gly Val Leu Leu Trp Glu Ile Val
   935 940 945
   Thr Leu Gly Gly Asn Pro Tyr Pro Gly Ile Pro Pro Glu Arg Leu
   950 955 960
   Phe Asn Leu Leu Lys Thr Gly His Arg Met Glu Arg Pro Asp Asn
   965 970 975
   Cys Ser Glu Glu Met Tyr Arg Leu Met Leu Gln Cys Trp Lys Gln
   980 985 990
   Glu Pro Asp Lys Arg Pro Val Phe Ala Asp Ile Ser Lys Asp Leu
   995 1000 1005
   Glu Lys Met Met Val Lys Arg Arg Asp Tyr Leu Asp Leu Ala Ala
   1010 1015 1020
   Ser Thr Pro Ser Asp Ser Leu Ile Tyr Asp Asp Gly Leu Ser Glu
   1025 1030 1035
   Glu Glu Thr Pro Leu Val Asp Cys Asn Asn Ala Pro Leu Pro Arg
   1040 1045 1050
   Ala Leu Pro Ser Thr Trp Ile Glu Asn Lys Leu Tyr Gly Arg Ile
   1055 1060 1065
   Ser His Ala Phe Thr Arg Phe
   1070
<210> 72
   <211> 403
   <212> PRT
   <213> Homo sapiens
<400> 72
   Met Asp Arg Ser Lys Glu Asn Cys Ile Ser Gly Pro Val Lys Ala
   1 5 10 15
   Thr Ala Pro Val Gly Gly Pro Lys Arg Val Leu Val Thr Gln Gln
   20 25 30
   Phe Pro Cys Gln Asn Pro Leu Pro Val Asn Ser Gly Gln Ala Gln
   35 40 45
   Arg Val Leu Cys Pro Ser Asn Ser Ser Gln Arg Ile Pro Leu Gln
   50 55 60
   Ala Gln Lys Leu Val Ser Ser His Lys Pro Val Gln Asn Gln Lys
   65 70 75
   Gln Lys Gln Leu Gln Ala Thr Ser Val Pro His Pro Val Ser Arg
   80 85 90
   Pro Leu Asn Asn Thr Gln Lys Ser Lys Gln Pro Leu Pro Ser Ala
   95 100 105
   Pro Glu Asn Asn Pro Glu Glu Glu Leu Ala Ser Lys Gln Lys Asn
   110 115 120
   Glu Glu Ser Lys Lys Arg Gln Trp Ala Leu Glu Asp Phe Glu Ile
   125 130 135
   Gly Arg Pro Leu Gly Lys Gly Lys Phe Gly Asn Val Tyr Leu Ala
   140 145 150
   Arg Glu Lys Gln Ser Lys Phe Ile Leu Ala Leu Lys Val Leu Phe
   155 160 165
   Lys Ala Gln Leu Glu Lys Ala Gly Val Glu His Gln Leu Arg Arg
   170 175 180
   Glu Val Glu Ile Gln Ser His Leu Arg His Pro Asn Ile Leu Arg
   185 190 195
   Leu Tyr Gly Tyr Phe His Asp Ala Thr Arg Val Tyr Leu Ile Leu
   200 205 210
   Glu Tyr Ala Pro Leu Gly Thr Val Tyr Arg Glu Leu Gln Lys Leu
   215 220 225
   Ser Lys Phe Asp Glu Gln Arg Thr Ala Thr Tyr Ile Thr Glu Leu
   230 235 240
   Ala Asn Ala Leu Ser Tyr Cys His Ser Lys Arg Val Ile His Arg
   245 250 255
   Asp Ile Lys Pro Glu Asn Leu Leu Leu Gly Ser Ala Gly Glu Leu
   260 265 270
   Lys Ile Ala Asp Phe Gly Trp Ser Val His Ala Pro Ser Ser Arg
   275 280 285
   Arg Thr Thr Leu Cys Gly Thr Leu Asp Tyr Leu Pro Pro Glu Met
   290 295 300
   Ile Glu Gly Arg Met His Asp Glu Lys Val Asp Leu Trp Ser Leu
   305 310 315
   Gly Val Leu Cys Tyr Glu Phe Leu Val Gly Lys Pro Pro Phe Glu
   320 325 330
   Ala Asn Thr Tyr Gln Glu Thr Tyr Lys Arg Ile Ser Arg Val Glu
   335 340 345
   Phe Thr Phe Pro Asp Phe Val Thr Glu Gly Ala Arg Asp Leu Ile
   350 355 360
   Ser Arg Leu Leu Lys His Asn Pro Ser Gln Arg Pro Met Leu Arg
   365 370 375
   Glu Val Leu Glu His Pro Trp Ile Thr Ala Asn Ser Ser Lys Pro
   380 385 390
   Ser Asn Cys Gln Asn Lys Glu Ser Ala Ser Lys Gln Ser
   395 400
<210> 73
   <211> 567
   <212> PRT
   <213> Homo sapiens
<400> 73
   Met Gly Arg Gly Leu Leu Arg Gly Leu Trp Pro Leu His Ile Val
   1 5 10 15
   Leu Trp Thr Arg Ile Ala Ser Thr Ile Pro Pro His Val Gln Lys
   20 25 30
   Ser Val Asn Asn Asp Met Ile Val Thr Asp Asn Asn Gly Ala Val
   35 40 45
   Lys Phe Pro Gln Leu Cys Lys Phe Cys Asp Val Arg Phe Ser Thr
   50 55 60
   Cys Asp Asn Gln Lys Ser Cys Met Ser Asn Cys Ser Ile Thr Ser
   65 70 75
   Ile Cys Glu Lys Pro Gln Glu Val Cys Val Ala Val Trp Arg Lys
   80 85 90
   Asn Asp Glu Asn Ile Thr Leu Glu Thr Val Cys His Asp Pro Lys
   95 100 105
   Leu Pro Tyr His Asp Phe Ile Leu Glu Asp Ala Ala Ser Pro Lys
   110 115 120
   Cys Ile Met Lys Glu Lys Lys Lys Pro Gly Glu Thr Phe Phe Met
   125 130 135
   Cys Ser Cys Ser Ser Asp Glu Cys Asn Asp Asn Ile Ile Phe Ser
   140 145 150
   Glu Glu Tyr Asn Thr Ser Asn Pro Asp Leu Leu Leu Val Ile Phe
   155 160 165
   Gln Val Thr Gly Ile Ser Leu Leu Pro Pro Leu Gly Val Ala Ile
   170 175 180
   Ser Val Ile Ile Ile Phe Tyr Cys Tyr Arg Val Asn Arg Gln Gln
   185 190 195
   Lys Leu Ser Ser Thr Trp Glu Thr Gly Lys Thr Arg Lys Leu Met
   200 205 210
   Glu Phe Ser Glu His Cys Ala Ile Ile Leu Glu Asp Asp Arg Ser
   215 220 225
   Asp Ile Ser Ser Thr Cys Ala Asn Asn Ile Asn His Asn Thr Glu
   230 235 240
   Leu Leu Pro Ile Glu Leu Asp Thr Leu Val Gly Lys Gly Arg Phe
   245 250 255
   Ala Glu Val Tyr Lys Ala Lys Leu Lys Gln Asn Thr Ser Glu Gln
   260 265 270
   Phe Glu Thr Val Ala Val Lys Ile Phe Pro Tyr Glu Glu Tyr Ala
   275 280 285
   Ser Trp Lys Thr Glu Lys Asp Ile Phe Ser Asp Ile Asn Leu Lys
   290 295 300
   His Glu Asn Ile Leu Gln Phe Leu Thr Ala Glu Glu Arg Lys Thr
   305 310 315
   Glu Leu Gly Lys Gln Tyr Trp Leu Ile Thr Ala Phe His Ala Lys
   320 325 330
   Gly Asn Leu Gln Glu Tyr Leu Thr Arg His Val Ile Ser Trp Glu
   335 340 345
   Asp Leu Arg Lys Leu Gly Ser Ser Leu Ala Arg Gly Ile Ala His
   350 355 360
   Leu His Ser Asp His Thr Pro Cys Gly Arg Pro Lys Met Pro Ile
   365 370 375
   Val His Arg Asp Leu Lys Ser Ser Asn Ile Leu Val Lys Asn Asp
   380 385 390
   Leu Thr Cys Cys Leu Cys Asp Phe Gly Leu Ser Leu Arg Leu Asp
   395 400 405
   Pro Thr Leu Ser Val Asp Asp Leu Ala Asn Ser Gly Gln Val Gly
   410 415 420
   Thr Ala Arg Tyr Met Ala Pro Glu Val Leu Glu Ser Arg Met Asn
   425 430 435
   Leu Glu Asn Ala Glu Ser Phe Lys Gln Thr Asp Val Tyr Ser Met
   440 445 450
   Ala Leu Val Leu Trp Glu Met Thr Ser Arg Cys Asn Ala Val Gly
   455 460 465
   Glu Val Lys Asp Tyr Glu Pro Pro Phe Gly Ser Lys Val Arg Glu
   470 475 480
   His Pro Cys Val Glu Ser Met Lys Asp Asn Val Leu Arg Asp Arg
   485 490 495
   Gly Arg Pro Glu Ile Pro Ser Phe Trp Leu Asn His Gln Gly Ile
   500 505 510
   Gln Met Val Cys Glu Thr Leu Thr Glu Cys Trp Asp His Asp Pro
   515 520 525
   Glu Ala Arg Leu Thr Ala Gln Cys Val Ala Glu Arg Phe Ser Glu
   530 535 540
   Leu Glu His Leu Asp Arg Leu Ser Gly Arg Ser Cys Ser Glu Glu
   545 550 555
   Lys Ile Pro Glu Asp Gly Ser Leu Asn Thr Thr Lys
   560 565
<210> 74
   <211> 393
   <212> PRT
   <213> Homo sapiens
<400> 74
<210> 75
   <211> 1308
   <212> PRT
   <213> Homo sapiens
<400> 75
<210> 76
   <211> 821
   <212> PRT
   <213> Homo sapiens
<400> 76
<210> 77
   <211> 1166
   <212> PRT
   <213> Homo sapiens
<400> 77
   Met Ala Asn Asp Ser Pro Ala Lys Ser Leu Val Asp Ile Asp Leu
   1 5 10 15
   Ser Ser Leu Arg Asp Pro Ala Gly Ile Phe Glu Leu Val Glu Val
   20 25 30
   Val Gly Asn Gly Thr Tyr Gly Gln Val Tyr Lys Gly Arg His Val
   35 40 45
   Lys Thr Gly Gln Leu Ala Ala Ile Lys Val Met Asp Val Thr Glu
   50 55 60
   Asp Glu Glu Glu Glu Ile Lys Leu Glu Ile Asn Met Leu Lys Lys
   65 70 75
   Tyr Ser His His Arg Asn Ile Ala Thr Tyr Tyr Gly Ala Phe Ile
   80 85 90
   Lys Lys Ser Pro Pro Gly His Asp Asp Gln Leu Trp Leu Val Met
   95 100 105
   Glu Phe Cys Gly Ala Gly Ser Ile Thr Asp Leu Val Lys Asn Thr
   110 115 120
   Lys Gly Asn Thr Leu Lys Glu Asp Trp Ile Ala Tyr Ile Ser Arg
   125 130 135
   Glu Ile Leu Arg Gly Leu Ala His Leu His Ile His His Val Ile
   140 145 150
   His Arg Asp Ile Lys Gly Gln Asn Val Leu Leu Thr Glu Asn Ala
   155 160 165
   Glu Val Lys Leu Val Asp Phe Gly Val Ser Ala Gln Leu Asp Arg
   170 175 180
   Thr Val Gly Arg Arg Asn Thr Phe Ile Gly Thr Pro Tyr Trp Met
   185 190 195
   Ala Pro Glu Val Ile Ala Cys Asp Glu Asn Pro Asp Ala Thr Tyr
   200 205 210
   Asp Tyr Arg Ser Asp Leu Trp Ser Cys Gly Ile Thr Ala Ile Glu
   215 220 225
   Met Ala Glu Gly Ala Pro Pro Leu Cys Asp Met His Pro Met Arg
   230 235 240
   Ala Leu Phe Leu Ile Pro Arg Asn Pro Pro Pro Arg Leu Lys Ser
   245 250 255
   Lys Lys Trp Ser Lys Lys Phe Phe Ser Phe Ile Glu Gly Cys Leu
   260 265 270
   Val Lys Asn Tyr Met Gln Arg Pro Ser Thr Glu Gln Leu Leu Lys
   275 280 285
   His Pro Phe Ile Arg Asp Gln Pro Asn Glu Arg Gln Val Arg Ile
   290 295 300
   Gln Leu Lys Asp His Ile Asp Arg Thr Arg Lys Lys Arg Gly Glu
   305 310 315
   Lys Asp Glu Thr Glu Tyr Glu Tyr Ser Gly Ser Glu Glu Glu Glu
   320 325 330
   Glu Glu Val Pro Glu Gln Glu Gly Glu Pro Ser Ser Ile Val Asn
   335 340 345
   Val Pro Gly Glu Ser Thr Leu Arg Arg Asp Phe Leu Arg Leu Gln
   350 355 360
   Gln Glu Asn Lys Glu Arg Ser Glu Ala Leu Arg Arg Gln Gln Leu
   365 370 375
   Leu Gln Glu Gln Gln Leu Arg Glu Gln Glu Glu Tyr Lys Arg Gln
   380 385 390
   Leu Leu Ala Glu Arg Gln Lys Arg Ile Glu Gln Gln Lys Glu Gln
   395 400 405
   Arg Arg Arg Leu Glu Glu Gln Gln Arg Arg Glu Arg Glu Ala Arg
   410 415 420
   Arg Gln Gln Glu Arg Glu Gln Arg Arg Arg Glu Gln Glu Glu Lys
   425 430 435
   Arg Arg Leu Glu Glu Leu Glu Arg Arg Arg Lys Glu Glu Glu Glu
   440 445 450
   Arg Arg Arg Ala Glu Glu Glu Lys Arg Arg Val Glu Arg Glu Gln
   455 460 465
   Glu Tyr Ile Arg Arg Gln Leu Glu Glu Glu Gln Arg His Leu Glu
   470 475 480
   Val Leu Gln Gln Gln Leu Leu Gln Glu Gln Ala Met Leu Leu His
   485 490 495
   Asp His Arg Arg Pro His Pro Gln His Ser Gln Gln Pro Pro Pro
   500 505 510
   Pro Gln Gln Glu Arg Ser Lys Pro Ser Phe His Ala Pro Glu Pro
   515 520 525
   Lys Ala His Tyr Glu Pro Ala Asp Arg Ala Arg Glu Val Pro Val
   530 535 540
   Arg Thr Thr Ser Arg Ser Pro Val Leu Ser Arg Arg Asp Ser Pro
   545 550 555
   Leu Gln Gly Ser Gly Gln Gln Asn Ser Gln Ala Gly Gln Arg Asn
   560 565 570
   Ser Thr Ser Ser Ile Glu Pro Arg Leu Leu Trp Glu Arg Val Glu
   575 580 585
   Lys Leu Val Pro Arg Pro Gly Ser Gly Ser Ser Ser Gly Ser Ser
   590 595 600
   Asn Ser Gly Ser Gln Pro Gly Ser His Pro Gly Ser Gln Ser Gly
   605 610 615
   Ser Gly Glu Arg Phe Arg Val Arg Ser Ser Ser Lys Ser Glu Gly
   620 625 630
   Ser Pro Ser Gln Arg Leu Glu Asn Ala Val Lys Lys Pro Glu Asp
   635 640 645
   Lys Lys Glu Val Phe Arg Pro Leu Lys Pro Ala Gly Glu Val Asp
   650 655 660
   Leu Thr Ala Leu Ala Lys Glu Leu Arg Ala Val Glu Asp Val Arg
   665 670 675
   Pro Pro His Lys Val Thr Asp Tyr Ser Ser Ser Ser Glu Glu Ser
   680 685 690
   Gly Thr Thr Asp Glu Glu Asp Asp Asp Val Glu Gln Glu Gly Ala
   695 700 705
   Asp Glu Ser Thr Ser Gly Pro Glu Asp Thr Arg Ala Ala Ser Ser
   710 715 720
   Leu Asn Leu Ser Asn Gly Glu Thr Glu Ser Val Lys Thr Met Ile
   725 730 735
   Val His Asp Asp Val Glu Ser Glu Pro Ala Met Thr Pro Ser Lys
   740 745 750
   Glu Gly Thr Leu Ile Val Arg Gln Thr Gln Ser Ala Ser Ser Thr
   755 760 765
   Leu Gln Lys His Lys Ser Ser Ser Ser Phe Thr Pro Phe Ile Asp
   770 775 780
   Pro Arg Leu Leu Gln Ile Ser Pro Ser Ser Gly Thr Thr Val Thr
   785 790 795
   Ser Val Val Gly Phe Ser Cys Asp Gly Met Arg Pro Glu Ala Ile
   800 805 810
   Arg Gln Asp Pro Thr Arg Lys Gly Ser Val Val Asn Val Asn Pro
   815 820 825
   Thr Asn Thr Arg Pro Gln Ser Asp Thr Pro Glu Ile Arg Lys Tyr
   830 835 840
   Lys Lys Arg Phe Asn Ser Glu Ile Leu Cys Ala Ala Leu Trp Gly
   845 850 855
   Val Asn Leu Leu Val Gly Thr Glu Ser Gly Leu Met Leu Leu Asp
   860 865 870
   Arg Ser Gly Gln Gly Lys Val Tyr Pro Leu Ile Asn Arg Arg Arg
   875 880 885
   Phe Gln Gln Met Asp Val Leu Glu Gly Leu Asn Val Leu Val Thr
   890 895 900
   Ile Ser Gly Lys Lys Asp Lys Leu Arg Val Tyr Tyr Leu Ser Trp
   905 910 915
   Leu Arg Asn Lys Ile Leu His Asn Asp Pro Glu Val Glu Lys Lys
   920 925 930
   Gln Gly Trp Thr Thr Val Gly Asp Leu Glu Gly Cys Val His Tyr
   935 940 945
   Lys Val Val Lys Tyr Glu Arg Ile Lys Phe Leu Val Ile Ala Leu
   950 955 960
   Lys Ser Ser Val Glu Val Tyr Ala Trp Ala Pro Lys Pro Tyr His
   965 970 975
   Lys Phe Met Ala Phe Lys Ser Phe Gly Glu Leu Val His Lys Pro
   980 985 990
   Leu Leu Val Asp Leu Thr Val Glu Glu Gly Gln Arg Leu Lys Val
   995 1000 1005
   Ile Tyr Gly Ser Cys Ala Gly Phe His Ala Val Asp Val Asp Ser
   1010 1015 1020
   Gly Ser Val Tyr Asp Ile Tyr Leu Pro Thr His Val Arg Lys Asn
   1025 1030 1035
   Pro His Ser Met Ile Gln Cys Ser Ile Lys Pro His Ala Ile Ile
   1040 1045 1050
   Ile Leu Pro Asn Thr Asp Gly Met Glu Leu Leu Val Cys Tyr Glu
   1055 1060 1065
   Asp Glu Gly Val Tyr Val Asn Thr Tyr Gly Arg Ile Thr Lys Asp
   1070 1075 1080
   Val Val Leu Gln Trp Gly Glu Met Pro Thr Ser Val Ala Tyr Ile
   1085 1090 1095
   Arg Ser Asn Gln Thr Met Gly Trp Gly Glu Lys Ala Ile Glu Ile
   1100 1105 1110
   Arg Ser Val Glu Thr Gly His Leu Asp Gly Val Phe Met His Lys
   1115 1120 1125
   Arg Ala Gln Arg Leu Lys Phe Leu Cys Glu Arg Asn Asp Lys Val
   1130 1135 1140
   Phe Phe Ala Ser Val Arg Ser Gly Gly Ser Ser Gln Val Tyr Phe
   1145 1150 1155
   Met Thr Leu Gly Arg Thr Ser Leu Leu Ser Trp
   1160 1165
<210> 78
   <211> 436
   <212> PRT
   <213> Homo sapiens
<400> 78
   Met Arg Leu Ala Arg Leu Leu Arg Gly Ala Ala Leu Ala Gly Pro
   1 5 10 15
   Gly Pro Gly Leu Arg Ala Ala Gly Phe Ser Arg Ser Phe Ser Ser
   20 25 30
   Asp Ser Gly Ser Ser Pro Ala Ser Glu Arg Gly Val Pro Gly Gln
   35 40 45
   Val Asp Phe Tyr Ala Arg Phe Ser Pro Ser Pro Leu Ser Met Lys
   50 55 60
   Gln Phe Leu Asp Phe Gly Ser Val Asn Ala Cys Glu Lys Thr Ser
   65 70 75
   Phe Met Phe Leu Arg Gln Glu Leu Pro Val Arg Leu Ala Asn Ile
   80 85 90
   Met Lys Glu Ile Ser Leu Leu Pro Asp Asn Leu Leu Arg Thr Pro
   95 100 105
   Ser Val Gln Leu Val Gln Ser Trp Tyr Ile Gln Ser Leu Gln Glu
   110 115 120
   Leu Leu Asp Phe Lys Asp Lys Ser Ala Glu Asp Ala Lys Ala Ile
   125 130 135
   Tyr Asp Phe Thr Asp Thr Val Ile Arg Ile Arg Asn Arg His Asn
   140 145 150
   Asp Val Ile Pro Thr Met Ala Gln Gly Val Ile Glu Tyr Lys Glu
   155 160 165
   Ser Phe Gly Val Asp Pro Val Thr Ser Gln Asn Val Gln Tyr Phe
   170 175 180
   Leu Asp Arg Phe Tyr Met Ser Arg Ile Ser Ile Arg Met Leu Leu
   185 190 195
   Asn Gln His Ser Leu Leu Phe Gly Gly Lys Gly Lys Gly Ser Pro
   200 205 210
   Ser His Arg Lys His Ile Gly Ser Ile Asn Pro Asn Cys Asn Val
   215 220 225
   Leu Glu Val Ile Lys Asp Gly Tyr Glu Asn Ala Arg Arg Leu Cys
   230 235 240
   Asp Leu Tyr Tyr Ile Asn Ser Pro Glu Leu Glu Leu Glu Glu Leu
   245 250 255
   Asn Ala Lys Ser Pro Gly Gln Pro Ile Gln Val Val Tyr Val Pro
   260 265 270
   Ser His Leu Tyr His Met Val Phe Glu Leu Phe Lys Asn Ala Met
   275 280 285
   Arg Ala Thr Met Glu His His Ala Asn Arg Gly Val Tyr Pro Pro
   290 295 300
   Ile Gln Val His Val Thr Leu Gly Asn Glu Asp Leu Thr Val Lys
   305 310 315
   Met Ser Asp Arg Gly Gly Gly Val Pro Leu Arg Lys Ile Asp Arg
   320 325 330
   Leu Phe Asn Tyr Met Tyr Ser Thr Ala Pro Arg Pro Arg Val Glu
   335 340 345
   Thr Ser Arg Ala Val Pro Leu Ala Gly Phe Gly Tyr Gly Leu Pro
   350 355 360
   Ile Ser Arg Leu Tyr Ala Gln Tyr Phe Gln Gly Asp Leu Lys Leu
   365 370 375
   Tyr Ser Leu Glu Gly Tyr Gly Thr Asp Ala Val Ile Tyr Ile Lys
   380 385 390
   Ala Leu Ser Thr Asp Ser Ile Glu Arg Leu Pro Val Tyr Asn Lys
   395 400 405
   Ala Ala Trp Lys His Tyr Asn Thr Asn His Glu Ala Asp Asp Trp
   410 415 420
   Cys Val Pro Ser Arg Glu Pro Lys Asp Met Thr Thr Phe Arg Ser
   425 430 435
   Ala
<210> 79
   <211> 928
   <212> PRT
   <213> Homo sapiens
<400> 79